# EUROPÄISCHE PATENTSCHRIFT

(11) **EP 3 414 238 B1**
(45) Veröffentlichungstag und Bekanntmachung des Hinweises auf die Patenterteilung: **24.03.2021**
(21) Anmeldenummer: 17703135.8
(22) Anmeldetag: 03.02.2017
(51) Int. Cl.: C07D 403/12, C07D 407/14, C07D 411/14, A01N 37/22

(54) **SUBSTITUIERTE IMIDAZOLYL-CARBOXAMIDE ALS SCHÄDLINGSBEKÄMPFUNGSMITTEL**
SUBSTITUTED IMIDAZOLYL-CARBOXAMIDES AS PESTICIDES
2-IMIDAZOLYLE-CARBOXAMIDE SUBSTITUE COMME PRODUIT DE LUTTE CONTRE LES PARASITES

(30) Priorität: 11.02.2016 EP 16155135
(43) Veröffentlichungstag der Anmeldung: 19.12.2018
(73) Patentinhaber: Bayer CropScience Aktiengesellschaft, 40789 Monheim am Rhein (DE)
(72) Erfinder: HEIL, Markus, 42799 Leichlingen (DE); FISCHER, Reiner, 40789 Monheim (DE); JANSEN, Johannes-Rudolf, 40789 Monheim (DE); WILLOT, Matthieu, 40215 Düsseldorf (DE); KÜBBELER, Susanne, 40589 Düsseldorf (DE); WILCKE, David, 40219 Düsseldorf (DE); ILG, Kerstin, 50670 Köln (DE); LÖSEL, Peter, 51371 Leverkusen (DE); EILMUS, Sascha, 42799 Leichlingen (DE); ANDERSCH, Wolfram, 51469 Bergisch Gladbach (DE); PORTZ, Daniela, 52391 Vettweiß (DE); GÖRGENS, Ulrich, 40882 Ratingen (DE)
(74) Vertreter: BIP Patents
(86) Internationale Anmeldenummer: PCT/EP2017/052350
(87) Internationale Veröffentlichungsnummer: WO 2017/137319

(56) Entgegenhaltungen:
- WO-A1-02/070483
- WO-A2-2011/009804

## Beschreibung

Die vorliegende Anmeldung betrifft neue heterocyclische Verbindungen und ihre Verwendung zur Bekämpfung von tierischen Schädlingen.

In WO 2011/009804 A2 sind heterocyclische Verbindungen unter anderem auch Imidazolylcarboxamide beschrieben, die als Insektizide verwendet werden können. Es werden jedoch keine Imidazolylcarboxamide beschrieben, die durch die erfindungsgemäßen funktionellen Gruppen X direkt am Imidazolylrest substituiert sind. Die in der WO 2011/009804 A2 aufgeführten Imidazolylcarboxamide zeigen dabei deutliche Schwächen in ihrer insektiziden Wirkung.

Moderne Insektizide müssen vielen Anforderungen genügen, beispielsweise in Bezug auf Höhe, Dauer und Breite ihrer Wirkung und möglichen Verwendung. Es spielen Fragen der Toxizität, der Nützling- und Bestäuberschonung, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten ergänzt wird.

Gelöst wird die Aufgabe, sowie weitere nicht explizit genannte Aufgaben, die aus den hierin diskutierten Zusammenhängen ableitbar oder erschließbar sind, durch die Bereitstellung von Verbindungen der Formel (I) 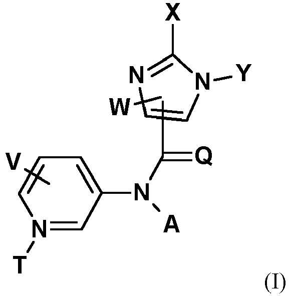 in welcher (Ausführungsform (0-1))
- Q: für Sauerstoff oder Schwefel steht,
- V: für einen Rest aus der Reihe Wasserstoff, Halogen, Alkyl, Halogenalkyl, Alkoxy, Halogenalkoxy und Cyano steht,
- W: für einen Rest aus der Reihe Wasserstoff, Halogen, Alkyl, Halogenalkyl, Alkoxy, Halogenalkoxy und Cyano steht,
- Y: für einen Rest aus der Reihe gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Arylalkyl oder Hetarylalkyl und Cyano steht,
- A: für einen Rest aus der Reihe Wasserstoff, gegebenenfalls substituiertes Alkyl, Alkenyl oder Alkinyl und gegebenenfalls durch Heteroatome unterbrochenes und gegebenenfalls substituiertes Cycloalkyl oder Cycloalkyl-alkyl steht,
- T: für Sauerstoff oder ein Elektronenpaar steht,
- X: für C(E)U¹, C(O)OR², C(O)N(R³)(R⁴), C(S)N(R³)(R⁴), C(O)N(R³¹)-N(R³²)(R⁴¹), oder C(S)N(R³¹)-N(R³²)(R⁴¹) steht,
- E: für die Reste O, N-OR²¹ und N-N(R³³)(R⁴³) oder für einen Cyclus der Formel (X-1) steht, 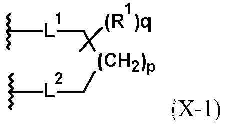
- U¹: für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Aryl, Hetaryl, Arylalkyl oder Hetarylalkyl steht,
- L¹, L²: jeweils unabhängig voneinander für O und S(O)ₘ stehen,
- R¹: für einen Rest aus der Reihe Halogen, Cyano, gegebenenfalls substituiertes Alkyl, Alkenyl, Alkinyl oder Alkoxy und gegebenenfalls substituiertes Aryl steht, oder
- R¹: für eine gegebenenfalls substituierte C₂-C₄-Alkylkette steht, die zwei am selben C-Atom gebunde H -Atome ersetzt, wobei q dann für 2 steht,
oder
- R¹: für eine gegebenenfalls substituierte C₁-C₄-Alkenylkette steht, wobei die Doppelbindung zwei am selben C-Atom gebunde H -Atome ersetzt, wobei q dann für 1 steht,
- R²: für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Aryl, Arylalkyl, Hetaryl oder Hetarylalkyl steht,
- R²¹: für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, für gegebenenfalls substituiertes Cycloalkylalkyl oder für gegebenenfalls substituiertes Arylalkyl steht,
- R³: für einen Rest aus der Reihe Wasserstoff, gegebenenfalls substituiertes Alkyl, Alkenyl, Alkinyl, Cycloalkyl, Alkoxycarbonyl, Alkylcarbonyl oder Arylalkoxycarbonyl steht,
- R⁴: für einen Rest aus der Reihe Wasserstoff, gegebenenfalls substituiertes Alkyl, Alkenyl, Alkinyl oder Alkoxy, gegebenenfalls durch Heteroatome unterbrochenes und gegebenenfalls substituiertes Cycloalkyl oder Cycloalkenyl, gegebenenfalls durch Heteroatome unterbrochenes und gegebenenfalls substituiertes Cycloalkylalkyl oder für Aryl, Arylalkyl, Hetaryl und Hetarylalkyl steht,
oder
- R³ und R⁴: zusammen einen gegebenenfalls substituierten fünf- bis siebengliedrigen aliphatischen Ring ausbilden, der gegebenenfalls ein N-, S- oder O Atom enthält,,
- R³¹, R³² und R³³: jeweils unabhängig voneinander für einen Rest aus der Reihe Wasserstoff, gegebenenfalls substituiertes Alkyl, Alkenyl, Alkinyl, Cycloalkyl, Alkoxycarbonyl, Alkylcarbonyl, Arylalkylcarbonyl oder Arylalkylalkoxycarbonyl stehen,
- R⁴¹, R⁴³: jeweils unabhängig voneinander für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Aryl, Arylalkyl, Hetaryl oder Hetarylalkyl stehen,
- m: für eine Zahl 0, 1 oder 2 steht,
- p: für eine Zahl 0, 1 oder 2 steht,
- q: für eine Zahl 0, 1 oder 2 steht
und deren Salze.

Weiterhin betrifft die Erfindung Verbindungen der Formel (I), 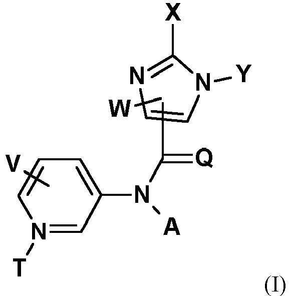 in welcher (Ausführungsform (0-2))
Q, V, W, Y, A, T, X, E, U¹, L¹, L², R¹, R², R²¹, R³¹, R³², R³³, R⁴¹, R⁴³, m, p, q definiert sind wie in Ausführungsform (0-1) und
R³ für einen Rest aus der Reihe Wasserstoff, gegebenenfalls substituiertes Alkyl, Alkenyl, Alkinyl, Cycloalkyl, Alkylsulfonyl, Cycloalkylsulfonyl, Alkoxycarbonyl, Alkylcarbonyl oder Arylalkoxycarbonyl steht,
R⁴ für einen Rest aus der Reihe Wasserstoff, gegebenenfalls substituiertes Alkyl, Alkenyl, Alkinyl oder Alkoxy, gegebenenfalls durch Heteroatome unterbrochenes und gegebenenfalls substituiertes Cycloalkyl oder Cycloalkenyl, gegebenenfalls durch Heteroatome unterbrochenes und gegebenenfalls substituiertes Cycloalkylalkyl oder für gegebenenfalls substituiertes Aryl, Arylalkyl, Hetaryl und Hetarylalkyl steht,
   oder
R³ und R⁴ zusammen einen gegebenenfalls substituierten vier- bis siebengliedrigen aliphatischen Ring ausbilden, der gegebenenfalls ein N-, S- oder O Atom enthält.

Bevorzugte Substituenten bzw. Bereiche der in den Verbindungen der Formel (I) aufgeführten Reste werden im Folgenden erläutert. Ihre Kombination bildet den Vorzugsbereich (1-1), wobei
- Q: für Sauerstoff oder Schwefel steht,
- V: für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
- W: für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
- Y: für einen Rest aus der Reihe gegebenenfalls einfach bis mehrfach unabhängig voneinander durch Halogen, C₁-C₄-Alkoxy, C₁-C₄-Alkyl-S(O)ₙ- oder Cyano substituiertes C₁-C₆-Alkyl, C₃-C₆-Alkenyl oder C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach bis zweifach unabhängig voneinander durch O, S(O)ₙ, CO oder NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl-C₁-C₄-alkyl oder Hetaryl-C₁-C₄-alkyl steht,
- A: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes C₁-C₆-Alkyl, C₃-C₆-Alkenyl oder C₃-C₆-Alkinyl und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₆-Cycloalkyl oder C₃-C₈-Cycloalkyl-C₁-C₄-alkyl steht,
- T: für Sauerstoff oder ein Elektronenpaar steht
- X: für C(E)U¹, C(O)OR², C(O)N(R³)(R⁴), C(S)N(R³)(R⁴), C(O)N(R³¹)-N(R³²)(R⁴¹) oder C(S)N(R³¹)-N(R³²)(R⁴¹) steht,
- E: für die Reste O, N-OR²¹, N-N(R³³)(R⁴³) oder für einen Cyclus der Formel (X-1) steht, 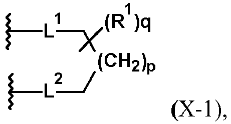
- U¹: für einen Rest aus der Reihe Wasserstoff, gegebenenfalls einfach bis mehrfach unabhängig voneinander durch Halogen, C₁-C₄-Alkoxy oder C₁-C₄-Alkyl-S(O)ₙ-, substituiertes C₁-C₈-Alkyl, C₃-C₈-Alkenyl oder C₃-C₈-Alkinyl, gegebenenfalls einfach durch O, S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach durch O oder S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl oder Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
- L¹, L²: jeweils unabhängig voneinander für O und S(O)ₘ stehen
- R¹: für einen Rest aus der Reihe Halogen, gegebenenfalls durch Halogen, Cyano, C₁-C₄-Alkoxy oder C₁-C₄-Alkyl-S(O)ₙ-, substituiertes C₁-C₆-Alkyl, C₂-C₆-Alkenyl, C₃-C₆-Alkinyl oder C₁-C₆-Alkoxy, und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl steht,
oder
- R¹: für eine gegebenenfalls durch Halogen, Cyano, C₁-C₄-Alkyl oder C₁-C₄-Alkoxy substituierte C₂-C₄-Alkylkette steht, die zwei am selben C-Atom gebunde H -Atome ersetzt, wobei n dann für 2 steht,
oder
- R¹: für eine gegebenenfalls durch Halogen, Cyano, oder C₁-C₄-Alkoxy substituierte C₁-C₄-Alkenylkette steht, wobei die Doppelbindung zwei am selben C-Atom gebunde H -Atome ersetzt und wobei q dann für 1 steht,
- R²: für einen Rest aus der Reihe Wasserstoff, gegebenenfalls einfach bis mehrfach unabhängig voneinander durch Halogen, C₁-C₄-Alkoxy oder C₁-C₄-Alkyl-S(O)ₙ-, substituiertes C₁-C₈-Alkyl, C₃-C₈-Alkenyl oder C₃-C₈-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl oder Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
- R²¹: für einen Rest aus der Reihe Wasserstoff, gegebenenfalls einfach bis mehrfach unabhängig voneinander durch Halogen, C₁-C₄-Alkoxy oder C₁-C₄-Alkyl-S(O)ₙ-, substituiertes C₁-C₈-Alkyl, C₃-C₈-Alkenyl, C₃-C₈-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, und für gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
- R³: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ₋ oder Cyano substituiertes substituiertes C₁-C₄-Alkyl, C₃-C₆-Alkenyl oder C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₆-Cycloalkyl, C₁-C₄-Alkylcarbonyl oder C₁-C₄-Alkoxycarbonyl und für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl-C₁-C₂-Alkoxycarbonyl steht,
- R⁴: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch O, S(O)ₙ, CO oder NR^{4a} unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl oder C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl, Hetaryl, Arylalkyl oder Hetarylalkyl steht,
oder
- R³ und R⁴: zusammen einen gegebenenfalls einfach bis dreifach durch Halogen, C₁-C₄-Alkyl oder C₁-C₄-Alkoxy substituierten fünf- bis siebengliedrigen aliphatischen Ring ausbilden, der gegebenenfalls ein N-, S- oder O Atom enthält,
- R³¹, R³², R³³: jeweils unabhängig voneinander für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ-oder Cyano substituiertes C₁-C₆-Alkyl, C₃-C₆-Alkenyl oder C₃-C₆-Alkinyl, gegebenenfalls einfach bis dreifach unabhängig voneinander durch Halogen, C₁-C₄-Alkyl, C₁-C₄-Halogenalkyl, C₁-C₄-Alkoxy oder Cyano substituiertes C₃-C₆-Cycloalkyl, C₁-C₆-Alkylcarbonyl, C₁-C₆-Alkoxycarbonyl, Aryl-C₁-C₄-alkyl oder Aryl-C₁-C₄-alkoxy stehen,
- R⁴¹, R⁴³: jeweils unabhängig voneinander für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ-oder Cyano substituiertes C₁-C₄-Alkyl, C₃-C₆-Alkenyl oder C₃-C₆-Alkinyl, gegebenenfalls einfach bis zweifach unabhängig voneinander durch O, S(O)ₙ, CO oder NR^{4a} unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl oder C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl, Hetaryl, Arylalkyl oder Hetarylalkyl stehen,
- m: für eine Zahl 0, 1 oder 2 steht,
- n: für eine Zahl 0, 1 oder 2 steht,
- p: für eine Zahl 0 oder 1 steht,
- q: für eine Zahl 0, 1 oder 2 steht
und deren Salze.

Bevorzugte Substituenten bzw. Bereiche der in den Verbindungen der Formel (I) aufgeführten Reste werden im Folgenden erläutert. Ihre Kombination bildet den Vorzugsbereich (1-2), wobei
Q, V, W, Y, A, T, X, E, L¹, L², R¹, R², R²¹, R³¹, R³², R³³, R⁴¹, R⁴³, m, n, p, q definiert sind wie in Vorzugsbereich (1-1) und
U¹ für einen Rest aus der Reihe Wasserstoff, gegebenenfalls einfach bis mehrfach unabhängig voneinander durch Halogen, Cyano, C₁-C₄-Alkoxy oder C₁-C₄-Alkyl-S(O)ₙ-, substituiertes C₁-C₈-Alkyl, C₃-C₈-Alkenyl oder C₃-C₈-Alkinyl, gegebenenfalls einfach durch O oder S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach durch O oder S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl oder Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
R³ für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes substituiertes C₁-C₄-Alkyl, C₃-C₆-Alkenyl oder C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₆-Cycloalkyl, C₁-C₄-Alkylsulfonyl, C₃-C₆-Cycloalkylsulfonyl, C₁-C₄-Alkylcarbonyl oder C₁-C₄-Alkoxycarbonyl und für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl-C₁-C₂-Alkoxycarbonyl steht,
R⁴ für einen Rest aus der Reihe Wasserstoff, gegebenenfalls einfach bis mehrfach unabhängig voneinander durch Halogen, Hydroxy, C₁-C₄-Alkoxy, C₁-C₄-Alkoxycarbonyl, - C₁-C₄-Alkyl-S(O)ₙ- oder Cyano substituiertes substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch O, S(O)ₙ, CO oder NR^{4a} unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl oder C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, Hydroxy, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl, Hetaryl, Arylalkyl oder Hetarylalkyl steht, oder
R³ und R⁴ zusammen einen gegebenenfalls einfach bis dreifach durch Halogen, C₁-C₄-Alkyl oder C₁-C₄-Alkoxy substituierten vier- bis siebengliedrigen aliphatischen Ring ausbilden, der gegebenenfalls ein N-, S- oder O Atom enthält.

Besonders bevorzugte Substituenten bzw. Bereiche der in den Verbindungen der Formel (I) aufgeführten Reste werden im Folgenden erläutert. Ihre Kombination bildet den Vorzugsbereich (2-1), wobei
- Q: für Sauerstoff steht
- V: für einen Rest aus der Reihe Wasserstoff, Fluor, Chlor, Brom, Methyl, Ethyl steht,
- W: für einen Rest aus der Reihe Wasserstoff, Fluor, Chlor, Brom, Cyano, Methyl steht,
- Y: für einen Rest aus der Reihe gegebenenfalls einfach bis fünffach unabhängig voneinander durch Fluor, Chlor, Brom, Methoxy oder Ethoxy substituiertes C₁-C₄-Alkyl, C₃-C₄-Alkenyl oder C₃-C₄-Alkinyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl oder Aryl-C₁-C₄-alkyl steht,
- A: für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl und gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl steht,
- T: für ein Elektronenpaar steht,
- X: für C(E)U¹, C(O)OR², C(O)N(R³)(R⁴) oder C(S)N(R³)(R⁴) steht
- E: für die Reste O, N-OR²¹ , N-N(R³³)(R⁴³) oder für einen Cyclus der Formel (X-1) steht, 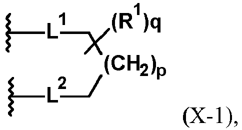
- U¹: für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl und gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl, für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl, Pyridyl, Pyrimidinyl oder Thienyl, für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
- L¹, L²: jeweils unabhängig voneinander für O und S(O)ₘ stehen
- R¹: für gegebenenfalls durch Halogen, Methoxy, Ethoxy, substituiertes C₁-C₄-Alkyl, C₂-C₄-Alkenyl, C₃-C₄-Alkinyl oder C₁-C₄-Alkoxy steht,
oder
- R¹: für eine C₂-C₄-Alkylkette steht, die zwei am selben C-Atom gebunde H -Atome ersetzt, wobei n dann für 2 steht,
oder
- R¹: für eine C₁-C₄-Alkenylkette steht, wobei die Doppelbindung zwei am selben C-Atom gebundene H -Atome ersetzt und wobei n dann für 1 steht,
- R²: für einen Rest aus der Reihe Wasserstoff, einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl-C₁-C₄-alkyl, für gegebenenfalls einfach bis dreifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl, Methyl-S(O)ₙ-, Difluorethyl-S(O)ₙ-, Trifluorethyl-S(O)ₙ-, Nitro oder Cyano substituiertes Phenyl, Pyridyl oder Thienyl und für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
- R²¹: für einen Rest aus der Reihe Wasserstoff, einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl-C₁-C₄-alkyl und für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
- R³: für einen Rest aus der Reihe Wasserstoff, gegebenenfalls einfach bis dreifach unabhängig voneinander durch Fluor oder Chlor substituiertes substituiertes C₁-C₄-Alkyl, C₃-C₄-Alkenyl oder C₃-C₄-Alkinyl, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl oder für gegebenenfalls einfach bis dreifach unabhängig voneinander durch Fluor, Chlor, Methoxy oder Ethoxy substituiertes C₁-C₄-Alkylcarbonyl oder C₁-C₄-Alkoxycarbonyl und für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl-C₁-C₂-Alkoxycarbonyl steht,
- R⁴: für einen Rest aus der Reihe Wasserstoff, gegebenenfalls einfach bis dreifach unabhängig voneinander durch Halogen Methoxy, Ethoxy, n- oder iPropoxy oder Methyl-S(O)ₙ-, substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl, oder C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl, Naphtyl Pyridyl, Pyrimidyl, Pyridazinyl, Pyrazinyl, Triazinyl, Furanyl, Thienyl, Oxazolyl, Isoxazolyl, Thiazolyl, Isothiazolyl, Pyrazolyl, Imidazolyl, Oxdiazolyl oder Thiadiazolyl, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
oder
- R³ und R⁴: zusammen einen gegebenenfalls einfach bis zweifach durch Fluor Chlor, Brom, Methyl, Ethyl, Methoxy oder Ethoxy substituierten fünf- bis sechsgliedrigen aliphatischen Ring ausbilden
- R³³: für einen Rest aus der Reihe Wasserstoff, gegebenenfalls einfach bis dreifach unabhängig voneinander durch Fluor oder Chlor substituiertes substituiertes C₁-C₄-Alkyl, für gegebenenfalls einfach bis dreifach unabhängig voneinander durch Fluor, Chlor, Methoxy oder Ethoxy substituiertes substituiertes C₁-C₆-Alkylcarbonyl oder C₁-C₆-Alkoxycarbonyl und für gegebenenfalls einfach bis zweifach unabhängig voneinander durch Fluor, Chlor, Brom, Methyl, Ethyl, Trifluormethyl, Methoxy, Ethoxy, Trifluormethoxy, Nitro oder Cyano substituiertes Phenyl-C₁-C₂-Alkoxycarbonyl steht,
- R⁴³: für einen Rest aus der Reihe Wasserstoff oder für gegebenenfalls einfach bis dreifach unabhängig voneinander durch Halogen, Methoxy oder Ethoxy substituiertes C₁-C₄-Alkyl steht,
- m: für 0 steht,
- n: für eine Zahl 0, 1 oder 2 steht,
- p: für eine Zahl 0 oder 1 steht,
- q: für eine Zahl 0, 1 oder 2 steht
und deren Salze.

Besonders bevorzugte Substituenten bzw. Bereiche der in den Verbindungen der Formel (I) aufgeführten Reste werden im Folgenden erläutert. Ihre Kombination bildet den Vorzugsbereich (2-2), wobei
Q, V, W, Y, A, T, X, U¹, E, L¹, L², R¹, R², R²¹, R³³, R⁴³, m, n, p, q definiert sind wie in Vorzugsbereich (2-1) und
- R³: für einen Rest aus der Reihe Wasserstoff, gegebenenfalls einfach bis dreifach unabhängig voneinander durch Fluor oder Chlor substituiertes substituiertes C₁-C₄-Alkyl, C₃-C₄-Alkenyl oder C₃-C₄-Alkinyl, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl oder für gegebenenfalls einfach bis dreifach unabhängig voneinander durch Fluor, Chlor, Methoxy oder Ethoxy substituiertes C₁-C₄-Alkylsulfonyl, C₃-C₆-Cycloalkylsulfonyl, C₁-C₄-Alkylcarbonyl oder C₁-C₄-Alkoxycarbonyl und für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl-C₁-C₂-Alkoxycarbonyl steht,
- R⁴: für einen Rest aus der Reihe Wasserstoff, gegebenenfalls einfach bis dreifach unabhängig voneinander durch Halogen, Hydroxy, Methoxy, Ethoxy, n- oder iPropoxy, Methoxycarbonyl, Ethoxycarbonyl, Cyano, oder Methyl-S(O)ₙ-, substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl, oder C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl, Naphtyl Pyridyl, Pyrimidyl, Pyridazinyl, Pyrazinyl, Triazinyl, Furanyl, Thienyl, Oxazolyl, Isoxazolyl, Thiazolyl, Isothiazolyl, Pyrazolyl, Imidazolyl, Oxdiazolyl oder Thiadiazolyl, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht oder
- R³ und R⁴: zusammen einen gegebenenfalls einfach bis zweifach durch Fluor Chlor, Brom, Methyl, Ethyl, Methoxy oder Ethoxy substituierten vier- bis sechsgliedrigen aliphatischen Ring ausbilden, der gegebenenfalls ein N-, S- oder O Atom enthält.

Ganz besonders bevorzugte Substituenten bzw. Bereiche der in den Verbindungen der Formel (I) aufgeführten Reste werden im Folgenden erläutert. Unter Berücksichtigung der Position der Carboxamidgruppe am Imidazolrest ergibt sich die ganz besonders bevorzugte Struktur (I-1). Ihre Kombination bildet den Vorzugsbereich (3-A-1), 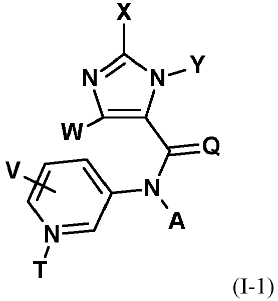 wobei
- Q: für Sauerstoff steht
- V: für einen Rest aus der Reihe Wasserstoff, Fluor, Chlor, Methyl und Cyano steht,
- W: für einen Rest aus der Reihe Wasserstoff, Fluor, Chlor, Brom. Methyl steht,
- Y: für einen Rest aus der Reihe gegebenenfalls einfach bis dreifach unabhängig voneinander durch Fluor, Methoxy, Ethoxy oder Cyano substituiertes Methyl, Ethyl, Propyl, Allyl oder Propargyl steht,
- A: für einen Rest aus der Reihe Wasserstoff, gegebenenfalls einfach bis dreifach unabhängig voneinander durch Fluor, Methoxy, Ethoxy oder Cyano substituiertes Methyl, Ethyl, Propyl, Allyl, Propargyl oder Cylopropyl steht,
- T: für ein Elektronenpaar steht,
- X: für C(E)U¹, C(O)OR², C(O)N(R³)(R⁴) oder C(S)N(R³)(R⁴) steht,
- E: für die Reste O, N-OR²¹, N-N(R³³)(R⁴³) oder für einen Cyclus der Formel (X-1) steht, 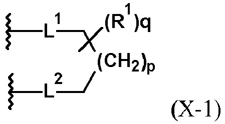
- U¹: für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl steht,
- L¹, L²: jeweils unabhängig voneinander für O und S(O)ₘ stehen,
- R¹: für gegebenenfalls durch Fluor, Chlor, Methoxy oder Ethoxy substituiertes C₁-C₄-Alkyl steht,
oder
- R¹: für eine C₂-C₄-Alkylkette steht, die zwei am selben C-Atom gebunde H -Atome ersetzt, wobei n dann für 2 steht,
- R²: für einen Rest aus der Reihe Wasserstoff, einfach bis dreifach unabhängig voneinander durch Fluor, Chlor, Brom, Methoxy, Ethoxy oder Cyano substituiertes C₁-C₆-Alkyl oder für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, Phenylmethyl steht,
- R²¹: für einen Rest aus der Reihe Wasserstoff, einfach bis dreifach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl, gegebenenfalls einfach bis zweifach unabhängig voneinander durch Fluor, Chlor, Brom, Methyl, Ethyl, Methoxy oder Ethoxy substituiertes C₃-C₆-Cycloalkyl, gegebenenfalls durch Fluor, Chlor, Brom, Methyl, Ethyl, Methoxy oder Ethoxy substituiertes C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, Phenylmethyl oder Pyridylmehyl steht,
- R³: für einen Rest aus der Reihe Wasserstoff, gegebenenfalls einfach bis dreifach unabhängig voneinander durch Fluor, Chlor, Methoxy oder Ethoxy substituiertes substituiertes C₁-C₄-Alkyl, C₁-C₄-Alkylcarbonyl oder C₁-C₄-Alkoxycarbonyl und gegebenenfalls einfach bis dreifach unabhängig voneinander durch Fluor, Chlor, Brom, Methyl, Ethyl, Trifluormethyl, Methoxy, oder Ethoxy substituiertes Arylmethoxycarbonyl steht,
- R⁴: für einen Rest aus der Reihe gegebenenfalls einfach bis dreifach unabhängig voneinander durch Halogen, Methoxy oder Ethoxy substituiertes C₁-C₄-Alkyl, C₃-C₆-Alkenyl oder C₃-C₆-Alkinyl, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl oder C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Trifluormethoxy, Nitro oder Cyano substituiertes Phenyl, Pyridyl oder Thienyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Trifluormethoxy, Nitro oder Cyano substituiertes Phenyl-C₁-C₂-alkyl oder Pyridyl-C₁-C₂-alkyl steht,
oder
- R³ und R⁴: zusammen einen gegebenenfalls einfach bis zweifach durch Fluor Chlor, Brom, Methyl, Ethyl, Methoxy oder Ethoxy substituierten fünf- bis sechsgliedrigen aliphatischen Ring ausbilden,
- R³³: für einen Rest aus der Reihe Wasserstoff, gegebenenfalls durch Methoxy oder Ethoxy substituiertes C₁-C₄-Alkylcarbonyl oder C₁-C₄-Alkoxycarbonyl und für gegebenenfalls einfach bis dreifach unabhängig voneinander durch Fluor, Chlor, Methyl oder Ethyl substituiertes Phenylmethoxycarbonyl steht,
- R⁴³: für einen Rest aus der Reihe Wasserstoff oder C₁-C₄-Alkyl steht,
- m: für 0 steht,
- n: für eine Zahl 0, 1 oder 2 steht,
- p: für eine Zahl 0 oder 1 steht,
- q: für eine Zahl 0, 1 oder 2 steht
und deren Salze.

Aus Formel (I) ergibt sich unter Berücksichtigung der Position der Carboxamidgruppe am Imidazolrest die ganz besonders bevorzugte Struktur (I-1). Ganz besonders bevorzugte Substituenten bzw. Bereiche der in den Verbindungen der Formel (I-1) aufgeführten Reste werden im Folgenden erläutert. Ihre Kombination bildet den Vorzugsbereich (3-A-2),
wobei Q, V, W, Y, A, T, X, E, L¹, L², R¹, R², R²¹, R³³, R⁴³, m, n, p, q definiert sind wie in Vorzugsbereich (3-A-1) und
- U¹: für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl oder für C₃-C₆-Cycloalkyl steht,
- R³: für einen Rest aus der Reihe Wasserstoff, gegebenenfalls einfach bis dreifach unabhängig voneinander durch Fluor, Chlor, Methoxy oder Ethoxy substituiertes substituiertes C₁-C₄-Alkyl, C₁-C₄-Alkylsulfonyl, C₃-C₆-Cycloalkylsulfonyl, C₁-C₄-Alkylcarbonyl oder C₁-C₄-Alkoxycarbonyl und gegebenenfalls einfach bis dreifach unabhängig voneinander durch Fluor, Chlor, Brom, Methyl, Ethyl, Trifluormethyl, Methoxy oder Ethoxy substituiertes Arylmethoxycarbonyl steht,
- R⁴: für einen Rest aus der Reihe Wasserstoff, gegebenenfalls einfach bis dreifach unabhängig voneinander durch Halogen, Hydroxy, Methoxy, Ethoxy, Methoxycarbonyl, Ethoxycarbonyl oder Cyano substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl oder C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Trifluormethoxy, Nitro oder Cyano substituiertes Phenyl, Pyridyl oder Thienyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Trifluormethoxy, Nitro oder Cyano substituiertes Phenyl-C₁-C₂-alkyl oder Pyridyl-C₁-C₂-alkylsteht oder
- R³ und R⁴: zusammen einen gegebenenfalls einfach bis zweifach durch Fluor Chlor, Brom, Methyl, Ethyl, Methoxy oder Ethoxy substituierten vier- bis sechsgliedrigen aliphatischen Ring ausbilden, der gegebenenfalls ein N-, S- oder O Atom enthält.

Insbesonders bevorzugte Substituenten bzw. Bereiche der in den Verbindungen der Formel (I-1) aufgeführten Reste werden im Folgenden erläutert. Ihre Kombination mit den insbesonders bevorzugten Substituenten bildet den Vorzugsbereich (4-A-1), wobei
- Q: für Sauerstoff steht
- V: für Wasserstoff steht,
- W: für Wasserstoff steht,
- Y: für einen Rest aus der Reihe Methyl, Ethyl, Propyl, Allyl und Propargyl steht,
- A: für einen Rest aus der Reihe Wasserstoff, Methyl oder Ethyl steht,
- T: für ein Elektronenpaar steht,
- X: für C(E)U¹, C(O)OR², C(O)N(R³)(R⁴) oder C(S)N(R³)(R⁴) steht,
- E: für die Reste O, N-OR²¹, N-N(R³³)(R⁴³) oder für einen Cyclus der Formel (X-1) steht, 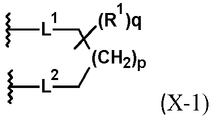
- U¹: für einen Rest aus der Reihe Wasserstoff, Methyl oder Ethyl, n-oder iso-Propyl steht,
- L¹, L²: jeweils unabhängig voneinander für O und S(O)ₘ stehen
- R¹: für Methyl, Ethyl, n-, iso-Propyl oder Methoxymethyl steht,
oder
- R¹: für -(CH₂)₂- steht, wobei eine spiro-Cyclopropylgruppe gebildet wird,
- R²: für einen Rest aus der Reihe Wasserstoff, Methyl, Ethyl, n-oder iso Propyl steht,
- R²¹: für einen Rest aus der Reihe für einen Rest aus der Reihe Wasserstoff, Methyl, Ethyl, n-oder iso Propyl, n-, iso-, sec- oder tert-Butyl, 2,2-Dimethylpropyl, Propenyl, Propinyl, 2,2,2-Trifluorethyl, Cyclopropyl, Cyclobutyl, Cyclopentyl, Cyclohexyl, Cyclopropylmethyl, 2-Cl-Phenylmethyl, 3-Cl-Phenylmethyl, 4-Cl-Phenylmethyl, Pyrid-2-ylmethyl, Pyrid-3-ylmethyl oder Pyrid-4-ylmethyl steht,
- R³: für einen Rest aus der Reihe Wasserstoff, Methyl, Ethyl, Methoxycarbonyl, Ethoxycarbonyl, 2-Methoxyethoxycarbonyl n-, iso, sec- oder t-Butoxycarbonyl steht,
- R⁴: für einen Rest aus der Reihe Wasserstoff, Methyl, Ethyl, n-oder iso Propyl, n-, iso, sec- oder tert. Butyl, 2,2,2-Trifluorethyl, Cylopropyl, Cylobutyl, Cylopentyl, Cylohexyl, Phenyl, 2-Cl-Phenyl, 3-Cl-Phenyl, 4-Cl-Phenyl, Phenylmethyl, 2-Cl-Phenylmethyl, 3-Cl-Phenylmethyl, 4-Cl-Phenylmethyl steht,
oder
- R³ und R⁴: zusammen für (CH₂)₅ oder (CH₂)₄ stehen,
- R³³: für einen Rest aus der Reihe Methoxycarbonyl, Ethoxycarbonyl, n- oder iso Propoxycarbonyl, n-, iso, sec- oder t-Butoxycarbonyl, iso Pentoxycarbonyl, 2-Methoxyethoxycarbonyl oder Phenylmethoxycarbonyl steht,
- R⁴³: für einen Rest aus der Reihe Wasserstoff, Methyl, Ethyl, n-oder iso Propyl, n-, iso, sec- oder tert. Butyl steht,
- m: für 0 steht,
- p: für eine Zahl 0 oder 1 steht,
- q: für eine Zahl 0, 1 oder 2 steht
und deren Salze.

Insbesonders bevorzugte Substituenten bzw. Bereiche der in den Verbindungen der Formel (I-1) aufgeführten Reste werden im Folgenden erläutert. Ihre Kombination mit den insbesonders bevorzugten Substituenten bildet den Vorzugsbereich (4-A-2), wobei
Q, W, Y, T, X, E, L¹, L², R¹, R², R²¹, R³³, R⁴³, m, p, q definiert sind wie in Vorzugsbereich (4-A-1) und
V für Wasserstoff oder Methyl steht,
A für einen Rest aus der Reihe Wasserstoff, Methyl, Ethyl oder Cyclopropyl steht,
U¹ für einen Rest aus der Reihe Wasserstoff, Methyl, Ethyl, n-oder iso-Propyl, 1-Cyano-1-methylethyl oder Cyclopropyl steht,
R³ für einen Rest aus der Reihe Wasserstoff, Methyl, Ethyl, Methylsulfonyl, Cyclopropylsulfonyl, Methoxycarbonyl, Ethoxycarbonyl, 2-Methoxyethoxycarbonyl n-, iso, sec- oder t-Butoxycarbonyl steht,
R⁴ für einen Rest aus der Reihe Wasserstoff, Methyl, Ethyl, n-oder iso Propyl, n-, iso, sec- oder tert. Butyl, 2,2,2-Trifluorethyl, 2-Hydroxyethyl, 2-Methoxyethyl, Cyanomethyl, Methoxycarbonylmethyl, Ethoxycarbonylmethyl, Cylopropyl, 1-Cyanocyclopropyl, Cyclopropylmethyl, Cylobutyl, Cylopentyl, Cylohexyl, Methoxy, Ethoxy, Phenyl, 2-Cl-Phenyl, 3-Cl-Phenyl, 4-Cl-Phenyl, Pyridyl, Phenylmethyl, 1-Phenyl-2-hydroxyethyl, 2-Cl-Phenylmethyl, 3-Cl-Phenylmethyl, 4-Cl-Phenylmethyl steht oder
R³ und R⁴ zusammen für (CH₂)₅, (CH₂)₄, (CH₂)₃ oder (CH₂)₂O(CH₂)₂ stehen.

Ganz besonders bevorzugte Substituenten bzw. Bereiche der in den Verbindungen der Formel (I-1) aufgeführten Reste werden im Folgenden erläutert. Ihre Kombination bildet den Vorzugsbereich (5-A-1), wobei
- Q: für Sauerstoff steht
- V: für Wasserstoff steht,
- W: für Wasserstoff steht,
- Y: für Methyl steht,
- A: für Methyl oder Ethyl steht,
- T: für ein Elektronenpaar steht,
- X: für C(E)U¹, C(O)OR², C(O)N(R³)(R⁴) oder C(S)N(R³)(R⁴) steht,
- E: für die Reste O, N-OR²¹, N-N(R³³)(R⁴³) oder für einen Cyclus der Formel (X-1) steht, 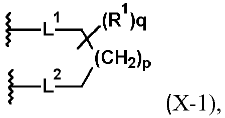
- U¹: für Wasserstoff steht,
- L¹, L²: jeweils unabhängig voneinander für O und S(O)ₘ stehen
- R¹: für Methyl, Ethyl oder Methoxymethyl steht,
oder
- R¹: für -(CH₂)₂- steht, wobei eine spiro-Cyclopropylgruppe gebildet wird
- R²: für Methyl oder Ethyl steht,
- R²¹: für einen Rest aus der Reihe für einen Rest aus der Reihe Methyl, Ethyl, n-oder iso Propyl, n-, iso-, oder tert-Butyl, 2,2-Dimethylpropyl, Propenyl, Propinyl, 2,2,2-Trifluorethyl, Cyclopentyl, Cyclopropylmethyl, 4-Cl-Phenylmethyl, Pyrid-2-ylmethyl, Pyrid-3-ylmethyl,
- R³: für einen Rest aus der Reihe Wasserstoff, Methyl oder Ethyl steht.
- R⁴: für einen Rest aus der Reihe Wasserstoff, Methyl, Ethyl, iso Propyl, tert. Butyl, 2,2,2-Trifluorethyl, Phenyl oder Phenylmethyl steht,
oder
- R³ und R⁴: zusammen für (CH₂)₅ oder (CH₂)₄ stehen,
- R³³: für einen Rest aus der Reihe Methoxycarbonyl, Ethoxycarbonyl, t-Butoxycarbonyl, 2-Methoxyethoxycarbonyl oder Phenylmethoxycarbonyl steht,
- R⁴³: für einen Rest aus der Reihe Wasserstoff, Methyl, oder n-Butyl steht,
- m: für 0 steht,
- p: für eine Zahl 0 oder 1 steht,
- q: für eine Zahl 0, 1 oder 2 steht
und deren Salze.

Ganz besonders bevorzugte Substituenten bzw. Bereiche der in den Verbindungen der Formel (I-1) aufgeführten Reste werden im Folgenden erläutert. Ihre Kombination bildet den Vorzugsbereich (5-A-2), wobei
Q, W, Y, T, X, E, L¹, L², R¹, R², R²¹, R³³, R⁴³, m, p, q definiert sind wie in Vorzugsbereich (5-A-1) und
V für Wasserstoff oder Methyl steht,
A für Methyl, Ethyl oder Cyclopropyl steht,
U¹ für Wasserstoff, Methyl, Ethyl, iso-Propyl, 1-Cyano-1-methylethyl oder Cyclopropyl steht,
R³ für einen Rest aus der Reihe Wasserstoff, Methyl, Ethyl, Methylsulfonyl, oder Cyclopropylsulfonyl steht.
R⁴ für einen Rest aus der Reihe Wasserstoff, Methyl, Ethyl, iso Propyl, iso-Butyl, tert. Butyl, 2,2,2-Trifluorethyl, 2-Hydroxyethyl, 2-Methoxyethyl, Cyanomethyl, Methoxycarbonylmethyl, Ethoxycarbonylmethyl, Cylopropyl, 1-Cyanocyclopropyl, Cyclopentyl, Cyclopropylmethyl, Methoxy, Ethoxy, Phenyl, Pyrid-3-yl, Phenylmethyl, 1-Phenyl-2-hydroxyethyl steht,
R³ und R⁴ zusammen für (CH₂)₅, (CH₂)₄, (CH₂)₃ oder (CH₂)₂O(CH₂)₂ stehen.

In den folgenden bevorzugten Ausführungsformen I-1a bis I-1f können alle Gruppen, Reste und Substituenten die oben in allen Ausführungsformen und Vorzugsbereichen genannten Bedeutungen haben, insbesondere haben sie die in Ausführungsform (0-1) oder (0-2) oder Vorzugsbereiche (1-1) oder (1-2) oder in Vorzugsbereich (2-1) oder (2-2) oder in Vorzugsbereich (3-A-1) oder (3-A-2) oder in Vorzugsbereich (4-A-1) oder (4-A-2) oder in Vorzugsbereich (5-A-1) oder (5-A-2) beschriebene Bedeutung.

In einer bevorzugten Ausführungsform betrifft die Erfindung die Verbindungen der Formel (I-1a).

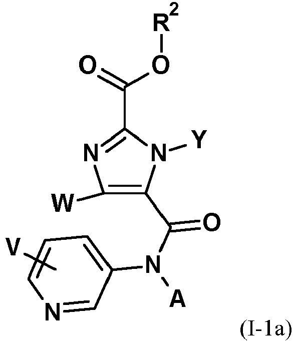

In einer weiteren bevorzugten Ausführungsform betrifft die Erfindung die Verbindungen der Formel (I-1b).

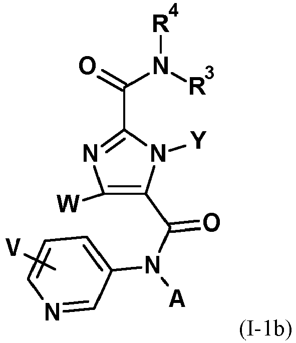

In einer weiteren bevorzugten Ausführungsform betrifft die Erfindung die Verbindungen der Formel (I-1c).

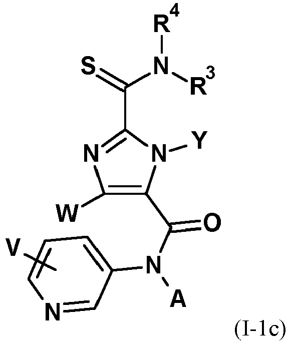

In einer weiteren bevorzugten Ausführungsform betrifft die Erfindung die Verbindungen der Formel (I-1d).

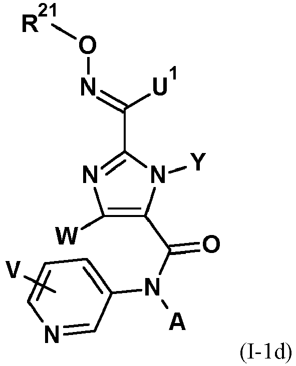

In einer weiteren bevorzugten Ausführungsform betrifft die Erfindung die Verbindungen der Formel (I-1e).

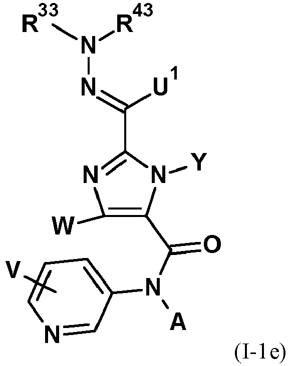

In einer weiteren bevorzugten Ausführungsform betrifft die Erfindung die Verbindungen der Formel (I-1f).

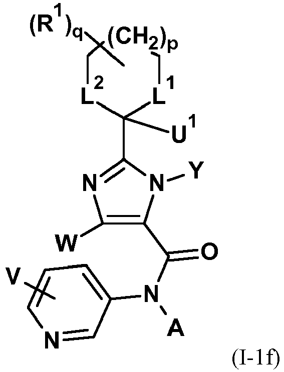

In einer bevorzugten Ausführungsform betrifft die Erfindung die Verbindungen der Formel (I) oder der Formel (I-1) oder der Formeln (I-1a bis I-1f), wobei die Reste Q, V, W, Y, A und T definiert sind wie in Vorzugsbereich (1-1) oder Vorzugsbereich (1-2) oder Vorzugsbereich (2-1) oder Vorzugsbereich (2-2) oder Vorzugsbereich (3-A-1) oder Vorzugsbereich (3-A-2) oder Vorzugsbereich (4-A-1) oder Vorzugsbereich (4-A-2) oder Vorzugsbereich (5-A-1) oder Vorzugsbereich (5-A-2) und
X, E, L¹, L², R¹, R², R²¹, R³ R⁴, R³¹, R³², R³³, R⁴¹, R⁴³, m, n, p und q die in Ausführungsform (0-1) oder (0-2) beschriebenen Bedeutungen haben.

In einer bevorzugten Ausführungsform betrifft die Erfindung die Verbindungen der Formel (I) oder der Formel (I-1) oder der Formeln (I-1a bis I-1f), wobei die Reste Q, V, W, Y, A und T definiert sind wie in Ausführungsform (0-1) oder Ausführungsform (0-2) oder Vorzugsbereich (2-1) oder Vorzugsbereich (2-2) oder Vorzugsbereich (3-A-1) oder Vorzugsbereich (3-A-2) oder Vorzugsbereich (4-A-1) oder Vorzugsbereich (4-A-2) oder Vorzugsbereich (5-A-1) oder Vorzugsbereich (5-A-2) und
X, E, L¹, L², R¹, R², R²¹, R³, R⁴ R³¹, R³² R³³, R⁴¹, R⁴³, m, n, p und q die in Vorzugsbereich (1-1) oder (1-2) beschriebenen Bedeutungen haben.

In einer bevorzugten Ausführungsform betrifft die Erfindung die Verbindungen der Formel (I) oder der Formel (I-1) oder der Formeln (I-1a bis I-1f), wobei die Reste Q, V, W, Y, A und T definiert sind wie in Ausführungsform (0-1) oder Ausführungsform (0-2) oder Vorzugsbereich (1-1) oder Vorzugsbereich (1-2) oder Vorzugsbereich (3-A-1) oder Vorzugsbereich (3-A-2) oder Vorzugsbereich (4-A-1) oder Vorzugsbereich (4-A-2) oder Vorzugsbereich (5-A-1) oder Vorzugsbereich (5-A-2) und
X, E, L¹, L², R¹, R², R²¹, R³, R⁴, R³³, R⁴³, m, n, p und q die in Vorzugsbereich (2-1) oder (2-2) beschriebenen Bedeutungen haben.

In einer bevorzugten Ausführungsform betrifft die Erfindung die Verbindungen der Formel (I) oder der Formel (I-1) oder der Formeln (I-1a bis I-1f), wobei die Reste Q, V, W, Y, A und T definiert sind wie in Ausführungsform (0-1) oder Ausführungsform (0-2) oder Vorzugsbereich (1-1) oder Vorzugsbereich (1-2) oder Vorzugsbereich (2-1) oder Vorzugsbereich (2-2) oder Vorzugsbereich (4-A-1) oder Vorzugsbereich (4-A-2) oder Vorzugsbereich (5-A-1) oder Vorzugsbereich (5-A-2) und
X, E, L¹, L², R¹, R², R²¹, R³, R⁴, R³³, R⁴³, m, n, p, q die in Vorzugsbereich (3-A-1) oder (3-A-2) beschriebenen Bedeutungen haben.

In einer bevorzugten Ausführungsform betrifft die Erfindung die Verbindungen der Formel (I) oder der Formel (I-1) oder der Formeln (I-1a bis I-1f), wobei die Reste Q, V, W, Y, A und T definiert sind wie in Ausführungsform (0-1) oder Ausführungsform (0-2) oder Vorzugsbereich (1-1) oder Vorzugsbereich (1-2) oder Vorzugsbereich (2-1) oder Vorzugsbereich (2-2) oder Vorzugsbereich (3-A-1) oder Vorzugsbereich (3-A-2) oder Vorzugsbereich (5-A-1) oder Vorzugsbereich (5-A-2) und
X, E, L¹, L², R¹, R², R²¹, R³, R⁴, R³³, R⁴³, m, p und q die in Vorzugsbereich (4-A-1) oder Vorzugsbereich (4-A-2) beschriebenen Bedeutungen haben.

In einer bevorzugten Ausführungsform betrifft die Erfindung die Verbindungen der Formel (I) oder der Formel (I-1) oder der Formeln (I-1a bis I-1f), wobei die Reste Q, V, W, Y, A, und T definiert sind wie in Ausführungsform (0-1) oder Ausführungsform (0-2) oder Vorzugsbereich (1-1) oder Vorzugsbereich (1-2) oder Vorzugsbereich (2-1) oder Vorzugsbereich (2-2) oder Vorzugsbereich (3-A-1) oder Vorzugsbereich (3-A-2) oder Vorzugsbereich (4-A-1) oder Vorzugsbereich (4-A-2) und
X, E, L¹, L², R¹, R², R²¹, R³, R⁴, R³³, R⁴³, m, p und q die in Vorzugsbereich (5-A-1) oder Vorzugsbereich (5-A-2) beschriebenen Bedeutungen haben.

Im Vorzugsbereich (1-1) oder (1-2) ist, sofern nichts anderes angegeben ist,
Halogen ausgewählt aus der Reihe Fluor, Chlor, Brom und Iod, bevorzugt wiederum aus der Reihe Fluor, Chlor und Brom,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl für einen gesättigten 3-, 4-, 5- oder 6-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für Aziridinyl, Azetidinyl, Azolidinyl, Azinanyl, Oxiranyl, Oxetanyl, Oxolanyl, Oxanyl, Dioxanyl, Thiiranyl, Thietanyl, Thiolanyl, Thianyl, Tetrahydrofuryl.

Im Vorzugsbereich (2-1) oder (2-2) steht, sofern nichts anderes angegeben ist,
Halogen für Fluor, Chlor, Brom und Iod, bevorzugt für Fluor, Chlor und Brom,
Hetaryl (auch als Teil einer größeren Einheit, wie Hetarylalkyl) für Pyridyl, Pyrimidyl, Thiazolyl, Oxazolyl, Pyrazolyl, Thienyl, Furanyl, Benzyl, Pyridinylmethyl und Thiazolylmethyl sowie
Heterocyclyl (auch als Teil einer größeren Einheit, wie Heterocyclylalkyl) für einen gesättigten oder ungesättigten 3-, 4- oder 5-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für 1- oder 2-Aziridinyl, 2-Oxiranyl, 2-Thiiranyl, 1- oder 2-Azetidinyl, 2- oder 3-Oxetanyl, 2- oder 3-Thietanyl, 1,3-Dioxetan-2-yl, 1-, 2- oder 3-Pyrrolidinyl.

Im Vorzugsbereich (3-1) oder (3-2) steht, sofern nichts anderes angegeben ist,
Halogen für Fluor, Chlor, Brom und Iod, bevorzugt für Fluor, Chlor und Brom und
Heterocyclyl (auch als Teil einer größeren Einheit, wie Heterocyclylalkyl) für einen gesättigten oder ungesättigten 3- oder 4-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für 1- oder 2-Aziridinyl, 2-Oxiranyl, 2-Thiiranyl, 1- oder 2-Azetidinyl, 2- oder 3-Oxetanyl, 2- oder 3-Thietanyl oder 1,3-Dioxetan-2-yl. Durch Halogen substituierte Reste, z.B. Halogenalkyl (= Haloalkyl), sind, sofern nichts anderes gesagt ist, einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Wenn T in den Verbindungen der Formel (I) für Sauerstoff steht, liegen diese Verbindungen als N-Oxide vor.

Wenn T in den Verbindungen der Formel (I) für ein Elektronenpaar steht, liegen diese Verbindungen als Pyridine vor.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt (Vorzugsbereich (1-1) oder (1-2)).

Erfindungsgemäß besonders bevorzugt verwendet werden Verbindungen der Formel (I), in welchen eine Kombination der vorstehend als besonders bevorzugt aufgeführten Bedeutungen vorliegt (Vorzugsbereich (2-1) oder (2-2)).

Erfindungsgemäß ganz besonders bevorzugt verwendet werden Verbindungen der Formel (I), in welchen eine Kombination der vorstehend als ganz besonders bevorzugt aufgeführten Bedeutungen vorliegt (Vorzugsbereich (3-A-1) oder (3-A-2)).

Erfindungsgemäß insbesondere bevorzugt verwendet werden Verbindungen der Formel (I), in welchen eine Kombination der vorstehend als ganz besonders bevorzugt aufgeführten Bedeutungen vorliegt (Vorzugsbereich (4-A-1) oder (4-A-2)).

Erfindungsgemäß noch weiter bevorzugt verwendet werden Verbindungen der Formel (I), in welchen eine Kombination der vorstehend als ganz besonders bevorzugt aufgeführten Bedeutungen vorliegt (Vorzugsbereich (5-A-1) oder (5-A-2)).

Im Folgenden betreffen die Ausführungen zu den Verbindungen der Formel (I) selbstverständlich auch dieVerbindungen der Formel (I-1) und (I-1a bis f), welche in Formel (I) umfasst sind.

### Isomere

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit sowohl reine Stereoisomere als auch beliebige Gemische dieser Isomere.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel. Hierunter ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Im Rahmen der vorliegenden Anmeldung umfasst der Begriff Schädlingsbekämpfungsmittel jeweils immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, insbesondere Nematoden, und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen.

Im Rahmen der vorliegenden Patentanmeldung ist der Begriff "Hygiene" so zu verstehen, dass damit jegliche und alle Maßnahmen, Vorschriften und Verfahrensweisen gemeint sind, deren Ziel es ist, Krankheiten, insbesondere Infektionskrankheiten, zu verhindern, und die dazu dienen, die Gesundheit von Menschen und Tieren zu schützen und/oder die Umwelt zu schützen, und/oder die Sauberkeit aufrechterhalten. Erfindungsgemäß schließt dies insbesondere Maßnahmen zur Reinigung, Desinfektion und Sterilisation beispielsweise von Textilien oder harten Oberflächen, insbesondere Oberflächen aus Glas, Holz, Zement, Porzellan, Keramik, Kunststoff oder auch Metall(en) ein, um sicherzustellen, dass diese frei von Hygieneschädlingen und/oder ihren Ausscheidungen sind. Vorzugsweise ausgeschlossen vom Schutzbereich der Erfindung sind in dieser Hinsicht chirurgische oder therapeutische, auf den menschlichen Körper oder die Körper von Tieren anzuwendende Behandlungsvorschriften und diagnostische Vorschriften, die am menschlichen Körper oder den Körpern von Tieren durchgeführt werden.

Hinblick auf Hygiene in Küchen, Bäckereien, Flughäfen, Badezimmern, Schwimmbecken, Kaufhäusern, Hotels, Krankenhäusern, Ställen, Tierhaltungen usw.

Der Begriff "Hygieneschädling" ist daher so zu verstehen, dass damit ein oder mehrere Tierschädlinge gemeint sind, deren Gegenwart im Hygienesektor problematisch ist, insbesondere aus Gesundheitsgründen. Es ist daher ein Hauptziel, das Vorhandensein von Hygieneschädlingen und/oder das Ausgesetztsein ihnen gegenüber im Hygienesektor zu vermeiden oder auf ein Mindestmaß zu begrenzen. Dies lässt sich insbesondere durch die Anwendung eines Pestizids erreichen, das sich sowohl zum Verhindern eines Befalls als auch zum Verhindern eines bereits vorhandenen Befalls einsetzen lässt. Man kann auch Zubereitungen verwenden, die eine Exposition gegenüber Schädlingen verhindern oder reduzieren. Hygieneschädlinge schließen zum Beispiel die unten erwähnten Organismen ein.

Der Begriff "Hygieneschutz" deckt somit alle Handlungen ab, mit denen diese Hygienemaßnahmen, -vorschriften und -verfahrensweisen aufrechterhalten und/oder verbessert werden.

Die Verbindungen der Formel (I) können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z. B. Acarus spp., z. B. Acarus siro, Aceria kuko, Aceria sheldoni, Aculops spp., Aculus spp., z. B. Aculus fockeui, Aculus schlechtendali, Amblyomma spp., Amphitetranychus viennensis, Argas spp., Boophilus spp., Brevipalpus spp., z. B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z. B. Eotetranychus hicoriae, Epitrimerus pyri, Eutetranychus spp., z. B. Eutetranychus banksi, Eriophyes spp., z. B. Eriophyes pyri, Glycyphagus domesticus, Halotydeus destructor, Hemitarsonemus spp., z. B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z. B. Oligonychus coffeae, Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z. B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptcs spp., Scorpio maurus, Stcncotarsoncmus spp., Stcncotarsoncmus spinki, Tarsonemus spp., z. B. Tarsonemus confusus, Tarsonemus pallidus, Tetranychus spp., z. B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z. B. Geophilus spp., Scutigera spp.;
aus der Ordnung oder der Klasse der Collembola z. B. Onychiurus armatus; Sminthurus viridis;
aus der Klasse der Diplopoda z. B. Blaniulus guttulatus;
aus der Klasse der Insecta, z. B. aus der Ordnung der Blattodea z. B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z. B. Periplaneta americana, Periplaneta australasiae, Pycnoscelus surinamensis, Supella longipalpa;
aus der Ordnung der Coleoptera z. B. Acalymma vittatum, Acanthoscelides obtectus, Adoretus spp., Aethina tumida, Agelastica alni, Agriotes spp., z. B. Agriotes linneatus, Agriotes mancus, Alphitobius diaperinus, Amphimallon solstitialis, Anobium punctatum, Anoplophora spp., Anthonomus spp., z. B. Anthonomus grandis, Anthrenus spp., Apion spp., Apogonia spp., Atomaria spp., z. B. Atomaria linearis, Attagenus spp., Baris caerulescens, Bruchidius obtectus, Bruchus spp., z. B. Bruchus pisorum, Bruchus rufimanus, Cassida spp., Cerotoma trifurcata, Ceutorrhynchus spp., z. B. Ceutorrhynchus assimilis, Ceutorrhynchus quadridens, Ceutorrhynchus rapae, Chaetocnema spp., z. B. Chaetocnema confinis, Chaetocnema denticulata, Chaetocnema ectypa, Cleonus mendicus, Conoderus spp., Cosmopolites spp., z. B. Cosmopolites sordidus, Costelytra zealandica, Ctenicera spp., Curculio spp., z. B. Curculio caryae, Curculio caryatrypes, Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z. B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z. B. Epilachna borealis, Epilachna varivestis, Epitrix spp., z. B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z. B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z. B. Leucoptera coffeella, Lissorhoptrus oryzophilus, Listronotus (=Hyperodes) spp., Lixus spp., Luperodes spp., Luperomorpha xanthodera, Lyctus spp., Megascelis spp., Melanotus spp., z. B. Melanotus longulus oregonensis, Meligethes aeneus, Melolontha spp., z. B. Melolontha melolontha, Migdolus spp., Monochamus spp., Naupactus xanthographus, Necrobia spp., Neogalerucella spp., Niptus hololeucus, Oryctes rhinoceros, Oryzaephilus surinamensis, Oryzaphagus oryzae, Otiorhynchus spp., z. B. Otiorhynchus cribricollis, Otiorhynchus ligustici, Otiorhynchus ovatus, Otiorhynchus rugosostriarus, Otiorhynchus sulcatus, Oulema spp., z. B. Oulema melanopus, Oulema oryzae, Oxycetonia jucunda, Phaedon cochleariae, Phyllophaga spp., Phyllophaga helleri, Phyllotreta spp., z. B. Phyllotreta armoraciae, Phyllotreta pusilla, Phyllotreta ramosa, Phyllotreta striolata, Popillia japonica, Premnotrypes spp., Prostephanus truncatus, Psylliodes spp., z. B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z. B. Sitophilus granarius, Sitophilus linearis, Sitophilus oryzae, Sitophilus zeamais, Sphenophorus spp., Stegobium paniceum, Sternechus spp., z. B. Sternechus paludatus, Symphyletes spp., Tanymecus spp., z. B. Tanymecus dilaticollis, Tanymecus indicus, Tanymecus palliatus, Tenebrio molitor, Tenebrioides mauretanicus, Tribolium spp., z. B. Tribolium audax, Tribolium castaneum, Tribolium confusum, Trogoderma spp., Tychius spp., Xylotrechus spp., Zabrus spp., z. B. Zabrus tenebrioides;
aus der Ordnung der Dermaptera z. B. Anisolabis maritime, Forficula auricularia, Labidura riparia;
aus der Ordnung der Diptera z. B. Aedes spp., z. B. Aedes aegypti, Aedes albopictus, Aedes sticticus, Aedes vexans, Agromyza spp., z. B. Agromyza frontella, Agromyza parvicornis, Anastrepha spp., Anopheles spp., z. B. Anopheles quadrimaculatus, Anopheles gambiae, Asphondylia spp., Bactrocera spp., z. B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z. B. Contarinia johnsoni, Contarinia nasturtii, Contarinia pyrivora, Contarinia schulzi, Contarinia sorghicola, Contarinia tritici, Cordylobia anthropophaga, Cricotopus sylvestris, Culex spp., z. B. Culex pipiens, Culex quinquefasciatus, Culicoides spp., Culiseta spp., Cuterebra spp., Dacus oleae, Dasineura spp., z. B. Dasineura brassicae, Delia spp., z. B. Delia antiqua, Delia coarctata, Delia florilega, Delia platura, Delia radicum, Dermatobia hominis, Drosophila spp., z. B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z. B. Liriomyza brassicae, Liriomyza huidobrensis, Liriomyza sativae, Lucilia spp., z. B. Lucilia cuprina, Lutzomyia spp., Mansonia spp., Musca spp., z. B. Musca domestica, Musca domestica vicina, Oestrus spp., Oscinella frit, Paratanytarsus spp., Paralauterborniella subcincta, Pegomya oder Pegomyia spp., z. B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z. B. Rhagoletis cingulata, Rhagoletis completa, Rhagoletis fausta, Rhagoletis indifferens, Rhagoletis mendax, Rhagoletis pomonella, Sarcophaga spp., Simulium spp., z. B. Simulium meridionale, Stomoxys spp., Tabanus spp., Tetanops spp., Tipula spp., z. B. Tipula paludosa, Tipula simplex, Toxotrypana curvicauda;
aus der Ordnung der Hemiptera z. B. Acizzia acaciaebaileyanae, Acizzia dodonaeae, Acizzia uncatoides, Acrida turrita, Acyrthosipon spp., z. B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z. B. Amrasca bigutulla, Amrasca devastans, Anuraphis cardui, Aonidiella spp., z. B. Aonidiella aurantii, Aonidiella citrina, Aonidiella inornata, Aphanostigma piri, Aphis spp., z. B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z. B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z. B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z. B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Doralis spp., Drosicha spp., Dysaphis spp., z. B. Dysaphis apiifolia, Dysaphis plantaginea, Dysaphis tulipae, Dysmicoccus spp., Empoasca spp., z. B. Empoasca abrupta, Empoasca fabae, Empoasca maligna, Empoasca solana, Empoasca stevensi, Eriosoma spp., z. B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z. B. Icerya purchasi, Idiocerus spp., Idioscopus spp., Laodelphax striatellus, Lecanium spp., z. B. Lecanium corni (=Parthenolecanium corni), Lepidosaphes spp., z. B. Lepidosaphes ulmi, Lipaphis erysimi, Lopholeucaspis japonica, Lycorma delicatula, Macrosiphum spp., z. B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z. B. Myzus ascalonicus, Myzus cerasi, Myzus ligustri, Myzus ornatus, Myzus persicae, Myzus nicotianae, Nasonovia ribisnigri, Neomaskellia spp., Nephotettix spp., z. B. Nephotettix cincticeps, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z. B. Paratrioza cockerelli, Parlatoria spp., Pemphigus spp., z. B. Pemphigus bursarius, Pemphigus populivenae, Peregrinus maidis, Perkinsiella spp., Phenacoccus spp., z. B. Phenacoccus madeirensis, Phloeomyzus passerinii, Phorodon humuli, Phylloxera spp., z. B. Phylloxera devastatrix, Phylloxera notabilis, Pinnaspis aspidistrae, Planococcus spp., z. B. Planococcus citri, Prosopidopsylla flava, Protopulvinaria pyriformis, Pseudaulacaspis pentagona, Pseudococcus spp., z. B. Pseudococcus calceolariae, Pseudococcus comstocki, Pseudococcus longispinus, Pseudococcus maritimus, Pseudococcus viburni, Psyllopsis spp., Psylla spp., z. B. Psylla buxi, Psylla mali, Psylla pyri, Pteromalus spp., Pulvinaria spp., Pyrilla spp., Quadraspidiotus spp., z. B. Quadraspidiotus juglansregiae, Quadraspidiotus ostreaeformis, Quadraspidiotus perniciosus, Quesada gigas, Rastrococcus spp., Rhopalosiphum spp., z. B. Rhopalosiphum maidis, Rhopalosiphum oxyacanthae, Rhopalosiphum padi, Rhopalosiphum rufiabdominale, Saissetia spp., z. B. Saissetia coffeae, Saissetia miranda, Saissetia neglecta, Saissetia oleae, Scaphoideus titanus, Schizaphis graminum, Selenaspidus articulatus, Sipha flava, Sitobion avenae, Sogata spp., Sogatella furcifera, Sogatodes spp., Stictocephala festina, Siphoninus phillyreae, Tenalaphara malayensis, Tetragonocephela spp., Tinocallis caryaefoliae, Tomaspis spp., Toxoptera spp., z. B. Toxoptera aurantii, Toxoptera citricidus, Trialeurodes vaporariorum, Trioza spp., z. B. Trioza diospyri, Typhlocyba spp., Unaspis spp., Viteus vitifolii, Zygina spp.;
aus der Unterordnung der Heteroptera z. B. Aelia spp., Anasa tristis, Antestiopsis spp., Boisea spp., Blissus spp., Calocoris spp., Campylomma livida, Cavelerius spp., Cimex spp., z. B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z. B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z. B. Lygocoris pabulinus, Lygus spp., z. B. Lygus elisus, Lygus hesperus, Lygus lineolaris, Macropes excavatus, Megacopta cribraria, Miridae, Monalonion atratum, Nezara spp., z. B. Nezara viridula, Nysius spp., Oebalus spp., Pentomidae, Piesma quadrata, Piezodorus spp., z. B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z. B. Acromyrmex spp., Athalia spp., z. B. Athalia rosae, Atta spp., Camponotus spp., Dolichovespula spp., Diprion spp., z. B. Diprion similis, Hoplocampa spp., z. B. Hoplocampa cookei, Hoplocampa testudinea, Lasius spp., Linepithema (Iridiomyrmex) humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z. B. Vespa crabro, Wasmannia auropunctata, Xeris spp.;
aus der Ordnung der Isopoda z. B. Armadillidium vulgare, Oniscus asellus, Porcellio scaber;
aus der Ordnung der Isoptera z. B. Coptotermes spp., z. B. Coptotermes formosanus, Cornitermes cumulans, Cryptotermes spp., Incisitermes spp., Kalotermes spp., Microtermes obesi, Nasutitermis spp., Odontotermes spp., Porotermes spp., Reticulitermes spp., z. B. Reticulitermes flavipes, Reticulitermes hesperus;
aus der Ordnung der Lepidoptera z. B. Achroia grisella, Acronicta major, Adoxophyes spp., z. B. Adoxophyes orana, Aedia leucomelas, Agrotis spp., z. B. Agrotis segetum, Agrotis ipsilon, Alabama spp., z. B. Alabama argillacea, Amyelois transitella, Anarsia spp., Anticarsia spp., z. B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z. B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z. B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z. B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z. B. Euproctis chrysorrhoea, Euxoa spp., Feltia spp., Galleria mellonella, Gracillaria spp., Grapholitha spp., z. B. Grapholita molesta, Grapholita prunivora, Hedylepta spp., Helicoverpa spp., z. B. Helicoverpa armigera, Helicoverpa zea, Heliothis spp., z. B. Heliothis virescens Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z. B. Leucoptera coffeella, Lithocolletis spp., z. B. Lithocolletis blancardella, Lithophane antennata, Lobesia spp., z. B. Lobesia botrana, Loxagrotis albicosta, Lymantria spp., z. B. Lymantria dispar, Lyonetia spp., z. B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z. B. Ostrinia nubilalis, Panolis flammea, Parnara spp., Pectinophora spp., z. B. Pectinophora gossypiella, Perileucoptera spp., Phthorimaea spp., z. B. Phthorimaea operculella, Phyllocnistis citrella, Phyllonorycter spp., z. B. Phyllonorycter blancardella, Phyllonorycter crataegella, Pieris spp., z. B. Pieris rapae, Platynota stultana, Plodia interpunctella, Plusia spp., Plutella xylostella (=Plutella maculipennis), Prays spp., Prodenia spp., Protoparce spp., Pseudaletia spp., z. B. Pseudaletia unipuncta, Pseudoplusia includens, Pyrausta nubilalis, Rachiplusia nu, Schoenobius spp., z. B. Schoenobius bipunctifer, Scirpophaga spp., z. B. Scirpophaga innotata, Scotia segetum, Sesamia spp., z. B. Sesamia inferens, Sparganothis spp., Spodoptera spp., z. B. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z. B. Trichoplusia ni, Tryporyza incertulas, Tuta absoluta, Virachola spp.;
aus der Ordnung der Orthoptera oder Saltatoria z. B. Acheta domesticus, Dichroplus spp., Gryllotalpa spp., z. B. Gryllotalpa gryllotalpa, Hieroglyphus spp., Locusta spp., z. B. Locusta migratoria, Melanoplus spp., z. B. Melanoplus devastator, Paratlanticus ussuriensis, Schistocerca gregaria;
aus der Ordnung der Phthiraptera z. B. Damalinia spp., Haematopinus spp., Linognathus spp., Pediculus spp., Phylloxera vastatrix, Phthirus pubis, Trichodectes spp.;
aus der Ordnung der Psocoptera z. B. Lepinotus spp., Liposcelis spp.;
aus der Ordnung der Siphonaptera z. B. Ceratophyllus spp., Ctenocephalides spp., z. B. Ctenocephalides canis, Ctenocephalides felis, Pulex irritans, Tunga penetrans, Xenopsylla cheopis;
aus der Ordnung der Thysanoptera z. B. Anaphothrips obscurus, Baliothrips biformis, Chaetanaphothrips leeuweni, Drepanothrips reuteri, Enneothrips flavens, Frankliniella spp., z. B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Kakothrips spp., Rhipiphorothrips cruentatus, Scirtothrips spp., Taeniothrips cardamomi, Thrips spp., z. B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z. B. Scutigerella spp., z. B. Scutigerella immaculata;
Schädlinge aus dem Stamm der Mollusca, z. B. aus der Klasse der Bivalvia, z. B. Dreissena spp.;
sowie aus der Klasse der Gastropoda z. B. Arion spp., z. B. Arion ater rufus, Biomphalaria spp., Bulinus spp., Deroceras spp., z. B. Deroceras laeve, Galba spp., Lymnaea spp., Oncomelania spp., Pomacea spp., Succinea spp.;
Pflanzenschädlinge aus dem Stamm der Nematoda, d. h. pflanzenparasitäre Nematoden, insbesondere Aglenchus spp., z. B. Aglenchus agricola, Anguina spp., z. B. Anguina tritici, Aphelenchoides spp., z. B. Aphelenchoides arachidis, Aphelenchoides fragariae, Belonolaimus spp., z. B. Belonolaimus gracilis, Belonolaimus longicaudatus, Belonolaimus nortoni, Bursaphelenchus spp., z. B. Bursaphelenchus cocophilus, Bursaphelenchus eremus, Bursaphelenchus xylophilus, Cacopaurus spp., z. B. Cacopaurus pestis, Criconemella spp., z. B. Criconemella curvata, Criconemella onoensis, Criconemella ornata, Criconemella rusium, Criconemella xenoplax (= Mesocriconema xenoplax), Criconemoides spp., z. B. Criconemoides ferniae, Criconemoides onoense, Criconemoides ornatum, Ditylenchus spp., z. B. Ditylenchus dipsaci, Dolichodorus spp., Globodera spp., z. B. Globodera pallida, Globodera rostochiensis, Helicotylenchus spp., z. B. Helicotylenchus dihystera, Hemicriconemoides spp., Hemicycliophora spp., Heterodera spp., z. B. Heterodera avenae, Heterodera glycines, Heterodera schachtii, Hirschmaniella spp., Hoplolaimus spp., Longidorus spp., z. B. Longidorus africanus, Meloidogyne spp., z. B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z. B. Paratrichodorus minor, Paratylenchus spp., Pratylenchus spp., z. B. Pratylenchus penetrans, Pseudohalenchus spp., Psilenchus spp., Punctodera spp., Quinisulcius spp., Radopholus spp., z. B. Radopholus citrophilus, Radopholus similis, Rotylenchulus spp., Rotylenchus spp., Scutellonema spp., Subanguina spp., Trichodorus spp., z. B. Trichodorus obtusus, Trichodorus primitivus, Tylenchorhynchus spp., z. B. Tylenchorhynchus annulatus, Tylenchulus spp., z. B. Tylenchulus semipenetrans, Xiphinema spp., z. B. Xiphinema index.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasmalike-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. pflanzliche Öle wie beispielsweise Rapsöl, Sonnenblumenöl, Mineralöle wie beispielsweise Paraffinöle, Alkylester pflanzlicher Fettsäuren wie beispielsweise Rapsöl- oder Sojaölmethylester oder Alkanol-alkoxylate und/oder Spreitmittel wie beispielsweise Alkylsiloxane und/oder Salze, z. B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar-Polymere und/oder Humectants wie z. B. Glycerin und/oder Dünger wie beispielsweise Ammonium, Kalium oder Phosphor enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 bcschricbcn. Gegebenenfalls enthalten die Formulierungen neben einer oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z. B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktiven Stoffen. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z. B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z. B. Wasser, polare und unpolare organische chemische Flüssigkeiten z. B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z. B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasserstoffe wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z. B. Xylol, Toluol oder Alkylnaphthaline, chlorierte aromatische oder chlorierte aliphatische Kohlenwasserstoffe wie z. B. Chlorbenzol, Chlorethylen, oder Methylenchlorid, aliphatische Kohlenwasserstoffe wie z. B. Cyclohexan, Paraffine, Erdölfraktionen, mineralische und pflanzliche Öle, Alkohole wie z. B. Methanol, Ethanol, iso-Propanol, Butanol oder Glykol sowie deren Ether und Ester, Ketone wie z. B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z. B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und natürliche oder synthetische Silikate, Harze, Wachse und/oder feste Düngemittel. Mischungen solcher Trägerstoffe können ebenfalls verwendet werden. Als Trägerstoffe für Granulate kommen infrage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z. B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulfonsäure, Salze von Phenolsulfonsäure oder Naphthalinsulfonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulfobernsteinsäureestern, Taurinderivate (vorzugsweise Alkyltaurate), Phosphorsäureester von polyethoxylierten Alkoholen oder Phenolen, Fettsäureester von Polyolen und Derivate der Verbindungen enthaltend Sulfate, Sulfonate und Phosphate, z. B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z. B. Eisenoxid, Titanoxid, Ferrocyanblau und organische Farbstoffe wie Alizarin-, Azo- und Mctallphthalocyaninfarbstoffc und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und pflanzliche Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Feuchthaltemittel, Spreitmittel. Im Allgemeinen können die Verbindungen der Formel (I) mit jedem festen oder flüssigen Zusatzstoff, welcher für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar-Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Beweglichkeit der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettaminalkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbiziden, Düngemitteln, Vogelrepellentien, Phytotonics, Sterilantien, Safenern, Semiochemicals und/oder Pflanzenwachstumsregulatoren verwendet werden, um so z. B. das Wirkungsspektrum zu verbreitern, die Wirkdauer zu verlängern, die Wirkgeschwindigkeit zu steigern, Repellenz zu verhindern oder Resistenzentwicklungen vorzubeugen. Des Weiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteertrag steigern, die Reife beeinflussen, die Qualität und/oder der Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann, sind auch diese Formen mit umfasst, auch wenn sie sie nicht in jedem Fall explizit genannt wurden. Alle genannten Mischungspartner können außerdem, wenn sie auf Grund ihrer funktionellen Gruppen dazu imstande sind, gegebenenfalls mit geeigneten Basen oder Säuren Salze bilden.

### Insektizide/Akarizide/Nematizide

Die hier mit ihrem "Common Name" genannten Wirkstoffe sind bekannt und beispielsweise im Pestizidhandbuch ("The Pesticide Manual" 16th Ed., British Crop Protection Council 2012) beschrieben oder im Internet recherchierbar (z. B. http://www.alanwood.net/pesticides). Die Klassifizierung basiert auf dem zum Zeitpunkt der Einreichung dieser Patentanmeldung gültigen IRAC Mode of Action Classification Scheme.
(1) Acetylcholinesterase(AChE)-Inhibitoren, wie beispielsweise Carbamate, z. B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder Organophosphate, z. B. Acephat, Azamethiphos, Azinphos-ethyl, Azinphos-methyl, Cadusafos, Chlorethoxyfos, Chlorfenvinphos, Chlormephos, Chlorpyrifos-methyl, Coumaphos, Cyanophos, Demeton-S-methyl, Diazinon, Dichlorvos/DDVP, Dicrotophos, Dimethoat, Dimethylvinphos, Disulfoton, EPN, Ethion, Ethoprophos, Famphur, Fenamiphos, Fenitrothion, Fenthion, Fosthiazat, Heptenophos, Imicyafos, Isofenphos, Isopropyl-O-(methoxyaminothio-phosphoryl)salicylat, Isoxathion, Malathion, Mecarbam, Methamidophos, Methidathion, Mevinphos, Monocrotophos, Naled, Omethoate, Oxydemeton-methyl, Parathion-methyl, Phenthoat, Phorat, Phosalon, Phosmet, Phosphamidon, Phoxim, Pirimiphos-methyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Blocker, wie beispielsweise Cyclodien-organochlorine, z. B. Chlordan und Endosulfan oder Phenylpyrazole (Fiprole), z. B. Ethiprol und Fipronil.
(3) Natrium-Kanal-Modulatoren, wie beispielsweise Pyrethroide, z. B. Acrinathrin, Allethrin, d-cistrans-Allethrin, d-trans-Allethrin, Bifenthrin, Bioallethrin, Bioallethrin-S-cyclopentenyl-Isomer, Bioresmethrin, Cycloprothrin, Cyfluthrin, beta-Cyfluthrin, Cyhalothrin, lambda-Cyhalothrin, gamma-Cyhalothrin, Cypermethrin, alpha-Cypermethrin, beta-Cypermethrin, theta-Cypermethrin, zeta-Cypermethrin, Cyphenothrin [(1R)-trans-Isomer], Deltamethrin, Empenthrin [(EZ)-(1R)-Isomer], Esfenvalerat, Etofenprox, Fenpropathrin, Fenvalerat, Flucythrinat, Flumethrin, tau-Fluvalinat, Halfenprox, Imiprothrin, Kadethrin, Momfluorothrin, Permethrin, Phenothrin [(1R)-trans-Isomer], Prallethrin, Pyrethrine (pyrethrum), Resmethrin, Silafluofen, Tefluthrin, Tetramethrin, Tetramethrin [(1R)-Isomer], Tralomethrin und Transfluthrin oder DDT oder Methoxychlor.
(4) Kompetitive Modulatoren des nicotinischen Acetylcholin-Rezeptors (nAChR), wie beispielsweise Neonicotinoide, z. B. Acetamiprid, Clothianidin, Dinotefuran, Imidacloprid, Nitenpyram, Thiacloprid und Thiamcthoxam oder Nicotin oder Sulfoxaflor oder Flupyradifuronc.
(5) Allosterische Modulatoren des nicotinischen Acetylcholin-Rezeptors (nAChR), wie beispielsweise Spinosyne, z. B. Spinetoram und Spinosad.
(6) Allosterische Modulatoren des Glutamat-abhängigen Chloridkanals(GluCl), wie beispielsweise Avermectine/Milbemycine, z. B. Abamectin, Emamectin-benzoat, Lepimectin und Milbemectin.
(7) Juvenilhormon-Mimetika, wie beispielsweise Juvenilhormon-Analoge, z. B. Hydropren, Kinopren und Methopren oder Fenoxycarb oder Pyriproxyfen.
(8) Verschiedene nicht spezifische (multi-site) Inhibitoren, wie beispielsweise Alkylhalogenide, z. B. Methylbromid und andere Alkylhalogenide; oder Chloropicrin oder Sulfurylfluorid oder Borax oder Brechweinstein oder Methylisocyanaterzeuger, z. B. Diazomet und Metam.
(9) Modulatoren chordotonaler Organe, z. B. Pymetrozin oder Flonicamid.
(10) Milbenwachstumsinhibitoren, wie z. B. Clofentezin, Hexythiazox und Diflovidazin oder Etoxazol.
(11) Mikrobielle Disruptoren der Insektendarmmembran, wie z. B. *Bacillus thuringiensis* Subspezies *israelensis, Bacillus sphaericus, Bacillus thuringiensis* Subspezies *aizawai, Bacillus thuringiensis* Subspezies *kurstaki, Bacillus thuringiensis* Subspezies *tenebrionis* und *B.t*.-Pflanzenproteine: Cry1Ab, Cry1Ac, Cry1Fa, Cry1A.105, Cry2Ab, VIP3A, mCry3A, Cry3Ab, Cry3Bb, Cry34Ab1/35Ab1.
(12) Inhibitoren der mitochondrialen ATP-Synthase, wie ATP-Disruptoren, wie beispielsweise Diafenthiuron oder Organozinnverbindungen, z. B. Azocyclotin, Cyhexatin und Fenbutatin-oxid oder Propargit oder Tetradifon.
(13) Entkoppler der oxidativen Phoshorylierung durch Störung des Protonengradienten, wie beispielsweise Chlorfenapyr, DNOC und Sulfluramid.
(14) Blocker des nicotinischen Acetylcholinrezeptorkanals, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungsdisruptor (insbesondere bei Dipteren, d. h. Zweiflüglern), wie beispielsweise Cyromazin.
(18) Ecdyson-Rezeptor-Agonisten, wie beispielsweise Chromafenozid, Halofenozid, Methoxyfenozid und Tebufenozid.
(19) Oktopamin-Rezeptor-Agonisten, wie beispielsweise Amitraz.
(20) Mitochondriale Komplex-III-Elektronentransportinhibitoren, wie beispielsweise Hydramethylnon oder Acequinocyl oder Fluacrypyrim.
(21) Mitochondriale Komplex-I-Elektronentransportinhibitoren, wie beispielsweise METI-Akarizide, z. B. Fenazaquin, Fenpyroximat, Pyrimidifen, Pyridaben, Tebufenpyrad und Tolfenpyrad oder Rotenon (Derris).
(22) Blocker des spannungsabhängigen Natriumkanals, wie z. B. Indoxacarb oder Metaflumizone.
(23) Inhibitoren der Acetyl-CoA-Carboxylase, wie beispielsweise Tetron- und Tetramsäurederivate, z. B. Spirodiclofen, Spiromesifen und Spirotetramat.
(24) Inhibitoren des mitochondrialen Komplex-IV-Elektronentransports, wie beispielsweise Phosphine, z. B. Aluminiumphosphid, Calciumphosphid, Phosphin und Zinkphosphid oder Cyanide, Calciumcyanid, Kaliumcyanid und Natriumcyanid.
(25) Inhibitoren des mitochondrialen Komplex-II-Elektronentransports, wie beispielsweise beta-Ketonitrilderivate, z. B. Cyenopyrafen und Cyflumetofen und Carboxanilide, wie beispielsweise Pyflubumid.
(28) Ryanodinrezeptor-Modulatoren, wie beispielsweise Diamide, z. B. Chlorantraniliprol, Cyantraniliprol und Flubendiamid,
weitere Wirkstoffe wie beispielsweise Afidopyropen, Afoxolaner, Azadirachtin, Benclothiaz, Benzoximat, Bifenazat, Broflanilid, Bromopropylat, Chinomethionat, Chloroprallethrin, Cryolit, Cyclaniliprol, Cycloxaprid, Cyhalodiamid, Dicloromezotiaz, Dicofol, epsilon-Metofluthrin, epsilon-Momfluthrin, Flometoquin, Fluazaindolizin, Fluensulfon, Flufenerim, Flufenoxystrobin, Flufiprol, Fluhexafon, Fluopyram, Fluralaner, Fluxametamid, Fufenozid, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprol, Tetrachlorantraniliprol, Tioxazafen, Thiofluoximat, Triflumezopyrim und Iodmethan; des Weiteren Präparate auf Basis von *Bacillus firmus* (I-1582, BioNeem, Votivo), sowie folgende Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635) (CAS 885026-50-6), {1'-[(2E)-3-(4-Chlorphenyl)prop-2-en-1-yl]-5-fluorspiro[indol-3,4'-piperidin]-1(2H)-yl}(2-chlorpyridin-4-yl)methanon (bekannt aus WO2003/106457) (CAS 637360-23-7), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494) (CAS 872999-66-1), 3-(4-Chlor-2,6-dimethylphenyl)-4-hydroxy-8-methoxy-1,8-diazaspiro[4.5]dec-3-en-2-on (bekannt aus WO 2010052161) (CAS 1225292-17-0), 3-(4-Chlor-2, 6-dimethylphenyl)-8-methoxy-2-oxo-1,8-diazaspiro[4.5]dec-3-en-4-yl-ethylcarbonat (bekannt aus EP 2647626) (CAS-1440516-42-6), 4-(But-2-in-1-yloxy)-6-(3,5-dimethylpiperidin-1-yl)-5-fluorpyrimidin (bekannt aus WO2004/099160) (CAS 792914-58-0), PF1364 (bekannt aus JP2010/018586) (CAS-Reg.No. 1204776-60-2), N-[(2E)-1-[(6-Chlorpyridin-3-yl)methyl]pyridin-2(1H)-yliden]-2,2,2-trifluoracetamid (bekannt aus WO2012/029672) (CAS 1363400-41-2), (3E)-3-[1-[(6-Chlor-3-pyridyl)methyl]-2-pyridyliden]-1,1,1-trifluorpropan-2-on (bekannt aus WO2013/144213) (CAS 1461743-15-6), N-[3-(Benzylcarbamoyl)-4-chlorphenyl]-1-methyl-3-(pentafluorethyl)-4-(trifluormethyl)-1H-pyrazol-5-carboxamid (bekannt aus WO2010/051926) (CAS 1226889-14-0), 5-Brom-4-chlor-N-[4-chlor-2-methyl-6-(methylcarbamoyl)phenyl]-2-(3-chlor-2-pyridyl)pyrazol-3-carboxamid (bekannt aus CN103232431) (CAS 1449220-44-3), 4-[5-(3,5-Dichlorphenyl)-4,5-dihydro-5-(trifluormethyl)-3-isoxazolyl]-2-methyl-N-(cis-l-oxido-3-thietanyl)benzamid, 4-[5-(3,5-Dichlorphenyl)-4,5-dihydro-5-(trifluormethyl)-3-isoxazolyl]-2-methyl-N-(trans-1-oxido-3-thietanyl)benzamid und 4-[(5S)-5-(3,5-Dichlorphenyl)-4,5-dihydro-5-(trifluormethyl)-3-isoxazolyl]-2-methyl-N-(cis-1-oxido-3-thietanyl)benzamid (bekannt aus WO 2013/050317 A1) (CAS 1332628-83-7), N-[3-Chlor-1-(3-pyridinyl)-1H-pyrazol-4-yl]-N-ethyl-3-[(3,3,3-trifluorpropyl)sulfinyl]propanamid, (+)-N-[3-Chlor-1-(3-pyridinyl)-1H-pyrazol-4-yl]-N-ethyl-3-[(3,3,3-trifluorpropyl)sulfinyl]propanamid und (-)-N-[3-Chlor-1-(3-pyridinyl)-1H-pyrazol-4-yl]-N-ethyl-3-[(3,3,3-trifluorpropyl)sulfmyl]propanamid (bekannt aus WO 2013/162715 A2, WO 2013/162716 A2, US 2014/0213448 A1) (CAS 1477923-37-7), 5-[[(2E)-3-Chlor-2-propen-1-yl]amino]-1-[2,6-dichlor-4-(trifluormethyl)phenyl]-4-[(trifluormethyl)sulfinyl]-1H-pyrazol-3-carbonitrile (bekannt aus CN 101337937 A) (CAS 1105672-77-2), 3-Brom-N-[4-chlor-2-methyl-6-[(methylamino)thioxomethyl]phenyl]-1-(3-chlor-2-pyridinyl)-1H-pyrazol-5-carboxamid, (Liudaibenjiaxuanan, bekannt aus CN 103109816 A) (CAS 1232543-85-9); N-[4-Chlor-2-[[(1,1-dimethylethyl)amino]carbonyl]-6-methylphenyl]-1-(3-chlor-2-pyridinyl)-3-(fluormethoxy)-1H-pyrazol-5-carboxamid (bekannt aus WO 2012/034403 A1) (CAS 1268277-22-0), N-[2-(5-Amino-1,3,4-thiadiazol-2-yl)-4-chlor-6-methylphenyl]-3-brom-1-(3-chlor-2-pyridinyl)-1H-pyrazol-5-carboxamid (bekannt aus WO 2011/085575 A1) (CAS 1233882-22-8), 4-[3-[2,6-Dichlor-4-[(3,3-dichlor-2-propen-1-yl)oxy]phenoxy]propoxy]-2-methoxy-6-(trifluormethyl)pyrimidin (bekannt aus CN 101337940 A) (CAS 1108184-52-6); (2E)- und 2(Z)-2-[2-(4-Cyanophenyl)-1-[3-(trifluormethyl)phenyl]ethyliden]-N-[4-(difluormethoxy)phenyl]hydrazincarboxamid (bekannt aus CN 101715774 A) (CAS 1232543-85-9); Cyclopropancarbonsäure-3-(2,2-dichlorethenyl)-2,2-dimethy|-4-(1H-benzimidazol-2-yl)phenylester (bekannt aus CN 103524422 A) (CAS 1542271-46-4); (4aS)-7-Chlor-2,5-dihydro-2-[[(methoxycarbonyl)[4-[(trifluormethyl)thio]phenyl]amino]carbonyl]indeno[1,2-e] [1,3,4]oxadiazin-4a(3H)-carbonsäuremethylester (bekannt aus CN 102391261 A) (CAS 1370358-69-2); 6-Desoxy-3-O-ethyl-2,4-di-O-methyl-1-[N-[4-[1-[4-(1,1,2,2,2-pentaf|uorethoxy)phenyl]-1H-1,2,4-triazol-3-yl]phenyl]carbamat]-α-L-mannopyranose (bekannt aus US 2014/0275503 A1) (CAS 1181213-14-8); 8-(2-Cyclopropylmethoxy-4-trifluormethylphenoxy)-3-(6-trifluormethylpyridazin-3-yl)-3-azabicyclo[3.2.1]octan (CAS 1253850-56-4), (8-anti)-8-(2-Cyclopropylmethoxy-4-trifluormethylphenoxy)-3-(6-trifluormethylpyridazin-3-yl)-3-azabicyclo[3.2.1]octan (CAS 933798-27-7), (8-syn)-8 -(2-Cyclopropylmethoxy-4-trifluormethylphenoxy)-3 -(6-trifluormethylpyridazin-3-yl)-3-azabicyclo[3.2.1]octan (bekannt aus WO 2007040280 A1, WO 2007040282 A1) (CAS 934001-66-8) und N-[3 -Chlor-1-(3-pyridinyl)-1H-pyrazol-4-yl]-N-ethyl-3-[(3,3,3-trifluorpropyl)thio]-propanamid (bekannt aus WO 2015/058021 A1, WO 2015/058028 A1) (CAS 1477919-27-9).

### Fungizide

Die hier mit ihrem "Common Name" spezifizierten Wirkstoffe sind bekannt und beispielsweise im "Pesticide Manual" (16. Aufl. British Crop Protection Council) oder im Internet recherchierbar (beispielsweise: http://www.alanwood.net/pesticides) beschrieben.

Alle genannten Mischungspartner der Klassen (1) bis (15) können, wenn sie auf Grund ihrer funktionellen Gruppen dazu imstande sind, gegebenenfalls mit geeigneten Basen oder Säuren Salze bilden. Alle genannten fungiziden Mischungspartner der Klassen (1) bis (15) können gegebenenfalls tautomere Formen einschließen.
1) Inhibitoren der Ergosterol-Biosynthese, beispielsweise (1.001) Cyproconazol, (1.002) Difenoconazol, (1.003) Epoxiconazol, (1.004) Fenhexamid, (1.005) Fenpropidin, (1.006) Fenpropimorph, (1.007) Fenpyrazamin, (1.008) Fluquinconazol, (1.009) Flutriafol, (1.010) Imazalil, (1.011) Imazalil Sulfat, (1.012) Ipconazol, (1.013) Metconazol, (1.014) Myclobutanil, (1.015) Paclobutrazol, (1.016) Prochloraz, (1.017) Propiconazol, (1.018) Prothioconazol, (1.019) Pyrisoxazol, (1.020) Spiroxamin, (1.021) Tebuconazol, (1.022) Tetraconazol, (1.023) Triadimenol, (1.024) Tridemorph, (1.025) Triticonazol, (1.026) (1R,2S,5S)-5-(4-Chlorbenzyl)-2-(chlormethyl)-2-methyl-1-(1H-1,2,4-triazol-1-ylmethyl)cyclopentanol, (1.027) (1S,2R,5R)-5-(4-Chlorbenzyl)-2-(chlormethyl)-2-methyl-1-(1H-1,2,4-triazol-l-ylmethyl)cyclopentanol, (1.028) (2R)-2-(1-Chlorcyclopropyl)-4-[(1R)-2,2-dichlorcyclopropyl]-1-(1H-1,2,4-triazol-1-yl)butan-2-ol (1.029) (2R)-2-(1-Chlorcyclopropyl)-4-[(1S)-2,2-dichlorcyclopropyl]-1-(1H-1,2,4-triazol-1-yl)butan-2-ol, (1.030) (2R)-2-[4-(4-Chlorphenoxy)-2-(trifluormethyl)phenyl]-1-(1H-1,2,4-triazol-1-yl)propan-2-ol, (1.031) (2S)-2-(1-Chlorcyclopropyl)-4-[(1R)-2,2-dichlorcyclopropyl]-1-(1H-1,2,4-triazol-1-yl)butan-2-ol, (1.032) (2S)-2-(1-Chlorcyclopropyl)-4-[(1S)-2,2-dichlorcyclopropyl]-1-(1H-1,2,4-triazol-1-yl)butan-2-ol, (1.033) (2S)-2-[4-(4-Chlorphenoxy)-2-(trifluormethyl)phenyl]-1-(1H-1,2,4-triazol-1-yl)propan-2-ol, (1.034) (R)-[3-(4-Chlor-2-fluorphenyl)-5-(2,4-difluorphenyl)-1,2-oxazol-4-yl](pyridin-3-yl)methanol, (1.035) (S)-[3-(4-Chlor-2-fluorphenyl)-5-(2,4-difluorphenyl)-1,2-oxazol-4-yl](pyridin-3-yl)methanol, (1.036) [3-(4-Chlor-2-fluorphenyl)-5-(2,4-difluorphenyl)-1,2-oxazol-4-yl](pyridin-3-yl)methanol, (1.037) 1-({(2R,4S)-2-[2-Chlor-4-(4-chlorphenoxy)phenyl]-4-methyl-1,3-dioxolan-2-yl}methyl)-1H-1,2,4-triazol, (1.038) 1-({(2S,4S)-2-[2-Chlor-4-(4-chlorphenoxy)phenyl]-4-methyl-1,3-dioxolan-2-yl}methyl)-1H-1,2,4-triazol, (1.039) 1-{[3-(2-Chlorphenyl)-2-(2,4-difluorphenyl)oxiran-2-yl]methyl}-1H-1,2,4-triazol-5-yl-thiocyanat, (1.040) 1-{[rel(2R,3R)-3-(2-Chlorphenyl)-2-(2,4-difluorphenyl)oxiran-2-yl]methyl}-1H-1,2,4-triazol-5-yl-thiocyanat, (1.041) 1-{[rel(2R,3S)-3-(2-Chlorphenyl)-2-(2,4-difluorphenyl)oxiran-2-yl]methyl}-1H-1,2,4-triazol-5-yl-thiocyanat, (1.042) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.043) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.044) 2-[(2R,4S,5R)-1-(2,4-Dichlorophenyl)-5-hydroxy-2,6,6-trimethylheptan-4-yl]-2,4-dihydro-3H-1,2,4-triazol-3-thion, (1.045) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.046) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.047) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.048) 2-[(2S,4S,5R)-1-(2,4- Dichlorphenyl)-5- hydroxy-2,6,6-trimethylheptan-4-yl]-2,4-dihydro- 3H-1,2,4-triazol-3-thion, (1.049) 2-[(2S,4S,SS)-1-(2,4-Dichlorphenyl)-5-hydroxy-2,6,6-trimethylheptan-4-yl]-2,4-dihydro-3H-1,2,4-triazol-3-thion, (1.050) 2-[1-(2,4-Dichlorphenyl)-5-hydroxy-2,6,6-trimethylheptan-4-yl]-2,4-dihydro-3H-1,2,4-triazol-3-thion, (1.051) 2-[2-Chlor-4-(2,4-dichlorophenoxy)phenyl]-1-(1H-1,2,4-triazol-1-yl)propan-2-ol, (1.052) 2-[2-Chlor-4-(4-chlorphenoxy)phenyl]-1-(1H-1,2,4-triazol-1-yl)butan-2-ol, (1.053) 2-[4-(4-Chlorphenoxy)-2-(trifluormethyl)phenyl]-1-(1H-1,2,4-triazol-1-yl)butan-2-ol, (1.054) 2-[4-(4-Chlorphenoxy)-2-(trifluormethyl)phenyl]-1-(IH-1,2,4-triazo1-1-yl)pentan-2-ol, (1.055) 2-[4-(4-Chlorphenoxy)-2-(trifluormethyl)phenyl]-1-(1H-1,2,4-triazol-1-yl)propan-2-ol, (1.056) 2-{[3-(2-Chlorphenyl)-2-(2,4-difluorphenyl)oxiran-2-yl]methyl}-2,4-dihydro-3H-1,2,4-triazol-3-thion, (1.057) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.058) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.059) 5-(4-Chlorbenzyl)-2-(chlormethyl)-2-methyl-1-(1H-1,2,4-triazol-1-ylmethyl)cyclopentanol, (1.060) 5-(Allylsulfanyl)-1- {[3-(2-chlorphenyl)-2-(2,4-difluorphenyl)oxiran-2-yl]methyl} -1H-1,2,4-triazol, (1.061) 5-(Allylsulfanyl)-1 - {[rel(2R,3R)-3-(2-chlorphenyl)-2-(2,4-difluorphenyl)oxiran-2-yl]methyl}-1H-1,2,4-triazol, (1.062) 5-(Allylsulfanyl)-1-{[rel(2R,3S)-3-(2-chlorphenyl)-2-(2,4-difluorphenyl)oxiran-2-yl]methyl}-1H-1,2,4-triazol, (1.063) N'-(2,5-Dimethyl-4-{[3-(1,1,2,2-tetrafluorethoxy)phenyl]sulfanyl}phenyl)-N-ethyl-N-methylimidoformamid, (1.064) N'-(2,5-Dimethyl-4-{[3-(2,2,2-trifluorethoxy)phenyl]sulfanyl}phenyl)-N-ethyl-N-methylimidoformamid, (1.065) N'-(2,5-Dimethyl-4- {[3-(2,2,3,3-tetrafluorpropoxy)phenyl]sulfanyl}phenyl)-N-ethyl-N-methylimidoformamid, (1.066) N'-(2,5-Dimethyl-4- {[3-(pentafluorethoxy)phenyl]sulfanyl}phenyl)-N-ethyl-N-methylimidoformamid, (1.067) N'-(2,5-Dimethyl-4-{3-[(1,1,2,2-tetrafluorethyl)sulfanyl]phenoxy}phenyl)-N-ethyl-N-methylimidoformamid, (1.068) N'-(2,5-Dimethyl-4- {3-[(2,2,2-trifluorethyl)sulfanyl]phenoxy}phenyl)-N-ethyl-N-methylimidoformamid, (1.069) N'-(2,5-Dimethyl-4- {3-[(2,2,3,3-tetrafluorpropyl)sulfanyl]phenoxy}phenyl)-N-ethyl-N-methylimidofomiamid, (1.070) N'-(2,5-Dimethyl-4- {3-[(pentafluorethyl)sulfanyl]phenoxy}phenyl)-N-ethyl-N-methylimidoformamid, (1.071) N'-(2,5-Dimethyl-4-phenoxyphenyl)-N-ethyl-N-methylimidoformamid, (1.072) N'-(4- {[3-(Difluormethoxy)phenyl]sulfanyl} -2,5-dimethylphenyl)-N-ethyl-N-methylimidoformamid, (1.073) N'-(4-{3-[(Difluormethyl)sulfanyl]phenoxy}-2,5-dimethylphenyl)-N-ethyl-N-methylimidoformamid, (1.074) N'-[5-Brom-6-(2,3-dihydro-1H-inden-2-yloxy)-2-methylpyridin-3-yl]-N-ethyl-N-methylimidoformamid, (1.075) N'-{4-[(4,5-Dichlor-l,3-thiazol-2-yl)oxy]-2,5-dimethylphenyl}-N-ethyl-N-methylimidoformamid, (1.076) N'-{5-Brom-6-[(lR)-l-(3,5-difluorophenyl)ethoxy]-2-methylpyridin-3-yl} -N-ethyl-N-methylimidoformamid, (1.077) N'-{5-Brom-6-[(1S)-1-(3,5-difluorphenyl)ethoxy]-2-methylpyridin-3-yl} -N-ethyl-N-methylimidoformamid, (1.078) N'- {5-Brom-6-[(cis-4-isopropylcyclohexyl)oxy]-2-methylpyridin-3-yl}-N-ethyl-N-methylimidoformamid, (1.079) N'-{5-Brom-6-[(trans-4-isopropylcyclohexyl)oxy]-2-methylpyridin-3-yl}-N-ethyl-N-methylimidoformamid, (1.080) N'- {5-Bromo-6-[1-(3,5-difluorphenyl)ethoxy]-2-methylpyridin-3-yl}-N-ethyl-N-methylimidoformamid.
2) Inhibitoren der Atmungskette am Komplex I oder II beispielsweise (2.001) Benzovindiflupyr, (2.002) Bixafen, (2.003) Boscalid, (2.004) Carboxin, (2.005) Fluopyram, (2.006) Flutolanil, (2.007) Fluxapyroxad, (2.008) Furametpyr, (2.009) Isofetamid, (2.010) Isopyrazam (anti-epimeres Enantiomer 1R,4S,9S), (2.011) Isopyrazam (anti-epimeres Enantiomer 1S,4R,9R), (2.012) Isopyrazam (anti-epimeres Racemat 1RS,4SR,9SR), (2.013) Isopyrazam (Mischung des syn-epimeren Razemates 1RS,4SR,9RS und des anti-epimeren Razemates 1RS,4SR,9SR), (2.014) Isopyrazam (syn-epimeres Enantiomer 1R,4S,9R), (2.015) Isopyrazam (syn-epimeres Enantiomer 1S,4R,9S), (2.016) Isopyrazam (syn-epimeres Racemat 1RS,4SR,9RS), (2.017) Penflufen, (2.018) Penthiopyrad, (2.019) Pydiflumetofen, (2.020) Pyraziflumid, (2.021) Sedaxane, (2.022) 1,3-Dimethyl-N-(1,1,3-trimethyl-2,3-dihydro-1H-inden-4-yl)-1H-pyrazol-4-carboxamid, (2.023) 1,3-Dimethyl-N-[(3R)-1,1,3-trimethyl-2,3-dihydro-1H-inden-4-yl]-1H-pyrazol-4-carboxamid, (2.024) 1,3-Dimethyl-N-[(3S)-1,1,3-trimethyl-2,3-dihydro-1H-inden-4-yl]-1H-pyrazol-4-carboxamid, (2.025) 1-Methyl-3-(trifluormethyl)-N-[2'-(trifluormethyl)biphenyl-2-yl]-1H-pyrazol-4-carboxamid, (2.026) 2-Fluor-6-(trifluoromethyl)-N-(1,1,3-trimethyl-2,3-dihydro-1H-inden-4-yl)benzamid, (2.027) 3-(Difluormethyl)-1-methyl-N-(1,1,3-trimethyl-2,3-dihydro-1H-inden-4-yl)-1H-pyrazol-4-carboxamid, (2.028) 3-(Difluormethyl)-1-methyl-N-[(3R)-1,1,3-trimethyl-2,3-dihydro-1H-inden-4-yl]-1H-pyrazol-4-carboxamid, (2.029) 3-(Difluormethyl)-1-methyl-N-[(3S)-1,1,3-trimethyl-2,3-dihydro-1H-inden-4-yl]-1H-pyrazol-4-carboxamid, (2.030) 3-(Difluormethyl)-N-(7-fluor-1,1,3-trimethyl-2,3-dihydro-1H-inden-4-yl)-1-methyl-1H-pyrazol-4-carboxamid, (2.031) 3-(Difluormethyl)-N-[(3R)-7-fluor-1,1,3-trimethyl-2,3-dihydro-1H-inden-4-yl]-1-methyl-1H-pyrazol-4-carboxamid, (2.032) 3-(Difluoromethyl)-N-[(3S)-7-fluor-1,1,3-trimethyl-2,3-dihydro-1H-inden-4-yl]-1-methyl-1H-pyrazol-4-carboxamid, (2.033) 5,8-Difluor-N-[2-(2-fluor-4- {[4-(trifluormethyl)pyridin-2-yl]oxy}phenyl)ethyl]quinazolin-4-amin, (2.034) N-(2-Cyclopentyl-5-fluorbenzyl)-N-cyclopropyl-3-(difluormethyl)-5-fluor-1-methyl-1H-pyrazol-4-carboxamid, (2.035) N-(2-tert-Butyl-5-methylbenzyl)-N-cyclopropyl-3-(difluormethyl)-5-fluor-1-methyl-1H-pyrazol-4-carboxamid, (2.036) N-(2-tert-Butylbenzyl)-N-cyclopropyl-3-(difluormethyl)-5-fluor-1-methyl-1H-pyrazol-4-carboxamid, (2.037) N-(5-Chlor-2-ethylbenzyl)-N-cyclopropyl-3-(difluormethyl)-5-fluor-1-methyl-1H-pyrazol-4-carboxamid, (2.038) N-(5-Chlor-2-isopropylbenzyl)-N-cyclopropyl-3-(difluormethyl)-5-fluor-1-methyl-1 H-pyrazol-4-carboxamid, (2.039) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.040) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid, (2.041) N-(2,4-Dichlorphenyl)-1-methoxypropan-2-yl]-3-(difluormethyl)-1-methyl-1H-pyrazol-4-carboxamid, (2.042) N-[2-Chlor-6-(trifluormethyl)benzyl]-N-cyclopropyl-3-(difluormethyl)-5-fluor-1-methyl-1H-pyrazol-4-carboxamid, (2.043) N-[3-Chlor-2-fluor-6-(trifluormethyl)benzyl]-N-cyclopropyl-3-(difluormethyl)-5-fluor-1-methyl-1H-pyrazol-4-carboxamid, (2.044) N-[5-Chlor-2-(trifluormethyl)benzyl]-N-cyclopropyl-3-(difluormethyl)-5-fluor-1-methyl-1H-pyrazol-4-carboxamid, (2.045) N-Cyclopropyl-3-(difluormethyl)-5-fluor-1-methyl-N-[5-methyl-2-(trifluormethyl)benzyl]-1H-pyrazol-4-carboxamid, (2.046) N-Cyclopropyl-3-(difluormethyl)-5-fluor-N-(2-fluor-6-isopropylbenzyl)-1-methyl-1H-pyrazol-4-carboxamid, (2.047) N-Cyclopropyl-3-(difluormethyl)-5-fluor-N-(2-isopropyl-5-methylbenzyl)-1-methyl-1H-pyrazol-4-carboxamid, (2.048) N-Cyclopropyl-3-(difluormethyl)-5-fluor-N-(2-isopropylbenzyl)-1-methyl-1H-pyrazol-4-carbothioamid, (2.049) N-Cyclopropyl-3-(difluoromethyl)-5-fluor-N-(2-isopropylbenzyl)-1-methyl-1H-pyrazol-4-carboxamid, (2.050) N-Cyclopropyl-3-(difluormethyl)-5-fluor-N-(5-fluor-2-isopropylbenzyl)-1-methyl-1H-pyrazol-4-carboxamid, (2.051) N-Cyclopropyl-3-(difluonnethyl)-N-(2-ethyl-4,5-dimethylbenzyl)-5-fluor-1-methyl-1H-pyrazol-4-carboxamid, (2.052) N-Cyclopropyl-3-(difluormethyl)-N-(2-ethyl-5-fluorbenzyl)-5-fluor-1-methyl-1H-pyrazol-4-carboxamid, (2.053) N-Cyclopropyl-3-(difluormethyl)-N-(2-ethyl-5-methylbenzyl)-5-fluor-1-methyl-1H-pyrazole-4-carboxamid, (2.054) N-Cyclopropyl-N-(2-cyclopropyl-5-fluorbenzyl)-3-(difluormethyl)-5-fluor-1-methyl-1H-pyrazole-4-carboxamid, (2.055) N-Cyclopropyl-N-(2-cyclopropyl-5-methylbenzyl)-3-(difluormethyl)-5-fluor-1-methyl-1H-pyrazole-4-carboxamid, (2.056) N-Cyclopropyl-N-(2-cyclopropylbenzyl)-3-(difluormethyl)-5-fluor-1-methyl-1H-pyrazole-4-carboxamid.
3) Inhibitoren der Atmungskette am Komplex III, beispielsweise (3.001) Ametoctradin, (3.002) Amisulbrom, (3.003) Azoxystrobin, (3.004) Coumethoxystrobin, (3.005) Coumoxystrobin, (3.006) Cyazofamid, (3.007) Dimoxystrobin, (3.008) Enoxastrobin, (3.009) Famoxadon, (3.010) Fenamidon, (3.011) Flufenoxystrobin, (3.012) Fluoxastrobin, (3.013) Kresoxim-Methyl, (3.014) Metominostrobin, (3.015) Orysastrobin, (3.016) Picoxystrobin, (3.017) Pyraclostrobin, (3.018) Pyrametostrobin, (3.019) Pyraoxystrobin, (3.020) Trifloxystrobin (3.021) (2E)-2-{2-[({[(lE)-1-(3-{[(E)-1-Fluor-2-phenylvinyl]oxy}phenyl)ethyliden]amino}oxy)methyl]phenyl}-2-(methoxyimino)-N-methylacetamid, (3.022) (2E,3Z)-5-{[1-(4-Chlorphenyl)-1H-pyrazol-3-yl]oxy}-2-(methoxyimino)-N,3-dimethylpent-3-enamid, (3.023) (2R)-2-{2-[(2,5-Dimethylphenoxy)methyl]phenyl}-2-methoxy-N-methylacetamid, (3.024) (2S)-2- {2-[(2,5-Dimethylphenoxy)methyl]phenyl}-2-methoxy-N-methylacetamid, (3.025) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl-2-methylpropanoat, (3.026) 2-{2-[(2,5-Dimethylphenoxy)methyl]phenyl} -2-methoxy-N-methylacetamid, (3.027) N-(3-Ethyl-3,5,5-trimethylcyclohexyl)-3-formamido-2-hydroxybenzamid, (3.028) (2E,3Z)-5-{[1-(4-Chlor-2-fluorphenyl)-1H-pyrazol-3-yl]oxy}-2-(methoxyimino)-N,3-dimethylpent-3-enamid.
4) Inhibitoren der Mitose und Zellteilung, beispielsweise (4.001) Carbendazim, (4.002) Diethofencarb, (4.003) Ethaboxam, (4.004) Fluopicolid, (4.005) Pencycuron, (4.006) Thiabendazol, (4.007) Thiophanat-Methyl, (4.008) Zoxamid, , (4.009) 3-Chlor-4-(2,6-difluorphenyl)-6-methyl-5-phenylpyridazin, (4.010) 3-Chlor-5-(4-chlorphenyl)-4-(2,6-difluorphenyl)-6-methylpyridazin, (4.011) 3-Chlor-5-(6-chlorpyridin-3-yl)-6-methyl-4-(2,4,6-trifluorphenyl)pyridazin, (4.012) 4-(2-Brom-4-fluorphenyl)-N-(2,6-difluorphenyl)-1,3-dimethyl-1H-pyrazol-5-amin, (4.013) 4-(2-Brom-4-fluorphenyl)-N-(2-brom-6-fluorphenyl)-1,3-dimethyl-1H-pyrazol-5-amin, (4.014) 4-(2-Brom-4-fluorphenyl)-N-(2-bromphenyl)-1,3-dimethyl-1H-pyrazol-5-amin, (4.015) 4-(2-Brom-4-fluorphenyl)-N-(2-chlor-6-fluorphenyl)-1,3-dimethyl-1H-pyrazol-5-amin, (4.016) 4-(2-Brom-4-fluorphenyl)-N-(2-chlorphenyl)-1,3-dimethyl-1H-pyrazol-5-amin, (4.017) 4-(2-Brom-4-fluorphenyl)-N-(2-fluorphenyl)-1,3-dimethyl-1H-pyrazol-5-amin, (4.018) 4-(2-Chlor-4-fluorphenyl)-N-(2,6-difluorphenyl)-1,3-dimethyl-1H-pyrazol-5-amin, (4.019) 4-(2-Chlor-4-fluorphenyl)-N-(2-chlor-6-fluorphenyl)-1,3-dimethyl-1H-pyrazol-5-amin, (4.020) 4-(2-Chlor-4-fluorphenyl)-N-(2-chlorphenyl)-1,3-dimethyl-1H-pyrazol-5-amin, (4.021) 4-(2-Chlor-4-fluorphenyl)-N-(2-fluorphenyl)-1,3-dimethyl-1H-pyrazol-5-amin, (4.022) 4-(4-Chlorphenyl)-5-(2,6-difluorphenyl)-3,6-dimethylpyridazin, (4.023) N-(2-Brom-6-fluorphenyl)-4-(2-chlor-4-fluorphenyl)-1,3-dimethyl-1H-pyrazol-5-amin, (4.024) N-(2-Bromphenyl)-4-(2-chlor-4-fluorphenyl)-1,3-dimethyl-1H-pyrazol-5-amin, (4.025) N-(4-Chlor-2,6-difluorphenyl)-4-(2-chlor-4-fluorphenyl)-1,3-dimethyl-1H-pyrazol-5-amin.
5) Verbindungen mit Befähigung zu Multisite-Aktivität, beispielsweise (5.001) Bordeauxmischung, (5.002) Captafol, (5.003) Captan, (5.004) Chlorthalonil, (5.005) Kupferhydroxid, (5.006) Kupfernaphthenat, (5.007) Kupferoxid, (5.008) Kupferoxychlorid, (5.009) Kupfer(2+)-sulfat, (5.010) Dithianon, (5.011) Dodin, (5.012) Folpet, (5.013) Mancozeb, (5.014) Maneb, (5.015) Metiram, (5.016) Zinkmetiram, (5.017) Kupfer-Oxin, (5.018) Propineb, (5.019) Schwefel und Schwefelzubereitungen einschließlich Calciumpolysulfid, (5.020) Thiram, (5.021) Zineb, (5.022) Ziram.
6) Verbindungen, die zum Auslösen einer Wirtsabwehr befähigt sind, beispielsweise (6.001) Acibenzolar-S-Methyl, (6.002) Isotianil, (6.003) Probenazol, (6.004) Tiadinil.
7) Inhibitoren der Aminosäure- und/oder Protein-Biosynthese, beispielsweise (7.001) Cyprodinil, (7.002) Kasugamycin, (7.003) Kasugamycinhydrochlorid-hydrat, (7.004) Oxytetracyclin (7.005) Pyrimethanil, (7.006) 3-(5-Fluor-3,3,4,4-tetramethyl-3,4-dihydroisochinolin-1-yl)chinolin.
(8) Inhibitoren der ATP-Produktion, beispielsweise (8.001) Silthiofam.
9) Inhibitoren der Zellwandsynthese, beispielsweise (9.001) Benthiavalicarb, (9.002) Dimethomorph, (9.003) Flumorph, (9.004) Iprovalicarb, (9.005) Mandipropamid, (9.006) Pyrimorph, (9.007) Valifenalat, (9.008) (2E)-3-(4-tert.-Butylphenyl)-3-(2-chlorpyridin-4-yl)-1-(morpholin-4-yl)prop-2-en-1-on, (9.009) (2Z)-3-(4-tert.-Butylphenyl)-3-(2-chlorpyridin-4-yl)-1-(morpholin-4-yl)prop-2-en-1-on.
10) Inhibitoren der Lipid- und Membran-Synthese, beispielsweise (10.001) Propamocarb, (10.002) Propamocarbhydrochlorid, (10.003) Tolclofos-Methyl.
11) Inhibitoren der Melanin-Biosynthese, beispielsweise (11.001) Tricyclazol, (11.002) 2,2,2-Trifluorethyl-{3-methyl-1-[(4-methylbenzoyl)amino]butan-2-yl}carbamat.
12) Inhibitoren der Nukleinsäuresynthese, beispielsweise (12.001) Benalaxyl, (12.002) Benalaxyl-M (Kiralaxyl), (12.003) Metalaxyl, (12.004) Metalaxyl-M (Mefenoxam).
13) Inhibitoren der Signaltransduktion, beispielsweise (13.001) Fludioxonil, (13.002) Iprodion, (13.003) Procymidon, (13.004) Proquinazid, (13.005) Quinoxyfen, (13.006) Vinclozolin.
14) Verbindungen, die als Entkoppler wirken können, beispielsweise (14.001) Fluazinam, (14.002) Meptyldinocap.
15) Weitere Verbindungen, beispielsweise (15.001) Abscisinsäure, (15.002) Benthiazol, (15.003) Bethoxazin, (15.004) Capsimycin, (15.005) Carvon, (15.006) Chinomethionat, (15.007) Cufraneb, (15.008) Cyflufenamid, (15.009) Cymoxanil, (15.010) Cyprosulfamid, (15.011) Flutianil, (15.012) Fosetyl-Aluminium, (15.013) Fosetyl-Calcium, (15.014) Fosetyl-Natrium, (15.015) Methylisothiocyanat, (15.016) Metrafenon, (15.017) Mildiomycin, (15.018) Natamycin, (15.019) Nickel-Dimethyldithiocarbamat, (15.020) Nitrothal-Isopropyl, (15.021) Oxamocarb, (15.022) Oxathiapiprolin, (15.023) Oxyfenthiin, (15.024) Pentachlorphenol und Salze, (15.025) Phosphonsäure und deren Salze, (15.026) Propamocarb-fosetylat, (15.027) Pyriofenone (Chlazafenone) (15.028) Tebufloquin, (15.029) Tecloftalam, (15.030) Tolnifanide, (15.031) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.032) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.033) 2-(6-Benzylpyridin-2-yl)quinazolin, (15.034) 2,6-Dimethyl-1H,5H-[1,4]dithiino[2,3-c:5,6-c']dipyrrol-1,3,5,7(2H,6H)-tetron, (15.035) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.036) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.037) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.038) 2-[6-(3-Fluor-4-methoxyphenyl)-5-methylpyridin-2-yl]quinazolin, (15.039) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.040) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl methanesulfonat, (15.041) 2-{2-[(7,8-Difluor-2-methylquinolin-3-yl)oxy]-6-fluorphenyl}propan-2-ol, (15.042) 2- {2-Fluor-6-[(8-fluor-2-methylquinolin-3-yl)oxy]phenyl}propan-2-ol, (15.043) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenyl-methansulfonat, (15.044) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenyl methanesulfonat, (15.045) 2-Phenylphenol und deren Salze, (15.046) 3-(4,4,5-Trifluor-3,3-dimethyl-3,4-dihydroisoquinolin-1-yl)quinolin, (15.047) 3-(4,4-Difluor-3,3-dimethyl-3,4-dihydroisoquinolin-1-yl)quinolin, (15.048) 4-Amino-5-fluorpyrimidin-2-ol (Tautomere Form: 4-Amino-5-fluorpyrimidin-2(1H)-on), (15.049) 4-Oxo-4-[(2-phenylethyl)amino]buttersäure, (15.050) 5-Amino-1,3,4-thiadiazol-2-thiol, (15.051) 5-Chlor-N'-phenyl-N'-(prop-2-yn-1-yl)thiophen-2-sulfonohydrazid, (15.052) 5-Fluor-2-[(4-fluorbenzyl)oxy]pyrimidin-4-amin, (15.053) 5-Fluor-2-[(4-methylbenzyl)oxy]pyrimidin-4-amin, (15.054) 9-Fluor-2,2-dimethyl-5-(quinolin-3-yl)-2,3-dihydro-1,4-benzoxazepin, (15.055) But-3-yn-1-yl {6-[({[(Z)-(1-methyl-1H-tetrazol-5-yl)(phenyl)methylen]amino}oxy)methyl]pyridin-2-yl}carbamat, (15.056) Ethyl (2Z)-3-amino-2-cyano-3-phenylacrylat, (15.057) Phenazin-1-carbonsäure, (15.058) Propyl 3,4,5-trihydroxybenzoat, (15.059) Quinolin-8-ol, (15.060) Quinolin-8-ol sulfat (2:1), (15.061) tert-Butyl {6-[({[(1-methyl-1H-tetrazol-5-yl)(phenyl)methylene]ammo}oxy)methyl]pyridm-2-yl}carbamat.

### Biologische Schädlingsbekämpfungsmittel als Mischungskomponenten

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens,* Stamm FZB42 (DSM 231179), oder *Bacillus cereus,* insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421), *Bacillus thuringiensis,* insbesondere *B. thuringiensis* Subspezies *israelensis* (Serotyp H-14), Stamm AM65-52 (Accession No. ATCC 1276), oder *B. thuringiensis subsp. aizawai,* insbesondere Stamm ABTS-1857 (SD-1372), oder *B. thuringiensis* *subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii* (ehemals bekannt als *Verticillium lecanii),* insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia), Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense, Fortune Aza, Fungastop, Heads Up (Chenopodium quinoa-Saponinextrakt), Pyrethrum/Pyrethrine, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischungskomponenten

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloracetyl)-1-oxa-4-azaspiro[4.5]decan (CAS 71526-07-3), 2,2,5-Trimethyl-3-(dichloracetyl)-1,3-oxazolidin (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Paprika, Gurke, Melone, Möhre, Wassermelone, Zwiebel, Salat, Spinat, Porree, Bohnen, *Brassica oleracea* (z. B. Kohl) und andere Gemüsesorten, Baumwolle, Tabak, Raps, sowie Obstpflanzen (mit den Früchten Äpfel, Birnen, Zitrusfrüchte und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzen sollen alle Entwicklungsstadien wie Saatgut, Stecklinge, junge (unausgereifte) Pflanzen bis hin zu ausgereiften Pflanzen verstanden werden. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehören auch geerntete Pflanzen oder geerntete Pflanzenteile sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung der Verbindungen auf die Umgebung, den Lebensraum oder den Lagerraum nach den üblichen Behandlungsmethoden, z. B. durch Eintauchen, Spritzen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die durch konventionelle Züchtung, durch Mutagenese oder durch rekombinante DNA-Techniken erhalten worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie Insekten, Spinnentiere, Nematoden, Milben, Schnecken, bewirkt z. B. durch in den Pflanzen entstehende Toxine, insbesondere solche, die durch das genetische Material aus Bacillus Thuringiensis (z. B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z. B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinone, Sulfonylharnstoffe, Glyphosat oder Phosphinotricin (z. B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchte und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z. B. durch Tauchen, Spritzen, Sprühen, Berieseln, Verdampfen, Zerstäuben, Vernebeln, Verstreuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d. h. die Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Wirkstoffen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d. h. der Standort der Pflanze (z. B. Boden oder hydroponische Systeme) wird mit einer flüssigen Form der Verbindungen der Formel (I) getränkt, oder durch die Bodenapplikation, d. h. die erfindungsgemäßen Verbindungen der Formel (I) werden in fester Form (z. B. in Form eines Granulats) in den Standort der Pflanzen eingebracht. Bei Wasserreiskulturen kann das auch durch Zudosieren der Verbindung der Formel (I) in einer festen Anwendungsform (z. B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. -toleranter transgener Pflanzen einbeziehen, um einen optimalen Schutz des Saatguts und auch der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und einer Mischungskomponente behandelt wird. Es umfasst ferner auch ein Verfahren, in dem das Saatgut zu unterschiedlichen Zeiten mit einer Verbindung der Formel (I) und einer Mischungskomponente behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer erfindungsgemäßen Verbindung der Formel (I) behandelt wurde. Die Erfindung bezieht sich auch auf Saatgut, welches zur gleichen Zeit mit einer Verbindung der Formel (I) und einer Mischungskomponente behandelt wurde. Die Erfindung bezieht sich weiterhin auf Saatgut, welches zu unterschiedlichen Zeiten mit einer Verbindung der Formel (I) und einer Mischungskomponente behandelt wurde. Bei Saatgut, welches zu unterschiedlichen Zeiten mit einer Verbindung der Formel (I) und einer Mischungskomponente behandelt wurde, können die einzelnen Substanzen in unterschiedlichen Schichten auf dem Saatgut vorhanden sein. Dabei können die Schichten, die eine Verbindung der Formel (I) und Mischungskomponenten enthalten, gegebenenfalls durch eine Zwischenschicht getrennt sein. Die Erfindung bezieht sich auch auf Saatgut, bei dem eine Verbindung der Formel (I) und eine Mischungskomponente als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating-Verfahren unterzogen wird, um Staubabrieb am Saatgut zu vermeiden.

Einer der auftretenden Vorteile, wenn eine Verbindung der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baumwolle, Soja, Reis, Kartoffeln, Sonnenblume, Kaffee, Tabak, Canola, Raps, Rübe (z. B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps, Gemüse und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hüllen, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z. B. mit Wasser behandelt und dann erneut getrocknet wurde, zum Beispiel Priming. Im Fall von Reis-Saatgut ist es auch möglich, Saatgut zu verwenden, das getränkt wurde, zum Beispiel in Wasser bis zu einem bestimmten Stadium des Reisembryos ("Pigeon Breast Stage"), wodurch die Keimung und ein einheitlicheres Auflaufen stimuliert wird.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man die Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalinsulfonate, wie Diisopropyl- oder Diisobutylnaphthalinsulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid-Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder dem daraus durch Zugabe von Wasser hergestellten Anwendungsformen kommen alle üblicherweise für die Beizung einsetzbaren Mischgeräte in Betracht. Im Einzelnen geht man bei der Beizung so vor, dass man das Saatgut in einen Mischer im diskontinuierlichen oder kontinuierlichen Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann inner-halb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d. h. dem Gebiet der Tiermedizin, sind die Verbindungen der Formel (I) gegen Tierparasiten, insbesondere Ektoparasiten oder Endoparasiten, wirksam. Der Begriff Endoparasit umfasst insbesondere Helminthen und Protozoen wie Kokzidien. Ektoparasiten sind typischerweise und bevorzugt Arthropoden, insbesondere Insekten oder Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; oder Geflügel wie Truthähne, Enten, Gänse und insbesondere Hühner; oder Fische oder Krustentiere, z. B. in der Aquakultur, oder gegebenenfalls Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel; Reptilien, Amphibien oder Aquariumfische.

Gemäß einer bestimmten Ausführungsform werden die Verbindungen der Formel (I) an Säugetiere verabreicht.

Gemäß einer weiteren bestimmten Ausführungsform werden die Verbindungen der Formel (I) an Vögel, nämlich Stubenvögel oder insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen" im vorliegenden Zusammenhang, dass durch die Verbindungen der Formel (I) wirksam das Auftreten des jeweiligen Parasiten in einem Tier, das mit solchen Parasiten in einem harmlosen Ausmaß infiziert ist, reduziert wird. Genauer gesagt bedeutet "bekämpfen" im vorliegenden Zusammenhang, dass die Verbindungen der Formel (I) den jeweiligen Parasiten abtöten, sein Wachstum verhindern oder seine Vermehrung verhindern.

Zu den Arthropoden zählen beispielsweise, ohne hierauf beschränkt zu sein,
aus der Ordnung Anoplurida zum Beispiel Haematopinus spp., Linognathus spp., Pediculus spp., Phtirus spp., Solenopotes spp.;
aus der Ordnung Mallophagida und den Unterordnungen Amblycerina und Ischnocerina, zum Beispiel Bovicola spp., Damalina spp., Felicola spp.; Lepikentron spp., Menopon spp., Trichodectes spp., Trimenopon spp., Trinoton spp., Werneckiella spp;
aus der Ordnung Diptera und den Unterordnungen Nematocerina und Brachycerina, zum Beispiel Aedes spp., Anopheles spp., Atylotus spp., Braula spp., Calliphora spp., Chrysomyia spp., Chrysops spp., Culex spp., Culicoides spp., Eusimulium spp., Fannia spp., Gasterophilus spp., Glossina spp., Haematobia spp., Haematopota spp., Hippobosca spp., Hybomitra spp., Hydrotaea spp., Hypoderma spp., Lipoptena spp., Lucilia spp., Lutzomyia spp., Melophagus spp., Morellia spp., Musca spp., Odagmia spp., Oestrus spp., Philipomyia spp., Phlebotomus spp., Rhinoestrus spp., Sarcophaga spp., Simulium spp., Stomoxys spp., Tabanus spp., Tipula spp., Wilhelmia spp., Wohlfahrtia spp.;
aus der Ordnung Siphonapterida, zum Beispiel Ceratophyllus spp., Ctenocephalides spp., Pulex spp., Tunga spp., Xenopsylla spp.;
aus der Ordnung Heteropterida, zum Beispiel Cimex spp., Panstrongylus spp., Rhodnius spp., Triatoma spp.; sowie Lästlinge und Hygieneschädlinge aus der Ordnung Blattarida.

Weiterhin sind bei den Arthropoden beispielhaft, ohne hierauf beschränkt zu sein, die folgenden Akari zu nennen:
Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Amblyomma spp., Dermacentor spp., Haemaphysalis spp., Hyalomma spp., Ixodes spp., Rhipicephalus (Boophilus) spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Sternostoma spp., Tropilaelaps spp., Varroa spp.; aus der Ordnung Actinedida (Prostigmata), zum Beispiel Acarapis spp., Cheyletiella spp., Demodex spp., Listrophorus spp., Myobia spp., Neotrombicula spp., Ornithocheyletia spp., Psorergates spp., Trombicula spp.; und aus der Ordung der Acaridida (Astigmata), zum Beispiel Acarus spp., Caloglyphus spp., Chorioptes spp., Cytodites spp., Hypodectes spp., Knemidocoptes spp., Laminosioptes spp., Notoedres spp., Otodectes spp., Psoroptes spp., Pterolichus spp., Sarcoptes spp., Trixacarus spp., Tyrophagus spp.

Zu Beispielen für parasitäre Protozoen zählen, ohne hierauf beschränkt zu sein:
Mastigophora (Flagellata), wie:
Metamonada: aus der Ordnung Diplomonadida zum Beispiel Giardia spp., Spironucleus spp.

Parabasala: aus der Ordnung Trichomonadida zum Beispiel Histomonas spp., Pentatrichomonas spp., Tetratrichomonas spp., Trichomonas spp., Tritrichomonas spp.

Euglenozoa: aus der Ordnung Trypanosomatida zum Beispiel Leishmania spp., Trypanosoma spp.

Sarcomastigophora (Rhizopoda), wie Entamoebidae, zum Beispiel Entamoeba spp., Centramoebidae, zum Beispiel Acanthamoeba sp., Euamoebidae, z. B. Hartmanella sp.

Alveolata wie Apicomplexa (Sporozoa): z. B. Cryptosporidium spp.; aus der Ordnung Eimeriida zum Beispiel Besnoitia spp., Cystoisospora spp., Eimeria spp., Hammondia spp., Isospora spp., Neospora spp., Sarcocystis spp., Toxoplasma spp.; aus der Ordnung Adeleida z. B. Hepatozoon spp., Klossiella spp.; aus der Ordnung Haemosporida z. B. Leucocytozoon spp., Plasmodium spp.; aus der Ordnung Piroplasmida z. B. Babesia spp., Ciliophora spp., Echinozoon spp., Theileria spp.; aus der Ordnung Vesibuliferida z. B. Balantidium spp., Buxtonella spp.

Microspora wie Encephalitozoon spp., Enterocytozoon spp., Globidium spp., Nosema spp., und außerdem z. B. Myxozoa spp.

Zu den für Menschen oder Tiere pathogenen Helminthen zählen zum Beispiel Acanthocephala, Nematoden, Pentastoma und Platyhelminthen (z.B. Monogenea, Cestodes und Trematodes).

Zu beispielhaften Helminthen zählen, ohne hierauf beschränkt zu sein:
Monogenea: z. B.: Dactylogyrus spp., Gyrodactylus spp., Microbothrium spp., Polystoma spp., Troglecephalus spp.;
Cestodes: aus der Ordnung Pseudophyllidea zum Beispiel: Bothridium spp., Diphyllobothrium spp., Diplogonoporus spp. Ichthyobothrium spp., Ligula spp., Schistocephalus spp., Spirometra spp.

Aus der Ordnung Cyclophyllida zum Beispiel: Andyra spp., Anoplocephala spp., Avitellina spp., Bertiella spp., Cittotaenia spp., Davainea spp., Diorchis spp., Diplopylidium spp., Dipylidium spp., Echinococcus spp., Echinocotyle spp., Echinolepis spp., Hydatigera spp., Hymenolepis spp., Joyeuxiella spp., Mesocestoides spp., Moniezia spp., Paranoplocephala spp., Raillietina spp., Stilesia spp., Taenia spp., Thysaniezia spp., Thysanosoma spp.

Trematodes: aus der Klasse Digenea zum Beispiel: Austrobilharzia spp., Brachylaima spp., Calicophoron spp., Catatropis spp., Clonorchis spp. Collyriclum spp., Cotylophoron spp., Cyclocoelum spp., Dicrocoelium spp., Diplostomum spp., Echinochasmus spp., Echinoparyphium spp., Echinostoma spp., Eurytrema spp., Fasciola spp., Fasciolides spp., Fasciolopsis spp., Fischoederius spp., Gastrothylacus spp., Gigantobilharzia spp., Gigantocotyle spp., Heterophyes spp., Hypoderaeum spp., Leucochloridium spp., Metagonimus spp., Metorchis spp., Nanophyetus spp., Notocotylus spp., Opisthorchis spp., Ornithobilharzia spp., Paragonimus spp., Paramphistomum spp., Plagiorchis spp., Posthodiplostomum spp., Prosthogonimus spp., Schistosoma spp., Trichobilharzia spp., Troglotrema spp., Typhlocoelum spp.

Nematoden: aus der Ordnung Trichinellida zum Beispiel: Capillaria spp., Trichinella spp., Trichomosoides spp., Trichuris spp.

Aus der Ordnung Tylenchida zum Beispiel: Micronema spp., Parastrangyloides spp., Strongyloides spp.

Aus der Ordnung Rhabditina zum Beispiel: Aelurostrongylus spp., Amidostomum spp., Ancylostoma spp., Angiostrongylus spp., Bronchonema spp., Bunostomum spp., Chabertia spp., Cooperia spp., Cooperioides spp., Crenosoma spp., Cyathostomum spp., Cyclococercus spp., Cyclodontostomum spp., Cylicocyclus spp., Cylicostephanus spp., Cylindropharynx spp., Cystocaulus spp., Dictyocaulus spp., Elaphostrongylus spp., Filaroides spp., Globocephalus spp., Graphidium spp., Gyalocephalus spp., Haemonchus spp., Heligmosomoides spp., Hyostrongylus spp., Marshallagia spp., Metastrongylus spp., Muellerius spp., Necator spp., Nematodirus spp., Neostrongylus spp., Nippostrongylus spp., Obeliscoides spp., Oesophagodontus spp., Oesophagostomum spp., Ollulanus spp.; Ornithostrongylus spp., Oslerus spp., Ostertagia spp., Paracooperia spp., Paracrenosoma spp., Parafilaroides spp., Parelaphostrongylus spp., Pneumocaulus spp., Pneumostrongylus spp., Poteriostomum spp., Protostrongylus spp., Spicocaulus spp., Stephanurus spp., Strongylus spp., Syngamus spp., Teladorsagia spp., Trichonema spp., Trichostrongylus spp., Triodontophorus spp., Troglostrongylus spp., Uncinaria spp.

Aus der Ordnung Spirurida zum Beispiel: Acanthocheilonema spp., Anisakis spp., Ascaridia spp.; Ascaris spp., Ascarops spp., Aspiculuris spp., Baylisascaris spp., Brugia spp., Cercopithifilaria spp., Crassicauda spp., Dipetalonema spp., Dirofilaria spp., Dracunculus spp.; Draschia spp., Enterobius spp., Filaria spp., Gnathostoma spp., Gongylonema spp., Habronema spp., Heterakis spp.; Litomosoides spp., Loa spp., Onchocerca spp., Oxyuris spp., Parabronema spp., Parafilaria spp., Parascaris spp., Passalurus spp., Physaloptera spp., Probstmayria spp., Pseudofilaria spp., Setaria spp., Skjrabinema spp., Spirocerca spp., Stephanofilaria spp., Strongyluris spp., Syphacia spp., Thelazia spp., Toxascaris spp., Toxocara spp., Wuchereria spp.

Acanthocephala: aus der Ordnung Oligacanthorhynchida z.B: Macracanthorhynchus spp., Prosthenorchis spp.; aus der Ordnung Moniliformida zum Beispiel: Moniliformis spp.,
Aus der Ordnung Polymorphida zum Beispiel: Filicollis spp.; aus der Ordnung Echinorhynchida zum Beispiel Acanthocephalus spp., Echinorhynchus spp., Leptorhynchoides spp.

Pentastoma: aus der Ordnung Porocephalida zum Beispiel Linguatula spp.

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch; metaphylaktisch oder therapeutisch erfolgen.

So bezieht sich eine Ausführungsform der vorliegenden Erfindung auf die Verbindungen der Formel (I) zur Verwendung als Arzneimittel.

Ein weiterer Aspekt bezieht sich auf die Verbindungen der Formel (I) zur Verwendung als Antiendoparasitikum.

Ein weiterer spezieller Aspekt der Erfindung betrifft die Verbindungen der Formel (I) zur Verwendung als Antihelminthikum, insbesondere zur Verwendung als Nematizid, Platymelminthizid, Acanthocephalizid oder Pentastomizid.

Ein weiterer spezieller Aspekt der Erfindung betrifft die Verbindungen der Formel (I) zur Verwendung als Antiprotozoikum.

Ein weiterer Aspekt betrifft die Verbindungen der Formel (I) zur Verwendung als Antiektoparasitikum, insbesondere ein Arthropodizid, ganz besonders ein Insektizid oder ein Akarizid.

Weitere Aspekte der Erfindung sind veterinärmedizinische Formulierungen, die eine wirksame Menge mindestens einer Verbindung der Formel (I) und mindestens einen der folgenden umfassen: einen pharmazeutisch unbedenklichen Exzipienten (z.B. feste oder flüssige Verdünnungsmittel), ein pharmazeutisch unbedenkliches Hilfsmittel (z.B. Tenside), insbesondere einen herkömmlicherweise in veterinärmedizinischen Formulierungen verwendeten pharmazeutisch unbedenklichen Exzipienten und/oder ein herkömmlicherweise in veterinärmedizinischen Formulierungen verwendetes pharmazeutisch unbedenkliches Hilfsmittel.

Ein verwandter Aspekt der Erfindung ist ein Verfahren zur Herstellung einer wie hier beschriebenen veterinärmedizinischen Formulierung, welches den Schritt des Mischens mindestens einer Verbindung der Formel (I) mit pharmazeutisch unbedenklichen Exzipienten und/oder Hilfsmitteln, insbesondere mit herkömmlicherweise in veterinärmedizinischen Formulierungen verwendeten pharmazeutisch unbedenklichen Exzipienten und/oder herkömmlicherweise in veterinärmedizinischen Formulierungen verwendeten Hilfsmitteln umfasst.

Ein anderer spezieller Aspekt der Erfindung sind veterinärmedizinische Formulierungen ausgewählt aus der Gruppe ektoparasitizider und endoparasitizider Formulierungen, insbesondere ausgewählt aus der Gruppe anthelmintischer, antiprotozolischer und arthropodizider Formulierungen, ganz besonders ausgewählt aus der Gruppe nematizider, platyhelminthizider, acanthocephalizider, pentastomizider, insektizider und akkarizider Formulierungen, gemäß den erwähnten Aspekten, sowie Verfahren zu ihrer Herstellung.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wirksamen Menge einer Verbindung der Formel (I) bei einem Tier, insbesondere einem nichthumanen Tier, das dessen bedarf.

Ein anderer Aspekt bezieht sich auf ein Verfahren zur Behandlung einer parasitischen Infektion, insbesondere einer Infektion durch einen Parasiten ausgewählt aus der Gruppe der hier erwähnten Ektoparasiten und Endoparasiten, durch Anwendung einer wie hier definierten veterinärmedizinischen Formulierung bei einem Tier, insbesondere einem nichthumanen Tier, das dessen bedarf.

Ein anderer Aspekt bezieht sich auf die Verwendung der Verbindungen der Formel (I) bei der Behandlung einer Parasiteninfektion, insbesondere einer Infektion durch einen Parasiten ausgewählt aus der Gruppe der hier erwähnten Ektoparasiten und Endoparasiten, bei einem Tier, insbesondere einem nichthumanen Tier.

Im vorliegenden tiergesundheitlichen oder veterinärmedizinischen Zusammenhang schließt der Begriff "Behandlung" die prophylaktische, die metaphylaktische und die therapeutische Behandlung ein.

Bei einer bestimmten Ausführungsform werden hiermit Mischungen mindestens einer Verbindung der Formel (I) mit anderen Wirkstoffen, insbesondere mit Endo- und Ektoparasitiziden, für das veterinärmedizinische Gebiet bereitgestellt.

Auf dem Gebiet der Tiergesundheit bedeutet "Mischung" nicht nur, dass zwei (oder mehr) verschiedene Wirkstoffe in einer gemeinsamen Formulierung formuliert werden und entsprechend zusammen angewendet werden, sondern bezieht sich auch auf Produkte, die für jeden Wirkstoff getrennte Formulierungen umfassen. Dementsprechend können, wenn mehr als zwei Wirkstoffe angewendet werden sollen, alle Wirkstoffe in einer gemeinsamen Formulierung formuliert werden oder alle Wirkstoffe in getrennten Formulierungen formuliert werden; ebenfalls denkbar sind gemischte Formen, bei denen einige der Wirkstoffe gemeinsam formuliert und einige der Wirkstoffe getrennt formuliert sind. Getrennte Formulierungen erlauben die getrennte oder aufeinanderfolgende Anwendung der in Rede stehenden Wirkstoffe.

Die hier mit ihrem "Common Name" spezifizierten Wirkstoffe sind bekannt und beispielsweise im "Pesticide Manual" (siehe oben) beschrieben oder im Internet recherchierbar (z.B. http://www.alanwood.net/pesticides).

Beispielhafte Wirkstoffe aus der Gruppe der Ektoparasitizide als Mischungspartner schließen, ohne dass dies eine Einschränkung darstellen soll, die oben ausführlich aufgelisteten Insektizide und Akkarizide ein. Weitere verwendbare Wirkstoffe sind unten gemäß der oben erwähnten Klassifikation, die auf dem aktuellen IRAC Mode of Action Classification Scheme beruht, aufgeführt: (1) Acetylcholinesterase (AChE)-Inhibitoren; (2) GABA-gesteuerte Chlorid-Kanal-Blocker; (3) Natrium-Kanal-Modulatoren; (4) kompetitive Modulatoren des nicotinischen Acetylcholin-Rezeptors (nAChR); (5) allosterische Modulatoren des nicotinischen Acetylcholin-Rezeptors (nAChR); (6) allosterische Modulatoren des Glutamat-abhängigen Chloridkanals (GluCl); (7) Juvenilhormon-Mimetika; (8) verschiedene nichtspezifische (Multi-Site) Inhibitoren; (9) Modulatoren Chordotonaler Organe; (10) Milbenwachstumsinhibitoren; (12) Inhibitoren der mitochondrialen ATP-Synthase, wie ATP-Disruptoren; (13) Entkoppler der oxidativen Phosphorylierung durch Störung des Protonengradienten; (14) Blocker des nicotinischen Acetylcholinrezeptorkanals; (15) Inhibitoren der Chitinbiosynthese, Typ 0; (16) Inhibitoren der Chitinbiosynthese, Typ 1; (17) Häutungsdisruptor (insbesondere bei Dipteren, d.h. Zweiflüglern); (18) Ecdyson-Rezeptor-Agonisten; (19) Octopamin-Rezeptor-Agonisten; (21) mitochondriale Komplex-I-Elektronentransportinhibitoren; (25) mitochondriale Komplex-II-Elektronentransportinhibitoren; (20) mitochondriale Komplex-III-Elektronentransportinhibitoren; (22) Blocker des spannungsabhängigen Natriumkanals; (23) Inhibitoren der Acetyl-CoA-Carboxylase; (28) Ryanodinrezeptor-Modulatoren;
Wirkstoffe mit unbekannten oder nicht spezifischen Wirkmechanismen, z. B. Fentrifanil, Fenoxacrim, Cyclopren, Chlorobenzilat, Chlordimeform, Flubenzimin, Dicyclanil, Amidoflumet, Quinomethionat, Triarathen, Clothiazoben, Tetrasul, Kaliumoleat, Petroleum, Metoxadiazon, Gossyplur, Flutenzin, Brompropylat, Cryolit;
Verbindungen aus anderen Klassen, z.B. Butacarb, Dimetilan, Cloethocarb, Phosphocarb, Pirimiphos(-ethyl), Parathion(-ethyl), Methacrifos, Isopropyl-o-salicylat, Trichlorfon, Sulprofos, Propaphos, Sebufos, Pyridathion, Prothoat, Dichlofenthion, Demeton-S-methylsulfon, Isazofos, Cyanofenphos, Dialifos, Carbophenothion, Autathiofos, Aromfenvinfos(-methyl), Azinphos(-ethyl), Chlorpyrifos(-ethyl), Fosmethilan, Iodofenphos, Dioxabenzofos, Formothion, Fonofos, Flupyrazofos, Fensulfothion, Etrimfos;
Organochlorverbindungen, z. B. Camphechlor, Lindan, Heptachlor; oder Phenylpyrazole, z. B. Acetoprol, Pyrafluprol, Pyriprol, Vaniliprol, Sisapronil; oder Isoxazoline, z. B. Sarolaner, Afoxolaner, Lotilaner, Fluralaner;
Pyrethroide, z. B. (cis-, trans-)Metofluthrin, Profluthrin, Flufenprox, Flubrocythrinat, Fubfenprox, Fenfluthrin, Protrifenbut, Pyresmethrin, RU15525, Terallethrin, cis-Resmethrin, Heptafluthrin, Bioethanomethrin, Biopermethrin, Fenpyrithrin, cis-Cypermethrin, cis-Permethrin, Clocythrin, Cyhalothrin (lambda-), Chlovaporthrin, oder halogenierte Kohlenwasserstoffverbindungen (HCHs),
Neonicotinoide, z. B. Nithiazin
Dicloromezotiaz, Triflumezopyrim
makrocyclische Lactone, z. B. Nemadectin, Ivermectin, Latidectin, Moxidectin, Selamectin, Eprinomectin, Doramectin, Emamectinbenzoat; Milbemycinoxim
Tripren, Epofenonan, Diofenolan;
Biologicals, Hormone oder Pheromone, zum Beispiel natürliche Produkte, z.B. Thuringiensin, Codlemon oder Neem-Komponenten
Dinitrophenole, z. B. Dinocap, Dinobuton, Binapacryl;
Benzoylharnstoffe, z. B. Fluazuron, Penfluron,
Amidinderivate, z. B. Chlormebuform, Cymiazol, Demiditraz
Bienenstockvarroa-Akarizide, zum Beispiel organische Säuren, z.B. Ameisensäure, Oxalsäure.

Zu beispielhaften Wirkstoffen aus der Gruppe der Endoparasitizide, als Mischungspartner, zählen, ohne hierauf beschränkt zu sein, anthelmintische Wirkstoffe und antiprotozoische Wirkstoffe.

Zu den anthelmintischen Wirkstoffen zählen, ohne hierauf beschränkt zu sein, die folgenden nematiziden, trematiziden und/oder cestoziden Wirkstoffe:
aus der Klasse der makrocyclischen Lactone zum Beispiel: Eprinomectin, Abamectin, Nemadectin, Moxidectin, Doramectin, Selamectin, Lepimectin, Latidectin, Milbemectin, Ivermectin, Emamectin, Milbemycin;
aus der Klasse der Benzimidazole und Probenzimidazole zum Beispiel: Oxibendazol, Mebendazol, Triclabendazol, Thiophanat, Parbendazol, Oxfendazol, Netobimin, Fenbendazol, Febantel, Thiabendazol, Cyclobendazol, Cambendazol, Albendazol-sulfoxid, Albendazol, Flubendazol;
aus der Klasse der Depsipeptide, vorzugsweise cyclischen Depsipetide, insbesondere 24-gliedrigen cyclischen Depsipeptide, zum Beispiel: Emodepsid, PF1022A;
aus der Klasse der Tetrahydropyrimidine zum Beispiel: Morantel, Pyrantel, Oxantel;
aus der Klasse der Imidazothiazole zum Beispiel: Butamisol, Levamisol, Tetramisol;
aus der Klasse der Aminophenylamidine zum Beispiel: Amidantel, deacyliertes Amidantel (dAMD), Tribendimidin;
aus der Klasse der Aminoacetonitrile zum Beispiel: Monepantel;
aus der Klasse der Paraherquamide zum Beispiel: Paraherquamid, Derquantel;
aus der Klasse der Salicylanilide zum Beispiel: Tribromsalan, Bromoxanid, Brotianid, Clioxanid, Closantel, Niclosamid, Oxyclozanid, Rafoxanid;
aus der Klasse der substituierten Phenole zum Beispiel: Nitroxynil, Bithionol, Disophenol, Hexachlorophen, Niclofolan, Meniclopholan;
aus der Klasse der Organophosphate zum Beispiel: Trichlorfon, Naphthalofos, Dichlorvos/DDVP, Crufomat, Coumaphos, Haloxon;
aus der Klasse der Piperazinone/Chinoline zum Beispiel: Praziquantel, Epsiprantel;
aus der Klasse der Piperazine zum Beispiel: Piperazin, Hydroxyzin;
aus der Klasse der Tetracycline zum Beispiel: Tetracyclin, Chlorotetracyclin, Doxycyclin, Oxytetracyclin, Rolitetracyclin;
aus diversen anderen Klassen zum Beispiel: Bunamidin, Niridazol, Resorantel, Omphalotin, Oltipraz, Nitroscanat, Nitroxynil, Oxamniquin, Mirasan, Miracil, Lucanthon, Hycanthon, Hetolin, Emetin, Diethylcarbamazin, Dichlorophen, Diamfenetid, Clonazepam, Bephenium, Amoscanat, Clorsulon.

Antiprotozoische Wirkstoffe, darunter, ohne hierauf beschränkt zu sein, die folgenden Wirkstoffe:
aus der Klasse der Triazine zum Beispiel: Diclazuril, Ponazuril, Letrazuril, Toltrazuril;
aus der Klasse Polyletherionophor zum Beispiel: Monensin, Salinomycin, Maduramicin, Narasin;
aus der Klasse der makrocyclischen Lactone zum Beispiel: Milbemycin, Erythromycin;
aus der Klasse der Chinolone zum Beispiel: Enrofloxacin, Pradofloxacin;
aus der Klasse der Chinine zum Beispiel: Chloroquin;
aus der Klasse der Pyrimidine zum Beispiel: Pyrimethamin;
aus der Klasse der Sulfonamide zum Beispiel: Sulfachinoxalin, Trimethoprim, Sulfaclozin;
aus der Klasse der Thiamine zum Beispiel: Amprolium;
aus der Klasse der Lincosamide zum Beispiel: Clindamycin;
aus der Klasse der Carbanilide zum Beispiel: Imidocarb;
aus der Klasse der Nitrofurane zum Beispiel: Nifurtimox;
aus der Klasse der Chinazolinonalkaloide zum Beispiel: Halofuginon;
aus diversen anderen Klassen zum Beispiel: Oxamniquin, Paromomycin;
aus der Klasse der Vakzine oder Antigene aus Mikroorganismen zum Beispiel: Babesia canis rossi, Eimeria tenella, Eimeria praecox, Eimeria necatrix, Eimeria mitis, Eimeria maxima, Eimeria brunetti, Eimeria acervulina, Babesia canis vogeli, Leishmania infantum, Babesia canis canis, Dictyocaulus viviparus.

Alle genannten Mischungspartner können außerdem, wenn sie auf Grund ihrer funktionellen Gruppen dazu imstande sind, gegebenenfalls mit geeigneten Basen oder Säuren Salze bilden.

### Vektorbekämpfung

Die Verbindungen der Formel (I) können auch in der Vektorbekämpfung eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z. B. Trachoma durch nicht-stechende Fliegen) auf einem Wirt, oder nach Injektion (z. B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von anderen Würmern;
   - Aedes: Gelbfieber, Dengue-Fieber, weitere virale Erkrankungen, Filariasis;
   - Simulien: Übertragung von Würmern, insbesondere Onchocerca volvulus;
   - Psychodidae: Übertragung von Leishmaniose
2) Läuse: Hautinfektionen, epidemisches Fleckfieber;
3) Flöhe: Pest, endemisches Fleckfieber, Bandwürmer;
4) Fliegen: Schlafkrankheit (Trypanosomiasis); Cholera, weitere bakterielle Erkrankungen;
5) Milben: Acariose, epidemisches Fleckfieber, Rickettsipocken, Tularämie, Saint-Louis-Enzephalitis, Frühsommer-Meningoenzephalitis (FSME), Krim-Kongo-Fieber, Borreliose;
6) Zecken: Borelliosen wie Borrelia bungdorferi sensu lato., Borrelia duttoni, Frühsommer-Meningoenzephalitis, Q-Fieber (Coxiella burnetii), Babesien (Babesia canis canis), Ehrlichiose.

Beispiele für Vektoren im Sinne der vorliegenden Erfindung sind Insekten, zum Beispiel Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z. B. A. gambiae, A. arabiensis, A. funestus, A. dirus (Malaria) und Culex, Psychodide wie Phlebotomus, Lutzomyia, Läuse, Flöhe, Fliegen, Milben und Zecken, die Krankheitserreger auf Tiere und/oder Menschen übertragen können.

Eine Vektorbekämpfung ist auch möglich, wenn die Verbindungen der Formel (I) Resistenz-brechend sind.

Verbindungen der Formel (I) sind zur Verwendung in der Prävention von Krankheiten und/oder Krankheitserregern, die durch Vektoren übertragen werden, geeignet. Somit ist ein weiterer Aspekt der vorliegenden Erfindung die Verwendung von Verbindungen der Formel (I) zur Vektorbekämpfung, z. B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z. B. aus den Ordnungen Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausführungsform werden die Verbindungen der Formel (I) zusammen mit mindestens einem weiteren Insektizid und/oder mindestens einem Fungizid eingesetzt.

In einer weiteren Ausführungsform liegen die Verbindungen der Formel (I) als ein anwendungsfertiges (ready-to-use) Schädlingsbekämpfungsmittel vor, d. h., sie können ohne weitere Änderungen auf das entsprechende Material aufgebracht werden. Als weitere Insektizide oder Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren, Zecken und Milben, die in geschlossenen Räumen, wie beispielsweise Wohnungen, Fabrikhallen, Büros, Fahrzeugkabinen, Tierzuchtanlagen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z. B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die Verbindungen der Formel (I-A), in denen Q für Sauerstoff und T für ein freies Elektronenpaar steht, können beispielsweise nach den in Schema 1 bis 10 angegebenen Verfahren synthetisiert werden. Die Verbindungen der Formel (I-B), in denen Q für Schwefel steht, können aus Verbindungen der Formel (I-A) beispielsweise nach Schema 11 erhalten werden. Bei den in Schema 1 bis 11 angegebenen Verfahren haben die in den Formeln verwendeten Reste W, Y, V, A, U¹, L¹, L², R¹, R², R²¹, R³, R³³, R⁴ und R⁴³ soweit nicht anders angegeben jeweils die in den Vorzugsbereichen (1) bis (5) angegebene Bedeutung.

Carbonsäureester der Formel (I-A¹) können wie in Schema 1 gezeigt, synthetisiert werden indem Verbindungen der Formel (IV) zunächst mit einer starken Base, wie z.B. n-Butyllithium, Lithium-diisoproylamin oder Lithium-tert.-butylat bei tiefer Temperaturen (-75 bis -100 °C) in Lösungsmitteln wie z.B. Tetrahydrofuran oder Diethylether deprotoniert und anschließend mit einem Chloroformiat der Formel (V) zu Estern der Formel (I-A¹) umgesetzt werden.

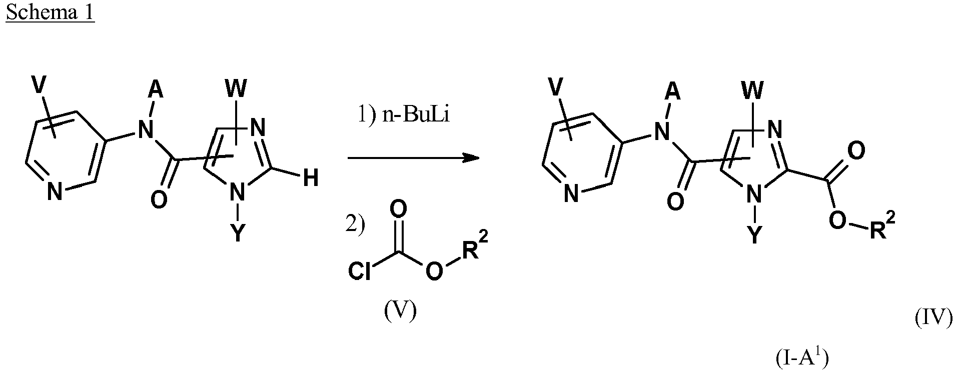

Für das Lithiierungsverfahren sind zahlreiche Reaktionsbedingungen beschrieben worden, z. B., Hoechst Aktiengesellschaft, Patent: US4764624 A1, 1988. Ohta; Yamamoto; Kawasaki; Yamashita; Katsuma; Nasako; Kobayashi; Ogawa, Chemical and Pharmaceutical Bulletin, 1992 , vol. 40, # 10 p. 2681 - 2685. Hara, Kenji; Iwahashi, Keiji; Kanamori, Yoshikazu; Naito, Satoshi; Takakusagi, Satoru; Uosaki, Kohei; Sawamura, Masaya Chemistry Letters, 2006 , vol. 35, # 8 p. 870 - 871.

Chloroformiate der Formel (V) sind kommerziell erhältlich oder können nach literaturbekannten Verfahren synthetisiert werden.

Verbindungen der Formel (I-A², R³=H) können in Analogie zum für Schema 1 beschriebenen Verfahren wie in Schema 2 gezeigt synthetisiert werden, indem zunächst Verbindungen der Formel (IV) mit einer starken Base, wie z.B. n-Butyllithium deprotoniert und anschließend mit einem Isocyanat der der Formel (VI) zu Amiden der Formel (I-A²) umgesetzt werden.

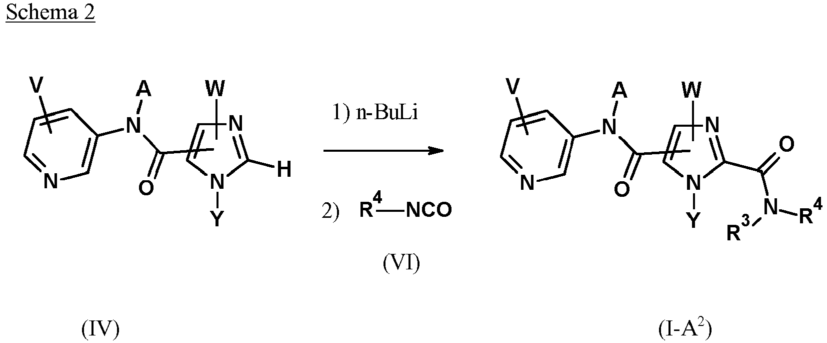

Isocyanate der Formel (VI) sind kommerziell erhältlich oder können nach literaturbekannten Verfahren synthetisiert werden.

Verbindungen der Formel (I-A²) können in Analogie zum für Schema 1 beschriebenen Verfahren auch wie in Schema 3 gezeigt synthetisiert werden, indem Verbindungen der Formel (IV) mit einer starken Base, wie z.B. n-Butyllithium deprotoniert und anschließend mit einer Verbindung der Formel (VII) zu Amiden der Formel (I-A²) umgesetzt werden.

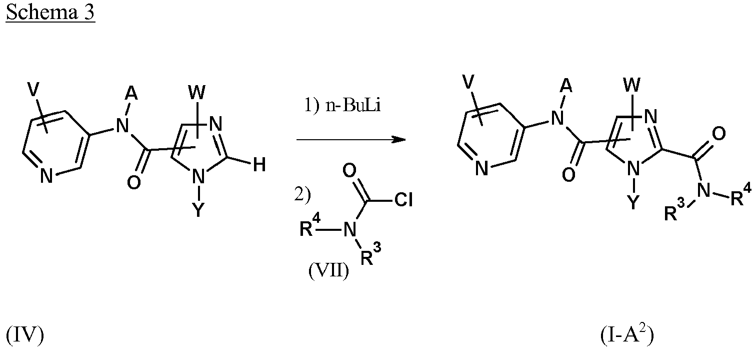

Verbindungen der Formel (VII) sind kommerziell erhältlich oder können nach literaturbekannten Verfahren synthetisiert werden.

Verbindungen der Formel (I-A³, R³=H) können in Analogie zum für Schema 1 beschriebenen Verfahren wie in Schema 4 gezeigt synthetisiert werden, indem Verbindungen der Formel (IV) mit einer starken Base, wie z.B. n-Butyllithium deprotoniert und anschließend mit einem Isothiocyanat der Formel (VIII) zu Thioamiden der Formel (I-A³) umgesetzt werden.

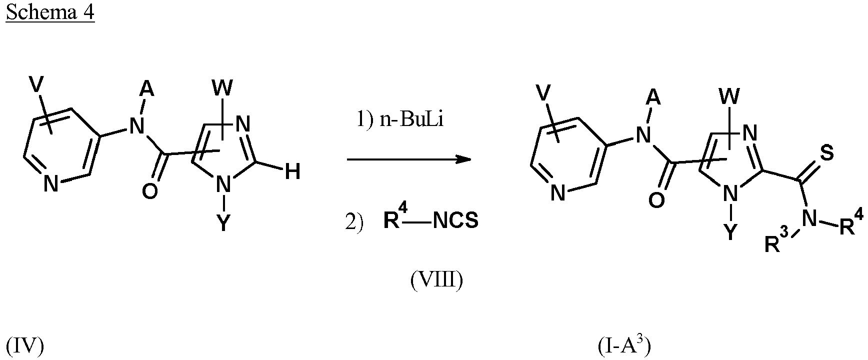

Isothiocyanate der Formel (VIII) sind kommerziell erhältlich oder können nach literaturbekannten Verfahren synthetisiert werden.

Carbonylverbindungen der Formel (I-A⁴) können in Analogie zum für Schema 1 beschriebenen Verfahren wie in Schema 6 gezeigt synthetisiert werden, indem Verbindungen der Formel (IV) zunächst mit n-Butyllithium deprotoniert werden und anschließend mit einer geeigneten Carbonylverbindung der Formel (X),
wobei G für eine Abgangsgruppe wie z.B. Cl, -OCH₃ oder -N(CH₃)₂ steht,
umgesetzt werden.

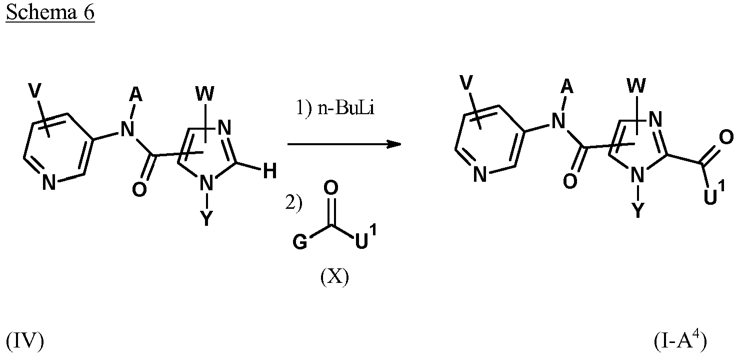

Verbindungen der Formel (X) sind kommerziell erhältlich oder können nach literaturbekannten Verfahren synthetisiert werden.

Oximether der Formel (I-A⁵) können wie in Schema 7 gezeigt, synthetisiert werden, indem Verbindungen der Formel (I-A⁴) mit Hydroxylaminen der Formel (XI) in Analogie zu allgemein bekannten Verfahren umgesetzt werden.

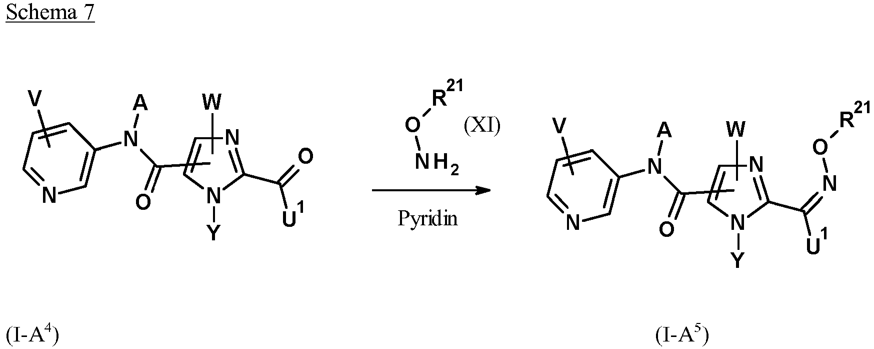

Hydroxylamine der Formel (XI) sind kommerziell erhältlich oder können nach literaturbekannten Verfahren synthetisiert werden.

Hydrazonderivate der Formel (I-A⁵) können wie in Schema 8 gezeigt, synthetisiert werden, indem Verbindungen der Formel (I-A⁵) mit Hydrazinderivaten der Formel (XII) in Analogie zu allgemein bekannten Verfahren umgesetzt werden.

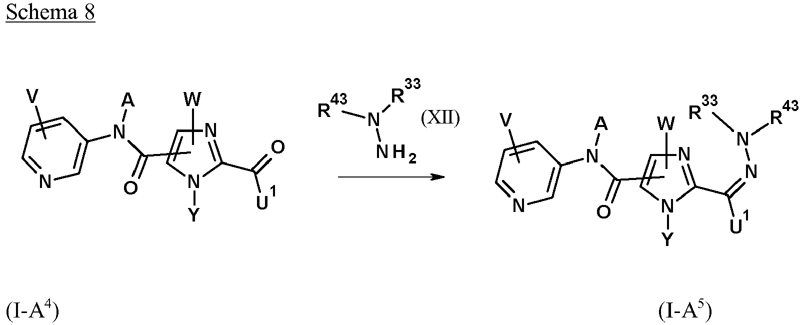

Hydrazinderivate der Formel (XII) sind kommerziell erhältlich oder können nach literaturbekannten Verfahren synthetisiert werden [vgl. z.B. WO2007/43677, WO2007/139346]
Verbindungen der Formel (I-A⁶) können wie in Schema 9 gezeigt, synthetisiert werden, indem Verbindungen der Formel (I-A⁴) mit Verbindungen der Formel (XIV), wobei L₁ und L₂ jeweils unabhängig voneinander für O oder S stehen, in Analogie zu allgemein bekannten Verfahren in Gegenwart einer Säure, wie z.B. Toluolsulfonsäure umgesetzt werden.

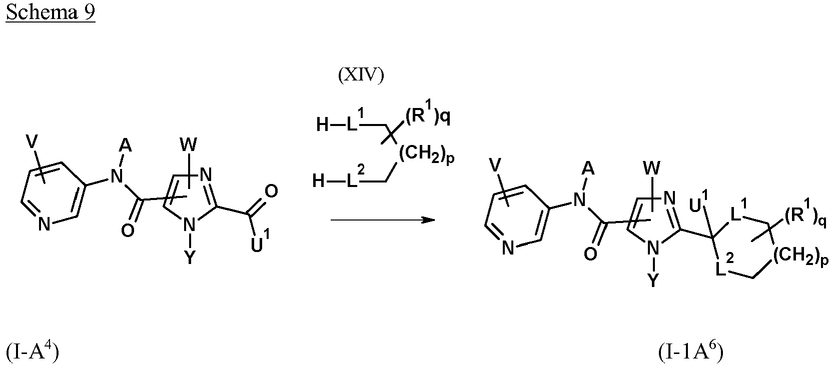

Verbindungen der Formel (XIV) sind kommerziell erhältlich oder können nach literaturbekannten Verfahren synthetisiert werden.

Thioamide der Formel (I-2) können wie in Schema 10 gezeigt, synthetisiert werden, indem Verbindungen der Formel (I-1) mit einem Schwefelungsreagenz, wie z.B. Diphosphorpentasulfid oder Lawessons Reagenz (vgl. z.B. C. P. Dell in Comprehensive Organic Functional Group Transformations, Vol. 5, Hrsg.: A. R. Katrizky, O. Meth-Cohn, C. W. Rees, Pergamon, Oxford, 1995, S. 565-628; M. Jesberger, T. P. Davis, L. Barner, Synthesis 2003, 13, 1929) in Analogie zu allgemein bekannten Verfahren umgesetzt werden.

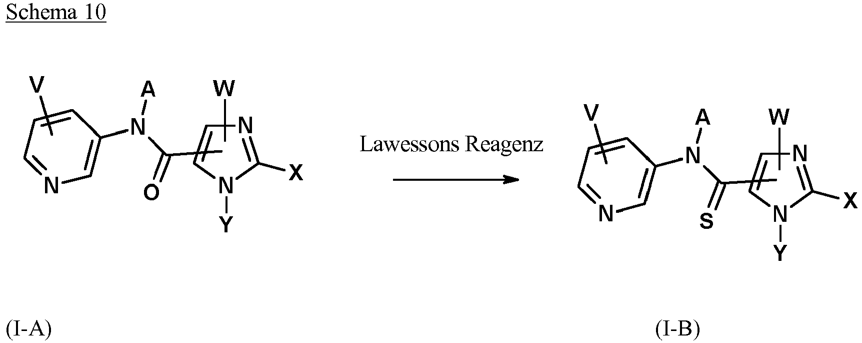

Die Imidazolintermediate der Formel (IV) können z.B. wie in Schema 11 gezeigt, synthetisiert werden, indem Imidazolcarbonsäuren der Formel (II) nach allgemein bekannten Amidierungsverfahren mit Aminopyridinen der Formel (III) umgesetzt werden.

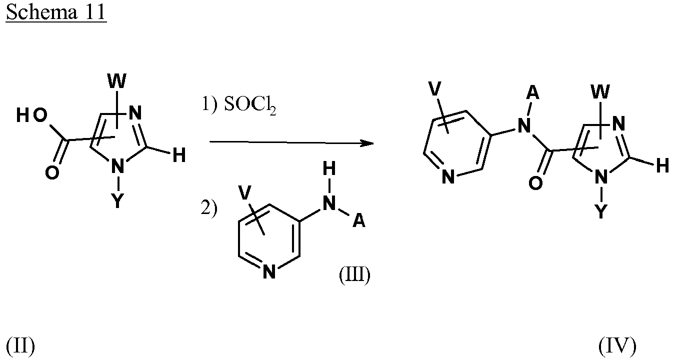

Für den Amidierungsschritt sind zahlreiche Reaktionsbedingungen beschrieben worden, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Einige dieser Reaktionen verlaufen über intermediäre Carbonsäurechloride, die isoliert oder in situ erzeugt eingesetzt werden können.

Die Amidierungsreaktionen erfolgen gegebenenfalls in Gegenwart eines Kondensationsmittels, gegebenenfalls in Gegenwart eines Säureakzeptors und gegebenenfalls in Gegenwart eines Lösungsmittels.

Als Kondensationsmittel kommen alle üblicherweise für derartige Amidierungsreaktionen verwendbaren Kondensationsmittel infrage. Beispielhaft genannt seien Aktivierungsreagenzien wie Phosgen, Phosphortrichlorid, Phosphoroxychlorid, Oxalylchlorid, Oxalylbromid oder Thionylchlorid; Carbodiimide, wie *N*,*N*'-Dicyclohexylcarbodiimid (DCC) und 1-(3-Dimethylaminopropyl)-3-ethyl-carbodiimid (EDCI), oder andere übliche Kondensationsmittel, wie Phosphorpentoxid, Polyphosphorsäure, *N*,*N*'-Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Brom-tripyrrolidino-phosphonium-hexafluorphosphat (BROP), O-(*1H*-Benzotriazol-1-yloxy)tris(dimethylamino)phosphonium-hexafluorphosphat (BOP), *N,N,N',N'-*Bis(tetramethylen)cloruronium-tetrafluorborat, O*-*(*1H-*Benzotriazol-1-yl)*-N,N,N',N'-*tetramethyluronium-hexafluorphosphat (HBTU), O*-*(*1H-Benzotriazol-1-yl)-N,N,N',N'-*bis(tetramethylen)uronium-hexafluorphosphat, O*-*(*1H-*Benzotriazol-1-yl)*-N,N,N,N'-*tetramethyluronium-tetrafluorborat (TBTU), O*-*(*1H-*Benzotriazol-1-yl)-*N*,*N*,*N'*,*N*'-bis(tetramethylen)uronium-tetrafluorborat, 0-(7-Azabenzotriazol-1-yl)-*N,N,N',N'*-tetramethyluronium-hexafluorphosphat (HATU), 1-Hydroxybenzotriazol (HOBt) und 4-(4,6-Dimethoxy-1,3,5-triazin-2-yl)-4-methylmorpholiniumsalz (DMT.MM), meistens als Chlorid verfügbar. Diese Reagenzien können separat oder in Kombination eingesetzt werden.

Als Säureakzeptor kommen alle üblichen anorganischen oder organischen Basen infrage, beispielsweise organische Amine wie Triethylamin, Diisopropylethylamin, *N*-Methylmorpholin, Pyridin oder *N,N-*Dimethylaminopyridin, Alkali- und Erdalkalicarbonate wie Lithiumcarbonat Natriumcarbonat, Kaliumcarbonat oder Caesiumcarbonat; Alkalyhydrogencarbonate wie Natrumhydrogencarbonat oder Kaliumhydrogencarbonat. Die Amidierungsreaktion in den erfindungsgemäßen Verfahren wird gegebenenfalls in Gegenwart eines geeigneten Reaktionshilfstoffes wie beispielsweise *N,N-*Dimethylformamid oder *N,N*-Dimethylaminopyridin durchgeführt. Als Lösungs- oder Verdünnungsmittel kommen alle interten organischen Lösungsmittel in Betracht, beispielsweise aliphatische oder aromatische Kohlenwasserstoffe (wie Petrolether, Toluol, Cyclohexan), halogenierte Kohlenwasserstoffe (wie Chlortoluol, Dichlorbenzol, Dichlormethan, Chloroform, 1,2-Dichlorethan), Ether (wie Diethylether, Dioxan, Tetrahydrofuran, 1,2-Dimethoxyethan), Ester (wie Essigsäureethylester- oder -methylester), Nitrokohlenwasserstoffe (wie Nitromethan, Nitroethan, Nitrobenzol), Nitrile (wie Acetonitril, Propionitril, Butyronitril. Benzonitril), Amide (wie *N,N-*Dimethylformamid, *N,N* Dimethylacetamide, *N*-Methylformanilid, *N*-Methylpyrrolidon, Hexamethylphosphorsäuretriamid) sowie Dimethylsulfoxid, Sulfolan oder Wasser oder Gemische der genannten Lösungsmittel.

Es können auch gemischte Anhydride zur Darstellung von Verbindungen der Formel (III) verwendet werden (vgl. J. Am. Chem. Soc. 1967, 5012). Bei diesem Verfahren können Chlorameisensäureester zum Einsatz kommen, wie z.B. Chlorameisensäuremethylester, Chlorameisensäureethylester, Chlorameisensäureisobutylester und Chlorameisensäureisopropylester. Ebenfalls können dafür Diethylacetylchlorid, Trimethylacetylchlorid und ähnliche Verbindungen verwendet werden. Imidazolyl-carbonsäuren der Formel (II) sind kommerziell erhältlich oder können beispielweise nach literaturbekannten Verfahren hergestellt werden, z. B., H. Rapoport et.al.; Synthesis 1988, 10, 767-771, BASF Aktiengesellschaft Patent: US4864030 A1, 1989, Takeda Pharmaceutical Company Limited Patent: EP2530078 A1, 2012, TAISHO PHARMACEUTICAL CO., LTD. Patent: US2012/10414 A1, 2012, Subrayan, Ramachandran P.; Thurber, Ernest L.; Rasmussen, Paul G. Tetrahedron, 1994 , 50, 2641 -2656.
3-Amino-pyridine der Formel (III) sind kommerziell erhältlich oder können beispielweise nach literaturbekannten Verfahren hergestellt werden, z. B., Liu, Zhen-Jiang; Vors, Jean-Pierre; Gesing, Ernst R. F.; Bolm, Carsten, Advanced Synthesis and Catalysis, 2010 , 352, 3158 - 3162, BAYER CROPSCIENCE AG Patent: US2010/305124 A1, 2010, Shafir, Alexandr; Buchwald, Stephen L., Journal of the American Chemical Society, 2006 , 128, 8742 - 8743.

### Herstellungsbeispiele

Die folgenden Herstellungs- und Verwendungsbeispiele illustrieren die Erfindung,

### Synthesebeispiel Nr. 1

### Methyl-1-methyl-5-[methyl(pyridin-3-yl)carbamoyl]-1H-imidazol-2-carboxylat (Verbindung Nr. I-1-026)

### Stufe 1: N,1 -Dimethyl-N-(pyridin-3 -yl)-1H-imidazol-5 -carboxamid

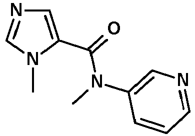

Eine Suspension von 12g (95.2 mmol) 1-Methyl-imidazol-5-carbonsäure in 72 ml Toluol wurde mit 12.71g (104.7 mmol) Thionylchlorid versetzt und über Nacht bei 130°C gerührt. Das Reaktionsgemisch wurde im Vakuum eingeengt. Der Rückstand wurde mit einer Lösung von 10.3g (95.2 mmol) 3-Methylamino-pyridin in 72ml Pyridin versetzt und die resultierende Reaktionsmischung 4h auf 115°C erwärmt. Anschließend wurde erneut im Vakuum eingeengt und der Rückstand an Kieselgel säulenchromatographisch mit Acetonitril/ Methanol 3:1 als Laufmittel gereinigt. Es wurden 8.1g (39.3 % d. Theorie) der Titelverbindung erhalten sowie 9.5g (37.1% d. Theorie) des HCl-Salzes der Titelverbindung.
HPLC-MS: logP[n] = 0,44 ; Masse (m/z): 217,1; ¹H-NMR (CD₃CN, 400MHz); δ = 3.39 (s, 3H), 3.81 (s, 3H), 6.17 (s, 1H), 7.36-7.40 (m, 2H), 7.67-7.70 (m, 1H), 8.41 (m, 1 H) 8.47 (m, 1H) ppm.

### Stufe 2: Methyl-1-methyl-5-[methyl(pyridin-3-yl)carbamoyl]-1H-imidazol-2-carboxylat (Verbindung Nr. 1-1-026)

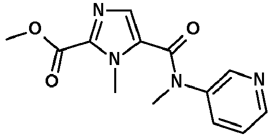

0.300 g (1.39 mmol) N,1-Dimethyl-N-(pyridin-3-yi)-1H-imidazol-5-carboxamid wurden in 7 mL THF gelöst und auf -90 °C abgekühlt. Die Temperatur wurde während der Reaktion zwischen -85 °C und -90 °C gehalten. 0.58 mL (1.45 mmol) 2.5 molare *n*-BuLi-Lösung in *n*-Hexan wurden innerhalb von 5 Minuten bei -90 °C zugetropft und 5 Minuten nachgerührt. Anschließend wurden innerhalb von 5 Minuten 0.393 g Methylcarbonochloridat (4.16 mmol), in 3 mL THF gelöst, zugetropft. Es wurden weitere 30 Minuten bei -80 °C nachgerührt und dann innerhalb von 30 Minuten auf Raumtemperatur erwärmt. Der Ansatz wurde mit halbkonzentrierter Natriumhydrogencarbonatlösung/Dichlormethan extrahiert. Die wässrige Phase wurde nochmals dreimal mit Dichlormethan extrahiert. Die organischen Phasen wurden vereinigt, mit wenig Wasser gewaschen, mit Natriumsulfat getrocknet, filtriert und eingeengt. Der Rückstand wurde an Kieselgel durch MPLC mit Essigsäureethylester/Methanol als Laufmittel gereinigt. Nach Aufreinigung wurden 59.0 mg (15.3% d. Theorie) erhalten.
logP[a]: 0.53; logP[n]: 0.84;
¹H-NMR (CD₃CN, 400MHz); δ = 3.42 (s, 3H), 3.83 (s, 3H), 4.05 (s, 3H), 6.46 (s, 1H), 7.32-7.36 (m, 1H), 7.64-7.67 (m, 1H), 8.387-8.393 (m, 1H), 8.43-8.45 (m, 1H) ppm.

### Synthesebeispiel Nr. 2

### N²-Ethyl-N⁵,1-dimethyl-N⁵-(pyridin-3-yl)-1H-imidazol-2,5-dicarboxamid (Verbindung Nr. 1-1-046)

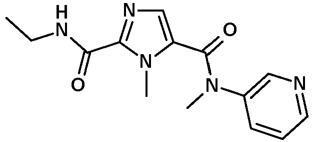

0.300 g (1.39 mmol) N,1-Dimethyl-N-(pyridin-3-yi)-1H-imidazol-5-carboxamid (Synthesebeispiel 1, Stufe 1) wurden in 11 mL THF gelöst und auf -90 °C abgekühlt. Die Temperatur wurde während der Reaktion zwischen -85 °C und -90 °C gehalten. 0.58 mL (1.45 mmol) 2.5 molare *n*-BuLi-Lösung in n-Hexan wurden innerhalb von 5 Minuten bei -90 °C zugetropft und 5 Minuten nachgerührt. Anschließend wurden innerhalb von 5 Minuten 0.325 mL Isocyanatoethan (4.16 mmol), in 1 mL THF gelöst, zugetropft. Es wurden weitere 30 Minuten bei -80 °C nachgerührt und dann innerhalb von 30 Minuten auf Raumtemperatur erwärmt. Der Ansatz wurde mit halbkonzentrierter Natriumhydrogencarbonatlösung/Dichlormethan extrahiert. Die wässrige Phase wurde nochmals dreimal mit Dichlormethan extrahiert. Die organischen Phasen wurden vereinigt, mit wenig Wasser gewaschen, mit Natriumsulfat getrocknet, filtriert und eingeengt. Der Rückstand wurde an Kieselgel durch MPLC mit Essigsäureethylester/Methanol als Laufmittel gereinigt. Nach Aufreinigung wurden 45.0 mg (11.3% d. Theorie) erhalten.
logP[a]: 0.80; logP[n]: 0.97;
¹H-NMR (d₆-DMSO, 400MHz); δ = 1.03-1.07 (t, 3H), 3.16-3.23 (m, 2H), 3.40 (s, 3H), 4.07 (s, 3H), 6.37 (s, 1H), 7.41-7.44 (m, 1H), 7.82-7.85 (m, 1H), 8.45-8.46 (m, 1H), 8.48-8.50 (m, 2H) ppm.

### Synthesebeispiel Nr. 3

### N,1-Dimethyl-N-(pyridin-3-yl)-2-(pyrrolidin-1-ylcarbonyl)-1H-imidazol-5-carboxamid (Verbindung Nr. I-1-055)

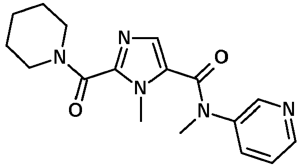

0.300 g (1.39 mmol) N,1-Dimethyl-N-(pyridin-3-yi)-1H-imidazol-5-carboxamid (Synthesebeispiel 1, Stufe 1) wurden in 11 mL THF gelöst und auf -90 °C abgekühlt. Die Temperatur wurde während der Reaktion zwischen -85 °C und -90 °C gehalten. 0.58 mL (1.45 mmol) 2.5 molare *n*-BuLi-Lösung in *n-*Hexan wurden innerhalb von 5 Minuten bei -90 °C zugetropft und 5 Minuten nachgerührt. Anschließend wurden innerhalb von 5 Minuten 0.460 mL Pyrrolidin-1-carbonylchlorid (4.16 mmol), in 1 mL THF gelöst, zugetropft. Es wurden weitere 30 Minuten bei -80 °C nachgerührt und dann innerhalb von 30 Minuten auf Raumtemperatur erwärmt. Der Ansatz wurde mit halbkonzentrierter Natriumhydrogencarbonatlösung/Dichlormethan extrahiert. Die wässrige Phase wurde nochmals dreimal mit Dichlormethan extrahiert. Die organischen Phasen wurden vereinigt, mit wenig Wasser gewaschen, mit Natriumsulfat getrocknet, filtriert und eingeengt. Der Rückstand wurde an Kieselgel durch MPLC mit Essigsäureethylester/Methanol als Laufmittel gereinigt. Nach Aufreinigung wurden 115 mg (25.9% d. Theorie) erhalten.
logP[a]: 0.81; logP[n]: 1.02;
¹H-NMR (d₆-DMSO, 400MHz); δ = 1.81 (m, 4H), 3.40 (s, 3H), 3.43-3.46 (m, 2H), 3.56-3.59 (m, 2H), 3.90 (s, 3H), 6.38 (s, 1H), 7.42-7.45 (m, 1H), 7.83-7.85 (m, 1H), 8.46-8.48 (m, 1H), 8.498-8.504 (m, 1H) ppm.

### Synthesebeispiel Nr. 4

### 2-(Ethylcarbamothioyl)-N,1-dimethyl-N-(pyridin-3-yl)-1H-imidazol-5-carboxamid (Verbindung Nr. I-1-057)

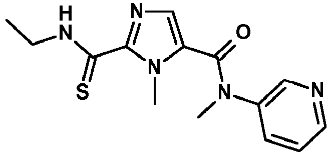

0.300 g (1.39 mmol) N,1-Dimethyl-N-(pyridin-3-yi)-1H-imidazol-5-carboxamid (Synthesebeispiel 1, Stufe 1) wurden in 11 mL THF gelöst und auf -90 °C abgekühlt. Die Temperatur wurde während der Reaktion zwischen -85 °C und -90 °C gehalten. 0.58 mL (1.45 mmol) 2.5 molare *n*-BuLi-Lösung in *n-*Hexan wurden innerhalb von 5 Minuten bei -90 °C zugetropft und 5 Minuten nachgerührt. Anschließend wurden innerhalb von 5 Minuten 0.365 mL Isothiocyanatoethan (4.16 mmol), in 1 mL THF gelöst, zugetropft. Es wurden weitere 30 Minuten bei -80 °C nachgerührt und dann innerhalb von 30 Minuten auf Raumtemperatur erwärmt. Der Ansatz wurde mit halbkonzentrierter Natriumhydrogencarbonatlösung/Dichlormethan extrahiert. Die wässrige Phase wurde nochmals dreimal mit Dichlormethan extrahiert. Die organischen Phasen wurden vereinigt, mit wenig Wasser gewaschen, mit Natriumsulfat getrocknet, filtriert und eingeengt. Der Rückstand wurde an Kieselgel durch MPLC mit Essigsäureethylester/Methanol als Laufmittel gereinigt. Nach Aufreinigung wurden 28.0 mg (6.1% d. Theorie) erhalten.
logP[a]: 1.21; logP[n]: 1.42;
¹H-NMR (d₆-DMSO, 400MHz); δ = 1.13-1.17 (t, 3H), 3.40 (s, 3H), 3.58-3.65 (m, 2H), 4.02 (s, 3H), 6.37 (s, 1H), 7.42-7.46 (m, 1H), 7.85-7.88 (m, 1H), 8.46-8.48 (m, 1H), 8.51-8.52 (m, 1H) ppm.

### Synthesebeispiel Nr. 5

### 2-Formyl-N,1-dimethyl-N-(pyridin-3-yl)-1H-imidazol-5-carboxamid (Verbindung Nr. I-1-007)

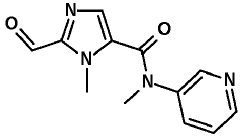

N,1-Dimethyl-N-(pyridin-3-yi)-1H-imidazol-5-carboxamid (Synthesebeispiel 1, Stufe 1) (5 g, 23,1 mmol) wurde in trockenem THF (150 mL) vorgelegt, unter Argon auf -90°C abgekühlt und 9,7 mL einer 2,5 N Lösung von n-Butyllithium in n-Hexan (1,55 g, 24,2 mmol) zugetropft. Das Reaktionsgemisch wurde 5 min bei -85°C° gerührt und anschließend langsam eine Lösung von N.N-Dimethylformamid (5,07 g, 69,3 mmol) in THF (50 mL) zugetropft. Das Reaktionsgemisch wurde zunächst 30 min bei -80 °C gerührt und anschließend innerhalb einer Stunde auf Raumtemperatur gebracht. Zur Aufarbeitung wurde der Ansatz in Dichlormethan/halkonzentrierte Natriumhydrogencarbonatlösung aufgenommen, die wässrige Phase mehrfach mit Dichlormethan extrahiert und die vereinigten organischen Phasen über Magnesiumsulfat getrocknet. Nach Abdestillieren des Lösungsmittels unter vermindertem Druck wurde der Rückstand anKieselgel mit Ethylacetat/Methanol (80:20) als Laufmittel chromatographiert. Es wurden 2,64 g (46,7 % der Theorie) 2-Formyl-N,1-dimethyl-N-(pyridin-3-yl)-1H-imidazol-5-carboxamid erhalten.
HPLC-MS: logP[n] = 0,59; Masse (m/z): 245,1; ¹HNMR (D⁶-DMSO): δ 3.42 (s, 3H), 4.05 (s, 3H), 6,57 (s, 1H), 7.36, (m, 1H), 7.67 (m, 1H), 8.40 (m, 1H), 8.47 (m, 1H), 9.66 (1, 1H).

### Synthesebeispiel Nr. 6

### 2-[(E)-(Cyclopropylmethoxyimino)methyl]-N,1-dimethyl-N-(pyridin-3-yl)-1H-imidazol-5-carboxamid (Verbindung Nr. I-1-008)

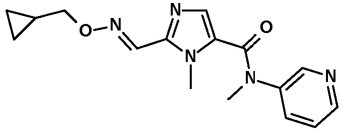

2-Formyl-N,1-dimethyl-N-(pyridin-3-yl)-1H-imidazol-5-carboxamid (Synthesebeispiel 5) (100 mg, 0,47 mmol) wurde in Pyridin (4,0 mL) vorgelegt und O-Cyclopropylmethylhydroxylamin Hydrochlorid (60,7 mg, 0,49 mmol) zugesetzt. Das Reaktionsgemisch wurde über Nacht bei Raumtemperatur gerührt und anschließend das Lösungsmittel vollständig abdestiliert. Der Rückstand wurde an Kieselgel mit Ethylacetat/Methanol (80:20) als Laufmittel chromatographiert. Es wurden 39 mg (30,3 % der Theorie) 2-[(E)-(Cyclopropylmethoxyimino)methyl] -N,1-dimethyl-N-(pyridin-3-yl)-1H-imidazol-5-carboxamid erhalten.
HPLC-MS: logP[n] = 1,78; Masse (m/z): 314,1; ¹HNMR (D⁶-DMSO): δ 0.29 (m, 2H), 0.54 (m, 2H), 1.27 (m, 1H), 3.40 (s, 3H), 3.97 (s, 3H), 3.99 (s, 2H), 6,40 (s, 1H), 7.35, (m, 1H), 7.67 (m, 1H), 8.04 (s, 1H), 8.44 (m, 2H).

### Synthesebeispiel Nr. 7

### Methyl-(2E)-1-methyl-2-({1-methyl-5-[methyl(pyridin-3-yl)carbamoyl]-1H-imidazol-2-yl}methylen)hydrazincarboxylat (Verbindung Nr.: 1-1-018)

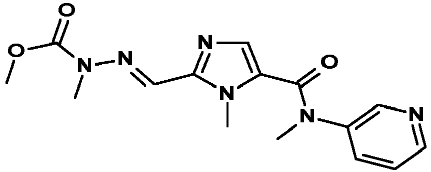

2-Formyl-N,1-dimethyl-N-(pyridin-3-yl)-1H-imidazol-5-carboxamid (Synthesebeispiel 5) (100 mg, 0,47 mmol) wurde in Ethanol (10,0 mL) vorgelegt, Methyl-1-methylhydrazincarboxylat [vgl. WO2007/43677] (60,7 mg, 0,49 mmol) zugesetzt und anschließend 2 Stunden bei Rückflußtemperatur gerührt. Das Lösungsmittel wurde vollständig abdestilliert und der Rückstand anschließend an Kieselgel mit Ethylacetat/Methanol (80:20) als Laufmittel chromatographiert. Es wurden 60 mg (45 % der Theorie) Methyl-(2E)-1-methyl-2-({1-methyl-5-[methyl(pyridin-3-yl)carbamoyl]-1H-imidazol-2-yl}methylen)hydrazincarboxylat erhalten.
HPLC-MS: logP[n] = 1,08; Masse (m/z): 331,1; ¹HNMR (D⁶-DMSO): δ 3.31 (s, 3H), 3.38 (s, 3H), 3.85 (s, 3H), 4.10 (s, 3H), 6.40 (s, 1H), 7.34, (m, 1H), 7.62 (s, 1H), 7.66 (m, 1H), 8.43 (m, 2H).

### Synthesebeispiel Nr. 8

### 2-(2,2-Dimethyl-1,3-dioxan-5-yl)-N,1-dimethyl-N-(pyridin-3-yl)-1H-imidazol-5-carboxamid (Verbindung Nr. I-1-016)

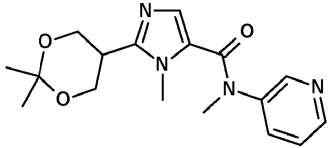

2-Formyl-N,1-dimethyl-N-(pyridin-3-yl)-1H-imidazol-5-carboxamid (Synthesebeispiel 5) (100 mg, 0,47 mmol) wurde in Toluol (10,0 mL) vorgelegt, 2,2-Dimethyl-1,3-propandiol (213,2 mg, 2,04 mmol) und Toluolsulfonsäure (14,1 mg, 0,08 mmol) zugesetzt und anschließend 24 Stunden am Wasserabscheider bei Rückflußtemperatur gerührt. Das Lösungsmittel wurde vollständig abdestilliert und der Rückstand anschließend an Kieselgel mit Ethylacetat/Methanol (80:20) als Laufmittel chromatographiert. Es wurden 120 mg (81 % der Theorie) 2-(2,2-Dimethyl-1,3-dioxan-5-yl)-N,1-dimethyl-N-(pyridin-3-yl)-1H-imidazol-5-carboxamid erhalten.
HPLC-MS: logP[n] = 1,48; Masse (m/z): 331,1; ¹HNMR (D⁶-DMSO): δ 0,77 (s, 3H), 0,81 (s, 3H), 1,08 (m, 1H), 3.39 (s, 3H), 3.97 (s, 3H), 3.68 (dd, 4H), 6.20 (s, 1H), 7.35, (m, 1H), 7.66 (s, 1H), 8.39 (m, 1H), 8.44 (m, 2H).

### Synthesebeispiel Nr. 9

### N²-Methoxy-N²,N⁵,1-trimethyl-N⁵-(pyridin-3-yl)-1H-imidazol-2,5-dicarboxamid (Verbindung Nr. I-1-69)

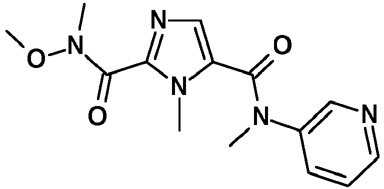

0.100 mg (1.02 mmol) N-Methoxymethanaminhydrochlorid wurden in 4.0 mL THF gelöst und auf 0 °C abgekühlt. 0.456 mL (0.911 mmol) Trimethylaluminiumlösung (2M in Toluol) wurden bei 0 °C zugetropft und 20 Minuten nachgerührt. 0.100 g (0.365 mmol) Methyl-1-methyl-5-[methyl(pyridin-3-yl)carbamoyl]-1H-imidazol-2-carboxylat (Synthesebeispiel Nr. 1), in 4.0 mL THF gelöst, wurden zugetropft. Es wurde 16 Stunden bei 50 °C gerührt. Der Ansatz wurde mit gesättigter wässriger Natriumkaliumtartratlösung/Dichlormethan extrahiert. Die organische Phase wurde mit Natriumsulfat getrocknet, filtriert und eingeengt. Nach Extraktion wurden 0.114 g (92.8% d. Theorie) erhalten.
HPLC-MS: logP[n] = 0.72; ¹H-NMR (d₆-DMSO, 400MHz); δ = 3.28 (s, 3H), 3.40 (s, 3H), 3.65 (s, 3H), 3.81 (s, 3H), 6.36 (s, 1H), 7.43-7.46 (m, 1H), 7.84-7.87 (m, 1H), 8.46-8.49 (m, 1H), 8.50-8.52 (m, 1H).

### Synthesebeispiel Nr. 10

### 2-Isobutyryl-N,1-dimethyl-N-(pyridin-3-yl)-1H-imidazol-5-carboxamid (Verbindung Nr. 1-1-82)

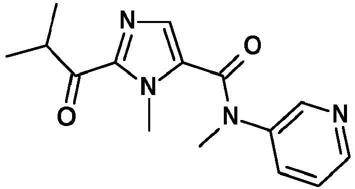

44.0 mg (0.145 mmol) N²-Methoxy-N²,N⁵,1-trimethyl-N⁵-(pyridin-3-yl)-1H-imidazol-2,5-dicarboxamid (Synthesebeispiel Nr. 9) wurden in 0.65 mL THF gelöst und auf -78 °C abgekühlt. 0.100 mL (0.290 mmol) Isopropylmagnesiumbromidlösung (2.9M in THF) wurden zugetropft. Es wurde innerhalb von 3 Stunden auf Raumtemperatur erwärmt. Der Ansatz wurde mit gesättigter wässriger Ammoniumchloridlösung/Essigsäureethylester extrahiert. Die organische Phase wurde mit Wasser und gesättigte wässrige Natriumchloridlösung gewaschen, mit Natriumsulfat getrocknet, filtriert und eingeengt. Der Rückstand wurde an Kieselgel durch MPLC mit Essigsäureethylester/Methanol als Laufmittel gereinigt. Die gewünschte Fraktion wurde über RP18 durch HPLC mit Acetonitril/Wasser als Laufmittel gereinigt. Nach Aufreinigung wurden 7.0 mg (16.9% d. Theorie) erhalten.
HPLC-MS: logP[n] = 1.55; ¹H-NMR (d₆-DMSO, 400MHz); δ = 1.04 (s, 3H), 1.06 (s, 1H), 3.41 (s, 3H), 3.66-3.73 (m, 1H), 4.015 (s, 3H), 6.57 (s, 1H), 7.42-7.45 (m, 1H), 7.84-7.86 (m, 1H), 8.46-8.47 (m, 1H), 8.497-8.503 (m, 1H).

**Tabelle 1**

| | | | | | | |
|---|---|---|---|---|---|---|
| Verbindungen der Formel (I-1) | | | | | | |
| 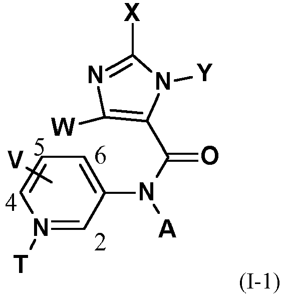 | | | | | | |
| in welcher T für ein freies Elektronenpaar steht, und die anderen Substituenten, die in der Tabelle angegebenen Bedeutungen haben: | | | | | | |

| **Bsp.-Nr.** | **X** | **Y** | **A** | **V** | **W** | **Logp [n] ^{a)}** |
|---|---|---|---|---|---|---|
| I-1-001 | 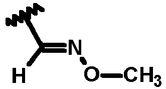 | CH₃ | CH₃ | H | H | 1,1 |
| I-1-002 | 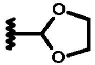 | CH₃ | CH₃ | H | H | 0,73 |
| I-1-003 | 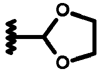 | CH₃ | C₂H₅ | H | H | 0,98 |
| T-1-004 | 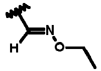 | CH₃ | CH₃ | H | H | 1,41 |
| I-1-005 | 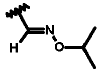 | CH₃ | CH₃ | H | H | 1,7 |
| I-1-006 | 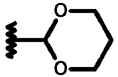 | CH₃ | CH₃ | H | H | 0,92 |
| I-1-007 | 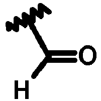 | CH₃ | CH₃ | H | H | 0,59 |
| I-1-008 | 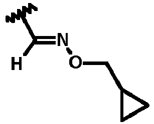 | CH₃ | CH₃ | H | H | 1,78 |
| I-1-009 | 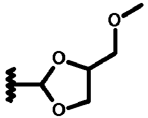 | CH₃ | CH₃ | H | H | 0,95 |
| I-1-010 | 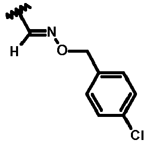 | CH₃ | CH₃ | H | H | 2,43 |
| I-1-011 | 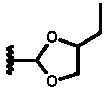 | CH₃ | CH₃ | H | H | 1,3 |
| I-1-012 | 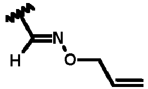 | CH₃ | CH₃ | H | H | 1,55 |
| I-1-013 | 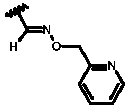 | CH₃ | CH₃ | H | H | 1,26 |
| I-1-014 | 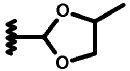 | CH₃ | CH₃ | H | H | 1,02 |
| I-1-015 | 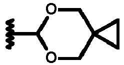 | CH₃ | CH₃ | H | H | 1,29 |
| I-1-016 | 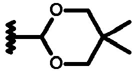 | CH₃ | CH₃ | H | H | 1,48 |
| I-1-017 | 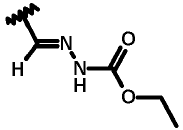 | CH₃ | CH₃ | H | H | 1,06 |
| I-1-018 | 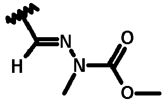 | CH₃ | CH₃ | H | H | 1,08 |
| I-1-019 | 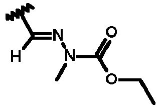 | CH₃ | CH₃ | H | H | 1,33 |
| I-1-020 | 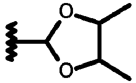 | CH₃ | CH₃ | H | H | 1,23 |
| I-1-021 | 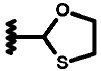 | CH₃ | CH₃ | H | H | 0,97 |
| I-1-022 | 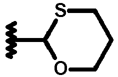 | CH₃ | CH₃ | H | H | 1,13 |
| I-1-023 | 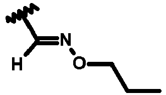 | CH₃ | CH₃ | H | H | 1,74 |
| I-1-024 | 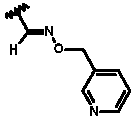 | CH₃ | CH₃ | H | H | 1,24 |
| I-1-025 | 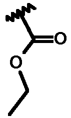 | CH₃ | CH₃ | H | H | 1,1 |
| I-1-026 | 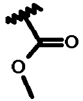 | CH₃ | CH₃ | H | H | 0,42 |
| I-1-027 | 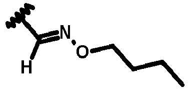 | CH₃ | CH₃ | H | H | 2,08 |
| I-1-028 | 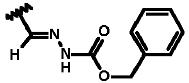 | CH₃ | CH₃ | H | H | 1,63 |
| I-1-029 | 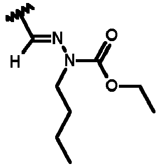 | CH₃ | CH₃ | H | H | 2,06 |
| I-1-030 | 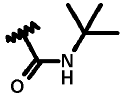 | CH₃ | CH₃ | H | H | 1,62 |
| I-1-031 | 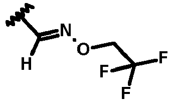 | CH₃ | CH₃ | H | H | 1,68 |
| I-1-032 | 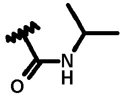 | CH₃ | CH₃ | H | H | 1,26 |
| I-1-033 | 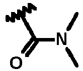 | CH₃ | CH₃ | H | H | 0,6 |
| I-1-034 | 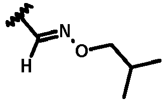 | CH₃ | CH₃ | H | H | 2,07 |
| I-1-035 | 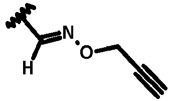 | CH₃ | CH₃ | H | H | 1,83 |
| I-1-036 | 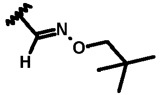 | CH₃ | CH₃ | H | H | 2,22 |
| I-1-037 | 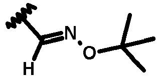 | CH₃ | CH₃ | H | H | 1,99 |
| I-1-038 | 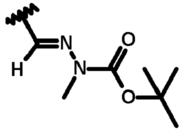 | CH₃ | CH₃ | H | H | 1,82 |
| I-1-039 | 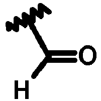 | CH₃ | C₂H₅ | H | H | 0,97 |
| I-1-040 | 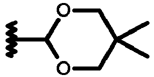 | CH₃ | C₂H₅ | H | H | 1,61 |
| I-1-041 | 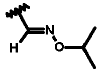 | CH₃ | C₂H₅ | H | H | 1,81 |
| I-1-042 | 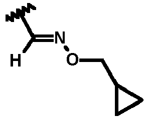 | CH₃ | C₂H₅ | H | H | 1,86 |
| I-1-043 | 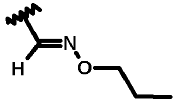 | CH₃ | C₂H₅ | H | H | 1,85 |
| I-1-044 | 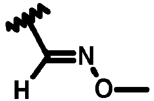 | CH₃ | C₂H₅ | H | H | 1,29 |
| I-1-045 | 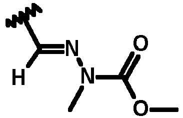 | CH₃ | C₂H₅ | H | H | 1,28 |
| I-1-046 | 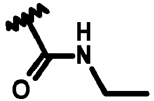 | CH₃ | CH₃ | H | H | 0,97 |
| I-1-047 | 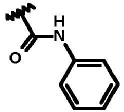 | CH₃ | CH₃ | H | H | 1,82 |
| I-1-048 | 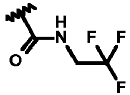 | CH₃ | CH₃ | H | H | 1,42 |
| I-1-049 | 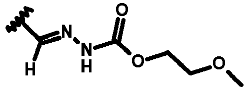 | CH₃ | CH₃ | H | H | 1,26 |
| I-1-050 | 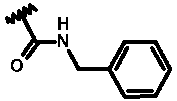 | CH₃ | CH₃ | H | H | 1,75 |
| I-1-051 | 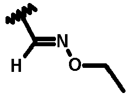 | CH₃ | C₂H₅ | H | H | 1,66 |
| I-1-052 | 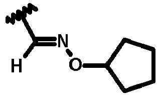 | CH₃ | CH₃ | H | H | 2,14 |
| I-1-053 | 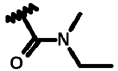 | CH₃ | CH₃ | H | H | 0,87 |
| I-1-054 | 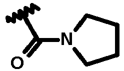 | CH₃ | CH₃ | H | H | 1,02 |
| I-1-055 | 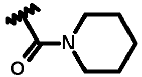 | CH₃ | CH₃ | H | H | 1,16 |
| I-1-056 | 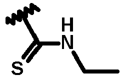 | CH₃ | CH₃ | H | H | 1,42 |
| I-1-057 | 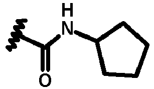 | CH₃ | CH₃ | H | H | 1,64 |
| I-1-058 | 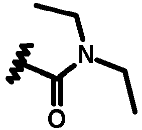 | CH₃ | CH₃ | H | H | 1,11 |
| I-1-059 | 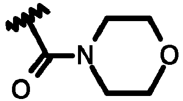 | CH₃ | CH₃ | H | H | 0,73 |
| I-1-060 | 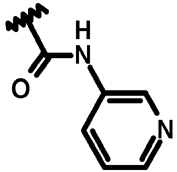 | CH₃ | CH₃ | H | H | 1,17 |
| I-1-061 | 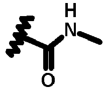 | CH₃ | CH₃ | H | H | 0,7 |
| I-1-062 | 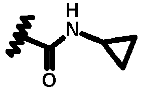 | CH₃ | CH₃ | H | H | 1,06 |
| I-1-063 | 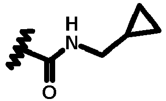 | CH₃ | CH₃ | H | H | 1,39 |
| I-1-064 | 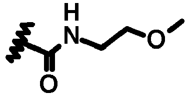 | CH₃ | CH₃ | H | H | 0,94 |
| I-1-065 | 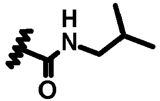 | CH₃ | CH₃ | H | H | 1,57 |
| I-1-066 | 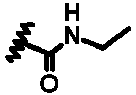 | CH₃ | C₂H₅ | H | H | 1,24 |
| I-1-067 | 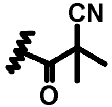 | CH₃ | CH₃ | H | H | 1,55 |
| I-1-068 | 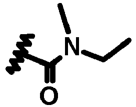 | CH₃ | C₂H₅ | H | H | 1,12 |
| I-1-069 | 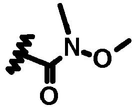 | CH₃ | CH₃ | H | H | 0,72 |
| I-1-070 | 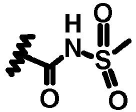 | CH₃ | CH₃ | H | H | -0,2 |
| I-1-071 | 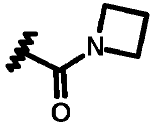 | CH₃ | CH₃ | H | H | 0,89 |
| I-1-072 | 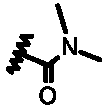 | CH₃ | C₂H₅ | H | H | 0,88 |
| I-1-073 | 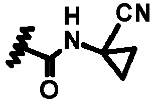 | CH₃ | CH₃ | H | H | 1,03 |
| I-1-074 | 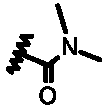 | C₂H₅ | CH₃ | H | H | 0,86 |
| I-1-075 | 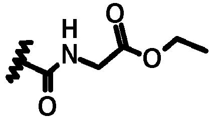 | CH₃ | CH₃ | H | H | 1,22 |
| I-1-076 | 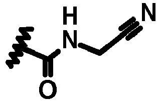 | CH₃ | CH₃ | H | H | 0,78 |
| I-1-077 | 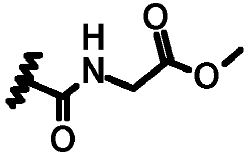 | CH₃ | CH₃ | H | H | 0,93 |
| I-1-078 | 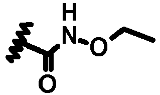 | CH₃ | CH₃ | H | H | 0,67 |
| I-1-079 | 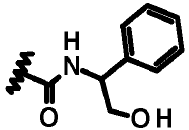 | CH₃ | CH₃ | H | H | 1,39 |
| I-1-080 | 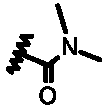 | CH₃ | CH₃ | 6-CH₃ | H | 0,81 |
| I-1-081 | 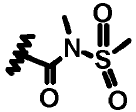 | CH₃ | CH₃ | H | H | 1,11 |
| I-1-082 | 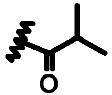 | CH₃ | CH₃ | H | H | 1,55 |
| I-1-083 | 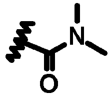 | CH₃ | 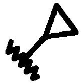 | H | H | 1,02 |
| I-1-084 | 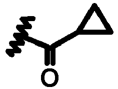 | CH₃ | CH₃ | H | H | 1,32 |
| I-1-085 | 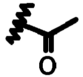 | CH₃ | CH₃ | H | H | 0,87 |
| I-1-086 | 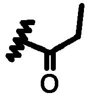 | CH₃ | CH₃ | H | H | 1,23 |
| I-1-087 | 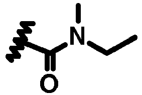 | CH₃ | 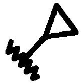 | H | H | 1,11 |
| I-1-088 | 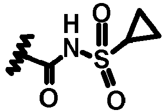 | CH₃ | CH₃ | H | H | 0,72 |
| I-1-089 | 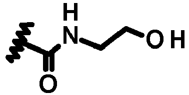 | CH₃ | CH₃ | H | H | 0,45 |

**Ausgewählte ¹H-NMR Daten ^{b}) der Verbindungen gemäß Tabelle 1**

| |
|---|
| Beispiel I-1-001: ¹H-NMR(400,0 MHz, CD3CN): δ= 8,449(1,6);8,441(1,5);8,438(1,7);8,407(2,0);8,402(2,3);8,028(4,9);7,963(0,8);7,827(0,6); 7,818(0,6);7,684(0,8);7,680(1,1);7,678(1,2);7,674(1,0);7,664(0,9);7,660(1,2);7,658(1,3);7,654(1,1);7,367(1,1);7,355(1,2);7,348(1,1);7,347(1, 1);7,335(1,0);7,098(0,4);7,086(0,5);7,077(0,6);7,066(0,5);6,902(0,6);6,898(0,7);6,895(0,6);6,881(0,5);6,877(0,6);6,874(0,5);6,400(2,6);5,448 (1,4);3,969(16,0);3,952(15,9);3,409(15,9);2,755(3,2);2,214(40,0);1,953(3,0);1,947(5,8);1,941(8,0);1,935(6,2);1,929(3,5);0,008(0,7);0,000(17 ,5) |
| Beispiel I-1-002: ¹H-NMR(400,0 MHz, CD3CN): δ= 8,459(1,4);8,456(1,3);8,447(1,4);8,444(1,3);8,407(1,9);8,400(1,9);7,682(0,8);7,678(0,9); 7,672(0,7);7,662(0,9);7,658(1,0);7,655(1,0);7,652(0,8);7,373(1,2);7,361(1,2);7,353(1,1);7,341(1,0);6,246(2,6);5,758(4,8);4,147(1,1);4,129(3, 1);4,120(1,4);4,112(1,5);4,085(0,4);4,017(0,5);3,990(1,6);3,981(1,5);3,973(3,1);3,955(1,0);3,924(0,9);3,818(16,0);3,395(16,0);2,762(0,4);2,1 47(10,3);2,113(0,4);2,107(0,4);1,963(1,0);1,957(2,6);1,951 (12,7);1,945(22,7);1,939(30,4);1,933(21,0);1,927(10,8);1,269(0,5);0,146(0,3);0,0 08(2,7);0,000(62,8) |
| Beispiel I-1-003: ¹H-NMR(400,0 MHz, CD3CN): δ= 8,477(1,4);8,474(1,5);8,465(1,4);8,462(1,4);8,372(1,9);8,366(2,0);7,965(1,1);7,958(1,1); 7,818(0,8);7,807(0,8);7,663(0,8);7,660(0,9);7,657(0,9);7,654(0,7);7,643(0,9);7,639(1,1);7,637(1,1);7,633(0,9);7,381(1,1);7,369(1,1);7,361(1, 0);7,349(0,9);7,080(0,6);7,068(0,6);7,059(0,7);7,047(0,7);6,907(0,6);6,903(0,6);6,886(0,5);6,882(0,5);6,199(2,8);5,887(0,3);5,745(4,9);4,164 (0,4);4,141(1,0);4,123(3,2);4,114(1,5);4,106(1,5);4,079(0,4);4,011(0,6);3,984(1,6);3,975(1,5);3,967(3,2);3,949(1,0);3,924(1,1);3,917(1,3);3,8 99(3,7);3,881(3,7);3,864(1,2);3,812(16,0);3,128(0,7);3,111(0,8);3,099(0,8);2,127(17,0);2,106(1,4);2,099(0,9);1,962(2,7);1,956(7,3);1,950(38 ,2);1,944(69,7);1,938(93,9);1,932(65,7);1,926(34,4);1,772(0,4);1,766(0,5);1,760(0,4);1,271(0,6);1,219(2,6);1,201(5,0);1,183(2,5);1,170(3,9); 1,152(7,9);1,135(3,8);0,146(0,5);0,008(4,0);0,000(109,1);-0,150(0,5) |
| Beispiel I-1-004: ¹H-NMR(400,0 MHz, CD3CN): δ= 8,452(0,4);8,440(0,4);8,407(0,4);8,401(0,4);8,022(0,9);7,367(0,4);7,354(0,3);6,394(0,6); 5,461(3,9);5,446(16,0);4,236(0,4);4,218(0,8);4,201(0,8);3,988(0,7);3,974(2,8);3,757(0,6);3,424(0,7);3,409(2,9);3,388(0,8);2,170(1,4);2,149( 2,1);1,952(2,3);1,946(3,1);1,940(3,4);1,933(2,2);1,927(1,1);1,386(0,4);1,302(1,1);1,285(2,0);1,267(1,2);0,014(0,4);0,000(1,7) |
| Beispiel I-1-005: ¹H-NMR(400,0 MHz, CD3CN): δ= 8,448(1,6);8,437(1,6);8,407(2,0);8,402(2,1);7,986(4,6);7,676(1,2);7,672(1,1);7,661(1,0); 7,657(1,2);7,365(1,4);7,354(1,4);7,345(1,2);7,333(1,1);6,394(2,0);5,447(6,2);4,467(0,4);4,451(1,1);4,436(1,4);4,420(1,1);4,404(0,4);3,981(1 3,8);3,763(0,7);3,756(1,0);3,409(14,1);3,387(1,7);2,179(1,8);1,952(2,7);1,946(4,6);1,940(6,1);1,934(4,5);1,928(2,5);1,385(0,6);1,341(1,3);1, 316(0,3);1,282(15,0);1,266(16,0);1,216(0,3);1,192(0,5);0,000(2,6) |
| Beispiel I-1-006: ¹H-NMR(400,0 MHz, CD3CN): δ= 8,448(0,5);8,439(0,6);8,436(0,6);8,391(0,8);8,385(0,8);7,676(0,4);7,674(0,4);7,670(0,3); 7,660(0,4);7,656(0,5);7,653(0,5);7,650(0,4);7,370(0,4);7,358(0,4);7,349(0,4);7,337(0,4);6,192(1,1);5,602(1,9);4,185(0,5);4,173(0,6);4,158(0, 7);4,145(0,7);3,950(0,5);3,944(0,6);3,931(6,1);3,919(1,0);3,914(0,9);3,888(0,5);3,882(0,6);3,866(0,4);3,614(8,8);3,599(16,0);3,583(8,9);3,38 9(6,1);2,734(1,6);2,225(0,4);2,210(0,4);2,198(0,4);2,179(0,4);2,166(0,4);2,133(0,4);1,953(1,7);1,947(3,1);1,941(4,1);1,935(2,9);1,928(1,6);1, 685(1,5);1,670(5,6);1,655(7,8);1,639(5,3);1,624(1,5);1,471(0,4);1,437(0,4);1,101(4,3);1,085(4,3);0,000(1,6) |
| Beispiel I-1-007: ¹H-NMR(400,0 MHz, CDCl₃): δ= 9,764(5,4);8,563(1,4);8,559(1,5);8,551(1,5);8,547(1,5);8,414(2,0);8,407(2,0);7,541(0,7);7, 537(0,8);7,535(0,8);7,531(0,7);7,521(0,9);7,517(1,0);7,515(1,0);7,511(0,8);7,366(1,2);7,354(1,2);7,346(1,0);7,334(0,9);7,269(4,4);6,512(1,5) ;4,204(16,0);4,178(0,5);3,983(0,4);3,509(15,6);1,715(0,5);0,000(1,7) |
| Beispiel I-1-008: ¹H-NMR(400,0 MHz, CD3CN): δ= 8,452(1,1);8,449(1,2);8,440(1,2);8,437(1,2);8,408(1,6);8,402(1,6);8,042(4,7);7,681(0,6); 7,677(0,8);7,675(0,7);7,671(0,6);7,661(0,7);7,657(0,8);7,654(0,8);7,651(0,7);7,367(0,9);7,355(0,9);7,347(0,8);7,335(0,8);6,399(2,4);5,446(3, 7);3,991(4,1);3,975(16,0);3,409(14,3);2,158(15,1);1,958(0,7);1,952(4,1);1,946(7,5);1,939(10,2);1,933(6,9);1,927(3,6);1,386(0,8);1,276(0,4); 1,268(0,8);1,195(0,4);1,193(0,4);1,187(0,4);1,175(0,7);1,163(0,4);1,155(0,4);1,100(0,4);1,084(0,4);0,577(0,5);0,566(1,6);0,562(1,7);0,551(0, 7);0,546(1,7);0,542(1,5);0,531(0,6);0,318(0,6);0,307(1,9);0,304(1,8);0,295(1,6);0,291(1,9);0,280(0,5);0,008(1,2);0,000(28,9);-0,009(1,1) |
| Beispiel I-1-009: ¹H-NMR(400,0 MHz, CD3CN): δ= 8,458(1,0);8,455(1,0);8,446(1,0);8,443(1,0);8,405(1,3);8,399(1,3);7,678(0,6);7,674(0,6); 7,668(0,3);7,663(0,4);7,658(0,7);7,654(0,6);7,648(0,3);7,371(0,8);7,359(0,8);7,351(0,7);7,339(0,7);6,256(1,0);6,243(1,1);5,868(2,0);5,777(2, 0);4,436(0,4);4,336(0,4);4,272(0,5);4,254(0,6);4,252(0,6);4,234(0,5);4,067(0,5);4,048(0,9);4,028(0,5);3,860(6,7);3,843(0,7);3,840(0,6);3,819 (6,8);3,745(0,6);3,728(0,6);3,724(0,6);3,707(0,6);3,680(0,7);3,668(0,7);3,654(0,4);3,557(1,3);3,546(1,0);3,542(1,0);3,505(1,1);3,497(1,3);3,4 92(1,4);3,487(1,1);3,480(0,7);3,466(0,8);3,454(0,5);3,428(0,5);3,414(0,9);3,397(7,8);3,394(7,7);3,383(1,0);3,371(1,0);3,358(2,0);3,342(10,6) ;3,326(2,0);3,311(2,0);3,303(16,0);3,286(0,8);2,971(0,8);2,961(0,7);2,753(0,4);2,739(0,7);2,727(0,4);2,164(10,9);1,957(0,7);1,952(4,0);1,94 5(7,2);1,939(9,7);1,933(6,6);1,927(3,4);1,100(2,7);1,084(2,7);0,008(0,9);0,000(22,3);-0,009(0,8) |
| Beispiel I-1-010: ¹H-NMR(400,0 MHz, CD3CN): δ= 8,445(0,7);8,434(0,7);8,394(0,9);8,081(4,2);7,668(0,6);7,664(0,7);7,661(0,7);7,658(0,6); 7,647(0,7);7,644(0,8);7,641(0,8);7,638(0,6);7,395(16,0);7,357(0,7);7,345(0,7);7,337(0,7);7,325(0,6);6,394(1,2);5,174(7,7);3,911(14,4);3,401 (14,3);2,142(12,0);1,963(0,5);1,957(1,2);1,951(6,8);1,945(12,3);1,939(16,6);1,933(11,3);1,927(5,8);1,100(5,2);1,085(5,2);0,008(2,0);0,000(4 5,5);-0,009(1,7) |
| Beispiel I-1-011: ¹H-NMR(400,0 MHz, CD3CN): δ= 9,660(0,5);8,459(2,1);8,455(2,2);8,447(2,1);8,443(2,1);8,405(2,5);8,402(2,5);8,399(2,5); 8,324(0,4);7,680(1,4);7,660(1,6);7,639(0,4);7,374(1,8);7,363(1,8);7,354(1,6);7,342(1,5);6,982(0,7);6,253(2,0);6,238(2,3);5,867(4,1);5,764(4, 0);4,293(0,8);4,277(1,3);4,258(1,4);4,251(0,5);4,235(0,7);4,220(0,9);4,203(0,6);4,129(0,5);4,112(0,7);4,096(1,6);4,088(0,9);4,079(1,5);4,069 (1,7);4,062(0,7);3,929(1,9);3,860(0,3);3,844(12,3);3,838(3,0);3,822(13,5);3,662(0,7);3,646(1,9);3,630(0,5);3,593(0,9);3,576(1,4);3,574(1,4); 3,557(1,0);3,548(0,8);3,428(2,8);3,397(15,0);3,395(16,0);3,306(0,5);3,283(0,6);2,809(0,7);2,752(0,5);2,740(0,5);2,547(0,4);2,244(0,5);2,226 (0,6);2,180(111,3);1,964(1,1);1,958(2,7);1,952(16,6);1,946(30,6);1,940(41,8);1,934(28,7);1,927(14,8);1,737(0,6);1,718(0,8);1,712(0,7);1,69 8(0,8);1,693(1,0);1,687(0,4);1,677(1,0);1,658(1,0);1,640(0,6);1,599(0,5);1,584(0,6);1,581(0,6);1,566(0,7);1,547(0,4);1,496(0,4);1,478(0,4);1,473(0,4);1,460(0,5);1,453(0,4);1,438(0,7);1,426(0,6);1,419(0,6);1,407(0,6);1,356(0,7);1,338(1,0);1,319(1,0);1,303(1,0);1,285(1,0);1,271(0,9) ;1,100(9,6);1,084(9,5);0,995(2,7);0,988(0,7);0,976(5,6);0,970(1,8);0,965(3,4);0,957(2,7);0,946(6,3);0,925(5,2);0,906(8,3);0,887(3,7);0,866(1 ,0);0,849(0,5);0,008(1,9);0,000(54,2);-0,009(2,1) |
| Beispiel I-1-012: ¹H-NMR(400,0 MHz, CD3CN): δ= 8,452(1,3);8,449(1,3);8,441(1,4);8,437(1,3);8,407(1,9);8,400(1,8);8,072(5,3);7,682(0,8); 7,679(0,9);7,676(0,9);7,672(0,7);7,662(0,9);7,658(1,0);7,656(1,0);7,652(0,8);7,368(1,1);7,356(1,1);7,347(1,0);7,335(0,9);6,402(2,6);6,076(0, 6);6,064(0,4);6,062(0,4);6,050(0,7);6,033(0,8);6,021(0,5);6,019(0,4);6,007(0,7);5,992(0,4);5,369(0,5);5,365(1,2);5,361(1,2);5,322(1,1);5,318 (1,1);5,260(1,2);5,257(1,1);5,234(1,1);5,231(1,0);4,672(3,0);4,661(2,0);4,658(2,9);3,957(16,0);3,409(16,0);2,198(15,9);1,964(0,3);1,958(0,8) ;1,952(4,3);1,946(7,7);1,940(10,2);1,934(6,8);1,928(3,4);1,100(3,6);1,084(3,5);0,007(1,6);0,000(26,4);-0,009(0,9) |
| Beispiel I-1-013: ¹H-NMR(400,0 MHz, CD3CN): δ= 8,561(0,8);8,550(0,9);8,444(1,1);8,434(1,1);8,432(1,1);8,394(1,4);8,388(1,5);8,162(2,1); 7,781(0,5);7,777(0,5);7,762(1,1);7,758(1,1);7,743(0,7);7,739(0,7);7,670(0,7);7,666(0,9);7,663(0,8);7,660(0,7);7,649(0,8);7,645(1,0);7,643(0, 9);7,639(0,8);7,441(1,1);7,421(1,0);7,358(1,0);7,346(1,0);7,338(0,9);7,326(0,9);7,290(0,6);7,277(0,7);7,272(0,7);7,259(0,6);6,401(1,2);5,447 (2,3);5,275(6,9);3,891(15,4);3,398(16,0);2,173(4,8);1,964(0,3);1,958(0,8);1,952(4,7);1,946(8,6);1,940(11,8);1,933(8,1);1,927(4,2);1269(0,6) ;1,100(0,5);1,085(0,5);0,008(0,6);0,000(17,5);-0,009(0,7) |
| Beispiel I-1-014: ¹H-NMR(400,0 MHz, CD3CN): δ= 8,458(2,0);8,455(2,1);8,446(2,1);8,443(2,1);8,406(1,8);8,401(2,9);8,397(1,8);8,322(0,6); 7,686(0,7);7,681(1,3);7,679(1,1);7,677(1,1);7,675(1,2);7,671(0,7);7,665(0,8);7,661(1,4);7,659(1,3);7,657(1,2);7,655(1,4);7,651(0,8);7,639(0, 5);7,373(1,8);7,361(1,7);7,353(1,5);7,341(1,5);6,982(1,0);6,251(2,2);6,239(2,3);5,900(4,2);5,763(4,8);5,612(0,3);4,444(0,5);4,429(0,9);4,411 (1,0);4,396(0,6);4,323(0,4);4,306(0,7);4,300(1,3);4,291(0,7);4,285(1,2);4,280(1,3);4,272(0,6);4,265(1,0);4,117(1,3);4,098(1,8);4,081(1,1);3,9 29(2,6);3,873(0,3);3,851(14,9);3,838(3,6);3,818(14,6);3,692(0,7);3,684(0,7);3,677(0,7);3,582(1,3);3,563(2,2);3,544(1,3);3,518(1,2);3,500(1, 4);3,498(1,5);3,480(1,1);3,398(16,0);3,394(15,8);3,378(0,7);3,365(0,9);3,354(0,5);3,277(0,5);3,267(0,6);3,261(0,6);3,250(0,9);3,241(0,4);3,2 34(0,4);3,222(0,4);2,813(1,1);2,761(0,8);2,396(0,4);2,244(0,6);2,226(0,7);2,207(0,5);2,174(109,5);2,107(0,3);1,964(1,4);1,958(3,3);1,952(19 ,5);1,946(35,8);1,940(48,6);1,934(33,0);1,927(16,8);1,496(0,5);1,478(0,5);1,368(6,9);1,353(6,8);1,306(0,5);1,297(0,7);1,283(7,3);1,267(7,3); 1,100(0,9);1,085(0,9);1,049(13,9);1,033(13,7);0,988(0,8);0,970(1,7);0,951(1,0);0,883(0,5);0,874(0,5);0,866(1,2);0,849(0,5);0,811(0,9);0,146 (0,5);0,008(3,8);0,000(108,3);-0,009(3,9);-0,150(0,5) |
| Beispiel I-1-015: ¹H-NMR(400,0 MHz, CD3CN): δ= 6,202(0,5);5,685(0,8);4,257(0,4);4,228(0,4);3,975(2,8);3,426(8,3);3,394(2,8);3,358(0,5); 3,328(0,4);2,786(0,6);2,183(0,4);1,952(1,1);1,946(1,9);1,940(2,6);1,934(1,8);1,927(0,9);1,100(2,7);1,085(2,7);0,435(0,4);0,415(0,4);0,393(1 6,0);0,000(6,2) |
| Beispiel I-1-016: ¹H-NMR(400,0 MHz, CD3CN): δ= 8,452(1,2);8,448(1,3);8,440(1,3);8,437(1,3);8,395(1,7);8,389(1,7);7,680(0,7);7,676(0,8); 7,674(0,8);7,670(0,7);7,660(0,8);7,656(0,9);7,653(0,9);7,650(0,8);7,368(1,0);7,367(1,0);7,357(1,0);7,348(0,9);7,336(0,9);7,335(0,8);6,982(0, 4);6,205(2,6);5,496(4,6);3,973(16,0);3,930(1,0);3,838(1,4);3,748(1,8);3,719(3,1);3,644(2,6);3,617(1,5);3,392(15,9);3,302(1,3);3,288(1,4);2,8 12(0,4);2,177(87,3);1,964(0,9);1,958(2,0);1,952(12,3);1,946(22,7);1,940(30,9);1,934(21,1);1,927(10,8);1,276(9,1);1,100(4,5);1,085(4,4);0,9 70(0,7);0,951(0,4);0,866(0,5);0,812(7,9);0,770(11,2);0,008(1,3);0,000(39,1);-0,009(1,4) |
| Beispiel I-1-017: ¹H-NMR(400,0 MHz, CD3CN): δ= 8,450(1,3);8,446(1,4);8,438(1,3);8,434(1,4);8,409(1,7);8,403(1,8);7,842(1,5);7,679(0,7); 7,675(0,8);7,672(0,8);7,668(0,7);7,658(0,8);7,654(0,9);7,652(0,9);7,648(0,8);7,363(1,0);7,362(1,0);7,351(1,0);7,343(0,9);7,331(0,9);6,386(2, 3);4,221(1,0);4,204(2,9);4,186(3,0);4,168(1,0);4,040(14,3);3,870(0,4);3,859(0,4);3,410(16,0);2,506(0,5);2,496(0,5);2,148(26,1);1,963(0,8);1, 957(1,8);1,952(11,1);1,945(20,4);1,939(27,8);1,933(19,0);1,927(9,7);1,281(3,4);1,263(6,7);1,245(3,3);1,100(10,5);1,085(10,4);0,008(1,9);0, 000(53,5);-0,009(1,9) |
| Beispiel I-1-018: ¹H-NMR(400,0 MHz, CD3CN): δ= 8,447(1,2);8,443(1,2);8,435(1,2);8,432(1,2);8,412(1,7);8,406(1,7);7,685(0,7);7,681(0,9); 7,679(0,8);7,675(0,7);7,665(0,8);7,661(1,0);7,658(0,9);7,655(0,7);7,620(2,5);7,364(1,1);7,352(1,0);7,344(0,9);7,332(0,9);6,399(1,8);4,102(1, 0);4,072(14,7);3,922(0,9);3,880(0,4);3,864(0,5);3,849(0,4);3,806(16,0);3,727(0,5);3,416(15,9);3,386(0,6);3,310(12,0);2,167(7,9);1,964(0,4); 1,958(1,0);1,952(5,4);1,946(9,8);1,940(13,2);1,933(8,9);1,927(4,5);1,296(0,3);1,270(0,6);1,100(8,0);1,085(7,8);0,899(0,4);0,889(0,5);0,881( 0,9);0,872(0,6);0,863(0,5);0,008(0,9);0,000(24,6);-0,009(0,8) |
| Beispiel I-1-019: ¹H-NMR(400,0 MHz, CD3CN): δ= 9,659(0,5);8,445(1,4);8,442(1,4);8,434(1,4);8,430(1,4);8,411(2,0);8,405(2,1);8,238(0,4); 7,685(0,9);7,681(0,9);7,678(0,9);7,675(0,8);7,664(1,0);7,661(1,1);7,658(1,0);7,654(0,8);7,632(0,3);7,608(2,8);7,364(1,1);7,362(1,1);7,352(1, 2);7,343(1,0);7,342(1,0);7,331(0,9);7,330(0,9);6,964(0,7);6,401(3,1);4,267(1,3);4,250(3,9);4,232(4,0);4,214(1,4);4,188(0,4);4,141(0,4);4,103 (2,2);4,094(0,7);4,081(15,7);4,069(2,0);4,059(1,6);4,041(0,7);3,922(1,7);3,806(1,7);3, 715(0,9);3,427(2,0);3,416(16,0);3,385(2,2);3,302(11,4) ;3,301(11,1);2,994(5,8);2,180(13,1);1,964(0,4);1,958(0,8);1,952(5,1);1,946(9,3);1,940(12,7);1,934(8,7);1,927(4,4);1,887(0,4);1,867(0,6);1,8 46(0,4);1,318(4,4);1,300(8,5);1,282(4,4);1,267(0,5);1,262(0,3);1,249(0,3);1,235(1,8);1,217(3,4);1,199(1,8);1,131(0,4);1,114(0,6);0,898(0,7); 0,888(0,7);0,881(1,5);0,871(0,9);0,862(0,8);0,853(0,4);0,008(1,0);0,000(26,9);-0,009(0,9) |
| Beispiel I-1-020: ¹H-NMR(400,0 MHz, CD3CN): δ= 8,457(1,0);8,454(1,0);8,445(1,1);8,442(1,0);8,403(1,4);8,397(1,5);7,682(0,6);7,678(0,7); 7,676(0,7);7,672(0,6);7,662(0,7);7,658(0,9);7,655(0,9);7,651(0,7);7,372(0,9);7,360(0,9);7,352(0,8);7,340(0,8);6,982(0,6);6,242(1,5);5,880(3, 6);5,687(0,4);5,446(16,0);3,935(0,5);3,929(1,8);3,888(1,4);3,860(0,7);3,848(12,1);3,838(2,6);3,826(0,9);3,814(0,7);3,804(0,5);3,789(0,9);3,7 74(1,2);3,759(0,3);3,755(0,5);3,528(0,7);3,396(12,8);2,857(0,4);2,658(1,0);2,244(0,4);2,226(0,4);2,155(25,5);1,964(0,7);1,957(1,6);1,952(9, 0);1,945(16,5);1,939(22,4);1,933(15,3);1,927(7,9);1,496(0,3);1,352(5,4);1,337(5,4);1,298(0,5);1,288(0,5);1,273(5,6);1,259(5,4);1,233(0,9);1, 218(0,8);1,170(0,4);1,155(0,4);1,059(2,9);1,049(8,8);1,044(4,2);1,033(7,9);0,988(0,5);0,970(1,0);0,951(0,5);0,883(0,4);0,875(0,4);0,866(0,8) ;0,851(0,4);0,008(1,6);0,000(42,3);-0,009(1,8) |
| Beispiel I-1-021: ¹H-NMR(400,0 MHz, CD3CN): δ= 8,457(1,3);8,453(1,4);8,445(1,4);8,441(1,4);8,402(1,9);8,396(1,9);7,682(0,7);7,678(0,9); 7,676(0,9);7,672(0,7);7,662(1,0);7,658(1,2);7,655(1,2);7,652(1,1);7,374(1,1);7,372(1,1);7,362(1,1);7,361(1,1);7,353(1,0);7,341(1,0);6,984(0, 5);6,233(2,7);6,118(4,4);4,522(0,5);4,510(0,9);4,499(1,1);4,489(0,7);4,485(0,7);4,475(0,6);4,069(0,4);3,938(0,9);3,932(1,4);3,918(0,7);3,900 (1,2);3,895(0,8);3,885(0,6);3,881(1,1);3,879(1,1);3,875(0,9);3,858(0,7);3,839(1,9);3,828(16,0);3,798(0,4);3,773(1,0);3,730(0,6);3,428(0,4);3, 393(16,0);3,233(1,2);3,227(1,2);3,218(1,9);3,213(1,7);3,200(2,3);2,839(0,5);2,823(0,9);2,807(0,4);2,244(0,4);2,227(0,5);2,169(30,3);2,113(0 ,4);2,107(0,4);1,964(0,9);1,958(2,1);1,952(12,3);1,946(22,5);1,940(30,3);1,933(20,8);1,927(10,7);1,303(0,5);1,297(0,5);1,288(0,4);1,283(0,4);1,270(0,5);1,100(0,9);1,085(0,8);0,988(0,4);0,970(0,8);0,951(0,4);0,880(1,4);0,866(0,7);0,851(0,4);0,833(0,5);0,069(0,7);0,008(1,8);0,000( 51,3);-0,009(2,0) |
| Beispiel I-1-022: ¹H-NMR(400,0 MHz, CD3CN): δ= 5,446(16,0);3,890(2,7);3,402(3,4);2,338(1,1);2,198(7,5);1,963(0,9);1,957(2,2);1,951(11, 8);1,945(21,4);1,939(28,7);1,933(19,6);1,927(10,0);1,386(0,5);1,269(0,5);0,008(2,4);0,000(56,5);-0,009(2,1) |
| Beispiel I-1-023: ¹H-NMR(400,0 MHz, CD3CN): δ= 8,452(1,3);8,448(1,3);8,440(1,3);8,436(1,4);8,405(1,7);8,400(1,8);8,030(5,2);7,681(0,7); 7,677(0,9);7,674(0,8);7,670(0,7);7,660(0,8);7,656(1,0);7,654(1,0);7,650(0,8);7,367(1,0);7,365(1,0);7,355(1,0);7,353(1,0);7,347(0,9);7,345(0, 9);7,335(0,9);7,333(0,9);6,394(2,7);4,137(2,4);4,120(5,0);4,104(2,5);3,968(16,0);3,409(16,0);2,140(61,9);2,113(0,3);2,106(0,4);1,963(1,3);1, 957(3,1);1,951(18,5);1,945(34,0);1,939(46,3);1,933(31,7);1,927(16,2);1,738(1,1);1,721(2,3);1,703(2,4);1,685(1,2);1,100(0,6);1,085(0,6);0,9 64(3,8);0,946(7,6);0,927(3,5);0,146(0,4);0,008(3,1);0,000(86,4);-0,009(3,2);-0,150(0,4) |
| Beispiel I-1-024: ¹H-NMR(400,0 MHz, CD3CN): δ= 8,617(1,6);8,613(1,6);8,540(1,1);8,536(1,1);8,528(1,1);8,524(1,1);8,443(1,3);8,440(1,3); 8,431(1,3);8,428(1,3);8,392(1,7);8,386(1,8);8,238(0,6);8,085(5,2);7,796(0,9);7,791(0,6);7,777(1,0);7,669(0,7);7,665(0,8);7,663(0,8);7,659(0, 7);7,648(0,8);7,645(0,9);7,642(0,9);7,638(1,0);7,369(0,9);7,357(1,8);7,348(1,0);7,345(1,2);7,336(1,7);7,323(1,3);6,966(1,1);6,698(0,5);6,394 (2,5);5,448(14,5);5,219(7,2);4,104(3,4);3,923(2,8);3,912(16,0);3,723(3,5);3,401(15,9);2,185(79,4);1,964(1,1);1,958(2,7);1,952(15,5);1,946(2 8,3);1,940(38,1);1,934(25,9);1,928(13,1);1,888(0,7);1,868(0,9);1,847(0,6);1,802(0,9);1,781(1,3);1,775(0,7);1,769(0,4);1,760(0,9);1,386(0,4); 1,313(0,7);1,297(1,1);1,275(1,4);1,270(1,4);1,259(0,8);1,251(0,7);1,237(0,4);1,230(0,4);1,218(0,4);1,199(0,5);1,179(0,5);1,160(0,4);1,146(0, 4);0,899(1,2);0,890(1,9);0,882(2,7);0,872(3,3);0,863(1,4);0,854(1,6);0,008(2,7);0,000(68,5);-0,009(2,3) |
| Beispiel I-1-025: ¹H-NMR(400,0 MHz, CD3CN): δ= 8,450(1,0);8,447(1,0);8,438(1,0);8,435(1,0);8,393(1,3);8,387(1,3);7,669(0,7);7,666(0,8); 7,663(0,8);7,659(0,7);7,649(0,8);7,645(0,9);7,643(0,9);7,639(0,7);7,357(0,9);7,345(0,9);7,337(0,8);7,325(0,8);6,457(1,2);4,331(1,3);4,314(3, 9);4,296(3,9);4,278(1,3);4,052(16,0);3,880(0,8);3,864(1,0);3,849(0,8);3,420(15,8);2,762(0,8);2,751(0,8);2,197(0,5);1,972(0,4);1,953(1,5);1,9 46(2,8);1,940(3,8);1,934(2,6);1,928(1,3);1,327(4,2);1,309(8,2);1,291(4,0);1,100(12,8);1,084(12,3);0,000(6,1) |
| Beispiel I-1-026: ¹H-NMR(400,0 MHz, CD3CN): δ= 8,450(1,3);8,447(1,3);8,438(1,3);8,435(1,3);8,393(1,5);8,387(1,5);7,669(0,7);7,665(0,8); 7,663(0,8);7,659(0,7);7,649(0,8);7,645(0,9);7,642(0,9);7,638(0,7);7,359(1,0);7,357(1,0);7,347(1,0);7,345(0,9);7,338(0,9);7,337(0,8);7,326(0, 8);7,325(0,8);6,459(1,1);4,053(16,0);3,831(15,9);3,420(16,0);2,151(13,8);1,964(0,3);1,958(0,7);1,952(4,0);1,946(7,2);1,940(9,6);1,933(6,5); 1,927(3,3);0,000(3,9) |
| Beispiel I-1-027: ¹H-NMR(400,0 MHz, d₆-DMSO): δ= 8,501(2,5);8,495(2,6);8,472(1,5);8,468(1,8);8,460(1,6);8,456(1,8);8,126(6,5);7,858(0,8 );7,854(1,0);7,852(1,1);7,848(0,9);7,837(0,9);7,834(1,1);7,832(1,3);7,828(1,0);7,449(1,4);7,437(1,4);7,428(1,3);7,416(1,3);6,408(2,8);4,156( 2,4);4,140(5,0);4,123(2,5);3,938(16,0);3,393(15,1);3,323(26,1);2,506(10,3);2,502(15,0);2,498(11,8);1,672(0,5);1,655(1,7);1,637(2,1);1,618( 1,7);1,601(0,7);1,394(1,2);1,375(2,1);1,357(2,1);1,338(1,2);1,046(0,5);1,031(0,5);0,927(4,2);0,908(8,2);0,890(3,7);0,008(0,4);0,000(11,9);-0,008(0,5) |
| Beispiel I-1-028: ¹H-NMR(400,0 MHz, d₆-DMSO): δ= 11,414(0,4);10,197(0,5);8,495(3,7);8,489(3,7);8,462(2,5);8,459(2,7);8,450(2,7);8,447(2 ,7);7,927(2,7);7,847(1,3);7,843(1,6);7,841(1,6);7,837(1,4);7,826(1,5);7,823(1,7);7,820(1,8);7,816(1,5);7,440(2,0);7,428(2,3);7,419(2,5);7,40 9(12,6);7,403(7,2);7,395(7,1);7,381(1,1);7,377(1,5);7,375(1,6);7,367(1,4);7,360(1,6);7,354(1,2);7,345(1,4);7,336(0,7);7,332(0,5);6,406(4,3); 5,184(5,4);4,342(0,5);4,332(0,6);4,018(16,0);3,391(21,7);3,320(199,2);2,675(0,6);2,670(0,8);2,666(0,6);2,523(1,8);2,510(43,2);2,505(92,2); 2,501(130,4);2,497(99,4);2,492(48,9);2,332(0,5);2,328(0,8);2,323(0,6);1,235(0,9);1,045(3,6);1,030(3,5);0,146(0,3);0,008(2,5);0,000(71,1);-0,008(2,9);-0,150(0,3) |
| Beispiel I-1-029: ¹H-NMR(400,0 MHz, d₆-DMSO): δ= 9,658(1,1);8,522(0,4);8,516(0,4);8,503(1,9);8,497(1,8);8,473(0,3);8,469(0,4);8,464(1,4 );8,461(1,5);8,453(1,4);8,449(1,4);7,856(0,8);7,852(1,0);7,850(0,9);7,846(0,8);7,836(0,8);7,832(0,9);7,829(0,9);7,826(0,8);7,769(1,8);7,445( 1,1);7,434(1,2);7,425(1,0);7,413(0,9);6,424(2,6);4,220(1,0);4,203(3,4);4,185(3,5);4,167(1,1);4,051(16,0);3,906(0,9);3,889(1,5);3,870(1,0);3, 416(3,3);3,397(12,9);3,318(122,9);2,674(0,6);2,670(0,6);2,665(0,5);2,523(1,4);2,519(2,2);2,510(34,0);2,505(74,7);2,501(106,6);2,496(79,6); 2,492(37,3);2,332(0,4);2,328(0,6);2,323(0,4);1,477(0,7);1,457(1,1);1,440(0,9);1,420(0,4);1,317(0,8);1,297(1,2);1,274(3,9);1,256(7,9);1,239( 3,6);0,903(3,1);0,885(6,5);0,867(2,7);0,008(2,0);0,000(65,6);-0,009(2,2) |
| Beispiel I-1-030: ¹H-NMR(400,0 MHz, CD3CN): δ= 8,445(0,4);8,435(0,4);8,390(0,5);8,385(0,5);7,648(0,3);7,644(0,4);7,641(0,4);7,360(0,4); 7,349(0,4);7,340(0,3);6,320(0,8);4,110(6,3);3,408(6,4);2,233(8,0);1,960(0,3);1,954(2,0);1,948(3,6);1,941(5,0);1,935(3,4);1,929(1,8);1,369(1 6,0);0,000(5,5) |
| Beispiel I-1-031: ¹H-NMR(400,0 MHz, d₆-DMSO): δ= 8,510(2,3);8,504(2,4);8,474(1,6);8,470(1,7);8,462(1,7);8,459(1,7);8,353(6,3);7,864(0,8 );7,860(1,0);7,858(1,0);7,854(0,9);7,843(0,9);7,840(1,1);7,837(1,1);7,833(0,9);7,450(1,3);7,438(1,3);7,430(1,2);7,418(1,1);6,457(2,1);4,865( 1,0);4,842(3,2);4,820(3,3);4,797(1,1);3,934(16,0);3,397(15,1);3,320(41,0);2,524(0,5);2,510(12,2);2,506(26,3);2,501(37,4);2,497(28,5);2,493 (14,1);0,782(0,4);0,008(0,3);0,000(11,0);-0,008(0,4) |
| Beispiel I-1-032: ¹H-NMR(400,0 MHz, d₆-DMSO): δ= 8,503(2,3);8,497(2,4);8,462(1,6);8,459(1,9);8,450(1,7);8,447(1,9);8,315(0,4);8,224(1,0 );8,203(1,0);7,852(0,9);7,848(1,1);7,845(1,1);7,842(1,0);7,832(1,0);7,828(1,2);7,825(1,2);7,822(1,0);7,439(1,2);7,427(1,2);7,419(1,2);7,407( 1,1);6,358(2,1);4,067(16,0);4,038(0,6);4,021(0,8);4,017(0,7);4,004(0,7);4,001(0,8);3,984(0,5);3,397(14,7);3,317(58,2);3,294(0,3);3,220(0,4); 2,674(0,4);2,670(0,6);2,666(0,4);2,523(1,4);2,509(30,3);2,505(64,3);2,501(91,5);2,497(74,3);2,332(0,4);2,328(0,5);2,323(0,4);1,115(13,3);1, 099(13,3);0,008(1,0);0,000(26,9) |
| Beispiel I-1-033: ¹H-NMR(400,0 MHz, d₆-DMSO): δ= 8,519(2,0);8,513(1,9);8,497(0,5);8,484(1,6);8,481(1,7);8,472(1,5);8,469(1,5);7,871(0,8 );7,867(0,9);7,864(0,9);7,861(0,8);7,851(1,0);7,847(1,0);7,844(1,0);7,840(0,9);7,459(1,1);7,447(1,2);7,439(1,0);7,428(0,9);7,427(1,0);6,342( 1,8);3,808(1,9);3,797(16,0);3,395(14,6);3,368(2,6);3,329(7,5);2,976(15,2);2,970(14,1);2,751(0,7);2,671(0,4);2,524(0,8);2,519(1,2);2,510(17, 8);2,506(38,0);2,501(53,8);2,497(39,8);2,492(18,7);0,008(0,5);0,000(16,6);-0,008(0,5) |
| Beispiel I-1-034: ¹H-NMR(400,0 MHz, d₆-DMSO): δ= 8,499(2,6);8,493(2,6);8,467(1,8);8,458(1,7);8,455(1,8);8,143(6,2);7,850(1,1);7,846(0,9 );7,835(1,0);7,830(1,2);7,825(1,0);7,448(1,4);7,436(1,4);7,427(1,3);7,415(1,2);6,406(2,9);3,936(16,0);3,930(6,4);3,913(5,3);3,393(15,0);3,318(17,9);2,505(20,0);2,502(27,2);2,497(21,1);2,021(0,5);2,004(1,0);1,987(1,3);1,971(1,0);1,954(0,5);0,921(15,7);0,904(15,4);0,000(13,6) |
| Beispiel I-1-035: ¹H-NMR(400,0 MHz, d₆-DMSO): δ= 8,510(2,5);8,504(2,6);8,469(1,8);8,461(1,8);8,457(1,8);8,313(1,3);8,210(6,2);7,854(1,1 );7,835(1,2);7,449(1,4);7,437(1,4);7,429(1,3);7,417(1,2);6,433(2,6);4,808(5,5);4,802(5,6);3,948(16,0);3,553(1,5);3,547(2,9);3,542(1,4);3,397 (15,3);3,314(157,0);2,670(2,9);2,505(345,7);2,501(467,9);2,497(358,0);2,328(2,7);0,146(0,8);0,008(7,0);0,000(166,0);-0,149(0,8) |
| Beispiel I-1-036: ¹H-NMR(400,0 MHz, d₆-DMSO): δ= 8,499(1,1);8,493(1,1);8,464(0,8);8,461(0,9);8,452(0,9);8,449(0,9);7,851(0,4);7,847(0,5 );7,845(0,5);7,841(0,5);7,831(0,5);7,827(0,5);7,824(0,6);7,820(0,5);7,442(0,6);7,430(0,6);7,421(0,6);7,409(0,5);6,405(1,5);4,034(5,8);3,828( 4,0);3,392(7,2);3,319(7,3);2,511(5,5);2,507(11,5);2,502(16,0);2,497(11,9);2,493(5,7);1,046(2,9);1,030(2,8);0,939(16,0);0,000(8,5) |
| Beispiel I-1-037: ¹H-NMR(400,0 MHz, d₆-DMSO): δ= 8,505(0,9);8,500(0,9);8,471(0,6);8,468(0,7);8,459(0,7);8,456(0,7);8,038(2,4);7,862(0,3 );7,858(0,4);7,856(0,4);7,852(0,4);7,842(0,4);7,838(0,4);7,835(0,5);7,832(0,4);7,451(0,5);7,450(0,5);7,439(0,5);7,438(0,5);7,431(0,4);7,430( 0,4);7,419(0,4);7,418(0,4);6,395(1,0);5,754(2,1);3,965(6,1);3,397(5,8);3,318(5,4);2,511(3,4);2,506(6,9);2,502(9,5);2,498(7,4);1,312(16,0);0, 000(4,7) |
| Beispiel I-1-038: ¹H-NMR(400,0 MHz, d₆-DMSO): δ= 8,497(1,0);8,490(1,0);8,460(0,7);8,456(0,8);8,448(0,8);8,445(0,8);7,846(0,4);7,843(0,4 );7,840(0,4);7,836(0,4);7,826(0,4);7,822(0,5);7,820(0,5);7,816(0,4);7,590(1,6);7,440(0,5);7,428(0,5);7,419(0,5);7,408(0,5);6,430(1,4);4,069( 6,7);3,397(6,6);3,319(5,4);3,240(5,0);2,511(4,3);2,506(8,9);2,502(12,3);2,497(9,2);2,493(4,4);1,485(16,0);0,000(5,9) |
| Beispiel I-1-039: ¹H-NMR(400,0 MHz, d₆-DMSO): δ= 9,644(5,2);8,503(1,6);8,500(1,7);8,488(3,7);8,481(2,3);7,866(0,8);7,862(1,0);7,860(1,0 );7,856(0,9);7,845(0,9);7,841(1,1);7,839(1,1);7,835(0,9);7,464(1,2);7,452(1,2);7,444(1,2);7,432(1,1);6,622(0,8);4,092(0,5);4,079(0,6);4,046( 16,0);3,921(1,0);3,903(3,1);3,886(3,2);3,868(1,1);3,802(0,8);3,315(37,9);3,283(0,8);3,175(2,0);3,162(1,9);2,670(0,5);2,666(0,4);2,523(1,1);2 ,506(60,9);2,501(84,1);2,497(63,5);2,332(0,4);2,328(0,5);2,324(0,4);1,140(3,3);1,122(7,1);1,104(3,3);1,045(0,4);1,030(0,4);0,008(2,1);0,000 (57,9);-0,008(2,5) |
| Beispiel I-1-040: ¹H-NMR(400,0 MHz, d₆-DMSO): δ= 8,487(1,5);8,483(1,6);8,475(1,6);8,472(1,6);8,443(2,2);8,437(2,2);7,822(0,8);7,818(1,0 );7,816(0,9);7,812(0,8);7,802(0,9);7,798(1,0);7,796(1,1);7,792(0,9);7,455(1,3);7,443(1,2);7,435(1,2);7,423(1,1);6,153(2,7);5,509(5,2);3,931( 16,0);3,876(0,9);3,858(2,8);3,840(2,9);3,822(0,9);3,701(1,7);3,674(3,7);3,636(3,3);3,609(1,4);3,317(18,0);3,175(0,4);3,162(0,4);2,523(0,8);2 ,510(16,2);2,506(33,7);2,501(46,9);2,497(35,2);2,492(17,1);1,228(9,9);1,120(3,2);1,102(7,2);1,084(3,1);0,744(10,2);0,000(7,5) |
| Beispiel I-1-041: ¹H-NMR(400,0 MHz, d₆-DMSO): δ= 8,869(7,0);8,866(10,3);8,853(10,1);8,850(8,0);8,524(3,7);8,520(3,3);8,512(1,8);8,508(1 ,7);8,448(1,4);8,444(2,6);8,441(1,5);8,429(2,9);8,425(5,5);8,421(3,1);8,409(1,6);8,406(3,0);8,402(1,7);8,120(5,6);8,115(1,1);7,943(7,1);7,92 7(7,8);7,924(7,9);7,908(7,5);7,894(1,0);7,890(1,0);7,888(1,1);7,884(0,8);7,515(1,1);7,503(1,1);7,495(1,0);7,482(1,0);6,569(0,4);6,486(2,9);4, 453(0,4);4,438(1,1);4,422(1,5);4,406(1,1);4,391(0,4);3,951(16,0);3,913(0,9);3,896(2,8);3,878(2,9);3,860(0,9);2,528(0,4);2,523(0,7);2,514(15 ,4);2,510(33,9);2,505(48,6);2,500(37,9);2,496(19,5);1,790(0,3);1,751(0,4);1,264(15,1);1,248(15,1);1,233(1,7);1,224(0,4);1,217(1,7);1,202(0, 4);1,151(0,4);1,134(3,4);1,117(7,3);1,099(3,2);0,000(10,6);-0,008(0,5) |
| Beispiel I-1-042: ¹H-NMR(400,0 MHz, d₆-DMSO): δ= 8,837(1,4);8,825(1,4);8,513(1,6);8,510(1,8);8,502(2,8);8,498(3,5);8,367(0,6);8,347(0,3 );8,165(6,2);7,889(1,3);7,885(1,1);7,882(1,3);7,879(1,6);7,875(1,3);7,869(1,3);7,862(1,4);7,859(1,6);7,497(1,1);7,484(1,1);7,477(1,0);7,465( 0,9);6,457(2,4);3,985(4,5);3,967(4,6);3,947(16,0);3,909(0,9);3,891(2,7);3,873(2,8);3,855(0,9);3,814(0,4);3,809(0,9);3,790(0,9);3,140(0,4);2, 525(0,6);2,520(1,0);2,511(16,7);2,507(35,5);2,502(49,8);2,498(37,0);2,493(17,8);1,172(0,5);1,165(0,4);1,153(0,8);1,145(0,4);1,141(0,5);1,1 33(3,4);1,115(7,1);1,097(3,0);0,746(0,5);0,563(0,4);0,558(0,8);0,547(1,9);0,542(1,9);0,538(0,8);0,532(0,9);0,527(1,9);0,522(1,7);0,512(0,7); 0,313(0,7);0,302(2,3);0,299(2,2);0,290(2,0);0,287(2,4);0,276(0,6);0,000(10,5);-0,009(0,4) |
| Beispiel I-1-043: ¹H-NMR(400,0 MHz, d₆-DMSO): δ= 8,766(1,2);8,762(1,7);8,759(0,8);8,754(0,8);8,750(1,7);8,747(1,3);8,501(1,5);8,498(1,7 );8,490(1,7);8,486(1,8);8,480(2,2);8,474(2,1);8,215(0,5);8,200(0,5);8,195(1,0);8,191(0,5);8,176(0,5);8,138(6,7);7,864(0,8);7,860(0,9);7,857( 0,9);7,853(0,8);7,843(0,9);7,839(1,0);7,837(1,0);7,833(0,9);7,749(1,3);7,745(0,8);7,737(0,8);7,733(1,4);7,729(1,4);7,726(0,7);7,718(0,6);7,7 14(1,2);7,477(1,2);7,465(1,1);7,456(1,1);7,444(1,0);6,408(2,8);4,113(2,6);4,096(5,3);4,079(2,7);3,937(16,0);3,925(0,4);3,903(0,9);3,886(2,7) ;3,868(2,8);3,850(0,9);2,512(9,4);2,507(21,1);2,502(30,4);2,498(23,2);2,493(11,5);1,695(1,1);1,678(2,3);1,659(2,5);1,642(1,2);1,624(0,4);1, 131(3,0);1,113(7,0);1,095(3,0);0,932(4,2);0,913(8,6);0,895(4,1);0,000(6,5) |
| Beispiel I-1-044:¹H-NMR(400,0 MHz, d₆-DMSO): δ= 8,753(7,9);8,749(11,7);8,742(6,2);8,738(11,6);8,734(9,5);8,502(1,6);8,499(1,7);8,490(1 ,7);8,487(1,9);8,482(2,4);8,476(2,3);8,184(1,6);8,180(3,0);8,176(1,8);8,165(3,2);8,160(6,3);8,156(3,7);8,145(1,9);8,141(3,6);8,137(2,4);8,13 3(6,4);7,865(0,8);7,861(1,0);7,858(1,0);7,854(0,9);7,844(0,9);7,840(1,1);7,838(1,2);7,834(1,0);7,719(8,5);7,716(5,8);7,707(5,3);7,704(9,4);7, 700(9,5);7,696(5,5);7,688(4,6);7,684(8,0);7,477(1,2);7,465(1,2);7,457(1,1);7,445(1,0);6,406(3,0);3,942(15,0);3,925(16,0);3,905(0,9);3,899(0 ,5);3,887(2,7);3,870(2,8);3,852(0,9);3,787(0,6);2,511(21,4);2,506(30,6);2,502(24,6);1,189(0,7);1,132(2,9);1,114(6,5);1,097(2,9);0,000(6,6) |
| Beispiel I-1-045: ¹H-NMR(400,0 MHz, d₆-DMSO): δ= 9,645(0,3);8,482(1,7);8,478(1,7);8,470(1,8);8,466(2,0);8,462(2,3);8,456(2,2);7,834(0,8 );7,830(1,0);7,827(0,9);7,824(0,8);7,813(0,9);7,810(1,0);7,807(1,1);7,803(0,9);7,667(3,3);7,453(1,2);7,443(1,2);7,433(1,1);7,422(1,0);6,390( 3,1);5,754(15,0);4,047(1,4);4,039(14,9);3,907(0,9);3,889(2,7);3,871(2,7);3,854(0,9);3,771(16,0);3,320(18,7);3,303(11,7);2,524(0,3);2,520(0, 5);2,511(8,3);2,507(17,7);2,502(24,8);2,497(18,4);2,493(8,8);1,138(3,2);1,120(7,2);1,102(3,1);0,000(1,8) |
| Beispiel I-1-046: ¹H-NMR(400,0 MHz, d₆-DMSO): δ= 8,501(2,9);8,495(3,4);8,479(0,6);8,461(1,7);8,458(1,7);8,449(1,7);8,447(1,7);7,846(0,9 );7,843(1,1);7,837(0,8);7,826(1,0);7,822(1,2);7,817(0,9);7,438(1,3);7,426(1,3);7,418(1,3);7,406(1,2);6,375(2,1);5,753(15,0);4,073(16,0);3,39 9(14,8);3,320(114,7);3,232(0,6);3,214(1,9);3,198(2,5);3,181(1,9);3,163(0,6);2,671(0,4);2,506(49,4);2,502(67,2);2,498(50,6);2,328(0,4);1,06 8(3,7);1,050(7,7);1,032(3,6);0,985(0,3);0,967(0,7);0,008(1,8);0,000(49,4) |
| Beispiel I-1-047: ¹H-NMR(400,0 MHz, d₆-DMSO): δ= 10,368(2,3);8,540(2,3);8,534(2,3);8,479(1,6);8,476(1,8);8,467(1,9);8,464(1,9);7,882(0, 8);7,879(1,0);7,876(1,0);7,872(0,9);7,862(1,0);7,858(1,1);7,856(1,1);7,852(0,9);7,766(2,7);7,747(3,0);7,455(1,3);7,443(1,3);7,434(1,3);7,424 (1,3);7,338(1,8);7,319(2,9);7,298(2,0);7,110(1,0);7,091(1,8);7,073(0,8);6,490(1,6);5,754(4,6);4,115(16,0);3,425(14,3);3,319(19,4);2,688(0,4) ;2,675(0,5);2,524(0,4);2,510(9,1);2,506(18,9);2,502(26,3);2,497(19,5);2,493(9,3);1,988(1,1);1,175(0,6);0,008(0,8);0,000(21,3);-0,008(0,7) |
| Beispiel I-1-048: ¹H-NMR(400,0 MHz, d₆-DMSO): δ= 9,068(0,6);9,052(1,2);9,035(0,6);8,520(2,3);8,513(2,4);8,468(1,6);8,465(1,7);8,457(1,7 );8,453(1,7);7,859(0,8);7,855(1,0);7,853(1,0);7,849(0,9);7,839(1,0);7,835(1,1);7,832(1,1);7,829(0,9);7,443(1,3);7,431(1,3);7,423(1,2);7,411( 1,2);6,457(1,5);5,754(1,6);4,081(16,0);4,006(0,4);3,982(1,3);3,966(1,3);3,958(1,4);3,942(1,3);3,935(0,6);3,918(0,4);3,411(14,8);3,322(16,4); 2,511(5,5);2,507(11,5);2,502(16,0);2,498(11,8);2,494(5,6);0,008(0,4);0,000(13,5);-0,008(0,5) |
| Beispiel I-1-049: ¹H-NMR(600,1 MHz, d₆-DMSO): δ= 12,924(0,6);9,123(0,6);8,507(4,0);8,476(2,9);8,469(3,0);7,869(2,1);7,856(2,3);7,591(2, 4);7,450(1,7);7,442(1,9);7,439(2,0);7,430(1,7);6,652(1,5);4,218(4,2);4,109(1,3);4,065(0,3);4,021(0,4);3,949(15,9);3,947(15,3);3,827(0,4);3,5 29(4,9);3,497(1,5);3,416(15,8);3,397(1,1);3,375(0,6);3,359(0,6);3,324(12,4);3,322(11,9);3,296(0,6);3,253(4,6);3,247(16,0);2,613(0,4);2,503( 19,0);2,501(19,3);0,000(0,9);-0,003(0,9) |
| Beispiel I-1-050: ¹H-NMR(400,0 MHz, d₆-DMSO): δ= 9,060(0,8);9,045(1,5);9,030(0,8);8,511(2,9);8,505(2,9);8,459(2,2);8,447(2,1);7,846(1,4 );7,842(1,2);7,826(1,5);7,822(1,2);7,438(1,5);7,426(1,6);7,418(1,5);7,406(1,3);7,316(0,8);7,296(3,4);7,280(9,2);7,262(1,4);7,237(1,0);7,232( 1,1);7,216(1,4);7,208(0,6);7,200(0,5);6,405(2,4);4,375(3,9);4,359(3,9);4,080(16,0);3,401(15,0);3,375(0,4);3,318(75,0);2,670(0,5);2,501(88,6 );2,497(73,6);2,327(0,5);1,091(0,5);0,146(0,4);0,006(9,7);0,000(73,6);-0,150(0,4) |
| Beispiel I-1-051: ¹H-NMR(400,0 MHz, d₆-DMSO): δ= 8,490(1,6);8,487(1,6);8,478(1,7);8,475(1,6);8,461(2,2);8,454(2,2);8,107(6,1);7,841(0,8 );7,837(1,0);7,834(0,9);7,831(0,8);7,820(1,0);7,816(1,1);7,814(1,0);7,810(0,9);7,459(1,2);7,448(1,2);7,440(1,1);7,428(1,0);6,365(2,7);4,207( 1,3);4,190(4,2);4,172(4,3);4,154(1,4);3,938(16,0);3,899(0,9);3,881(2,8);3,864(2,9);3,846(0,9);3,320(27,9);2,524(0,5);2,511(12,4);2,506(26,2 );2,502(36,5);2,497(27,1);2,493(13,0);1,270(4,3);1,253(9,0);1,235(4,2);1,130(3,2);1,112(7,2);1,094(3,1);0,008(1,3);0,000(40,6);-0,008(1,5) |
| Beispiel I-1-052: ¹H-NMR(400,0 MHz, d₆-DMSO): δ= 8,499(2,1);8,493(2,1);8,471(1,5);8,467(1,6);8,459(1,6);8,455(1,6);8,072(6,1);7,858(0,9 );7,854(1,0);7,851(1,0);7,847(0,9);7,837(1,0);7,833(1,1);7,831(1,1);7,827(0,9);7,450(1,2);7,438(1,2);7,429(1,1);7,417(1,0);6,398(2,3);4,763( 0,6);4,757(0,8);4,749(1,2);4,742(0,8);4,735(0,6);3,941(16,0);3,393(15,0);3,319(27,3);2,524(0,6);2,511(13,1);2,506(26,4);2,502(35,8);2,497( 26,7);2,493(13,1);1,824(0,6);1,808(1,5);1,802(1,2);1,794(2,3);1,789(2,1);1,779(1,8);1,668(0,4);1,657(0,7);1,648(0,8);1,638(1,0);1,629(0,8);1 ,621(0,9);1,598(0,6);1,590(0,6);1,573(1,0);1,555(1,1);1,535(0,6);0,008(1,7);0,000(42,7);-0,008(1,6) |
| Beispiel I-1-053: ¹H-NMR(400,0 MHz, d₆-DMSO): δ= 8,517(2,4);8,511(2,5);8,484(1,7);8,481(1,8);8,472(1,8);8,469(1,8);7,864(1,0);7,861(1,1 );7,858(1,0);7,844(1,0);7,841 (1,2);7,838(1,1);7,458(1,3);7,446(1,3);7,438(1,2);7,426(1,2);6,346(1,3);6,329(1,1);5,753(2,9);4,053(0,9);3,870( 0,5);3,865(0,6);3,793(7,0);3,784(9,3);3,467(0,5);3,449(1,4);3,432(1,8);3,413(0,5);3,395(15,5);3,352(0,6);3,335(1,6);3,317(24,5);3,300(0,6);3 ,001(0,5);2,968(0,5);2,944(16,0);2,732(0,4);2,524(0,4);2,506(21,9);2,502(30,2);2,497(22,7);1,119(1,5);1, 102(3,2);1,084(1,6);1,077(1,9);1,06 0(3,9);1,042(1,7);0,008(1,2);0,000(36,5);-0,008(1,5) |
| Beispiel I-1-054: ¹H-NMR(400,0 MHz, d₆-DMSO): δ= 8,504(3,0);8,498(2,9);8,475(2,2);8,463(2,3);7,851(1,5);7,848(1,3);7,834(1,4);7,831(1,6 );7,451(1,5);7,439(1,6);7,430(1,5);7,418(1,4);6,385(2,8);5,753(0,8);4,052(0,4);3,979(0,4);3,903(16,0);3,588(1,4);3,572(3,7);3,556(1,4);3,460 (1,4);3,443(3,5);3,434(1,7);3,398(15,4);3,316(26,9);2,505(45,3);2,502(52,5);1,809(5,8);1,238(0,3);0,000(43,4) |
| Beispiel I-1-055: ¹H-NMR(400,0 MHz, d₆-DMSO): δ= 8,520(2,2);8,514(2,2);8,487(1,5);8,483(1,7);8,475(1,6);8,471(1,7);7,871(0,8);7,867(1,0 );7,865(1,0);7,861(0,8);7,851(0,9);7,847(1,0);7,844(1,0);7,840(0,9);7,461(1,2);7,449(1,2);7,440(1,1);7,428(1,0);6,334(2,0);5,753(1 ,3);3,784( 16,0);3,600(0,5);3,581(1,3);3,567(1,9);3,554(1,2);3,420(0,5);3,393(15,3);3,371(1,4);3,319(92,8);2,675(0,3);2,671(0,4);2,524(0,8);2,510(19, );2,506(41,4);2,501(59,0);2,497(45,3);2,493(22,5);2,328(0,3);1,603(1,2);1,591(1,1);1,576(0,6);1,529(1,1);1,519(1,2);1,488(0,6);1,471(1,0);1, 461(1,3);1,433(0,3);1,236(0,4);0,008(0,8);0,000(28,7);-0,008(1,1) |
| Beispiel I-1-056: ¹H-NMR(400,0 MHz, d₆-DMSO): δ= 10,542(0,8);8,520(2,4);8,514(2,5);8,475(1,6);8,472(1,9);8,463(1,8);8,460(1,9);7,877(0, 9);7,874(1,1);7,871(1,1);7,867(1,0);7,857(1,0);7,853(1,2);7,851(1,3);7,847(1,1);7,456(1,3);7,444(1,3);7,435(1,2);7,423(1,2);6,371(2,6);5,752 (0,8);4,021(16,0);3,651(0,6);3,633(1,9);3,619(2,1);3,615(2,3);3,601(1,9);3,583(0,6);3,397(15,0);3,320(19,5);2,697(0,4);2,693(0,4);2,506(22, 0);2,501(30,7);2,497(24,7);1,171(3,8);1,153(7,9);1,135(3,6);0,000(19,3) |
| Beispiel I-1-057: ¹H-NMR(400,0 MHz, d₆-DMSO): δ= 8,502(2,3);8,495(2,3);8,462(1,5);8,458(1,7);8,450(1,7);8,446(1,7);8,300(1,0);8,281(1,0 );7,850(0,8);7,846(1,0);7,843(1,0);7,839(0,9);7,829(1,0);7,825(1,0);7,823(1,1);7,819(0,9);7,436(1,3);7,424(1,3);7,416(1,2);7,404(1,1);6,357( 2,0);5,754(3,6);4,158(0,4);4,140(0,6);4,122(0,6);4,104(0,4);4,064(16,0);3,398(14,3);3,322(19,2);2,511 (5,4);2,507(11,3);2,502(15,9);2,498(1 2,1);2,493(5,9);1,826(0,5);1,818(0,9);1,808(1,0);1,799(1,0);1,789(0,7);1,637(1,4);1,617(0,5);1,606(0,5);1,537(0,5);1,520(0,9);1,514(0,9);1,5 01(1,8);1,494(2,1);1,486(2,5);1,477(1,2);1,466(0,7);1,459(0,5);0,008(0,7);0,000(20,5);-0,008(0,8) |
| Beispiel I-1-058: ¹H-NMR(400.0 MHz, d₆-DMSO):δ= 8.516(2.29);8.51(2.4);8.484(1.66);8.48(1.65);8.472(1.75);8.469(1.64); 7.864(0.82);7.861(1.06);7.858(0.96);7.854(0.84);7.844(0.97);7.84(1.16);7.838(1.09);7 .834(0.88);7.457(1.24);7.445(1.26);7.437(1.18);7.425( 1.13);6.332(2.26);3.776(16);3.437(0.91);3.42(2.93);3.402(3.46);3.394(15.4);3.32(18.54);3.31(2.85);3.292(2.51);3.274(0.84);2.506(37.54);2.5 02(48.63);2.497(34.94);1.355(1.26);1.235(0.35);1.135(3.21);1.118(6.86);1.1(3.11);1.053(2.91);1.035(6.36);1.018(2.83);0.008(2.09);0(53.85) ;-0.008(2.47) |
| Beispiel I-1-059: ¹H-NMR(400.0 MHz, d₆-DMSO):δ= 8.52(1.81);8.514(1.88);8.485(1.28);8.481(1.36);8.473(1.36);8.47(1.35); 7.871(0.79);7.868(0.97);7.865(0.91);7.861(0.81);7.851(0.92);7.847(1.04);7.845(1.04);7.841(0.88);7.459(1.13);7.447(1.14);7.439(1.08);7.427 (1.04);6.364(1.85);3.834(16);3.617(3.41);3.61(3.49);3.558(6.71);3.396(14.76);3.328(6.11);2.671(0.35);2.524(0.83);2.51(20.54);2.506(43.69) ;2.501(61.09);2.497(45.13);2.493(21.49);2.328(0.35);0(3.46) |
| Beispiel I-1-060: ¹H-NMR(400.0 MHz, d₆-DMSO):δ= 10.653(2.37);8.93(2.26);8.924(2.27);8.54(2.29);8.534(2.3);8.479(1.63); 8.475(1.74);8.467(1.71);8.464(1.72);8.314(0.33);8.305(1.57);8.302(1.73);8.293(1.66);8 91);8.148(1.06);8.145(0.81);7.883(0.85);7.879(1.02);7.877(0.98);7 .873(0.89);7.863(0.98);7.859(1.05);7.856(1.12);7.853(0.94);7.455(1.22);7. 444(1.22);7.434(1.16);7.423(1.09);7.378(1.18);7.367(1.16);7.358(1.14);7.346(1.09);6.517(1.38);5.754(3.01);4.119(16);3.428(14.23);3.318(6 1.58);2.675(0.59);2.67(0.8);2.666(0.61);2.524(2.47);2.51 (47.91);2.506(97.52);2.501 (133.97);2.497(101.67);2.493(50.97);2.333(0.55);2.328(0.77);2.324(0.58);0.008(1.25);0(33.18);-0.008(1.35) |
| Beispiel I-1-061: ¹H-NMR(400.0 MHz, d₆-DMSO):δ= 8.5(2.49);8.494(2.7);8.46(2.18);8.457(2.35);8.449(2.89);8.445(2.69); 8.315(1.01);7.843(0.92);7.839(1.18);7.833(1);7.823(1.15);7.819(1.35);7.813(1.09);7.437(1.33);7.425(1.41);7.417(1.36);7.405(1.28);6.384(2. 18);4.071(16);3.398(15.08);3.375(0.67);3.32(30.36);3.301(1.18);2.702(6.94);2.69(7.44);2.67 4(1.52);2.506(47.2);2.502(63.65);2.497(50.7 4);2 .328(0.38);1.091(0.45);0(38.49);-0.018(1.17) |
| Beispiel I-1-062: ¹H-NMR(400.0 MHz, d₆-DMSO):δ= 8.497(3.06);8.49(3.45);8.456(1.56);8.452(1.73);8.444(1.65);8.441(1.72); 7.844(0.82);7.841(0.98);7.838(1);7.834(0.86);7.824(0.93);7.82(1.06);7.818(1.13);7.814(0.92);7.431(1.3);7.419(1.33);7.41 (1.21);7.399(1.15); 6.349(1.95);5.754(2.46);4.065(16);3.697(0.44);3.396(14.78);3.32(4.81);3.242(0.36);2.799(0.4);2.79(0.51);2.779(0.81);2.769(0.79);2.764(0.5 9);2.759(0.45);2.751(0.43);2.506(21.83);2.502(30.69);2.497(23.77);0.836(0.37);0.657(0.43);0.638(1.94);0.627(1.25);0.623(1.7);0.618(1.72); 0.61(1.49);0.595(0.55);0.59(1.03);0.58(2.97);0.571(1.76);0.566(1.51);0.551(0.53);0.008(0.76);0(22.4);-0.008(0.95) |
| Beispiel I-1-063: ¹H-NMR(400.0 MHz, d₆-DMSO):δ= 8.548(0.52);8.533(1.01);8.517(0.57);8.508(2.27);8.502(2.24);8.465(1.54); 8.461(1.73);8.453(1.65);8.45(1.72);7.853(0.79);7 .85(0.96);7.847(0.96);7.843(0.86);7.833(0.9);7.829(1.03);7.827(1.09);7 .823(0.92);7.441(1.2 );7.43(1.2);7.421(1.12);7.409(1.07);6.386(1.85);5.757(0.39);4.081(16);3.402(14.66);3.392(0.52);3.321(8.91);3.061 (1.93);3.045(3.26);3.029( 1.95);2.687(1.22);2.674(1.28);2.51(7.43);2.506(15.5);2.502(20.72);2.497(15.16);2.493(7.48);1.091(0.46);1(0.44);0.995(0.42);0.991(0.38);0.9 83(0.75);0.974(0.37);0.971(0.45);0.963(0.47);0.403(0.62);0.392(1.9);0.388(2.09);0.384(1.02);0.378(0.99);0.372(1.98);0.368(1.94);0.358(0.7 8);0.21(0.77);0.2(2.24);0.197(2.38);0.188(2.01);0.185(2.39);0.174(0.58);0.008(0.61);0(17.49);-0.008(0.67) |
| Beispiel I-1-064: ¹H-NMR(400.0 MHz, d₆-DMSO):δ= 8.505(1.81);8.499(1.83);8.463(1.34);8.46(1.27);8.451(1.39);8.448(1.31); 8.376(0.45);8.36(0.82);8.349(0.43);7.849(0.69);7.846(0.84);7.843(0.78);7.839(0.66);7.829(0.79);7.825(0.89);7.823(0.87);7.819(0.69);7.441( 1.02);7.429(1.02);7.42(0.94);7.409(0.94);6.389(1.46);4.077(12.96);3.411(0.87);3.4(13.8);3.385(2.41);3.364(1.24);3.35(2.22);3.336(1.78);3.3 18(101,32);3.226(16);2.675(1.85);2.67(2.48);2.666(1.8);2.523(6.31);2.51(160.29);2.506(317.13);2.501(406.88);2.497(286.18);2.332(1.73);2. 328(2.38);2.324(1.74);0.008(1.55);0(51.09);-0.008(1.82) |
| Beispiel I-1-065: ¹H-NMR(400.0 MHz, d₆-DMSO):δ= 8.512(2.18);8.506(2.28);8.481(0.52);8.466(2.53);8.463(2.31);8.454(2.04); 8.451(2.12);7.86(0.82);7.856(0.98);7.853(0.91);7 .849(0.83);7.839(0.95);7.835(1.05);7.833(1.05);7.829(0.88);7.445(1.22);7.433(1.22);7.424( 1.15);7.413(1.11);6.381(1.85);5.758(1.2);4.071(16);3.401(14.57);3.322(6.32);3.016(1.96);3(3.26);2.983(2);2.52(0.35);2.511(6.35);2.507(13. 28);2.502(17.55);2.498(12.4);2.494(5.89);1.825(0.37);1.808(0.76);1.791(0.97);1.774(0.79);1.757(0.4);0.836(14.45);0.819(13.81);0(7.63) |
| Beispiel I-1-066: ¹H-NMR(400.0 MHz, d₆-DMSO):δ= 8.498(0.62);8.48(2.35);8.467(4.14);7.833(0.84);7.829(1.1);7.824(0.85); 7.813(0.96);7.808(1.21);7.803(0.91);7.448(1.31);7.436(1.32);7.428(1.24);7.416(1.17);6.342(2.4);4.067(16);3.905(0.95);3.887(3.2);3.869(3.1 7);3.852(1.01);3.321(9.18);3.225(0.55);3.207(1.94);3.19(2.61);3.173(2);3.156(0.59);2.506(23.64);2.502(30.7);2.498(22.32);1.131(3.46);1.11 3(7.51);1.096(3.39);1.062(3.9);1.044(8.18);1.026(3.89);0(9.12) |
| Beispiel I-1-067: ¹H-NMR(400.0 MHz, d₆-DMSO):δ= 8.532(1.26);8.526(1.28);8.49(1.08);8.486(1.08);8.478(1.14);8.475(1.1); 7.896(0.55);7.892(0.65);7.889(0.62);7.885(0.56);7.875(0.61);7.871(0.7);7.869(0.7);7.865(0.6);7.465(0.8);7.453(0.81);7.444(0.78);7.432(0.71 );4.07(0.36);4.028(0.42);4.016(0.38);4.004(10.57);3.793(0.9);3.432(0.51);3.422(9.97);3.411(0.42);3.363(0.41);3.318(16.13);2.964(0.55);2.67 (0.41);2.524(0.93);2.519(1,47);2.51(26.06);2.506(54.51);2.501(72.19);2.497(51.1);2.492(24.09);2.328(0.42);1.645(16);0.008(0.74);0(26.03); -0.008(0.89) |
| Beispiel I-1-068: ¹H-NMR(400.0 MHz, d₆-DMSO):δ= 8.506(2.91);8.503(2.98);8.494(3.17);8.491(3.06);8.481(4.48);8.474(4.6); 7.847(1.79);7.826(1.99);7.47(2.31);7.458(2.32);7.45(2.18);7.438(2.08);6.289(2.56);6.274(2.08);5.757(1.11);3.896(1.66);3.878(5.33);3.86(5.5 6);3.843(1.75);3.789(12.3);3.781(15.69);3.46(0.79);3.443(2.5);3.425(2.55);3.407(0.84);3.337(0.98);3.323(14.78);3.302(2.91);3.285(0.95);2.9 38(15.41);2.931(12.62);2.506(24.59);2.502(31.9);2.498(23.24);1.132(6.18);1.114(16);1.096(11.71);1.079(2.89);1.067(3.32);1.049(6.9);1.032 (3.11);0.008(1.58);0(42.08);-0.008(1.95) |
| Beispiel I-1-069: ¹H-NMR(400.0 MHz, d₆-DMSO):δ= 8.523(5.67);8.517(5.71);8.5(0.45);8.488(4.18);8.484(4.18);8.476(4.29); 8.472(4.19);8.46(0.48);7.873(2.06);7.869(2.55);7.863(2.1);7.853(2.35);7.847(2.78);7.843(2.28);7.463(3.07);7.451(3.29);7.443(2.99);7.431(2. 94);7.25(0.41);7.232(0.37);7.182(0.45);7.163(0.38);6.871(0.34);6.362(3.93);6.277(0.4);5.756(0.39);4.838(0.52);4.458(0.62);4.01 (1.32);3.979 (0.38);3.902(0.36);3.812(13.52);3.746(1.98);3.696(0.35);3.648(16);3.57(0.34);3.444(0.48);3.407(2.37);3.396(36.96);3.384(2.78);3.364(0.49) ;3.348(0.39);3.321(39.44);3.282(11.34);2.671(0.57);2.628(1.6);2.506(71.56);2.502(92.32);2.497(66.63);2.48(2.04);2.329(0 .51) ;2.324(0 .39);2 .3(1.69);2.184(0.58);1.356(3.65);1.236(0.65);0.854(0.36);0.836(0.35);0.146(0.48);0.008(4.65);0(103.54);-0.008(4.8);-0.15(0.5) |
| Beispiel I-1-070: ¹H-NMR(400.0 MHz, d₆-DMSO):δ= 8.527(2.59);8.522(2.65);8.477(1.96);8.475(1.9);8.465(2.12);7.866(1.35); 7.849(1.28);7.846(1.52);7.449(1.48);7.437(1.5);7.429(1.44);7.417(1.32);6.806(0.51);6.587(1.13);5.756(11.35);4.094(0.39);4.066(0.93);4.054 (16);4.024(0.39);3.913(0.34);3.802(0.45);3.413(16.78);3.399(0.84);3.253(12.85);2.909(4.27);2.671(0.41);2.506(54.35);2.501(69.09);2.497(4 9.93);2.328(0.4);1.236(1.05);0.85(0.47);0.008(1.53);0(39.75) |
| Beispiel I-1-071: ¹H-NMR(400.0 MHz, d₆-DMSO):δ= 8.486(2.11);8.48(2.18);8.468(1.61);8.464(1.6);8.456(1.68);8.452(1.54); 7.841(0.81);7.837(1);7.835(0.9);7.831(0.78);7.821(0.92);7.817(1.09);7.814(1.02);7.811(0.81);7.443(1.16);7.431(1.16);7.423(1.06);7.411(1.0 3);6.437(1.66);4.38(1.43);4.362(2.32);4.342(1.48);4.02(16);4.008(1.67);3.99(2.27);3.97(1.53);3.392(14.92);3.32(36.59);2.67(0.37);2.524(0.9 6);2.506(50.78);2.502(65.6);2.497(46.28);2.328(0.38);2.23(0.5);2.211(1.21);2.192(1.94);2.173(1.14);2.153(0.47);1.235(0.46);0.146(0.32);0.0 08(2.8);0(77.28);-0.008(2.93);-0.15(0.34) |
| Beispiel I-1-072: ¹H-NMR(400.0 MHz, d₆-DMSO):δ= 8.506(0.85);8.502(0.87);8.494(0.92);8.49(0.9);8.482(1.2);8.476(1.23); 8.317(0.49);7.854(0.43);7.85(0.55);7.847(0.5);7.844(0.43);7.833(0.5);7.829(0.6);7.827(0.57);7.823(0.46);7.471(0.64);7.459(0.65);7.451(0.61 );7.439(0.58);6.285(1.21);5.757(2.13);3.897(0.48);3.879(1.56);3.861 (1.58);3.844(0.5);3.796(8.55);3.324(1.32);2.966(16);2.507(5.63);2.503( 7.28);2.498(5.18);1.132(1.76);1.115(3.89);1.097(1.69);0.008(0.45);0(10.99);-0.008(0.44) |
| Beispiel I-1-073: ¹H-NMR(400.0 MHz, d₆-DMSO): δ= 9.455(2.93);8.506(2.55);8.5(2.61);8.459(1.82);8.456(1.79);8.448(1.92);8.444(1.77);7.851(0.93);7.847(1.18);7.841 (0.9);7.83(1.07);7.827(1 .29);7.824(1.22);7.821(0.95);7.434(1.36);7.423(1.38);7.414(1.29);7.402(1.24);6.417(1.68);5.755(7.47);4.082(16);3.406(15.15);3.322(7.77);2. 506(17.43);2.502(22.1);2.498(15.85);1.989(1,09);1.51(1.12);1.496(3.06);1.489(3.22);1.476(1.45);1.283(1,47);1.27(3.06);1.263(3.15);1.248(1 .11);1.175(0.56);0.008(1.23);0(28.06);-0.008(1.17) |
| Beispiel I-1-074: ¹H-NMR(400.0 MHz, d₆-DMSO):δ= 8.491(1.77);8.487(1.83);8.479(2.03);8.474(3.19);8.467(2.43);8.103(0.57); 8.099(0.57);7.866(0.61);7.863(0.61);7.842(0.86);7.838(1.05);7.836(0.97);7.832(0.88);7.822(1,06);7.818(1,23);7.815(1.21);7.812(0.99);7.472 (1.3);7.461(1.3);7.452(1.24);7.44(1.15);6.365(2.4);5.756(0.34);4.315(0.82);4.297(2.72);4.279(2.76);4.262(0.88);4.254(0.41);3.398(15.83);3. 369(1.61);3.343(0.5);3.319(26.19);3.015(6.03);2.978(14.74);2.962(0.77);2.943(16);2.674(0.36);2.671(0.48);2.666(0.36);2.523(1.28);2.506(6 3.6);2.501(82.93);2.497(59.42);2.332(0.34);2.328(0.48);2.324(0.35); 1.373(0.35); 1.355(0.79);1.34(3.12); 1.322(6.87); 1.305(2.99);0.146(0.43); 0.008(3.66);0(96.34);-0.008(4.03);-0.15(0.44) |
| Beispiel I-1-075: ¹H-NMR(400.0 MHz, d₆-DMSO):δ= 8.799(0.64);8.784(1.32);8.768(0.65);8.525(2.37);8.519(2.46);8.47(1.66); 8.467(1.66);8.458(1.76);8.455(1.66);7.858(0.85);7.854(1.08);7 .848(0.83);7.837(0.99);7.833(1.17);7.828(0.89);7.446(1.29);7.435(1.3);7.426( 1.23);7.414(1.16);6.437(1.76);5.756(1.73);4.127(1.22);4.11 (3.86);4.092(3.99);4.075(16);3.92(3.84);3.905(3.86);3.635(0.39);3.408(14.1);3.32 1(12.56);2.506(35.54);2.502(45.81);2.497(33.2);1.205(4.16);1.187(8.52);1.169(4.03);0.008(0.38);0(9.92) |
| Beispiel I-1-076: ¹H-NMR(400.0 MHz, d₆-DMSO):δ= 9.171(0.72);9.156(1.47);9.141(0.79);8.515(2.46);8.509(2.45);8.466(1.77); 8.463(1.73);8.454(1.83);8.45(1.7);8.315(0.61);7.845(1.16);7.824(1.22);7.44(1.43);7.428(1.38);7.419(1.3);7.407(1.15);6.46(1.6);4.205(4.4);4. 191(4.42);4.086(16);4.038(0.54);4.02(0.51);3.408(15.05);3.319(120.93);2.674(2.18);2.67(2.79);2.666(2.13);2.505(376.5);2.501(481.2);2.497 (349.42);2.328(2.72);1.988(2.07);1.237(0.56);1.192(0.53);1.174(1.1);1.157(0.56);0.146(0.45);0(91.03);-0.15(0.48) |
| Beispiel I-1-077: ¹H-NMR(400.0 MHz, d₆-DMSO):δ= 8.824(0.89);8.808(1.46);8.793(0.76);8.525(2.63);8.518(2.66);8.47(1.8); 8.458(1.91);8.315(1.8);7.855(1.24);7.835(1.47);7.448(1.4);7.436(1.46);7.427(1.42);7.414(1.22);6.436(2.03);4.076(15.54);4.039(0.82);4.02(0. 68);3.942(4.06);3.926(4.12);3.822(0.49);3.635(16);3.408(14.66);3.387(0.51);3.356(0.84);3.318(306.44);2.67(8.71);2.602(0.55);2.576(0.76);2 .505(1213.22);2.501(1535.96);2.497(1114.25);2.42(0.75);2.386(0.56);2.328(9.07);2.302(0.59);2.283(0.51);2.28(0.49);1.988(2.65);1.235(2.0 2);1.193(0.73);1.174(1.33);1.157(0.84);0.146(1.23);0(290.84);-0.149(1.37);-3.236(0.53) |
| Beispiel I-1-078: ¹H-NMR(400.0 MHz, d₆-DMSO):δ= 11.725(2.66);8.51(3);8.504(3.16);8.467(2.05);8.463(2.16);8.455(2.18); 8.451(2.17);8.315(0.92);7.855(1.35);7.839(1.32);7.834(1.46);7.444(1.63);7.432(1 .69);7.424(1.55);7.412(1.44);6.367(2.26);4.034(16);3.886(1 ,46);3.868(4.81);3.851 (4.8);3.833(1.66);3.398(18.99);3.319(157.08);2.686(0.74);2.67(4.31);2.666(3.09);2.523(10.11);2.506(558.72);2.501(7 34.26);2.497(526.38);2.332(2.98);2.328(4.2);2.324(3.08);1.236(0.8);1.169(4.46);1.152(9.11 );1.134(4.34);1.056(0.42);0.147(0.69);0.008(5.32 );0(165.28);-0.008(6.15);-0.15(0.68) |
| Beispiel I-1-079: ¹H-NMR(400.0 MHz, d₆-DMSO):δ= 8.691(1.51);8.67(1.52);8.515(2.52);8.509(2.57);8.47(1.68);8.467(1.87); 8.459(1.82);8.455(1.87);7.858(0.9);7.855(1.12);7.852(1.15);7.848(0.99);7.838(1.04);7.834(1.22);7.832(1.31);7.828(1.04);7.445(1.35);7.433( 1.37);7.424(1.29);7.413(1.21);7.341(1.68);7.323(4.33);7.31(2.85);7.293(3.65);7.273(1.59);7.236(1.27);7.218(1.53);7.201(0.53);6.413(2.03);5 .756(3.01);4.962(1.41);4.948(3.61);4.934(2.14);4.915(0.49);4.047(16);3.696(0.41);3.685(0.88);3.669(1.29);3.661(0.99);3.647(1.64);3.634(1. 14);3.62(0.53);3.401(14.58);3.321(17.58);2.505(43.5);2.501(57.68);2.497(43.17);2.328(0.33);0(16.51) |
| Beispiel I-1-080: ¹H-NMR(400.0 MHz, d₆-DMSO):δ= 8.451(3.29);8.429(0.76);8.416(3.15);8.404(3.07);7.371(2.47);7.359(2.41); 6.087(1.58);5.756(0.96);3.804(7.46);3.687(0.51);3.32(35.22);3.275(5.01);3.112(0.63);3.008(0.79);2.962(6.79);2.938(5.29);2.67(0.62);2.506( 89.28);2.501(109.26);2.497(78.76);2.328(0.61);2.233(16);2.195(1.37);0(59.66) |
| Beispiel I-1-081: ¹H-NMR(400.0 MHz, d₆-DMSO):δ= 8.532(2.33);8.526(2.37);8.482(1.8);8.473(1.7);8.47(1.79);8.315(0.51); 7.857(1.08);7.837(1.21);7.83(0.98);7.451(1.28);7.438(1.2);7.43(1.19);7.418(1.08);6.467(1.2);3.854(16);3.498(14.44);3.407(14.96);3.37(2.25 );3.324(522.9);3.259(15.7);3.207(1.04);2.861(0.36);2.67(1.86);2.506(254.25);2.501 (341.84);2.497(253.8);2.328(1.93);0.146(1.16);0.007(9.8) ;0(242.01);-0.008(12.59);-0.15(1.11) |
| Beispiel I-1-082: ¹H-NMR(400.0 MHz, d₆-DMSO):δ= 8.503(2.35);8.497(2.42);8.472(1.75);8.46(1.79);8.314(0.55);7.861(1.03); 7.843(1.19);7.451(1.37);7.438(1.29);7.43(1.23);7.418(1.28);6.57(1.05);4.068(0.33);4.015(16);3.725(0.43);3.708(1);3.691(1.41);3.674(1.06); 3.657(0.55);3.411(15.1);3.319(180.01);2.67(2.22);2.666(1.76);2.505(299.26);2.501(396.17);2.497(299.13);2.327(2.24);2.323(1.9); 1.233(0.7 5);1.059(14.93);1.042(14.93);0.146(1.34);0(292.5);-0.068(0.34);-0.15(1.37) |
| Beispiel I-1-083: ¹H-NMR(400.0 MHz, d₆-DMSO):δ= 8.532(2.35);8.526(2.29);8.494(1.66);8.49(1.74);8.482(1.67);8.478(1.65); 7.831(0.81);7.825(1.06);7.822(0.86);7.811(0.93);7.805(1.2);7.801(0.9);7.473(1.23);7.461(1.25);7.453(1.14);7.441(1.06);6.895(2.46);3.776(1 6);3.381(0.64);3.372(0.85);3.363(1.25);3.354(0.95);3.346(0.82);3.323(21.02);3.018(15.11);2.99(13.52);2.506(21.9);2.502(28.64);2.497(21.2 6);0.858(0.57);0.84(2.31);0.826(2.32);0.822(1.93);0.808(0.65);0.566(0.72);0.554(2.05);0.548(2.22);0.544(2.09);0.539(1.96);0.526(0.57);0(1 8.02) |
| Beispiel I-1-084: ¹H-NMR(400.0 MHz, d₆-DMSO):δ= 8.507(2.52);8.501(2.59);8.476(1.98);8.464(1.99);7.861(1.27);7.841(1.39); 7.453(1.41);7.44(1.42);7.432(1.34);7.42(1.24);6.59(1.22);4.014(16);3.416(15.48);3.322(18.76);3.114(0.75);3.101(1.27);3.088(0.73);3.083(0. 7);2.502(37.69);1.259(0.36);1.235(0.63);1.013(3.26);0.995(5.43);0.987(4.48);0(33.58) |
| Beispiel I-1-085: ¹H-NMR(400.0 MHz, d₆-DMSO):δ= 8.499(1.96);8.492(2);8.474(1.45);8.471(1.53);8.462(1.54);8.459(1.58); 8.315(0.64);7.865(0.77);7.862(0.97);7.86(0.88);7.855(0.87);7.845(0.94);7.842(1.05);7.839(1.11);7.835(0.91);7.449(1.13);7.438(1.11);7.429( 1.01);7.417(1.09);6.539(0.89);4.07(0.34);4.009(16);3.407(15.22);3.368(0.48);3.32(252.15);2.674(1.83);2.67(2.6);2.666(1.91);2.523(8.15);2.5 1(162.92);2.506(330.93);2.501(443.6);2.497(331.71);2.492(168.97);2.479(20.1);2.332(1.81);2.328(2.52);2.323(1.89);1.234(0.58);0.865(0.34 );0.848(0.73);0.829(0.4);0.146(1.86);0.008(15.84);0(420.24);-0.008(20.19);-0.15(1.91) |
| Beispiel I-1-086: ¹H-NMR(400.0 MHz, d₆-DMSO):δ= 8.498(2.01);8.492(2.09);8.473(1.51);8.47(1.54);8.461(1.52);8.458(1.49); 7.862(1);7.856(0.79);7.845(0.89);7.841(1.03);7.835(0.84);7.45(1.15);7.438(1.17);7.43(1.07);7.418(1.01);6.533(0.99);4.017(16);3.407(15.16) ;3.327(89.99);2.999(1.03);2.98(3.52);2.962(3.63);2.944(1.12);2.675(0.85);2.67(1.17);2.666(0.87);2.506(160.11);2.501(209.18);2.497(153.02 );2.332(0.88);2.328(1.16);2.324(0.86);1.031(3.74);1.013(7.71);0.995(3.59);0.146(0.51);0.008(5.19);0(123.3);-0.008(5.31);-0.15(0.54) |
| Beispiel I-1-087: ¹H-NMR(400.0 MHz, d₆-DMSO):δ= 8.53(3.7);8.525(3.71);8.494(2.64);8.491(2.78);8.482(2.83);8.315(1.21); 7.823(1,67);7.807(1.68);7.804(1.85);7.473(2.24);7.462(2.24);7.453(2.03);7.441(1.89);6.894(2.71);6.886(2.17);3.878(0.54);3.788(0.62);3.768 (12.39);3.761(16);3.484(0.89);3.467(2.37);3.449(2.32);3.432(0.87);3.391(1.46);3.374(4.16);3.356(4.91);3.319(713.32);3.009(0.44);2.983(11. 59);2.963(13.87);2.947(0.38);2.938(0.53);2.839(0.47);2.675(5.04);2.67(6.95);2.666(5.28);2.592(0.7);2.51(443.49);2.506(865.16);2.501(1138 .51);2.497(859.59);2.332(5.08);2.328(6.73);2.324(5.08);1.55(0.34);1.266(0.46);1.259(0.74);1.234(2.83);1.135(2.73);1.117(5.63);1.109(3.49); 1.099(3.12);1.092(6.81);1.074(3.02);1.048(0.38);0.858(1.1);0.84(4.06);0.827(3.95);0.808(1.17);0.565(1.16);0.553(3.45);0.547(3.74);0.538(3. 25);0.524(0.99);0.146(2.04);0.008(20.12);0(477.57);-0.008(23.15);-0.049(0.39);-0.061(0.34);-0.15(2.14) |
| Beispiel I-1-088: ¹H-NMR(400.0 MHz, d₆-DMSO):δ= 8.539(0.35);8.497(4.02);8.491(4.18);8.459(2.79);8.456(2.92);8.447(2.86); 8.444(2.8);7.82(1.85);7.8(1.9);7.435(2.08);7.424(2.1);7.415(1.95);7.403(1.78);6.228(3.02);5.756(8.09);4.098(1.15);4.077(0.34);4.055(0.52); 4.034(0.46);3.996(16);3.796(1.32);3.787(0.5);3.436(1.69);3.38(23.01);3.363(3.41);3.323(31.94);2.85(0.46);2.838(0.95);2.83(1.07);2.818(1.8 );2.806(1.1);2.798(0.95);2.785(0.47);2.675(1.26);2.67(1.67);2.666(1.29);2.506(209.12);2.501(273.32);2.497(205.3);2.332(1.19);2.328(1.63); 2.324(1.2);1.908(0.51);1.259(0.37);1.236(0.35);0.825(3.21);0.818(3.16);0.778(0.38);0.754(0.35);0.728(2.35);0.723(2.37);0.709(2.55);0.146( 0.49);0.008(4.56);0(108.14);-0.15(0.51) |
| Beispiel I-1-089: ¹H-NMR(400.0 MHz, d₆-DMSO):δ= 8.502(3.15);8.496(2.55);8.462(2.08);8.459(2.01);8.451(2.08);8.447(1.69); 8.329(0.88);8.315(2.11);8.3(0.74);7.849(1,33);7.845(1,43);7.839(1,02);7.828(1,48);7.825(1,47);7.819(0.93);7.44(1,46);7.428(1,58);7.42(1.35 );7.408(1.2);6.389(2.27);4.086(6.8);4.081(16);3.506(0.58);3.462(2.5);3.446(4.75);3.431(3.41);3.4(16.04);3.359(5.22);3.345(6.52);3.331(4.89 );3.276(2.77);3.262(4.27);3.246(3.61);3.231(1.48);2.984(0.66);2.971(0.6);2.676(0.35);2.671(0.38);2.576(2.08);2.562(3.61);2.547(2.8);2.506( 53.66);2.502(58.47);2.498(40.03);2.333(0.32);2.329(0.35);0.005(7.57);0(19.13) |

a) Die Bestimmung der in den vorangegangenen Tabellen und Herstellungsbeispielen angegebenen logP-Werte erfolgte gemäß EEC Directive 79/831 Annex V.A8 durch HPLC (High Performance Liquid Chromatography) an einer Phasenumkehrsäule (C18). Temperatur 43 °C. Die Eichung erfolgt mit unverzweigten Alkan-2-onen (mit 3 bis 16 Kohlenstoffatomen), deren logP Werte bekannt sind.
   Die Bestimmung des M⁺ mit der LC-MS im sauren Bereich erfolgte bei pH 2,7 mit 0,1 % wässriger Ameisensäure und Acetonitril (enthält 0,1% Ameisensäure) als Eluenten; linearer Gradient von 10% Acetonitril bis 95% Acetonitril, Gerät: Agilent 1100 LC-System, Agilent MSD System, HTS PAL.
   Die Bestimmung des M⁺ mit der LC-MS im neutralen Bereich erfolgte bei pH 7.8 mit 0,001 molarer wässriger Ammoniumhydrogencarbonat-Lösung und Acetonitril als Eluenten; linearer Gradient von 10 % Acetonitril bis 95 % Acetonitril.
   In den vorangegangenen Tabellen und Herstellungsbeispielen wurden die logP Werte für den sauren Bereich (als logP [a]) und/oder für den neutralen Bereich (als logP [n]) angegeben.
b) Die Bestimmung der ¹H-NMR-Daten erfolgte mit einem Bruker Avance 400 ausgestattet mit einem Durchflussprobenkopf (60 µl Volumen), mit Tetramethylsilan als Referenz (0.0) und den Lösungsmitteln CD₃CN, CDCl₃ oder D₆-DMSO.

Die NMR-Daten ausgewählter Beispiele werden entweder in klassischer Form (δ-Werte, Multiplettaufspaltung, Anzahl der H-Atome) oder als NMR-Peak-Listen aufgeführt.

### NMR-Peak-Listenverfahren

Die ¹H-NMR-Daten ausgewählter Beispiele werden in Form von ¹H-NMR-Peaklisten notiert. Zu jedem Signalpeak wird erst der δ -Wert in ppm und dann die Signalintensität in runden Klammern aufgeführt. Die δ -Wert - Signalintensitäts- Zahlenpaare von verschiedenen Signalpeaks werden durch Semikolons voneinander getrennt aufgelistet.

Die Peakliste eines Beispieles hat daher die Form:
δ₁ (Intensität₁); δ₂ (Intensität₂);........; δᵢ (Intensität;); ......; δₙ (Intensitätₙ)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von ¹H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der ¹H-NMR-Peaks sind ähnlich den klassischen ¹H-NMR-Ausdrucken und enthalten somit gewöhnlich alle Peaks, die bei einer klassischen NMR-Interpretation aufgeführt werden.

Darüber hinaus können sie wie klassische ¹H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von ¹H-NMR-Peaks die gewöhnlichen Lösungsmittelpeaks, zum Beispiel Peaks von DMSO in DMSO-D₆ und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen ¹H-NMR-Interpretation.

Weitere Details zu ¹H-NMR-Peaklisten können der Research Disclosure Database Number 564025 entnommen werden.

### Anwendungsbeispiele

Die folgenden Beispiele zeigen die insektizide und akarizide Wirkung der erfindungsgemäßen Verbindungen. Dabei beziehen sich die genannten erfindungsgemäßen Verbindungen auf die in der Tabelle 1 mit den entsprechenden Referenzzeichen (Nr.) aufgeführten Verbindungen:

### Myzus persicae - Sprühtest

| | |
|---|---|
| Lösungsmittel: | 78 Gewichtsteile Aceton |
| | 1,5 Gewichtsteile Dimethylformamid |
| Emulgator: | Alkylarylpo lyglyko lether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach 5-6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500g/ha: 1-1-005, I-1-007, I-1-008, I-1-013, I-1-014, I-1-016, I-1-017, I-1-018, I-1-020, I-1-021, I-1-022, I-1-023, I-1-027, I-1-032, I-1-033, I-1-035, I-1-036, I-1-037, I-1-042, I-1-044, I-1-046, I-1-047, I-1-048, I-1-051, I-1-052, I-1-054, I-1-055, I-1-056, I-1-058, I-1-059, I-1-062, I-1-065, I-1-066, I-1-067, I-1-068, I-1-069, I-1-072, I-1-073, I-1-074, I-1-076, I-1-082.

Bei diesem Test zeigen z. B. die folgenden Verbindungen der Herstellungsbeispiele Wirkung von 90% bei einer Aufwandmenge von 500g/ha: I-1-001, I-1-002, I-1-003, I-1-004, I-1-009, I-1-010, I-1-011, I-1-012, I-1-015, I-1-019, I-1-025, I-1-026, I-1-028, I-1-029, I-1-030, I-1-031, I-1-034, I-1-038, I-1-040, I-1-041, I-1-043, I-1-045, I-1-049, I-1-050, I-1-053, I-1-057, I-1-060, I-1-064, I-1-071, I-1-075, I-1-078, I-1-079, I-1-080, I-1-081, I-1-083, I-1-084, I-1-085, I-1-086.

### Myzus persicae - Sprühtest

| | |
|---|---|
| Lösungsmittel: | 14 Gewichtsteile Dimethylformamid |
| Emulgator: | Alkylarylpo lyglyko lether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (*Capsicum annuum*), die stark von der Grünen Pfirsichblattlaus (*Myzus persicae*) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 98% bei einer Aufwandmenge von 20ppm: I-1-006

### Tetranychus urticae - Sprühtest, OP-resistent

| | | |
|---|---|---|
| Lösungsmittel: | 78,0 | GewichtsteileAceton |
| | 1,5 | Gewichtsteile Dimethylformamid |
| Emulgator : | Alkylarylpo lyglyko lether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (*Phaseolus vulgaris*), die von allen Stadien der Gemeinen Spinnmilbe (*Tetranychus urticae*) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 500g/ha: I-1-008, I-1-010, I-1-076.

### Myzus persicae - Oraltest

| | | |
|---|---|---|
| Lösungsmittel: | 100 | Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus (*Myzus persicae*), die sich in einer zweiten Mikrotiterplatte befindet, hindurchstechen und die Lösung aufnehmen kann.

Nach 5 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 20ppm: 1-1-001, 1-1-002, I-1-003, I-1-004, I-1-005, I-1-006, I-1-007, I-1-008, I-1-009, I-1-011, I-1-012, I-1-013, I-1-014, I-1-015, I-1-016, I-1-017, I-1-018, I-1-019, I-1-020, I-1-021, I-1-022, I-1-023, I-1-024, I-1-025, I-1-026, I-1-027, I-1-028, I-1-029, I-1-030, I-1-031, I-1-032, I-1-033, I-1-034, I-1-035, I-1-036, I-1-037, I-1-038, I-1-039, I-1-040, I-1-041, I-1-042, I-1-043, I-1-044, I-1-045, I-1-046, I-1-047, I-1-048, I-1-049, I-1-050, I-1-051, I-1-052, I-1-053, I-1-054, I-1-058, I-1-055, I-1-056, I-1-057, I-1-059, I-1-060, I-1-061, I-1-062, I-1-063, I-1-064, I-1-065, I-1-066, I-1-067, I-1-069, I-1-071, I-1-072, I-1-073, I-1-075, I-1-076, I-1-080, I-1-081, I-1-084, I-1-085.

Bei diesem Test zeigen z. B. die folgenden Verbindungen der Herstellungsbeispiele Wirkung von 90% bei einer Aufwandmenge von 20ppm: 1-1-010, I-1-068, I-1-070, I-1-074, I-1-077, I-1-079, I-1-083.

### Phaedon cochleariae - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpo lyglyko lether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*) werden mit einer Wirkstoffzubereitung der gewünschten Konzentration gespritzt und nach dem Abtrocknen mit Larven des Meerrettichblattkäfers (*Phaedon cochleariae*) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-1-039

### Spodoptera frugiperda - Sprühtest

| | | |
|---|---|---|
| Lösungsmittel: | 78,0 | Gewichtsteile Aceton |
| | 1,5 | Gewichtsteile Dimethylformamid |
| Emulgator: | Alkylarylpolyglykolether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Maisblattscheiben (*Zea mays*) werden mit einer Wirkstoffzubereitung der gewünschten Konzentration gespritzt und nach dem Abtrocknen mit Raupen des Heerwurms (*Spodoptera frugiperda*) besetzt.

Nach 7 Tagen wird die Wirkung in % bestimmt. Dabei bedeutet 100 %, dass alle Raupen abgetötet wurden; 0 % bedeutet, dass keine Raupe abgetötet wurde.

Bei diesem Test zeigen z. B. die folgenden Verbindungen der Herstellungsbeispiele Wirkung von 83% bei einer Aufwandmenge von 500g/ha: I-1-039.

### Boophilus microplus -Injektionstest

| | |
|---|---|
| Lösungsmittel: | Dimethylsulfoxid |

Zur Herstellung einer zweckmäßigen Wirkstoffzubereitung vermischt man 10 mg Wirkstoff mit 0,5 ml Lösungsmittel und verdünnt das Konzentrat mit Lösungsmittel auf die gewünschte Konzentration.

1µl der Wirkstofflösung wird in das Abdomen von 5 vollgesogenen, adulten, weiblichen Rinderzecken (*Boophilus microplus*) injiziert. Die Tiere werden in Schalen überführt und in einem klimatisierten Raum aufbewahrt.

Die Wirkungskontrolle erfolgt nach 7 Tagen auf Ablage fertiler Eier. Eier, deren Fertilität nicht äußerlich sichtbar ist, werden bis zum Larvenschlupf nach etwa 42 Tagen im Klimaschrank aufbewahrt. Eine Wirkung von 100 % bedeutet, dass keine der Zecken fertile Eier gelegt hat, 0% bedeutet, dass alle Eier fertil sind.

Bei diesem Test zeigen z.B. die folgenden Verbindungen der Herstellungsbeispiele eine Wirkung von 90% bei einer Aufwandmenge von 20µg/Tier: I-1-037
Bei diesem Test zeigen z.B. die folgenden Verbindungen der Herstellungsbeispiele eine Wirkung von 80% bei einer Aufwandmenge von 20µg/Tier: I-1-031

### Absetzbeispiele

### Myzus persicae - Sprühtest (MYZUPE)

| | | |
|---|---|---|
| Lösungsmittel: | 78,0 | Gewichtsteile Aceton |
| | 1,5 | Gewichtsteile Dimethylformamid |
| Emulgator: | Alkylarylpo lyglyko lether | |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (*Brassica pekinensis*), die von allen Stadien der Grünen Pfirsichblattlaus (*Myzus persicae*) befallen sind, werden mit einer Wirkstoffzubereitung der gewünschten Konzentration gespritzt.

Nach der gewünschten Zeit (dat) wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle 3

### Myzus persicae - Oraltest (MYZUPE O)

| | | |
|---|---|---|
| Lösungsmittel: | 100 | Gewichtsteile Aceton |

Zur Herstellung einer zweckmäßigen Wirkstoffzubereitung löst man 1 Gewichtsteil Wirkstoff mit den angegebenen Gewichtsteilen Lösungsmittel und füllt mit Wasser bis zum Erreichen der gewünschten Konzentration auf.

50 µl der Wirkstoffzubereitung werden in Mikrotiterplatten überführt und mit 150µl IPL41Insektenmedium (33% + 15% Zucker) auf eine Endvolumen von 200 µl aufgefüllt. Anschließend werden die Platten mit Parafilm verschlossen, durch den eine gemischte Population der Grünen Pfirsichblattlaus (*Myzus persicae*), die sich in einer zweiten Mikrotiterplatte befindet, hindurchstechen und die Lösung aufnehmen kann.

Nach der gewünschten Zeit (dat) wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle 3

**Tabelle 3:**

| Substanz | Struktur | Tierart | Konzentration | % Wirkung dat | |
|---|---|---|---|---|---|
| Bsp.Nr. 14 WO2011/009804 Stand der Technik | 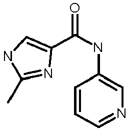 | MYZUPE | 100 g ai/ha | 0 | 5/6 dat |
| | | MYZUPE O | 4 ppm | 0 | 5 dat |
| | | | 0.8 ppm | 0 | 5 dat |
| Bsp.Nr. 13 WO2011/009804 Stand der Technik | 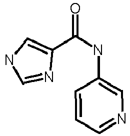 | MYZUPE | 100 g ai/ha | 0 | 6 dat |
| | | MYZUPE O | 4 ppm | 0 | 5 dat |
| | | | 0.8 ppm | 0 | 5 dat |
| Bsp.Nr. I-1-006 Erfindungsgemäß | 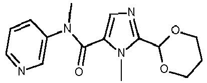 | MYZUPE O | 4 ppm | 100 | 5 dat |
| | | | 0.8 ppm | 100 | 5 dat |
| Bsp.Nr. I-1-024 Erfindungsgemäß | 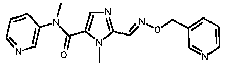 | MYZUPE O | 4 ppm | 90 | 6 dat |
| Bsp.Nr. I-1-025 Erfindungsgemäß | 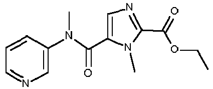 | MYZUPE | 100 g ai/ha | 90 | 6 dat |
| | | MYZUPE O | 4 ppm | 100 | 5 dat |
| | | | 0.8 ppm | 90 | 5 dat |
| Bsp.Nr. I-1-033 Erfindungsgemäß | 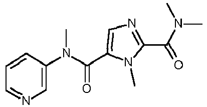 | MYZUPE | 100 g ai/ha | 90 | 5 dat |
| | | MYZUPE O | 4 ppm | 100 | 5 dat |
| | | | 0.8 ppm | 100 | 5 dat |
| Bsp.Nr. I-1-044 Erfindungsgemäß | 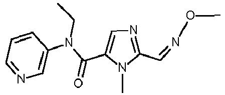 | MYZUPE | 100 g ai/ha | 90 | 5 dat |
| | | MYZUPE O | 4 ppm | 100 | 5 dat |
| Bsp.Nr. I-1-045 Erfindungsgemäß | 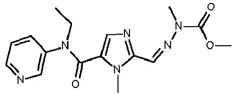 | MYZUPE | 100 g ai/ha | 90 | 5 dat |
| | | MYZUPE O | 4 ppm | 100 | 5 dat |
| Bsp.Nr. I-1-047 Erfindungsgemäß | 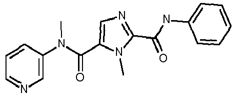 | MYZUPE | 100 g ai/ha | 90 | 5 dat |
| | | MYZUPE O | 4 ppm | 100 | 5 dat |
| Bsp.Nr. I-1-050 Erfindungsgemäß | 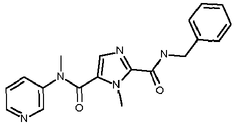 | MYZUPE | 100 g ai/ha | 90 | 5 dat |
| | | MYZUPE O | 4 ppm | 90 | 5 dat |
| | | | 0.8 ppm | 90 | 5 dat |
| Bsp.Nr. I-1-056 Erfindungsgemäß | 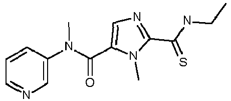 | MYZUPE | 100 g ai/ha | 100 | 5 dat |
| | | MYZUPE O | 4 ppm | 100 | 5 dat |
| | | | 0.8 ppm | 100 | 5 dat |
| Bsp.Nr. I-1-059 Erfindungsgemäß | 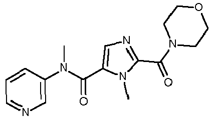 | MYZUPE | 100 g ai/ha | 100 | 5 dat |
| | | MYZUPE O | 4 ppm | 100 | 5 dat |
| Bsp.Nr. I-1-060 Erfindungsgemäß | 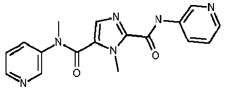 | MYZUPE | 100 g ai/ha | 90 | 5 dat |
| | | MYZUPE O | 4 ppm | 100 | 5 dat |
| Bsp.Nr. I-1-070 Erfindungsgemäß | 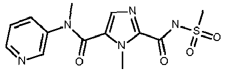 | MYZUPE O | 0.8 ppm | 100 | 5 dat |
| Bsp.Nr. I-1-074 Erfindungsgemäß | 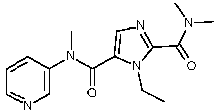 | MYZUPE | 100 g ai/ha | 90 | 5 dat |
| | | MYZUPE O | 4 ppm | 100 | 5 dat |
| | | | 0.8 ppm | 100 | 5 dat |
| Bsp.Nr. I-1-076 Erfindungsgemäß | 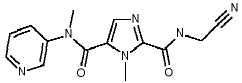 | MYZUPE | 100 g ai/ha | 90 | 5 dat |
| | | MYZUPE O | 4 ppm | 90 | 5 dat |
| | | | 0.8 ppm | 90 | 5 dat |
| Bsp.Nr. I-1-083 Erfindungsgemäß | 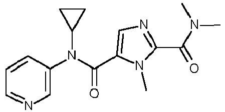 | MYZUPE | 100 g ai/ha | 90 | 5 dat |

## Patentansprüche

1. Verbindungen der Formel (I) 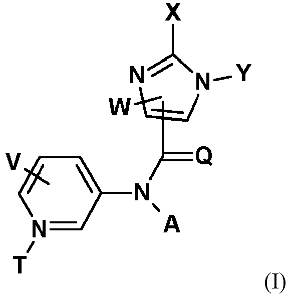 in welcher
Q für Sauerstoff oder Schwefel steht,
V für einen Rest aus der Reihe Wasserstoff, Halogen, Alkyl, Halogenalkyl, Alkoxy, Halogenalkoxy und Cyano steht,
W für einen Rest aus der Reihe Wasserstoff, Halogen, Alkyl, Halogenalkyl, Alkoxy, Halogenalkoxy und Cyano steht,
Y für einen Rest aus der Reihe gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Arylalkyl oder Hetarylalkyl und Cyano steht,
A für einen Rest aus der Reihe Wasserstoff, gegebenenfalls substituiertes Alkyl, Alkenyl oder Alkinyl und gegebenenfalls durch Heteroatome unterbrochenes und gegebenenfalls substituiertes Cycloalkyl oder Cycloalkyl-alkyl steht,
T für Sauerstoff oder ein Elektronenpaar steht,
X für C(E)U¹, C(O)OR², C(O)N(R³)(R⁴), C(S)N(R³)(R⁴), C(O)N(R³¹)-N(R³²)(R⁴¹), oder C(S)N(R³¹)-N(R³²)(R⁴¹) steht,
E für die Reste O, N-OR²¹ und N-N(R³³)(R⁴³) oder für einen Cyclus der Formel (X-1) steht, 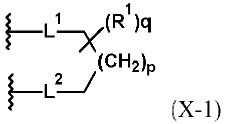
U¹ für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Aryl, Hetaryl, Arylalkyl oder Hetarylalkyl steht,
L¹, L² jeweils unabhängig voneinander für O und S(O)ₘ stehen,
R¹ für einen Rest aus der Reihe Halogen, Cyano, gegebenenfalls substituiertes Alkyl, Alkenyl, Alkinyl oder Alkoxy und gegebenenfalls substituiertes Aryl steht,
oder
R¹ für eine gegebenenfalls substituierte C₂-C₄-Alkylkette steht, die zwei am selben C-Atom gebunde H -Atome ersetzt, wobei q dann für 2 steht, oder
R¹ für eine gegebenenfalls substituierte C₁-C₄-Alkenylkette steht, wobei die Doppelbindung zwei am selben C-Atom gebunde H -Atome ersetzt, wobei q dann für 1 steht,
R² für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Aryl, Arylalkyl, Hetaryl oder Hetarylalkyl steht,
R²¹ für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, für gegebenenfalls substituiertes Cycloalkylalkyl oder für gegebenenfalls substituiertes Arylalkyl steht,
R³ für einen Rest aus der Reihe Wasserstoff, gegebenenfalls substituiertes Alkyl, Alkenyl, Alkinyl, Cycloalkyl, Alkylsulfonyl, Cycloalkylsulfonyl, Alkoxycarbonyl, Alkylcarbonyl oder Arylalkoxycarbonyl steht,
R⁴ für einen Rest aus der Reihe Wasserstoff, gegebenenfalls substituiertes Alkyl, Alkenyl, Alkinyl oder Alkoxy, gegebenenfalls durch Heteroatome unterbrochenes und gegebenenfalls substituiertes Cycloalkyl oder Cycloalkenyl, gegebenenfalls durch Heteroatome unterbrochenes und gegebenenfalls substituiertes Cycloalkylalkyl oder für gegebenenfalls substituiertes Aryl, Arylalkyl, Hetaryl und Hetarylalkyl steht,
oder
R³ und R⁴ zusammen einen gegebenenfalls substituierten vier- bis siebengliedrigen aliphatischen Ring ausbilden, der gegebenenfalls ein N-, S- oder O Atom enthält,
R³¹, R³² und R³³ jeweils unabhängig voneinander für einen Rest aus der Reihe Wasserstoff, gegebenenfalls substituiertes Alkyl, Alkenyl, Alkinyl, Cycloalkyl, Alkoxycarbonyl, Alkylcarbonyl, Arylalkylcarbonyl oder Arylalkylalkoxycarbonyl stehen,
R⁴¹, R⁴³ jeweils unabhängig voneinander für einen Rest aus der Reihe Wasserstoff, gegebenenfalls substituiertes Alkyl, Alkenyl oder Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Aryl, Arylalkyl, Hetaryl oder Hetarylalkyl stehen,
m für eine Zahl 0, 1 oder 2 steht,
p für eine Zahl 0, 1 oder 2 steht,
q für eine Zahl 0, 1 oder 2 steht
und deren Salze.

2. Verbindungen nach Anspruch 1, bei der
Q für Sauerstoff oder Schwefel steht,
V für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
W für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
Y für einen Rest aus der Reihe gegebenenfalls einfach bis mehrfach unabhängig voneinander durch Halogen, C₁-C₄-Alkoxy, C₁-C₄-Alkyl-S(O)ₙ- oder Cyano substituiertes C₁-C₆-Alkyl, C₃-C₆-Alkenyl oder C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach bis zweifach unabhängig voneinander durch O, S(O)ₙ, CO oder NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl-C₁-C₄-alkyl oder Hetaryl-C₁-C₄-alkyl steht,
A für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes C₁-C₆-Alkyl, C₃-C₆-Alkenyl oder C₃-C₆-Alkinyl und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₆-Cycloalkyl oder C₃-C₈-Cycloalkyl-C₁-C₄-alkyl steht,
T für Sauerstoff oder ein Elektronenpaar steht
X für C(E)U¹, C(O)OR², C(O)N(R³)(R⁴), C(S)N(R³)(R⁴), C(O)N(R³¹)-N(R³²)(R⁴¹) oder C(S)N(R³¹)-N(R³²)(R⁴¹) steht,
E für die Reste O, N-OR²¹, N-N(R³³)(R⁴³) oder für einen Cyclus der Formel (X-1) steht, 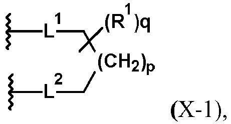
U¹ für einen Rest aus der Reihe Wasserstoff, gegebenenfalls einfach bis mehrfach unabhängig voneinander durch Halogen, Cyano, C₁-C₄-Alkoxy oder C₁-C₄-Alkyl-S(O)ₙ-, substituiertes C₁-C₈-Alkyl, C₃-C₈-Alkenyl oder C₃-C₈-Alkinyl, gegebenenfalls einfach durch O oder S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach durch O oder S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl oder Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
L¹, L² jeweils unabhängig voneinander für O und S(O)ₘ stehen,
R¹ für einen Rest aus der Reihe Halogen, gegebenenfalls durch Halogen, Cyano, C₁-C₄-Alkoxy oder C₁-C₄-Alkyl-S(O)ₙ-, substituiertes C₁-C₆-Alkyl, C₂-C₆-Alkenyl, C₃-C₆-Alkinyl oder C₁-C₆-Alkoxy, und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl steht,
oder
R¹ für eine gegebenenfalls durch Halogen, Cyano, C₁-C₄-Alkyl oder C₁-C₄-Alkoxy substituierte C₂-C₄-Alkylkette steht, die zwei am selben C-Atom gebunde H -Atome ersetzt, wobei n dann für 2 steht,
oder
R¹ für eine gegebenenfalls durch Halogen, Cyano, oder C₁-C₄-Alkoxy substituierte C₁-C₄-Alkenylkette steht, wobei die Doppelbindung zwei am selben C-Atom gebunde H -Atome ersetzt und wobei q dann für 1 steht,
R² für einen Rest aus der Reihe Wasserstoff, gegebenenfalls einfach bis mehrfach unabhängig voneinander durch Halogen, C₁-C₄-Alkoxy oder C₁-C₄-Alkyl-S(O)ₙ-, substituiertes C₁-C₈-Alkyl, C₃-C₈-Alkenyl oder C₃-C₈-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl oder Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
R²¹ für einen Rest aus der Reihe Wasserstoff, gegebenenfalls einfach bis mehrfach unabhängig voneinander durch Halogen, C₁-C₄-Alkoxy oder C₁-C₄-Alkyl-S(O)ₙ-, substituiertes C₁-C₈-Alkyl, C₃-C₈-Alkenyl, C₃-C₈-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₈-Cycloalkyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl-C₁-C₄-alkyl, und für gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes, geradkettiges oder verzweigtes Aryl-C₁-C₄-alkyl oder Hetaryl-C₁-C₄-alkyl steht,
R³ für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes substituiertes C₁-C₄-Alkyl, C₃-C₆-Alkenyl oder C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy oder Cyano substituiertes C₃-C₆-Cycloalkyl, C₁-C₄-Alkylsulfonyl, C₃-C₆-Cycloalkylsulfonyl, C₁-C₄-Alkylcarbonyl oder C₁-C₄-Alkoxycarbonyl und für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl-C₁-C₂-Alkoxycarbonyl steht,
R⁴ für einen Rest aus der Reihe Wasserstoff, gegebenenfalls einfach bis mehrfach unabhängig voneinander durch Halogen, Hydroxy, C₁-C₄-Alkoxy, C₁-C₄-Alkoxycarbonyl, - C₁-C₄-Alkyl-S(O)ₙ- oder Cyano substituiertes substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch O, S(O)ₙ, CO oder NR^{4a} unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl oder C₃-C₈-Cycloalkyl-C₁-C₄-aₗkyl, gegebenenfalls einfach bis dreifach unabhängig voneinander durch Halogen, C₁-C₄-Alkyl, C₁-C₄-Halogenalkyl, Hydroxy, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl, Hetaryl, Arylalkyl oder Hetarylalkyl steht, oder
R³ und R⁴ zusammen einen gegebenenfalls einfach bis dreifach durch Halogen, C₁-C₄-Alkyl oder C₁-C₄-Alkoxy substituierten vier- bis siebengliedrigen aliphatischen Ring ausbilden, der gegebenenfalls ein N-, S- oder O Atom enthält,
R³¹, R³², R³³ jeweils unabhängig voneinander für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes C₁-C₆-Alkyl, C₃-C₆-Alkenyl oder C₃-C₆-Alkinyl, gegebenenfalls einfach bis dreifach unabhängig voneinander durch Halogen, C₁-C₄-Alk-yl, C₁-C₄-Halogenalkyl, C₁-C₄-Alkoxy oder Cyano substituiertes C₃-C₆-Cycloalkyl, C₁-C₆-Alkylcarbonyl, C₁-C₆-Alkoxycarbonyl, Aryl-C₁-C₄-alkyl oder Aryl-C₁-C₄-alkoxy stehen,
R⁴¹, R⁴³ jeweils unabhängig voneinander für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₙ- oder Cyano substituiertes C₁-C₄-Alkyl, C₃-C₆-Alkenyl oder C₃-C₆-Alkinyl, gegebenenfalls einfach bis zweifach unabhängig voneinander durch O, S(O)ₙ, CO oder NR^{4a} unterbrochenes und gegebenenfalls einfach bis vierfach unabhängig voneinander durch Halogen, C₁-C₄-Alkyl, C₁-C₄-Halogenalkyl, C₁-C₄-Alkoxy oder Cyano substituiertes, geradkettiges oder verzweigtes C₃-C₈-Cycloalkyl oder C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₙ-, C₁-C₄-Halogenalkoxy, C₁-C₄-Halogenalkyl-S(O)ₙ-, Nitro oder Cyano substituiertes Aryl, Hetaryl, Arylalkyl oder Hetarylalkyl stehen,
m für eine Zahl 0, 1 oder 2 steht,
n für eine Zahl 0, 1 oder 2 steht,
p für eine Zahl 0 oder 1 steht,
q für eine Zahl 0, 1 oder 2 steht
und deren Salze.

3. Verbindungen nach Anspruch 1, bei der
Q für Sauerstoff steht
V für einen Rest aus der Reihe Wasserstoff, Fluor, Chlor, Brom, Methyl, Ethyl steht,
W für einen Rest aus der Reihe Wasserstoff, Fluor, Chlor, Brom, Cyano, Methyl steht,
Y für einen Rest aus der Reihe gegebenenfalls einfach bis fünffach unabhängig voneinander durch Fluor, Chlor, Brom, Methoxy oder Ethoxy substituiertes C₁-C₄-Alkyl, C₃-C₄-Alkenyl oder C₃-C₄-Alkinyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl oder Aryl-C₁-C₄-alkyl steht,
A für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl und gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl steht,
T für ein Elektronenpaar steht,
X für C(E)U¹, C(O)OR², C(O)N(R³)(R⁴) oder C(S)N(R³)(R⁴) steht,
E für die Reste O, N-OR²¹ , N-N(R³³)(R⁴³) oder für einen Cyclus der Formel (X-1) steht, 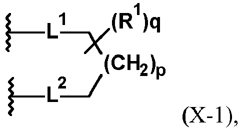
U¹ für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl und gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl, für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl, Pyridyl, Pyrimidinyl oder Thienyl, für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
L¹, L² jeweils unabhängig voneinander für O und S(O)ₘ stehen,
R¹ für gegebenenfalls durch Halogen, Methoxy, Ethoxy, substituiertes C₁-C₄-Alkyl, C₂-C₄-Alkenyl, C₃-C₄-Alkinyl oder C₁-C₄-Alkoxy steht,
oder
R¹ für eine C₂-C₄-Alkylkette steht, die zwei am selben C-Atom gebunde H -Atome ersetzt, wobei n dann für 2 steht, oder
R¹ für eine C₁-C₄-Alkenylkette steht, wobei die Doppelbindung zwei am selben C-Atom gebundene H -Atome ersetzt und wobei n dann für 1 steht,
R² für einen Rest aus der Reihe Wasserstoff, einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl-C₁-C₄-alkyl, für gegebenenfalls einfach bis dreifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl, Methyl-S(O)ₙ-, Difluorethyl-S(O)ₙ-, Trifluorethyl-S(O)ₙ-, Nitro oder Cyano substituiertes Phenyl, Pyridyl oder Thienyl und für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
R²¹ für einen Rest aus der Reihe Wasserstoff, einfach bis fünffach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl, gegebenenfalls einfach bis zweifach unabhängig voneinander durch Fluor, Chlor, Brom, Methyl, Ethyl, Trifluormethyl, Methoxy, Ethoxy oder Cyano substituiertes C₃-C₆-Cycloalkyl-C₁-C₄-alkyl und für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, geradkettiges oder verzweigtes Phenyl-C₁-C₂-alkyl oder Pyridyl-C₁-C₂-alkyl steht,
R³ für einen Rest aus der Reihe Wasserstoff, gegebenenfalls einfach bis dreifach unabhängig voneinander durch Fluor oder Chlor substituiertes substituiertes C₁-C₄-Alkyl, C₃-C₄-Alkenyl oder C₃-C₄-Alkinyl, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl oder für gegebenenfalls einfach bis dreifach unabhängig voneinander durch Fluor, Chlor, Methoxy oder Ethoxy substituiertes C₁-C₄-Alkylsulfonyl, C₃-C₆-Cycloalkylsulfonyl, C₁-C₄-Alkylcarbonyl oder C₁-C₄-Alkoxycarbonyl und für gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl-C₁-C₂-Alkoxycarbonyl steht,
R⁴ für einen Rest aus der Reihe Wasserstoff, gegebenenfalls einfach bis dreifach unabhängig voneinander durch Halogen, Hydroxy, Methoxy, Ethoxy, n- oder iPropoxy, Methoxycarbonyl, Ethoxycarbonyl, Cyano, oder Methyl-S(O)ₙ-, substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl, oder C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes Phenyl, Naphtyl Pyridyl, Pyrimidyl, Pyridazinyl, Pyrazinyl, Triazinyl, Furanyl, Thienyl, Oxazolyl, Isoxazolyl, Thiazolyl, Isothiazolyl, Pyrazolyl, Imidazolyl, Oxdiazolyl oder Thiadiazolyl, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ, Difluorethyl-S(O)ₙ-, Trifluorethyl-S(O)ₙ, Nitro oder Cyano substituiertes, geradkettiges oder verzweigtes Phenyl-C₁-C₂-alkyl oder Pyridyl-C₁-C₂-alkyl steht oder
R³ und R⁴ zusammen einen gegebenenfalls einfach bis zweifach durch Fluor Chlor, Brom, Methyl, Ethyl, Methoxy oder Ethoxy substituierten vier- bis sechsgliedrigen aliphatischen Ring ausbilden, der gegebenenfalls ein N-, S- oder O Atom enthält,
R³³ für einen Rest aus der Reihe Wasserstoff, gegebenenfalls einfach bis dreifach unabhängig voneinander durch Fluor oder Chlor substituiertes substituiertes C₁-C₄-Alkyl, für gegebenenfalls einfach bis dreifach unabhängig voneinander durch Fluor, Chlor, Methoxy oder Ethoxy substituiertes substituiertes C₁-C₆-Alkylcarbonyl oder C₁-C₆-Alkoxycarbonyl und für gegebenenfalls einfach bis zweifach unabhängig voneinander durch Fluor, Chlor, Brom, Methyl, Ethyl, Trifluormethyl, Methoxy, Ethoxy, Trifluormethoxy, Nitro oder Cyano substituiertes Phenyl-C₁-C₂-Alkoxycarbonyl steht,
R⁴³ für einen Rest aus der Reihe Wasserstoff oder für gegebenenfalls einfach bis dreifach unabhängig voneinander durch Halogen, Methoxy oder Ethoxy substituiertes C₁-C₄-Alkyl steht,
m für 0 steht,
n für eine Zahl 0, 1 oder 2 steht,
p für eine Zahl 0 oder 1 steht,
q für eine Zahl 0, 1 oder 2 steht
und deren Salze.

4. Verbindungen nach Anspruch 1 gemäß Formel (I-1) 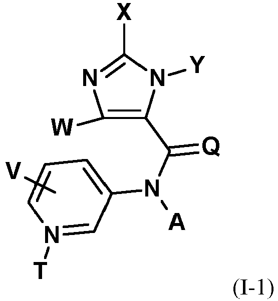
Q für Sauerstoff steht
V für einen Rest aus der Reihe Wasserstoff, Fluor, Chlor, Methyl und Cyano steht,
W für einen Rest aus der Reihe Wasserstoff, Fluor, Chlor, Brom. Methyl steht,
Y für einen Rest aus der Reihe gegebenenfalls einfach bis dreifach unabhängig voneinander durch Fluor, Methoxy, Ethoxy oder Cyano substituiertes Methyl, Ethyl, Propyl, Allyl oder Propargyl steht,
A für einen Rest aus der Reihe Wasserstoff, gegebenenfalls einfach bis dreifach unabhängig voneinander durch Fluor, Methoxy, Ethoxy oder Cyano substituiertes Methyl, Ethyl, Propyl, Allyl, Propargyl oder Cylopropyl steht,
T für ein Elektronenpaar steht,
X für C(E)U¹, C(O)OR², C(O)N(R³)(R⁴) oder C(S)N(R³)(R⁴) steht,
E für die Reste O, N-OR²¹, N-N(R³³)(R⁴³) oder für einen Cyclus der Formel (X-1) steht, 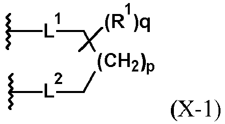
U¹ für einen Rest aus der Reihe Wasserstoff, gegebenenfalls einfach bis fünffach unabhängig voneinander durch Fluor, Chlor, Brom, Methoxy, Ethoxy oder Cyano substituiertes C₁-C₆-Alkyl oder für C₃-C₆-Cycloalkyl steht,
L¹, L² jeweils unabhängig voneinander für O und S(O)ₘ stehen,
R¹ für gegebenenfalls durch Fluor, Chlor, Methoxy oder Ethoxy substituiertes C₁-C₄-Alkyl steht,
oder
R¹ für eine C₂-C₄-Alkylkette steht, die zwei am selben C-Atom gebunde H -Atome ersetzt, wobei n dann für 2 steht,
R² für einen Rest aus der Reihe Wasserstoff, einfach bis dreifach unabhängig voneinander durch Fluor, Chlor, Brom, Methoxy, Ethoxy oder Cyano substituiertes C₁-C₆-Alkyl oder für gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, Phenylmethyl steht,
R²¹ für einen Rest aus der Reihe Wasserstoff, einfach bis dreifach unabhängig voneinander durch Fluor, Chlor, Brom, Methoxy, Ethoxy oder Cyano substituiertes C₁-C₆-Alkyl, C₃-C₆-Alkenyl oder C₃-C₆-Alkinyl, gegebenenfalls einfach bis zweifach unabhängig voneinander durch Fluor, Chlor, Brom, Methyl, Ethyl, Methoxy oder Ethoxy substituiertes C₃-C₆-Cycloalkyl, gegebenenfalls durch Fluor, Chlor, Brom, Methyl, Ethyl, Methoxy oder Ethoxy substituiertes C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₙ-, Et-S(O)ₙ-, Difluormethoxy, Trifluormethoxy, Trifluormethyl-S(O)ₙ-, Difluorethyl-S(O)ₙ-, Trifluorethyl-S(O)ₙ-, Nitro oder Cyano substituiertes, Phenylmethyl oder Pyridylmehyl steht,
R³ für einen Rest aus der Reihe Wasserstoff, gegebenenfalls einfach bis dreifach unabhängig voneinander durch Fluor, Chlor, Methoxy oder Ethoxy substituiertes substituiertes C₁-C₄-Alkyl, C₁-C₄-Alkylsulfonyl, C₃-C₆-Cycloalkylsulfonyl, C₁-C₄-Alkylcarbonyl oder C₁-C₄-Alkoxycarbonyl und gegebenenfalls einfach bis dreifach unabhängig voneinander durch Fluor, Chlor, Brom, Methyl, Ethyl, Trifluormethyl, Methoxy oder Ethoxy substituiertes Arylmethoxycarbonyl steht,
R⁴ für einen Rest aus der Reihe Wasserstoff, gegebenenfalls einfach bis dreifach unabhängig voneinander durch Halogen, Hydroxy, Methoxy, Ethoxy, Methoxycarbonyl, Ethoxycarbonyl oder Cyano substituiertes C₁-C₄-Alkyl, C₃-C₆-Alkenyl, C₃-C₆-Alkinyl oder C₁-C₄-Alkoxy, gegebenenfalls einfach bis zweifach unabhängig voneinander durch Fluor, Chlor, Methyl, Ethyl, Trifluormethyl, Methoxy, Ethoxy oder Cyano substituiertes C₃-C₆-Cycloalkyl oder C₃-C₆-Cycloalkyl-C₁-C₄-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Trifluormethoxy, Nitro oder Cyano substituiertes Phenyl, Pyridyl oder Thienyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₙ-, Ethyl-S(O)ₙ-, Trifluormethoxy, Nitro oder Cyano substituiertes Phenyl-C₁-C₂-alkyl oder Pyridyl-C₁-C₂-alkyl steht oder
R³ und R⁴ zusammen einen gegebenenfalls einfach bis zweifach durch Fluor Chlor, Brom, Methyl, Ethyl, Methoxy oder Ethoxy substituierten vier- bis sechsgliedrigen aliphatischen Ring ausbilden, der gegebenenfalls ein N-, S- oder O Atom enthält,
R³³ für einen Rest aus der Reihe Wasserstoff, gegebenenfalls durch Methoxy oder Ethoxy substituiertes C₁-C₄-Alkylcarbonyl oder C₁-C₄-Alkoxycarbonyl und für gegebenenfalls einfach bis dreifach unabhängig voneinander durch Fluor, Chlor, Methyl oder Ethyl substituiertes Phenylmethoxycarbonyl steht,
R⁴³ für einen Rest aus der Reihe Wasserstoff oder C₁-C₄-Alkyl steht,
m für 0 steht,
n für eine Zahl 0, 1 oder 2 steht,
p für eine Zahl 0 oderl steht,
q für eine Zahl 0, 1 oder 2 steht
und deren Salze.

5. Verbindungen nach Anspruch 4, bei der
Q für Sauerstoff steht,
V für Wasserstoff oder Methyl steht,
W für Wasserstoff steht,
Y für einen Rest aus der Reihe Methyl, Ethyl, Propyl, Allyl und Propargyl steht,
A für einen Rest aus der Reihe Wasserstoff, Methyl, Ethyl oder Cyclopropyl steht,
T für ein Elektronenpaar steht,
X für C(E)U¹, C(O)OR², C(O)N(R³)(R⁴) oder C(S)N(R³)(R⁴) steht,
E für die Reste O, N-OR²¹, N-N(R³³)(R⁴³) oder für einen Cyclus der Formel (X-1) steht, 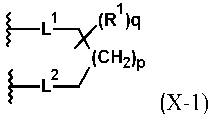
U¹ für einen Rest aus der Reihe Wasserstoff, Methyl, Ethyl, n-oder iso-Propyl, 1-Cyano-1-methylethyl oder Cyclopropyl steht,
L¹, L² jeweils unabhängig voneinander für O und S(O)ₘ stehen
R¹ für Methyl, Ethyl, n-, iso-Propyl oder Methoxymethyl steht,
oder
R¹ für -(CH₂)₂- steht, wobei eine spiro-Cyclopropylgruppe gebildet wird,
R² für einen Rest aus der Reihe Wasserstoff, Methyl, Ethyl, n-oder iso Propyl steht,
R²¹ für einen Rest aus der Reihe für einen Rest aus der Reihe Wasserstoff, Methyl, Ethyl, n-oder iso Propyl, n-, iso-, sec- oder tert-Butyl, 2,2-Dimethylpropyl, Propenyl, Propinyl, 2,2,2-Trifluorethyl, Cyclopropyl, Cyclobutyl, Cyclopentyl, Cyclohexyl, Cyclopropylmethyl, 2-C1-Phenylmethyl, 3-C1-Phenylmethyl, 4-C1-Phenylmethyl, Pyrid-2-ylmethyl, Pyrid-3-ylmethyl oder Pyrid-4-ylmethyl steht,
R³ für einen Rest aus der Reihe Wasserstoff, Methyl, Ethyl, Methylsulfonyl, Cyclopropylsulfonyl, Methoxycarbonyl, Ethoxycarbonyl, 2-Methoxyethoxycarbonyl n-, iso, sec- oder t-Butoxycarbonyl steht,
R⁴ für einen Rest aus der Reihe Wasserstoff, Methyl, Ethyl, n-oder iso Propyl, n-, iso, sec- oder tert. Butyl, 2,2,2-Trifluorethyl, 2-Hydroxyethyl, 2-Methoxyethyl, Cyanomethyl, Methoxycarbonylmethyl, Ethoxycarbonylmethyl, Cylopropyl, 1-Cyanocyclopropyl, Cyclopropylmethyl, Cylobutyl, Cylopentyl, Cylohexyl, Methoxy, Ethoxy, Phenyl, 2-Cl-Phenyl, 3-Cl-Phenyl, 4-Cl-Phenyl, Pyridyl, Phenylmethyl, 1-Phenyl-2-hydroxyethyl, 2-Cl-Phenylmethyl, 3-Cl-Phenylmethyl, 4-Cl-Phenylmethyl steht oder
R³ und R⁴ zusammen für (CH₂)₅, (CH₂)₄, (CH₂)₃ oder (CH₂)₂O(CH₂)₂ stehen,
R³³ für einen Rest aus der Reihe Methoxycarbonyl, Ethoxycarbonyl, n- oder iso Propoxycarbonyl, n-, iso, sec- oder t-Butoxycarbonyl, iso Pentoxycarbonyl, 2-Methoxyethoxycarbonyl oder Phenylmethoxycarbonyl steht,
R⁴³ für einen Rest aus der Reihe Wasserstoff, Methyl, Ethyl, n-oder iso Propyl, n-, iso, sec-oder tert. Butyl steht,
m für 0 steht,
p für eine Zahl 0 oderl steht,
q für eine Zahl 0, 1 oder 2 steht
und deren Salze.

6. Verbindungen nach Anspruch 4, bei der
Q für Sauerstoff steht
V für Wasserstoff oder Methyl steht,
W für Wasserstoff steht,
Y für Methyl steht,
A für Methyl, Ethyl oder Cyclopropyl steht,
T für ein Elektronenpaar steht,
X für C(E)U¹, C(O)OR², C(O)N(R³)(R⁴) oder C(S)N(R³)(R⁴) steht,
E für die Reste O, N-OR²¹, N-N(R³³)(R⁴³) oder für einen Cyclus der Formel (X-1) steht, 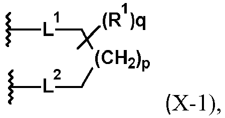
U¹ für Wasserstoff, Methyl, Ethyl, iso-Propyl, 1-Cyano-1-methylethyl oder Cyclopropyl steht,
L¹, L² jeweils unabhängig voneinander für O und S(O)ₘ stehen,
R¹ für Methyl, Ethyl oder Methoxymethyl steht, oder
R¹ für -(CH₂)₂- steht, wobei eine spiro-Cyclopropylgruppc gebildet wird
R² für Methyl oder Ethyl steht,
R²¹ für einen Rest aus der Reihe für einen Rest aus der Reihe Methyl, Ethyl, n-oder iso Propyl, n-, iso-, oder tert-Butyl, 2,2-Dimethylpropyl, Propenyl, Propinyl, 2,2,2-Trifluorethyl, Cyclopentyl, Cyclopropylmethyl, 4-C1-Phenylmethyl, Pyrid-2-ylmethyl, Pyrid-3-ylmethyl,
R³ für einen Rest aus der Reihe Wasserstoff, Methyl, Ethyl, Methylsulfonyl, oder Cyclopropylsulfonyl steht.
R⁴ für einen Rest aus der Reihe Wasserstoff, Methyl, Ethyl, iso Propyl, iso-Butyl, tert. Butyl, 2,2,2-Trifluorethyl, 2-Hydroxyethyl, 2-Methoxyethyl, Cyanomethyl, Methoxycarbonylmethyl, Ethoxycarbonylmethyl, Cylopropyl, 1-Cyanocyclopropyl, Cyclopentyl, Cyclopropylmethyl, Methoxy, Ethoxy, Phenyl, Pyrid-3-yl, Phenylmethyl, 1-Phenyl-2-hydroxyethyl steht,
R³ und R⁴ zusammen für (CH₂)₅, (CH₂)₄, (CH₂)₃ oder (CH₂)₂O(CH₂)₂ stehen,
R³³ für einen Rest aus der Reihe Methoxycarbonyl, Ethoxycarbonyl, t-Butoxycarbonyl, 2-Methoxyethoxycarbonyl oder Phenylmethoxycarbonyl steht,
R⁴³ für einen Rest aus der Reihe Wasserstoff, Methyl, oder n-Butyl steht,
m für 0 steht,
p für eine Zahl 0 oder1 steht,
q für eine Zahl 0, 1 oder 2 steht
und deren Salze.

7. Verbindung nach einem der Ansprüche 1 bis 6 gemäß einer der Formeln (I-1a) bis (I-1f)

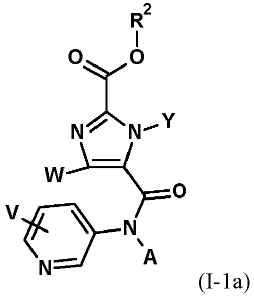

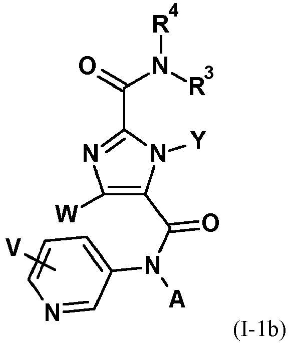

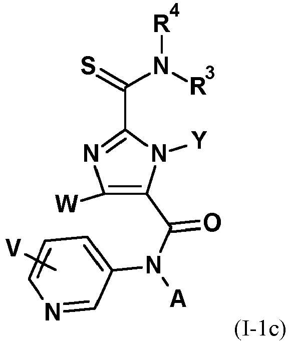

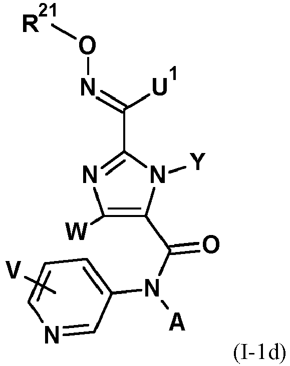

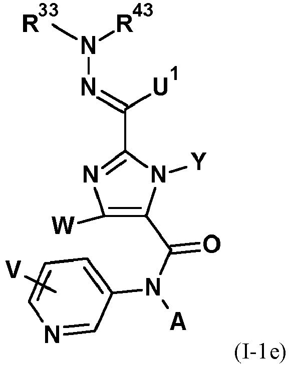

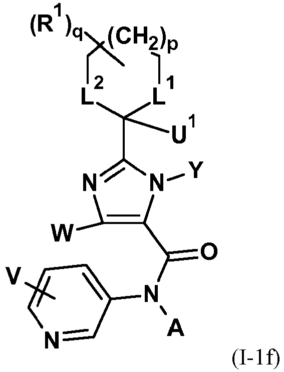

8. Mittel, mit einem Gehalt von mindestens einer Verbindung gemäß einem der Ansprüche 1 bis 7 und üblichen Streckmitteln und/oder oberflächenaktiven Substanzen insbesondere zur Bekämpfung von tierischen Schädlingen.

9. Verfahren zur Bekämpfung von tierischen Schädlingen, bei dem man wenigstens eine Verbindung gemäß einem der Ansprüche 1 bis 7 oder ein Mittel gemäß Anspruch 8 auf die tierischen Schädlinge und/oder ihren Lebensraum einwirken lässt, wobei die chirurgische, therapeutische und diagnostische Behandlung des menschlichen oder tierischen Körpers ausgeschlossen ist.

10. Verwendung wenigstens einer Verbindung gemäß einem der Ansprüche 1 bis 7 oder eines Mittels gemäß Anspruch 8 zur Bekämpfung von tierischen Schädlingen, wobei die chirurgische, therapeutische und diagnostische Behandlung des menschlichen oder tierischen Körpers ausgeschlossen ist.

11. Verwendung wenigstens einer Verbindung gemäß einem der Ansprüche 1 bis 7 zum Schutz des Vermehrungsmaterials von Pflanzen.

12. Agrochemische Formulierung enthaltend wenigstens eine Verbindung gemäß einem der Ansprüche 1 bis 7 in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

13. Agrochemische Formulierung gemäß Anspruch 12, zusätzlich enthaltend einen weiteren agrochemischen Wirkstoff.

## Claims

1. Compounds of the formula (I) 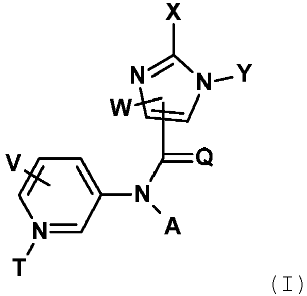 in which
Q represents oxygen or sulfur,
V represents a radical from the group consisting of hydrogen, halogen, alkyl, haloalkyl, alkoxy, haloalkoxy and cyano,
W represents a radical from the group consisting of hydrogen, halogen, alkyl, haloalkyl, alkoxy, haloalkoxy and cyano,
Y represents a radical from the group consisting of optionally substituted alkyl, alkenyl or alkynyl, optionally substituted cycloalkyl which is optionally interrupted by heteroatoms, optionally substituted cycloalkylalkyl which is optionally interrupted by heteroatoms, arylalkyl or hetarylalkyl and cyano,
A represents a radical from the group consisting of hydrogen, optionally substituted alkyl, alkenyl or alkynyl and optionally substituted cycloalkyl or cycloalkylalkyl which are optionally interrupted by heteroatoms,
T represents oxygen or an electron pair,
X represents C(E)U¹, C(O)OR², C(O)N(R³)(R⁴), C(S)N(R³)(R⁴), C(O)N(R³¹)-N(R³²)(R⁴¹), or C(S)N(R³¹)-N(R³²)(R⁴¹),
E represents the radicals O, N-OR²¹ and N-N(R³³)(R⁴³) or represents a cycle of the formula (X-1), 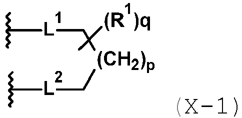
U¹ represents a radical from the group consisting of hydrogen, optionally substituted alkyl, alkenyl or alkynyl, optionally substituted cycloalkyl which is optionally interrupted by heteroatoms, optionally substituted cycloalkylalkyl, aryl, hetaryl, arylalkyl or hetarylalkyl which is optionally interrupted by heteroatoms,
L¹, L² each independently of one another represent O and S(O)ₘ,
R¹ represents a radical from the group consisting of halogen, cyano, optionally substituted alkyl, alkenyl, alkynyl or alkoxy and optionally substituted aryl,
or
R¹ represents an optionally substituted C₂-C₄-alkyl chain which replaces two H atoms attached to the same carbon atom, in which case q represents 2, or
R¹ represents an optionally substituted C₁-C₄-alkenyl chain, where the double bond replaces two H atoms attached to the same carbon atom, in which case q represents 1,
R² represents a radical from the group consisting of hydrogen, optionally substituted alkyl, alkenyl or alkynyl, optionally substituted cycloalkyl which is optionally interrupted by heteroatoms, optionally substituted cycloalkylalkyl, aryl, arylalkyl, hetaryl or hetarylalkyl which is optionally interrupted by heteroatoms,
R²¹ represents a radical from the group consisting of hydrogen, optionally substituted alkyl, alkenyl or alkynyl, optionally substituted cycloalkyl which is optionally interrupted by heteroatoms, represents optionally substituted cycloalkylalkyl or represents optionally substituted arylalkyl,
R³ represents a radical from the group consisting of hydrogen, optionally substituted alkyl, alkenyl, alkynyl, cycloalkyl, alkylsulfonyl, cycloalkylsulfonyl, alkoxycarbonyl, alkylcarbonyl or arylalkoxycarbonyl,
R⁴ represents a radical from the group consisting of hydrogen, optionally substituted alkyl, alkenyl, alkynyl or alkoxy, optionally substituted cycloalkyl or cycloalkenyl which is optionally interrupted by heteroatoms, optionally substituted cycloalkylalkyl which is optionally interrupted by heteroatoms, or represents optionally substituted aryl, arylalkyl, hetaryl and hetarylalkyl,
or
R³ and R⁴ together form an optionally substituted four- to seven-membered aliphatic ring which optionally contains a nitrogen, sulfur or oxygen atom,
R³¹, R³² and R³³ each independently of one another represent a radical from the group consisting of hydrogen, optionally substituted alkyl, alkenyl, alkynyl, cycloalkyl, alkoxycarbonyl, alkylcarbonyl, arylalkylcarbonyl or arylalkylalkoxycarbonyl,
R⁴¹, R⁴³ each independently of one another represent a radical from the group consisting of hydrogen, optionally substituted alkyl, alkenyl or alkynyl, optionally substituted cycloalkyl which is optionally interrupted by heteroatoms, optionally substituted cycloalkylalkyl, aryl, arylalkyl, hetaryl or hetarylalkyl which is optionally interrupted by heteroatoms,
m represents a number 0, 1 or 2,
p represents a number 0, 1 or 2,
q represents a number 0, 1 or 2,
and salts thereof.

2. Compounds according to Claim 1 in which
Q represents oxygen or sulfur,
V represents a radical from the group consisting of hydrogen, halogen, C₁-C₄-alkyl, C₁-C₄-haloalkyl, C₁-C₄-alkoxy, C₁-C₄-haloalkoxy and cyano,
W represents a radical from the group consisting of hydrogen, halogen, C₁-C₄-alkyl, C₁-C₄-haloalkyl, C₁-C₄-alkoxy, C₁-C₄-haloalkoxy and cyano,
Y represents a radical from the group consisting of C₁-C₆-alkyl, C₃-C₆-alkenyl or C₃-C₆-alkynyl, optionally mono- to polysubstituted independently of one another by halogen, C₁-C₄-alkoxy, C₁-C₄-alkyl-S(O)ₙ- or cyano, C₃-C₈-cycloalkyl, optionally mono- to disubstituted independently of one another by halogen, C₁-C₄-alkyl, C₁-C₄-haloalkyl, C₁-C₄-alkoxy or cyano, straight-chain or branched C₃-C₈-cycloalkyl-C₁-C₄-alkyl, optionally interrupted once or twice independently of one another by O, S(O)ₙ, CO or NR⁴ and optionally mono- to tetrasubstituted independently of one another by halogen, C₁-C₄-alkyl, C₁-C₄-haloalkyl, C₁-C₄-alkoxy or cyano, aryl-C₁-C₄-alkyl or hetaryl-C₁-C₄-alkyl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano,
A represents a radical from the group consisting of hydrogen, C₁-C₆-alkyl, C₃-C₆-alkenyl or C₃-C₆-alkynyl, optionally mono- to polysubstituted independently of one another by halogen, C₁-C₄-alkoxy, C₁-C₄-alkyl-S(O)n- or cyano and C₃-C₆-cycloalkyl or C₃-C₈-cycloalkyl-C₁-C₄-alkyl, optionally mono- to disubstituted independently of one another by halogen, C₁-C₄-alkyl, C₁-C₄-haloalkyl, C₁-C₄-alkoxy or cyano,
T represents oxygen or an electron pair,
X represents C(E)U¹, C(O)OR², C(O)N(R³)(R⁴), C(S)N(R³)(R⁴), C(O)N(R³¹)-N(R³²)(R⁴¹)or C(S)N(R³¹)-N(R³²)(R⁴¹),
E represents the radicals O, N-OR²¹, N-N(R³³)(R⁴³) or represents a cycle of the formula (X-1), 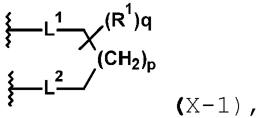
U¹ represents a radical from the group consisting of hydrogen, C₁-C₈-alkyl, C₃-C₈-alkenyl or C₃-C₈-alkynyl, optionally mono- to polysubstituted independently of one another by halogen, cyano, C₁-C₄-alkoxy or C₁-C₄-alkyl-S(O)ₙ-, C₃-C₈-cycloalkyl, optionally interrupted once by O or S(O)ₙ and optionally mono- to disubstituted independently of one another by halogen, C₁-C₄-alkyl, C₁-C₄-haloalkyl, C₁-C₄-alkoxy or cyano, straight-chain or branched C₃-C₈-cycloalkyl-C₁-C₄-alkyl, optionally interrupted once by O or S(O)ₙ and optionally mono- to disubstituted independently of one another by halogen, C₁-C₄-alkyl, C₁-C₄-haloalkyl, C₁-C₄-alkoxy or cyano, aryl or hetaryl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano, and straight-chain or branched aryl-C₁-C₄-alkyl or hetaryl-C₁-C₄-alkyl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano,
L¹, L² each independently of one another represent O and S(O)ₘ,
R¹ represents a radical from the group consisting of halogen, C₁-C₆-alkyl, C₂-C₆-alkenyl, C₃-C₆-alkynyl or C₁-C₆-alkoxy, optionally substituted by halogen, cyano, C₁-C₄-alkoxy or C₁-C₄-alkyl-S(O)ₙ-, and aryl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano,
or
R¹ represents a C₂-C₄-alkyl chain which replaces two H atoms attached to the same carbon atom, in which case n represents 2, and is optionally substituted by halogen, cyano, C₁-C₄-alkyl or C₁-C₄-alkoxy,
or
R¹ represents a C₁-C₄-alkenyl chain, where the double bond replaces two H atoms attached to the same carbon atom, in which case q represents 1, which C₁-C₄-alkenyl chain is optionally substituted by halogen, cyano or C₁-C₄-alkoxy,
R² represents a radical from the group consisting of hydrogen, C₁-C₈-alkyl, C₃-C₈-alkenyl or C₃-C₈-alkynyl, optionally mono- to polysubstituted independently of one another by halogen, C₁-C₄-alkoxy or C₁-C₄-alkyl-S(O)ₙ-, C₃-C₈-cycloalkyl, optionally mono- to disubstituted independently of one another by halogen, C₁-C₄-alkyl, C₁-C₄-haloalkyl, C₁-C₄-alkoxy or cyano, straight-chain or branched C₃-C₈-cycloalkyl-C₁-C₄-alkyl, optionally mono- to disubstituted independently of one another by halogen, C₁-C₄-alkyl, C₁-C₄-haloalkyl, C₁-C₄-alkoxy or cyano, aryl or hetaryl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano, and straight-chain or branched aryl-C₁-C₄-alkyl or hetaryl-C₁-C₄-alkyl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano,
R²¹ represents a radical from the group consisting of hydrogen, C₁-C₈-alkyl, C₃-C₈-alkenyl or C₃-C₈-alkynyl, optionally mono- to polysubstituted independently of one another by halogen, C₁-C₄-alkoxy or C₁-C₄-alkyl-S(O)ₙ-, C₃-C₈-cycloalkyl, optionally mono- to disubstituted independently of one another by halogen, C₁-C₄-alkyl, C₁-C₄-haloalkyl, C₁-C₄-alkoxy or cyano, straight-chain or branched C₃-C₈-cycloalkyl-C₁-C₄-alkyl, optionally mono- to disubstituted independently of one another by halogen, C₁-C₄-alkyl, C₁-C₄-haloalkyl, C₁-C₄-alkoxy or cyano, and represents straight-chain or branched aryl-C₁-C₄-alkyl or hetaryl-C₁-C₄-alkyl, optionally mono- to trisubstituted independently of one another by halogen, C₁-C₄-alkyl, C₁-C₄-haloalkyl, C₁-C₄-alkoxy, C₁-C₄-alkyl-S(O)ₙ-, C₁-C₄-haloalkoxy, C₁-C₄-haloalkyl-S(O)ₙ-, nitro or cyano,
R³ represents a radical from the group consisting of hydrogen, C₁-C₄-alkyl, C₃-C₆-alkenyl or C₃-C₆-alkynyl, optionally mono- to polysubstituted independently of one another by halogen, C₁-C₄-alkoxy, C₁-C₄-alkyl-S(O)ₙ- or cyano, C₃-C₆-cycloalkyl, C₁-C₄-alkylsulfonyl, C₃-C₆-cycloalkylsulfonyl, C₁-C₄-alkylcarbonyl or C₁-C₄-alkoxycarbonyl, optionally mono- to disubstituted independently of one another by halogen, C₁-C₄-alkyl, C₁-C₄-haloalkyl, C₁-C₄-alkoxy or cyano and represents phenyl-C₁-C₂-alkoxycarbonyl, optionally mono- to disubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
R⁴ represents a radical from the group consisting of hydrogen, C₁-C₄-alkyl, C₃-C₆-alkenyl, C₃-C₆-alkynyl or C₁-C₄-alkoxy, optionally mono- to polysubstituted independently of one another by halogen, hydroxy, C₁-C₄-alkoxy, C₁-C₄-alkoxycarbonyl, -C₁-C₄-alkyl-S(O)ₙ- or cyano, straight-chain or branched C₃-C₈-cycloalkyl or C₃-C₈-cycloalkyl-C₁-C₄-alkyl, optionally interrupted once or twice independently of one another by O, S(O)ₙ, CO or NR^{4a} and optionally mono- to tetrasubstituted independently of one another by halogen, C₁-C₄-alkyl, C₁-C₄-haloalkyl, C₁-C₄-alkoxy or cyano, aryl, hetaryl, arylalkyl or hetarylalkyl, optionally mono- to trisubstituted independently of one another by halogen, C₁-C₄-alkyl, C₁-C₄-haloalkyl, hydroxy, C₁-C₄-alkoxy, C₁-C₄-alkyl-S(O)ₙ-, C₁-C₄-haloalkoxy, C₁-C₄-haloalkyl-S(O)ₙ-, nitro or cyano, or
R³ and R⁴ together form a four- to seven-membered aliphatic ring which is optionally mono- to trisubstituted by halogen, C₁-C₄-alkyl or C₁-C₄-alkoxy and optionally contains a nitrogen, sulfur or oxygen atom,
R³¹, R³², R³³ each independently of one another represent a radical from the group consisting of hydrogen, C₁-C₆-alkyl, C₃-C₆-alkenyl or C₃-C₆-alkynyl, optionally mono- to polysubstituted independently of one another by halogen, C₁-C₄-alkoxy, C₁-C₄-alkyl-S (O)ₙ- or cyano or C₃-C₆-cycloalkyl, C₁-C₆-alkylcarbonyl, C₁-C₆-alkoxycarbonyl, aryl-C₁-C₄-alkyl or aryl-C₁-C₄-alkoxy, optionally mono- to trisubstituted independently of one another by halogen, C₁-C₄-alkyl, C₁-C₄-haloalkyl, C₁-C₄-alkoxy or cyano,
R⁴¹, R⁴³ each independently of one another represent a radical from the group consisting of hydrogen, C₁-C₄-alkyl, C₃-C₆-alkenyl or C₃-C₆-alkynyl, optionally mono- to polysubstituted independently of one another by halogen, C₁-C₄-alkoxy, C₁-C₄-alkyl-S(O)ₙ- or cyano, straight-chain or branched C₃-C₈-cycloalkyl or C₃-C₈-cycloalkyl-C₁-C₄-alkyl, optionally interrupted once or twice independently of one another by O, S(O)ₙ, CO or NR^{4a} and optionally mono- to tetrasubstituted independently of one another by halogen, C₁-C₄-alkyl, C₁-C₄-haloalkyl, C₁-C₄-alkoxy or cyano, aryl, hetaryl, arylalkyl or hetarylalkyl, optionally mono- to trisubstituted independently of one another by halogen, C₁-C₄-alkyl, C₁-C₄-haloalkyl, C₁-C₄-alkoxy, C₁-C₄-alkyl-S (O)ₙ-, C₁-C₄-haloalkoxy, C₁-C₄-haloalkyl-S(O)ₙ-, nitro or cyano,
m represents a number 0, 1 or 2,
n represents a number 0, 1 or 2,
p represents a number 0 or 1,
q represents a number 0, 1 or 2,
and salts thereof.

3. Compounds according to Claim 1 in which
Q represents oxygen,
V represents a radical from the group consisting of hydrogen, fluorine, chlorine, bromine, methyl and ethyl,
W represents a radical from the group consisting of hydrogen, fluorine, chlorine, bromine, cyano and methyl,
Y represents a radical from the group consisting of C₁-C₄-alkyl, C₃-C₄-alkenyl or C₃-C₄-alkynyl, optionally mono- to pentasubstituted independently of one another by fluorine, chlorine, bromine, methoxy or ethoxy, C₃-C₆-cycloalkyl or aryl-C₁-C₄-alkyl, optionally mono-to disubstituted independently of one another by fluorine, chlorine, bromine, methyl, ethyl, trifluoromethyl, methoxy, ethoxy or cyano,
A represents a radical from the group consisting of hydrogen, C₁-C₆-alkyl, C₃-C₆-alkenyl or C₃-C₆-alkynyl, optionally mono- to pentasubstituted independently of one another by fluorine, chlorine, bromine, methoxy, ethoxy or cyano, and C₃-C₆-cycloalkyl, optionally mono- to disubstituted independently of one another by fluorine, chlorine, bromine, methyl, ethyl, trifluoromethyl, methoxy, ethoxy or cyano,
T represents an electron pair,
X represents C(E)U¹, C(O)OR², C(O)N(R³)(R⁴) or C(S)N(R³) (R⁴) ,
E represents the radicals O, N-OR²¹, N-N(R³³) (R⁴³) or represents a cycle of the formula (X-1), 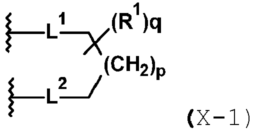
U¹ represents a radical from the group consisting of hydrogen, C₁-C₆-alkyl, C₃-C₆-alkenyl or C₃-C₆-alkynyl, optionally mono- to pentasubstituted independently of one another by fluorine, chlorine, bromine, methoxy, ethoxy or cyano, and C₃-C₆-cycloalkyl, optionally mono- to disubstituted independently of one another by fluorine, chlorine, bromine, methyl, ethyl, trifluoromethyl, methoxy, ethoxy or cyano, represents phenyl, pyridyl, pyrimidinyl or thienyl, optionally mono- to disubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano, represents straight-chain or branched phenyl-C₁-C₂-alkyl or pyridyl-C₁-C₂-alkyl, optionally mono- to disubstituted independently of one another by fluorine, chlorine, bromine, methyl, ethyl, difluoromethyl, trifluoromethyl, methoxy, ethoxy, Me-S(O)ₙ-, Et-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
L¹, L² each independently of one another represent O and S(O)ₘ,
R¹ represents C₁-C₄-alkyl, C₂-C₄-alkenyl, C₃-C₄-alkynyl or C₁-C₄-alkoxy, optionally substituted by halogen, methoxy, ethoxy,
or
R¹ represents a C₂-C₄-alkyl chain which replaces two H atoms attached to the same carbon atom, in which case n represents 2,
or
R¹ represents a C₁-C₄-alkenyl chain, where the double bond replaces two H atoms attached to the same carbon atom and in which case n represents 1,
R² represents a radical from the group consisting of hydrogen, C₁-C₆-alkyl, C₃-C₆-alkenyl or C₃-C₆-alkynyl, mono- to pentasubstituted independently of one another by fluorine, chlorine, bromine, methoxy, ethoxy or cyano, C₃-C₆-cycloalkyl, optionally mono- to disubstituted independently of one another by fluorine, chlorine, bromine, methyl, ethyl, trifluoromethyl, methoxy, ethoxy or cyano, C₃-C₆-cycloalkyl-C₁-C₄-alkyl, optionally mono-to disubstituted independently of one another by fluorine, chlorine, bromine, methyl, ethyl, trifluoromethyl, methoxy, ethoxy or cyano, represents phenyl, pyridyl or thienyl, optionally mono- to trisubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, difluoromethoxy, trifluoromethoxy, trifluoromethyl, methyl-S(O)ₙ-, difluoroethyl-S(O)ₙ-, trifluoroethyl-S(O)ₙ-, nitro or cyano, and represents straight-chain or branched phenyl-C₁-C₂-alkyl or pyridyl-C₁-C₂-alkyl, optionally mono- to disubstituted independently of one another by fluorine, chlorine, bromine, methyl, ethyl, difluoromethyl, trifluoromethyl, methoxy, ethoxy, Me-S(O)ₙ-, Et-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
R²¹ represents a radical from the group consisting of hydrogen, C₁-C₆-alkyl, C₃-C₆-alkenyl or C₃-C₆-alkynyl, mono- to pentasubstituted independently of one another by fluorine, chlorine, bromine, methoxy, ethoxy or cyano, C₃-C₆-cycloalkyl, optionally mono- to disubstituted independently of one another by fluorine, chlorine, bromine, methyl, ethyl, trifluoromethyl, methoxy, ethoxy or cyano, C₃-C₆-cycloalkyl-C₁-C₄-alkyl, optionally mono- to disubstituted independently of one another by fluorine, chlorine, bromine, methyl, ethyl, trifluoromethyl, methoxy, ethoxy or cyano, and represents straight-chain or branched phenyl-C₁-C₂-alkyl or pyridyl-C₁-C₂-alkyl, optionally mono- to disubstituted independently of one another by fluorine, chlorine, bromine, methyl, ethyl, difluoromethyl, trifluoromethyl, methoxy, ethoxy, Me-S(O)ₙ-, Et-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
R³ represents a radical from the group consisting of hydrogen, C₁-C₄-alkyl, C₃-C₄-alkenyl or C₃-C₄-alkynyl, optionally mono- to trisubstituted independently of one another by fluorine or chlorine, C₃-C₆-cycloalkyl, optionally mono- to disubstituted independently of one another by fluorine, chlorine, methyl, ethyl, trifluoromethyl, methoxy, ethoxy or cyano, or represents C₁-C₄-alkylsulfonyl, C₃-C₆-cycloalkylsulfonyl, C₁-C₄-alkylcarbonyl or C₁-C₄-alkoxycarbonyl, optionally mono- to trisubstituted independently of one another by fluorine, chlorine, methoxy or ethoxy, and represents phenyl-C₁-C₂-alkoxycarbonyl, optionally mono- to disubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
R⁴ represents a radical from the group consisting of hydrogen, C₁-C₄-alkyl, C₃-C₆-alkenyl, C₃-C₆-alkynyl or C₁-C₄-alkoxy, optionally mono- to trisubstituted independently of one another by halogen, hydroxy, methoxy, ethoxy, n- or isopropoxy, methoxycarbonyl, ethoxycarbonyl, cyano or methyl-S (O)ₙ-, C₃-C₆-cycloalkyl or C₃-C₆-cycloalkyl-C₁-C₄-alkyl, optionally mono- to disubstituted independently of one another by fluorine, chlorine, methyl, ethyl, trifluoromethyl, methoxy, ethoxy or cyano, phenyl, naphthyl, pyridyl, pyrimidyl, pyridazinyl, pyrazinyl, triazinyl, furanyl, thienyl, oxazolyl, isoxazolyl, thiazolyl, isothiazolyl, pyrazolyl, imidazolyl, oxdiazolyl or thiadiazolyl, optionally mono- to disubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano, straight-chain or branched phenyl-C₁-C₂-alkyl or pyridyl-C₁-C₂-alkyl, optionally mono- to disubstituted independently of one another by fluorine, chlorine, bromine, methyl, ethyl, difluoromethyl, trifluoromethyl, methoxy, ethoxy, Me-S(O)ₙ-, Et-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano, or
R³ and R⁴ together form a four- to six-membered aliphatic ring which optionally contains a nitrogen, sulfur or oxygen atom and is optionally mono- to disubstituted by fluorine, chlorine, bromine, methyl, ethyl, methoxy or ethoxy,
R³³ represents a radical from the group consisting of hydrogen, C₁-C₄-alkyl, optionally mono- to trisubstituted independently of one another by fluorine or chlorine, represents C₁-C₆-alkylcarbonyl or C₁-C₆-alkoxycarbonyl, optionally mono- to trisubstituted independently of one another by fluorine, chlorine, methoxy or ethoxy and represents phenyl-C₁-C₂-alkoxycarbonyl, optionally mono- to disubstituted independently of one another by fluorine, chlorine, bromine, methyl, ethyl, trifluoromethyl, methoxy, ethoxy, trifluoromethoxy, nitro or cyano,
R⁴³ represents a radical from the group consisting of hydrogen and C₁-C₄-alkyl which is optionally mono- to trisubstituted independently of one another by halogen, methoxy or ethoxy,
m represents 0,
n represents a number 0, 1 or 2,
p represents a number 0 or 1,
q represents a number 0, 1 or 2,
and salts thereof.

4. Compounds according to Claim 1 of the formula (I-1) 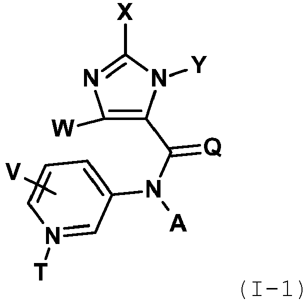
Q represents oxygen,
V represents a radical from the group consisting of hydrogen, fluorine, chlorine, methyl and cyano,
W represents a radical from the group consisting of hydrogen, fluorine, chlorine, bromine, methyl,
Y represents a radical from the group consisting of methyl, ethyl, propyl, allyl or propargyl, optionally mono- to trisubstituted independently of one another by fluorine, methoxy, ethoxy or cyano,
A represents a radical from the group consisting of hydrogen; methyl, ethyl, propyl, allyl, propargyl or cyclopropyl, optionally mono- to trisubstituted independently of one another by fluorine, methoxy, ethoxy or cyano,
T represents an electron pair,
X represents C(E)U¹, C(O)OR², C(O)N(R³) (R⁴) or C(S)N(R³) (R⁴) ,
E represents the radicals O, N-OR²¹, N-N(R³³) (R⁴³) or represents a cycle of the formula (X-1), 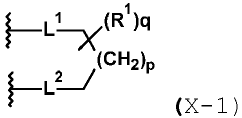
U¹ represents a radical from the group consisting of hydrogen and C₁-C₆-alkyl, optionally mono- to pentasubstituted independently of one another by fluorine, chlorine, bromine, methoxy, ethoxy or cyano, or represents C₃-C₆-cycloalkyl,
L¹, L² each independently of one another represent O and S(O)ₘ,
R¹ represents C₁-C₄-alkyl, optionally substituted by fluorine, chlorine, methoxy or ethoxy,
or
R¹ represents a C₂-C₄-alkyl chain which replaces two H atoms attached to the same carbon atom, in which case n represents 2,
R² represents a radical from the group consisting of hydrogen, C₁-C₆-alkyl, mono- to trisubstituted independently of one another by fluorine, chlorine, bromine, methoxy, ethoxy or cyano, or represents phenylmethyl, optionally mono- to disubstituted independently of one another by fluorine, chlorine, bromine, methyl, ethyl, difluoromethyl, trifluoromethyl, methoxy, ethoxy, Me-S(O)ₙ-, Et-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
R²¹ represents a radical from the group consisting of hydrogen, C₁-C₆-alkyl, C₃-C₆-alkenyl or C₃-C₆-alkynyl, mono- to trisubstituted independently of one another by fluorine, chlorine, bromine, methoxy, ethoxy or cyano, C₃-C₆-cycloalkyl, optionally mono- to disubstituted independently of one another by fluorine, chlorine, bromine, methyl, ethyl, methoxy or ethoxy, C₃-C₆-cycloalkyl-C₁-C₄-alkyl, optionally substituted by fluorine, chlorine, bromine, methyl, ethyl, methoxy or ethoxy, phenylmethyl or pyridylmethyl, optionally mono- to disubstituted independently of one another by fluorine, chlorine, bromine, methyl, ethyl, difluoromethyl, trifluoromethyl, methoxy, ethoxy, Me-S(O)ₙ-, Et-S(O)ₙ-, difluoromethoxy, trifluoromethoxy, trifluoromethyl-S(O)ₙ-, difluoroethyl-S(O)ₙ-, trifluoroethyl-S(O)ₙ-, nitro or cyano,
R³ represents a radical from the group consisting of hydrogen, C₁-C₄-alkyl, C₁-C₄-alkylsulfonyl, C₃-C₆-cycloalkylsulfonyl, C₁-C₄-alkylcarbonyl or C₁-C₄-alkoxycarbonyl, optionally mono- to trisubstituted independently of one another by fluorine, chlorine, methoxy or ethoxy, and arylmethoxycarbonyl, optionally mono- to trisubstituted independently of one another by fluorine, chlorine, bromine, methyl, ethyl, trifluoromethyl, methoxy or ethoxy,
R⁴ represents a radical from the group consisting of hydrogen, C₁-C₄-alkyl, C₃-C₆-alkenyl, C₃-C₆-alkynyl or C₁-C₄-alkoxy, mono- to trisubstituted independently of one another by halogen, hydroxy, methoxy, ethoxy, methoxycarbonyl, ethoxycarbonyl or cyano, C₃-C₆-cycloalkyl or C₃-C₆-cycloalkyl-C₁-C₄-alkyl, optionally mono- to disubstituted independently of one another by fluorine, chlorine, methyl, ethyl, trifluoromethyl, methoxy, ethoxy or cyano, phenyl, pyridyl or thienyl, optionally mono- to disubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, trifluoromethoxy, nitro or cyano, phenyl-C₁-C₂-alkyl or pyridyl-C₁-C₂-alkyl, optionally mono-to disubstituted independently of one another by fluorine, chlorine, bromine, methyl, ethyl, difluoromethyl, trifluoromethyl, methoxy, ethoxy, methyl-S(O)ₙ-, ethyl-S(O)ₙ-, trifluoromethoxy, nitro or cyano, or
R³ and R⁴ together form a four- to six-membered aliphatic ring which optionally contains a nitrogen, sulfur or oxygen atom and is optionally mono- to disubstituted by fluorine, chlorine, bromine, methyl, ethyl, methoxy or ethoxy,
R³³ represents a radical from the group consisting of hydrogen, C₁-C₄-alkylcarbonyl or C₁-C₄-alkoxycarbonyl, optionally substituted by methoxy or ethoxy, and represents phenylmethoxycarbonyl, optionally mono- to trisubstituted independently of one another by fluorine, chlorine, methyl or ethyl,
R⁴³ represents a radical from the group consisting of hydrogen and C₁-C₄-alkyl,
m represents 0,
n represents a number 0, 1 or 2,
p represents a number 0 or 1,
q represents a number 0, 1 or 2,
and salts thereof.

5. Compounds according to Claim 4 in which
Q represents oxygen,
V represents hydrogen or methyl,
W represents hydrogen,
Y represents a radical from the group consisting of methyl, ethyl, propyl, allyl and propargyl,
A represents a radical from the group consisting of hydrogen, methyl, ethyl and cyclopropyl,
T represents an electron pair,
X represents C(E)U¹, C(O)OR², C(O)N(R³) (R⁴) or C(S)N(R³) (R⁴) ,
E represents the radicals O, N-OR²¹, N-N(R³³) (R⁴³) or represents a cycle of the formula (X-1), 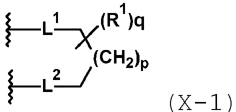
U¹ represents a radical from the group consisting of hydrogen, methyl, ethyl, n- or isopropyl, 1-cyano-1-methylethyl and cyclopropyl,
L¹, L² each independently of one another represent O and S(O)ₘ,
R¹ represents methyl, ethyl, n-, isopropyl or methoxymethyl,
or
R¹ represents -(CH₂)₂-, resulting in the formation of a spiro-cyclopropyl group,
R² represents a radical from the group consisting of hydrogen, methyl, ethyl, n- or isopropyl,
R²¹ represents a radical from the group consisting of hydrogen, methyl, ethyl, n- or isopropyl, n-, iso-, sec- or tert-butyl, 2,2-dimethylpropyl, propenyl, propynyl, 2,2,2-trifluoroethyl, cyclopropyl, cyclobutyl, cyclopentyl, cyclohexyl, cyclopropylmethyl, 2-Cl-phenylmethyl, 3-Cl-phenylmethyl, 4-Cl-phenylmethyl, pyrid-2-ylmethyl, pyrid-3-ylmethyl or pyrid-4-ylmethyl,
R³ represents a radical from the group consisting of hydrogen, methyl, ethyl, methylsulfonyl, cyclopropylsulfonyl, methoxycarbonyl, ethoxycarbonyl, 2-methoxyethoxycarbonyl n-, iso-, sec- or t-butoxycarbonyl,
R⁴ represents a radical from the group consisting of hydrogen, methyl, ethyl, n- or isopropyl, n-, iso-, sec- or tert-butyl, 2,2,2-trifluoroethyl, 2-hydroxyethyl, 2-methoxyethyl, cyanomethyl, methoxycarbonylmethyl, ethoxycarbonylmethyl, cyclopropyl, 1-cyanocyclopropyl, cyclopropylmethyl, cyclobutyl, cyclopentyl, cyclohexyl, methoxy, ethoxy, phenyl, 2-Cl-phenyl, 3-Cl-phenyl, 4-Cl-phenyl, pyridyl, phenylmethyl, 1-phenyl-2-hydroxyethyl, 2-Cl-phenylmethyl, 3-Cl-phenylmethyl, 4-Cl-phenylmethyl or
R³ and R⁴ together represent (CH₂)₅, (CH₂)₄, (CH₂)₃ or (CH₂)₂O(CH₂)₂,
R³³ represents a radical from the group consisting of methoxycarbonyl, ethoxycarbonyl, n- or isopropoxycarbonyl, n-, iso-, sec- or t-butoxycarbonyl, isopentoxycarbonyl, 2-methoxyethoxycarbonyl or phenylmethoxycarbonyl,
R⁴³ represents a radical from the group consisting of hydrogen, methyl, ethyl, n- or isopropyl, n-, iso-, sec- or tert-butyl,
m represents 0,
p represents a number 0 or 1,
q represents a number 0, 1 or 2,
and salts thereof.

6. Compounds according to Claim 4 in which
Q represents oxygen,
V represents hydrogen or methyl,
W represents hydrogen,
Y represents methyl,
A represents methyl, ethyl or cyclopropyl,
T represents an electron pair,
X represents C(E)U¹, C(O)OR², C(O)N(R³) (R⁴) or C(S)N(R³) (R⁴) ,
E represents the radicals O, N-OR²¹, N-N(R³³) (R⁴³) or represents a cycle of the formula (X-1), 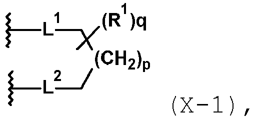
U¹ represents hydrogen, methyl, ethyl, isopropyl, 1-cyano-1-methylethyl or cyclopropyl,
L¹, L² each independently of one another represent O and S(O)ₘ,
R¹ represents methyl, ethyl or methoxymethyl, or
R¹ represents -(CH₂)₂-, resulting in the formation of a spiro-cyclopropyl group,
R² represents methyl or ethyl,
R²¹ represents a radical from the group consisting of methyl, ethyl, n- or isopropyl, n-, iso- or tert-butyl, 2,2-dimethylpropyl, propenyl, propynyl, 2,2,2-trifluoroethyl, cyclopentyl, cyclopropylmethyl, 4-Cl-phenylmethyl, pyrid-2-ylmethyl, pyrid-3-ylmethyl,
R³ represents a radical from the group consisting of hydrogen, methyl, ethyl, methylsulfonyl and cyclopropylsulfonyl.
R⁴ represents a radical from the group consisting of hydrogen, methyl, ethyl, isopropyl, isobutyl, tert-butyl, 2,2,2-trifluoroethyl, 2-hydroxyethyl, 2-methoxyethyl, cyanomethyl, methoxycarbonylmethyl, ethoxycarbonylmethyl, cyclopropyl, 1-cyanocyclopropyl, cyclopentyl, cyclopropylmethyl, methoxy, ethoxy, phenyl, pyrid-3-yl, phenylmethyl, 1-phenyl-2-hydroxyethyl,
R³ and R⁴ together represent (CH₂)₅, (CH₂)₄, (CH₂)₃ or (CH₂)₂O(CH₂)₂,
R³³ represents a radical from the group consisting of methoxycarbonyl, ethoxycarbonyl, t-butoxycarbonyl, 2-methoxyethoxycarbonyl and phenylmethoxycarbonyl,
R⁴³ represents a radical from the group consisting of hydrogen, methyl and n-butyl,
m represents 0,
p represents a number 0 or 1,
q represents a number 0, 1 or 2,
and salts thereof.

7. Compound according to any of Claims 1 to 6 of any of the formulae (I-1a) to (I-1f)

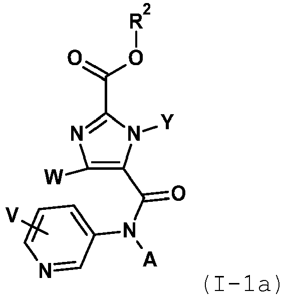

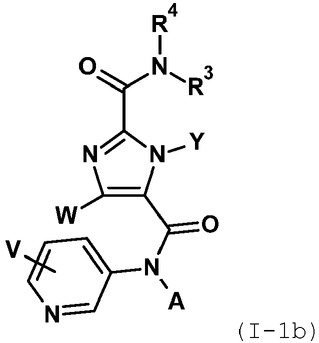

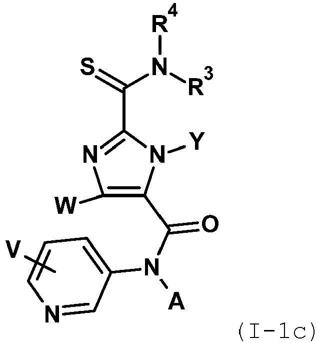

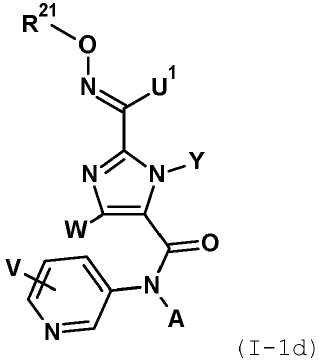

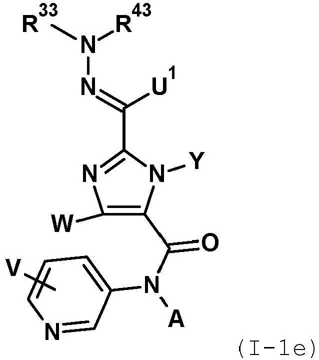

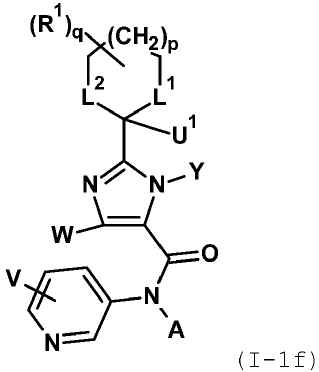

8. Composition, comprising at least one compound according to any of Claims 1 to 7 and customary extenders and/or surfactants in particular for controlling animal pests.

9. Method for controlling animal pests, where at least one compound according to any of Claims 1 to 7 or a composition according to Claim 8 is allowed to act on the animal pests and/or their habitat, where the surgical, therapeutic and diagnostic treatment of the human or animal body is excluded.

10. Use of at least one compound according to any of Claims 1 to 7 or of a composition according to Claim 8 for controlling animal pests, where the surgical, therapeutic and diagnostic treatment of the human or animal body is excluded.

11. Use of at least one compound according to any of Claims 1 to 7 for protecting the propagation material of plants.

12. Agrochemical formulation comprising at least one compound according to any of Claims 1 to 7 in biologically effective amounts of from 0.00000001 to 98% by weight, based on the weight of the agrochemical formulation, and also extenders and/or surfactants.

13. Agrochemical formulation according to Claim 12, additionally comprising a further agrochemically active compound.

## Revendications

1. Composés de formule (I) 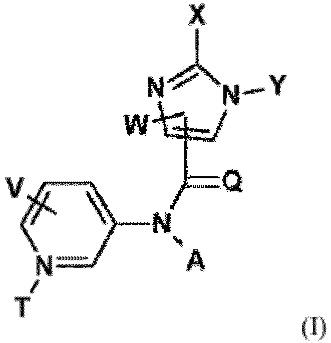 dans laquelle
Q représente oxygène ou soufre,
V représente un radical de la série hydrogène, halogène, alkyle, halogénoalkyle, alcoxy, halogénoalcoxy et cyano,
W représente un radical de la série hydrogène, halogène, alkyle, halogénoalkyle, alcoxy, halogénoalcoxy et cyano,
Y représente un radical de la série alkyle, alcényle ou alcynyle éventuellement substitué, cycloalkyle éventuellement interrompu par des hétéroatomes et éventuellement substitué, cycloalkylalkyle, arylalkyle ou hétarylalkyle éventuellement interrompu par des hétéroatomes et éventuellement substitué, et cyano,
A représente un radical de la série hydrogène, alkyle, alcényle ou alcynyle éventuellement substitué, cycloalkyle ou cycloalkyle alkyle éventuellement interrompu par des hétéroatomes et éventuellement substitué,
T représente oxygène ou une paire d'électrons,
X représente C(E)U¹, C(O)OR², C(O)N(R³) (R⁴) , C(S)N(R³) (R⁴), C(O)N(R³¹)-N(R³²)(R⁴¹), ou C(S)N(R³¹)-N(R³²)(R⁴¹),
E représente les radicaux O, N-OR²¹ et N-N(R³³) (R⁴³) ou un cycle de formule (X-1), 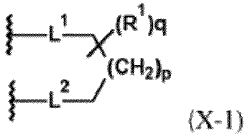
U¹ représente un radical de la série hydrogène, alkyle, alcényle ou alcynyle éventuellement substitué, cycloalkyle éventuellement interrompu par des hétéroatomes et éventuellement substitué, cycloalkylalkyle, aryle, hétaryle, arylalkyle ou hétarylalkyle éventuellement interrompu par des hétéroatomes et éventuellement substitué,
L¹, L² représentent à chaque fois indépendamment l'un de l'autre O et S(O)ₘ,
R¹ représente un radical de la série halogène, cyano, alkyle, alcényle, alcynyle ou alcoxy éventuellement substitué et aryle éventuellement substitué,
ou
R¹ représente une chaîne C₂-C₄-alkyle éventuellement substituée, qui remplace deux atomes de H liés au même atome de C, q représentant alors 2, ou
R¹ représente une chaîne C₁-C₄-alcényle éventuellement substituée, la double liaison remplaçant deux atomes de H liés au même atome de C, q représentant alors 1,
R² représente un radical de la série hydrogène, alkyle, alcényle ou alcynyle éventuellement substitué, cycloalkyle éventuellement interrompu par des hétéroatomes et éventuellement substitué, cycloalkylalkyle, aryle, arylalkyle, hétaryle ou hétarylalkyle éventuellement interrompu par des hétéroatomes et éventuellement substitué,
R²¹ représente un radical de la série hydrogène, alkyle, alcényle ou alcynyle éventuellement substitué, cycloalkyle éventuellement interrompu par des hétéroatomes et éventuellement substitué, cycloalkylalkyle éventuellement substitué ou arylalkyle éventuellement substitué,
R³ représente un radical de la série hydrogène, alkyle, alcényle, alcynyle, cycloalkyle, alkylsulfonyle, cycloalkylsulfonyle, alcoxycarbonyle, alkylcarbonyle ou arylalcoxycarbonyle éventuellement substitué,
R⁴ représente un radical de la série hydrogène, alkyle, alcényle, alcynyle ou alcoxy éventuellement substitué, cycloalkyle ou cycloalcényle éventuellement interrompu par des hétéroatomes et éventuellement substitué, cycloalkylalkyle éventuellement interrompu par des hétéroatomes et éventuellement substitué, ou aryle, arylalkyle, hétaryle et hétarylalkyle éventuellement substitués,
ou
R³ et R⁴ forment ensemble un cycle aliphatique ayant quatre à sept chaînons éventuellement substitué, qui contient éventuellement un atome de N, de S ou de O,
R³¹, R³² et R³³ représentent à chaque fois indépendamment l'un de l'autre un radical de la série hydrogène, alkyle, alcényle, alcynyle, cycloalkyle, alcoxycarbonyle, alkylcarbonyle, arylalkylcarbonyle ou arylalkylalcoxycarbonyle éventuellement substitué,
R⁴¹, R⁴³ représentent à chaque fois indépendamment l'un de l'autre un radical de la série hydrogène, alkyle, alcényle ou alcynyle éventuellement substitué, cycloalkyle éventuellement interrompu par des hétéroatomes et éventuellement substitué, cycloalkylalkyle, aryle, arylalkyle, hétaryle ou hétarylalkyle éventuellement interrompu par des hétéroatomes et éventuellement substitué,
m représente le nombre 0, 1 ou 2,
p représente le nombre 0, 1 ou 2,
q représente le nombre 0, 1 ou 2
et leurs sels.

2. Composés selon la revendication 1, dans lesquels
Q représente oxygène ou soufre,
V représente un radical de la série hydrogène, halogène, C₁-C₄-alkyle, C₁-C₄-halogénoalkyle, C₁-C₄-alcoxy, C₁-C₄-halogénoalcoxy et cyano,
W représente un radical de la série hydrogène, halogène, C₁-C₄-alkyle, C₁-C₄-halogénoalkyle, C₁-C₄-alcoxy, C₁-C₄-halogénoalcoxy et cyano,
Y représente un radical de la série C₁-C₆-alkyle, C₃-C₆-alcényle ou C₃-C₆-alcynyle éventuellement monosubstitué à polysubstitué indépendamment l'un de l'autre par halogène, C₁-C₄-alcoxy, C₁-C₄-alkyl-S(O)ₙ- ou cyano, C₃-C₈-cycloalkyle éventuellement monosubstitué ou disubstitué indépendamment l'un de l'autre par halogène, C₁-C₄-alkyle, C₁-C₄-halogénoalkyle, C₁-C₄-alcoxy ou cyano, C₃-C₈-cycloalkyl-C₁-C₄-alkyle linéaire ou ramifié, éventuellement interrompu une ou deux fois indépendamment l'un de l'autre par O, S(O)ₙ, CO ou NR⁴ et éventuellement monosubstitué à tétrasubstitué indépendamment l'un de l'autre par halogène, C₁-C₄-alkyle, C₁-C₄-halogénoalkyle, C₁-C₄-alcoxy ou cyano, aryl-C₁-C₄-alkyle ou hétaryl-C₁-C₄-alkyle éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S(O)ₙ-, C₁-C₄-halogénoalcoxy, C₁-C₄-halogénoalkyl-S(O)ₙ-, nitro ou cyano,
A représente un radical de la série hydrogène, C₁-C₆-alkyle, C₃-C₆-alcényle ou C₃-C₆-alcynyle éventuellement monosubstitué à polysubstitué indépendamment l'un de l'autre par halogène, C₁-C₄-alcoxy, C₁-C₄-alkyl-S (O)n- ou cyano et C₃-C₆-cycloalkyle ou C₃-C₈-cycloalkyl-C₁-C₄-alkyle éventuellement monosubstitué ou disubstitué indépendamment l'un de l'autre par halogène, C₁-C₄-alkyle, C₁-C₄-halogénoalkyle, C₁-C₄-alcoxy ou cyano,
T représente oxygène ou une paire d'électrons
X représente C(E)U¹, C(O)OR², C(O)N(R³) (R⁴) , C(S)N(R³) (R⁴), C(O)N(R³¹)-N(R³²)(R⁴¹) ou C(S)N(R³¹)-N(R³²)(R⁴¹),
E représente les radicaux O, N-OR²¹, N-N(R³³)(R⁴³) ou un cycle de formule (X-1), 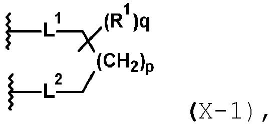
U¹ représente un radical de la série hydrogène, C₁-C₈-alkyle, C₃-C₈-alcényle ou C₃-C₈-alcynyle éventuellement monosubstitué à polysubstitué indépendamment l'un de l'autre par halogène, cyano, C₁-C₄-alcoxy ou C₁-C₄-alkyl-S (O)ₙ-, C₃-C₈-cycloalkyle éventuellement interrompu une fois par O ou S(O)ₙ et éventuellement monosubstitué ou disubstitué indépendamment l'un de l'autre par halogène, C₁-C₄-alkyle, C₁-C₄-halogénoalkyle, C₁-C₄-alcoxy ou cyano, C₃-C₈-cycloalkyl-C₁-C₄-alkyle linéaire ou ramifié, éventuellement interrompu une fois par O ou S(O)ₙ et éventuellement monosubstitué ou disubstitué indépendamment l'un de l'autre par halogène, C₁-C₄-alkyle, C₁-C₄-halogénoalkyle, C₁-C₄-alcoxy ou cyano, aryle ou hétaryle éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S(O)ₙ-, C₁-C₄-halogénoalcoxy, C₁-C₄-halogénoalkyl-S(O)ₙ-, nitro ou cyano, et aryl-C₁-C₄-alkyle ou hétaryl-C₁-C₄-alkyle linéaire ou ramifié, éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S (O)ₙ-, C₁-C₄-halogénoalcoxy, C₁-C₄-halogénoalkyl-S(O)ₙ-, nitro ou cyano,
L¹, L² représentent à chaque fois indépendamment l'un de l'autre O et S(O)ₘ,
R¹ représente un radical de la série halogène, C₁-C₆-alkyle, C₂-C₆-alcényle, C₃-C₆-alcynyle ou C₁-C₆-alcoxy éventuellement substitué par halogène, cyano, C₁-C₄-alcoxy ou C₁-C₄-alkyl-S (O)ₙ-, et aryle éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S (O)ₙ-, C₁-C₄-halogénoalcoxy, C₁-C₄-halogénoalkyl-S(O)ₙ-, nitro ou cyano,
ou
R¹ représente une chaîne C₂-C₄-alkyle éventuellement substituée par halogène, cyano, C₁-C₄-alkyle ou C₁-C₄-alcoxy, qui remplace deux atomes de H liés au même atome de C, n représentant alors 2,
ou
R¹ représente une chaîne C₁-C₄-alcényle éventuellement substituée par halogène, cyano, ou C₁-C₄-alcoxy, la double liaison remplaçant deux atomes de H liés au même atome de C et q représentant alors 1,
R² représente un radical de la série hydrogène, C₁-C₈-alkyle, C₃-C₈-alcényle ou C₃-C₈-alcynyle éventuellement monosubstitué à polysubstitué indépendamment l'un de l'autre par halogène, C₁-C₄-alcoxy ou C₁-C₄-alkyl-S (O)ₙ-, C₃-C₈-cycloalkyle éventuellement monosubstitué ou disubstitué indépendamment l'un de l'autre par halogène, C₁-C₄-alkyle, C₁-C₄-halogénoalkyle, C₁-C₄-alcoxy ou cyano, C₃-C₈-cycloalkyl-C₁-C₄-alkyle linéaire ou ramifié, éventuellement monosubstitué ou disubstitué indépendamment l'un de l'autre par halogène, C₁-C₄-alkyle, C₁-C₄-halogénoalkyle, C₁-C₄-alcoxy ou cyano, aryle ou hétaryle éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S (O)ₙ-, C₁-C₄-halogénoalcoxy, C₁-C₄-halogénoalkyl-S(O)ₙ-, nitro ou cyano, et aryl-C₁-C₄-alkyle ou hétaryl-C₁-C₄-alkyle linéaire ou ramifié, éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S(O)ₙ-, C₁-C₄-halogénoalcoxy, C₁-C₄-halogénoalkyl-S(O)ₙ-, nitro ou cyano,
R²¹ représente un radical de la série hydrogène, C₁-C₈-alkyle, C₃-C₈-alcényle ou C₃-C₈-alcynyle éventuellement monosubstitué à polysubstitué indépendamment l'un de l'autre par halogène, C₁-C₄-alcoxy ou C₁-C₄-alkyl-S (O)ₙ-, C₃-C₈-cycloalkyle éventuellement monosubstitué ou disubstitué indépendamment l'un de l'autre par halogène, C₁-C₄-alkyle, C₁-C₄-halogénoalkyle, C₁-C₄-alcoxy ou cyano, C₃-C₈-cycloalkyl-C₁-C₄-alkyle linéaire ou ramifié, éventuellement monosubstitué ou disubstitué indépendamment l'un de l'autre par halogène, C₁-C₄-alkyle, C₁-C₄-halogénoalkyle, C₁-C₄-alcoxy ou cyano, et aryl-C₁-C₄-alkyle ou hétaryl-C₁-C₄-alkyle linéaire ou ramifié, éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S(O)ₙ-, C₁-C₄-halogénoalcoxy, C₁-C₄-halogénoalkyl-S(O)ₙ-, nitro ou cyano,
R³ représente un radical de la série hydrogène, C₁-C₄-alkyle, C₃-C₆-alcényle ou C₃-C₆-alcynyle éventuellement monosubstitué à polysubstitué indépendamment l'un de l'autre par halogène, C₁-C₄-alcoxy, C₁-C₄-alkyl-S (O)ₙ- ou cyano, C₃-C₆-cycloalkyle, C₁-C₄-alkylsulfonyle, C₃-C₆-cycloalkylsulfonyle, C₁-C₄-alkylcarbonyle ou C₁-C₄-alcoxycarbonyle éventuellement monosubstitué ou disubstitué indépendamment l'un de l'autre par halogène, C₁-C₄-alkyle, C₁-C₄-halogénoalkyle, C₁-C₄-alcoxy ou cyano et phényl-C₁-C₂-alcoxycarbonyle éventuellement monosubstitué ou disubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
R⁴ représente un radical de la série hydrogène, C₁-C₄-alkyle, C₃-C₆-alcényle, C₃-C₆-alcynyle ou C₁-C₄-alcoxy éventuellement monosubstitué à polysubstitué indépendamment l'un de l'autre par halogène, hydroxy, C₁-C₄-alcoxy, C₁-C₄-alcoxycarbonyle, -C₁-C₄-alkyl-S(O)ₙ- ou cyano, C₃-C₈-cycloalkyle ou C₃-C₈-cycloalkyl-C₁-C₄-alkyle linéaire ou ramifié, éventuellement interrompu une ou deux fois indépendamment l'un de l'autre par O, S(O)ₙ, CO ou NR^{4a} et éventuellement monosubstitué à tétrasubstitué indépendamment l'un de l'autre par halogène, C₁-C₄-alkyle, C₁-C₄-halogénoalkyle, C₁-C₄-alcoxy ou cyano, aryle, hétaryle, arylalkyle ou hétarylalkyle éventuellement monosubstitué à trisubstitué indépendamment l'un de l'autre par halogène, C₁-C₄-alkyle, C₁-C₄-halogénoalkyle, hydroxy, C₁-C₄-alcoxy, C₁-C₄-alkyl-S (O)ₙ-, C₁-C₄-halogénoalcoxy, C₁-C₄-halogénoalkyl-S(O)ₙ-, nitro ou cyano, ou
R³ et R⁴ forment ensemble un cycle aliphatique ayant quatre à sept chaînons éventuellement monosubstitué à trisubstitué par halogène, C₁-C₄-alkyle ou C₁-C₄-alcoxy, qui contient éventuellement un atome de N, de S ou de O,
R³¹, R³², R³³ représentent à chaque fois indépendamment les uns des autres un radical de la série hydrogène, C₁-C₆-alkyle, C₃-C₆-alcényle ou C₃-C₆-alcynyle éventuellement monosubstitué à polysubstitué indépendamment l'un de l'autre par halogène, C₁-C₄-alcoxy, C₁-C₄-alkyl-S (O)ₙ- ou cyano ou C₃-C₆-cycloalkyle, C₁-C₆-alkylcarbonyle, C₁-C₆-alcoxycarbonyle, aryl-C₁-C₄-alkyle ou aryl-C₁-C₄-alcoxy éventuellement monosubstitué à trisubstitué indépendamment l'un de l'autre par halogène, C₁-C₄-alkyle, C₁-C₄-halogénoalkyle, C₁-C₄-alcoxy ou cyano,
R⁴¹, R⁴³ représentent à chaque fois indépendamment l'un de l'autre un radical de la série hydrogène, C₁-C₄-alkyle, C₃-C₆-alcényle ou C₃-C₆-alcynyle éventuellement monosubstitué à polysubstitué indépendamment l'un de l'autre par halogène, C₁-C₄-alcoxy, C₁-C₄-alkyl-S(O)ₙ- ou cyano, C₃-C₈-cycloalkyle ou C₃-C₈-cycloalkyl-C₁-C₄-alkyle linéaire ou ramifié, éventuellement interrompu une ou deux fois indépendamment l'un de l'autre par O, S(O)ₙ, CO ou NR^{4a} et éventuellement monosubstitué à tétrasubstitué indépendamment l'un de l'autre par halogène, C₁-C₄-alkyle, C₁-C₄-halogénoalkyle, C₁-C₄-alcoxy ou cyano, aryle, hétaryle, arylalkyle ou hétarylalkyle éventuellement monosubstitué à trisubstitué indépendamment l'un de l'autre par halogène, C₁-C₄-alkyle, C₁-C₄-halogénoalkyle, C₁-C₄-alcoxy, C₁-C₄-alkyl-S (O)ₙ-, C₁-C₄-halogénoalcoxy, C₁-C₄-halogénoalkyl-S(O)ₙ-, nitro ou cyano,
m représente le nombre 0, 1 ou 2,
n représente le nombre 0, 1 ou 2,
p représente le nombre 0 ou 1,
q représente le nombre 0, 1 ou 2,
et leurs sels.

3. Composés selon la revendication 1, dans lesquels
Q représente oxygène,
V représente un radical de la série hydrogène, fluor, chlore, brome, méthyle et éthyle,
W représente un radical de la série hydrogène, fluor, chlore, brome, cyano et méthyle,
Y représente un radical de la série C₁-C₄-alkyle, C₃-C₄-alcényle ou C₃-C₄-alcynyle éventuellement monosubstitué à pentasubstitué indépendamment l'un de l'autre par fluor, chlore, brome, méthoxy ou éthoxy, C₃-C₆-cycloalkyle ou aryl-C₁-C₄-alkyle éventuellement monosubstitué ou disubstitué indépendamment l'un de l'autre par fluor, chlore, brome, méthyle, éthyle, trifluorométhyle, méthoxy, éthoxy ou cyano,
A représente un radical de la série hydrogène, C₁-C₆-alkyle, C₃-C₆-alcényle ou C₃-C₆-alcynyle éventuellement monosubstitué à pentasubstitué indépendamment l'un de l'autre par fluor, chlore, brome, méthoxy, éthoxy ou cyano, et C₃-C₆-cycloalkyle éventuellement monosubstitué ou disubstitué indépendamment l'un de l'autre par fluor, chlore, brome, méthyle, éthyle, trifluorométhyle, méthoxy, éthoxy ou cyano,
T représente une paire d'électrons,
X représente C(E)U¹, C(O)OR², C(O)N(R³) (R⁴) ou C(S)N(R³) (R⁴) ,
E représente les radicaux O, N-OR²¹, N-N(R³³)(R⁴³) ou représente un cycle de formule (X-1), 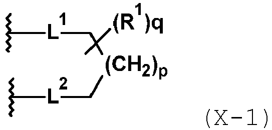
U¹ représente un radical de la série hydrogène, C₁-C₆-alkyle, C₃-C₆-alcényle ou C₃-C₆-alcynyle éventuellement monosubstitué à pentasubstitué indépendamment l'un de l'autre par fluor, chlore, brome, méthoxy, éthoxy ou cyano, et C₃-C₆-cycloalkyle éventuellement monosubstitué ou disubstitué indépendamment l'un de l'autre par fluor, chlore, brome, méthyle, éthyle, trifluorométhyle, méthoxy, éthoxy ou cyano, phényle, pyridinyle, pyrimidinyle ou thiényle éventuellement monosubstitué ou disubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano, phényl-C₁-C₂-alkyle ou pyridinyl-C₁-C₂-alkyle linéaire ou ramifié, éventuellement monosubstitué ou disubstitué indépendamment l'un de l'autre par fluor, chlore, brome, méthyle, éthyle, difluorométhyle, trifluorométhyle, méthoxy, éthoxy, Me-S(O)ₙ-, Et-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
L¹, L² représentent à chaque fois indépendamment l'un de l'autre O et S(O)ₘ,
R¹ représente C₁-C₄-alkyle, C₂-C₄-alcényle, C₃-C₄-alcynyle ou C₁-C₄-alcoxy éventuellement substitué par halogène, méthoxy, éthoxy,
ou
R¹ représente une chaîne C₂-C₄-alkyle, qui remplace deux atomes de H liés au même atome de C, n représentant alors 2,
ou
R¹ représente une chaîne C₁-C₄-alcényle, la double liaison remplaçant deux atomes de H liés au même atome de et n représentant alors 1,
R² représente un radical de la série hydrogène, C₁-C₆-alkyle, C₃-C₆-alcényle ou C₃-C₆-alcynyle, monosubstitué à pentasubstitué indépendamment l'un de l'autre par fluor, chlore, brome, méthoxy, éthoxy ou cyano, C₃-C₆-cycloalkyle éventuellement monosubstitué ou disubstitué indépendamment l'un de l'autre par fluor, chlore, brome, méthyle, éthyle, trifluorométhyle, méthoxy, éthoxy ou cyano, C₃-C₆-cycloalkyl-C₁-C₄-alkyle éventuellement monosubstitué ou disubstitué indépendamment l'un de l'autre par fluor, chlore, brome, méthyle, éthyle, trifluorométhyle, méthoxy, éthoxy ou cyano, phényle, pyridinyle ou thiényle éventuellement monosubstitué à trisubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, difluorométhoxy, trifluorométhoxy, trifluorométhyle, méthyl-S(O)ₙ-, difluoroéthyl-S(O)ₙ-, trifluoroéthyl-S(O)ₙ-, nitro ou cyano, et phényl-C₁-C₂-alkyle ou pyridinyl-C₁-C₂-alkyle linéaire ou ramifié, éventuellement monosubstitué ou disubstitué indépendamment l'un de l'autre par fluor, chlore, brome, méthyle, éthyle, difluorométhyle, trifluorométhyle, méthoxy, éthoxy, Me-S(O)ₙ-, Et-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
R²¹ représente un radical de la série hydrogène, C₁-C₆-alkyle, C₃-C₆-alcényle ou C₃-C₆-alcynyle, monosubstitué à pentasubstitué indépendamment l'un de l'autre par fluor, chlore, brome, méthoxy, éthoxy ou cyano, C₃-C₆-cycloalkyle éventuellement monosubstitué ou disubstitué indépendamment l'un de l'autre par fluor, chlore, brome, méthyle, éthyle, trifluorométhyle, méthoxy, éthoxy ou cyano, C₃-C₆-cycloalkyl-C₁-C₄-alkyle éventuellement monosubstitué ou disubstitué indépendamment l'un de l'autre par fluor, chlore, brome, méthyle, éthyle, trifluorométhyle, méthoxy, éthoxy ou cyano, et phényl-C₁-C₂-alkyle ou pyridinyl-C₁-C₂-alkyle linéaire ou ramifié, éventuellement monosubstitué ou disubstitué indépendamment l'un de l'autre par fluor, chlore, brome, méthyle, éthyle, difluorométhyle, trifluorométhyle, méthoxy, éthoxy, Me-S(O)ₙ-, Et-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
R³ représente un radical de la série hydrogène, C₁-C₄-alkyle, C₃-C₄-alcényle ou C₃-C₄-alcynyle éventuellement monosubstitué à trisubstitué indépendamment l'un de l'autre par fluor ou chlore, C₃-C₆-cycloalkyle éventuellement monosubstitué ou disubstitué indépendamment l'un de l'autre par fluor, chlore, méthyle, éthyle, trifluorométhyle, méthoxy, éthoxy ou cyano, ou C₁-C₄-alkylsulfonyle, C₃-C₆-cycloalkylsulfonyle, C₁-C₄-alkylcarbonyle ou C₁-C₄-alcoxycarbonyle éventuellement monosubstitué à trisubstitué indépendamment l'un de l'autre par fluor, chlore, méthoxy ou éthoxy, et phényl-C₁-C₂-alcoxycarbonyle éventuellement monosubstitué ou disubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
R⁴ représente un radical de la série hydrogène, C₁-C₄-alkyle, C₃-C₆-alcényle, C₃-C₆-alcynyle ou C₁-C₄-alcoxy éventuellement monosubstitué à trisubstitué indépendamment l'un de l'autre par halogène, hydroxy, méthoxy, éthoxy, n-propoxy ou isopropoxy, méthoxycarbonyle, éthoxycarbonyle, cyano ou méthyl-S(O)ₙ-, C₃-C₆-cycloalkyle ou C₃-C₆-cycloalkyl-C₁-C₄-alkyle éventuellement monosubstitué ou disubstitué indépendamment l'un de l'autre par fluor, chlore, méthyle, éthyle, trifluorométhyle, méthoxy, éthoxy ou cyano, phényle, naphtyle, pyridinyle, pyrimidinyle, pyridazinyle, pyrazinyle, triazinyle, furannyle, thiényle, oxazolyle, isoxazolyle, thiazolyle, isothiazolyle, pyrazolyle, imidazolyle, oxadiazolyle ou thiadiazolyle éventuellement monosubstitué ou disubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano, phényl-C₁-C₂-alkyle ou pyridinyl-C₁-C₂-alkyle linéaire ou ramifié, éventuellement monosubstitué ou disubstitué indépendamment l'un de l'autre par fluor, chlore, brome, méthyle, éthyle, difluorométhyle, trifluorométhyle, méthoxy, éthoxy, Me-S(O)ₙ-, Et-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano, ou
R³ et R⁴ forment ensemble un cycle aliphatique ayant quatre à six chaînons éventuellement monosubstitué ou disubstitué par fluor, chlore, brome, méthyle, éthyle, méthoxy ou éthoxy, qui contient éventuellement un atome de N, de S ou de O,
R³³ représente un radical de la série hydrogène, C₁-C₄-alkyle éventuellement monosubstitué à trisubstitué indépendamment l'un de l'autre par fluor ou chlore, C₁-C₆-alkylcarbonyle ou C₁-C₆-alcoxycarbonyle éventuellement monosubstitué à trisubstitué indépendamment l'un de l'autre par fluor, chlore, méthoxy ou éthoxy et phényl-C₁-C₂-alcoxycarbonyle éventuellement monosubstitué ou disubstitué indépendamment l'un de l'autre par fluor, chlore, brome, méthyle, éthyle, trifluorométhyle, méthoxy, éthoxy, trifluorométhoxy, nitro ou cyano,
R⁴³ représente un radical de la série hydrogène ou C₁-C₄-alkyle éventuellement monosubstitué à trisubstitué indépendamment l'un de l'autre par halogène, méthoxy ou éthoxy,
m représente 0,
n représente le nombre 0, 1 ou 2,
p représente le nombre 0 ou 1,
q représente le nombre 0, 1 ou 2, et leurs sels.

4. Composés selon la revendication 1 selon la formule (I-1) 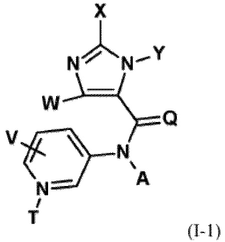
Q représente oxygène,
V représente un radical de la série hydrogène, fluor, chlore, méthyle et cyano,
W représente un radical de la série hydrogène, fluor, chlore, brome, méthyle,
Y représente un radical de la série méthyle, éthyle, propyle, allyle ou propargyle éventuellement monosubstitué à trisubstitué indépendamment l'un de l'autre par fluor, méthoxy, éthoxy ou cyano,
A représente un radical de la série hydrogène ; méthyle, éthyle, propyle, allyle, propargyle ou cyclopropyle éventuellement monosubstitué à trisubstitué indépendamment l'un de l'autre par fluor, méthoxy, éthoxy ou cyano,
T représente une paire d'électrons,
X représente C(E)U¹, C(O)OR², C(O)N(R³) (R⁴) ou C(S)N(R³) (R⁴) ,
E représente les radicaux O, N-OR²¹, N-N(R³³) (R⁴³) ou représente un cycle de formule (X-1), 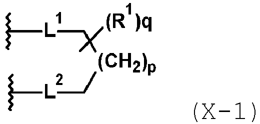
U¹ représente un radical de la série hydrogène, C₁-C₆-alkyle éventuellement monosubstitué à pentasubstitué indépendamment l'un de l'autre par fluor, chlore, brome, méthoxy, éthoxy ou cyano, ou C₃-C₆-cycloalkyle,
L¹, L² représentent à chaque fois indépendamment l'un de l'autre O et S(O)ₘ,
R¹ représente C₁-C₄-alkyle éventuellement substitué par fluor, chlore, méthoxy ou éthoxy,
ou
R¹ représente une chaîne C₂-C₄-alkyle, qui remplace deux atomes de H liés au même atome de C, n représentant alors 2,
R² représente un radical de la série hydrogène, C₁-C₆-alkyle, monosubstitué à trisubstitué indépendamment l'un de l'autre par fluor, chlore, brome, méthoxy, éthoxy ou cyano, ou phénylméthyle éventuellement monosubstitué ou disubstitué indépendamment l'un de l'autre par fluor, chlore, brome, méthyle, éthyle, difluorométhyle, trifluorométhyle, méthoxy, éthoxy, Me-S(O)ₙ-, Et-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
R²¹ représente un radical de la série hydrogène, C₁-C₆-alkyle, C₃-C₆-alcényle ou C₃-C₆-alcynyle, monosubstitué à trisubstitué indépendamment l'un de l'autre par fluor, chlore, brome, méthoxy, éthoxy ou cyano, C₃-C₆-cycloalkyle éventuellement monosubstitué ou disubstitué indépendamment l'un de l'autre par fluor, chlore, brome, méthyle, éthyle, méthoxy ou éthoxy, C₃-C₆-cycloalkyl-C₁-C₄-alkyle éventuellement substitué par fluor, chlore, brome, méthyle, éthyle, méthoxy ou éthoxy, phénylméthyle ou pyridinylméthyle éventuellement monosubstitué ou disubstitué indépendamment l'un de l'autre par fluor, chlore, brome, méthyle, éthyle, difluorométhyle, trifluorométhyle, méthoxy, éthoxy, Me-S(O)ₙ-, Et-S(O)ₙ-, difluorométhoxy, trifluorométhoxy, trifluorométhyl-S(O)ₙ-, difluoroéthyl-S(O)ₙ-, trifluoroéthyl-S(O)ₙ-, nitro ou cyano,
R³ représente un radical de la série hydrogène, C₁-C₄-alkyle, C₁-C₄-alkylsulfonyle, C₃-C₆-cycloalkylsulfonyle, C₁-C₄-alkylcarbonyle ou C₁-C₄-alcoxycarbonyle éventuellement monosubstitué à trisubstitué indépendamment l'un de l'autre par fluor, chlore, méthoxy ou éthoxy, et arylméthoxycarbonyle éventuellement monosubstitué à trisubstitué indépendamment l'un de l'autre par fluor, chlore, brome, méthyle, éthyle, trifluorométhyle, méthoxy ou éthoxy,
R⁴ représente un radical de la série hydrogène, C₁-C₄-alkyle, C₃-C₆-alcényle, C₃-C₆-alcynyle ou C₁-C₄-alcoxy éventuellement monosubstitué à trisubstitué indépendamment l'un de l'autre par halogène, hydroxy, méthoxy, éthoxy, méthoxycarbonyle, éthoxycarbonyle ou cyano, C₃-C₆-cycloalkyle ou C₃-C₆-cycloalkyl-C₁-C₄-alkyle éventuellement monosubstitué ou disubstitué indépendamment l'un de l'autre par fluor, chlore, méthyle, éthyle, trifluorométhyle, méthoxy, éthoxy ou cyano, phényle, pyridinyle ou thiényle éventuellement monosubstitué ou disubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, trifluorométhoxy, nitro ou cyano, phényl-C₁-C₂-alkyle ou pyridinyl-C₁-C₂-alkyle éventuellement monosubstitué ou disubstitué indépendamment l'un de l'autre par fluor, chlore, brome, méthyle, éthyle, difluorométhyle, trifluorométhyle, méthoxy, éthoxy, méthyl-S(O)ₙ-, éthyl-S(O)ₙ-, trifluorométhoxy, nitro ou cyano, ou
R³ et R⁴ forment ensemble un cycle aliphatique ayant quatre à six chaînons éventuellement monosubstitué ou disubstitué par fluor, chlore, brome, méthyle, éthyle, méthoxy ou éthoxy, qui contient éventuellement un atome de N, de S ou de O,
R³³ représente un radical de la série hydrogène, C₁-C₄-alkylcarbonyle ou C₁-C₄-alcoxycarbonyle éventuellement substitué par méthoxy ou éthoxy, et phénylméthoxycarbonyle éventuellement monosubstitué à trisubstitué indépendamment l'un de l'autre par fluor, chlore, méthyle ou éthyle,
R⁴³ représente un radical de la série hydrogène ou C₁-C₄-alkyle,
m représente 0,
n représente le nombre 0, 1 ou 2,
p représente le nombre 0 ou 1,
q représente le nombre 0, 1 ou 2,
et leurs sels.

5. Composés selon la revendication 4, dans lesquels
Q représente oxygène,
V représente hydrogène ou méthyle,
W représente hydrogène,
Y représente un radical de la série méthyle, éthyle, propyle, allyle et propargyle,
A représente un radical de la série hydrogène, méthyle, éthyle ou cyclopropyle,
T représente une paire d'électrons,
X représente C(E)U¹, C(O)OR², C(O)N(R³) (R⁴) ou C(S)N(R³) (R⁴) ,
E représente les radicaux O, N-OR²¹, N-N(R³³) (R⁴³) ou représente un cycle de formule (X-1), 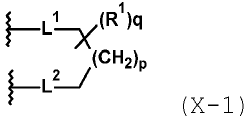
U¹ représente un radical de la série hydrogène, méthyle, éthyle, n-propyle ou isopropyle, 1-cyano-1-méthyléthyle et cyclopropyle,
L¹, L² représentent à chaque fois indépendamment l'un de l'autre O et S(O)ₘ,
R¹ représente méthyle, éthyle, n-propyle, isopropyle ou méthoxyméthyle,
ou
R¹ représente -(CH₂)₂-, un groupe spirocyclopropyle étant formé,
R² représente un radical de la série hydrogène, méthyle, éthyle, n-propyle ou isopropyle,
R²¹ représente un radical de la série hydrogène, méthyle, éthyle, n-propyle ou isopropyle, n-butyle, iso-butyle, sec-butyle ou tert-butyle, 2,2-diméthylpropyle, propényle, propynyle, 2,2,2-trifluoroéthyle, cyclopropyle, cyclobutyle, cyclopentyle, cyclohexyle, cyclopropylméthyle, 2-Cl-phénylméthyle, 3-Cl-phénylméthyle, 4-Cl-phénylméthyle, pyridin-2-ylméthyle, pyridin-3-ylméthyle ou pyridin-4-ylméthyle,
R³ représente un radical de la série hydrogène, méthyle, éthyle, méthylsulfonyle, cyclopropylsulfonyle, méthoxycarbonyle, éthoxycarbonyle, 2-méthoxyéthoxycarbonyle n-butoxycarbonyle, iso-butoxycarbonyle, sec-butoxycarbonyle ou t-butoxycarbonyle,
R⁴ représente un radical de la série hydrogène, méthyle, éthyle, n-propyle ou isopropyle, n-butyle, iso-butyle, sec-butyle ou tert-butyle, 2,2,2-trifluoroéthyle, 2-hydroxyéthyle, 2-méthoxyéthyle, cyanométhyle, méthoxycarbonylméthyle, éthoxycarbonylméthyle, cyclopropyle, 1-cyanocyclopropyle, cyclopropylméthyle, cyclobutyle, cyclopentyle, cyclohexyle, méthoxy, éthoxy, phényle, 2-Cl-phényle, 3-Cl-phényle, 4-Cl-phényle, pyridinyle, phénylméthyle, 1-phényl-2-hydroxyéthyle, 2-Cl-phénylméthyle, 3-Cl-phénylméthyle, 4-Cl-phénylméthyle ou
R³ et R⁴ représentent ensemble (CH₂)₅, (CH₂)₄, (CH₂)₃ ou (CH₂)₂O (CH₂)₂,
R³³ représente un radical de la série méthoxycarbonyle, éthoxycarbonyle, n-propoxycarbonyle ou isopropoxycarbonyle, n-butoxycarbonyle, iso-butoxycarbonyle, sec-butoxycarbonyle ou t-butoxycarbonyle, isopentoxycarbonyle, 2-méthoxyéthoxycarbonyle ou phénylméthoxycarbonyle,
R⁴³ représente un radical de la série hydrogène, méthyle, éthyle, n-propyle ou isopropyle, n-butyle, iso-butyle, sec-butyle ou tert-butyle,
m représente 0,
p représente le nombre 0 ou 1,
q représente le nombre 0, 1 ou 2,
et leurs sels.

6. Composés selon la revendication 4, dans lesquels
Q représente oxygène,
V représente hydrogène ou méthyle,
W représente hydrogène,
Y représente méthyle,
A représente méthyle, éthyle ou cyclopropyle,
T représente une paire d'électrons,
X représente C(E)U¹, C(O)OR², C(O)N(R³)(R⁴) ou C(S)N(R³)(R⁴),
E représente les radicaux O, N-OR²¹, N-N(R³³)(R⁴³) ou représente un cycle de formule (X-1), 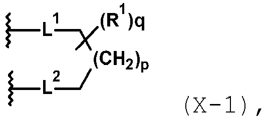
U¹ représente hydrogène, méthyle, éthyle, isopropyle, 1-cyano-1-méthyléthyle ou cyclopropyle,
L¹, L² représentent à chaque fois indépendamment l'un de l'autre O et S(O)ₘ,
R¹ représente méthyle, éthyle ou méthoxyméthyle,
ou
R¹ représente -(CH₂)₂-, un groupe spiro-cyclopropyle étant formé,
R² représente méthyle ou éthyle,
R²¹ représente un radical de la série méthyle, éthyle, n-propyle ou isopropyle, n-butyle, iso-butyle ou tert-butyle, 2,2-diméthylpropyle, propényle, propynyle, 2,2,2-trifluoroéthyle, cyclopentyle, cyclopropylméthyle, 4-Cl-phénylméthyle, pyridin-2-ylméthyle, pyridin-3-ylméthyle,
R³ représente un radical de la série hydrogène, méthyle, éthyle, méthylsulfonyle et cyclopropylsulfonyle,
R⁴ représente un radical de la série hydrogène, méthyle, éthyle, isopropyle, isobutyle, tert-butyle, 2,2,2-trifluoroéthyle, 2-hydroxyéthyle, 2-méthoxyéthyle, cyanométhyle, méthoxycarbonylméthyle, éthoxycarbonylméthyle, cyclopropyle, 1-cyanocyclopropyle, cyclopentyle, cyclopropylméthyle, méthoxy, éthoxy, phényle, pyridin-3-yle, phénylméthyle, 1-phényl-2-hydroxyéthyle,
R³ et R⁴ représentent ensemble (CH₂)₅, (CH₂)₄, (CH₂)₃ ou (CH₂)₂O(CH₂)₂,
R³³ représente un radical de la série méthoxycarbonyle, éthoxycarbonyle, t-butoxycarbonyle, 2-méthoxyéthoxycarbonyle et phénylméthoxycarbonyle,
R⁴³ représente un radical de la série hydrogène, méthyle et n-butyle,
m représente 0,
p représente le nombre 0 ou 1,
q représente le nombre 0, 1 ou 2
et leurs sels.

7. Composé selon l'une quelconque des revendications 1 à 6 selon l'une des formules (I-1a) à (I-1f)

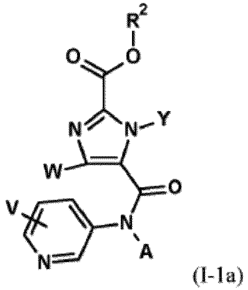

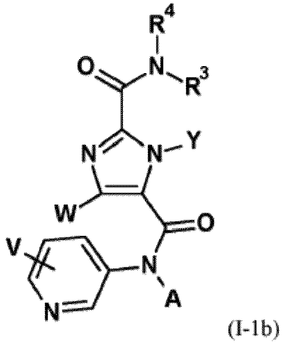

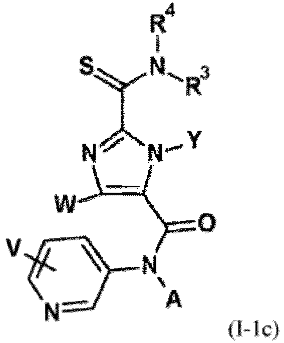

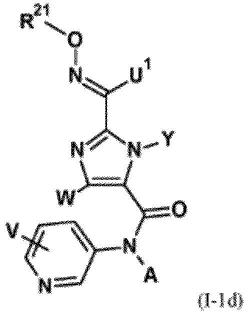

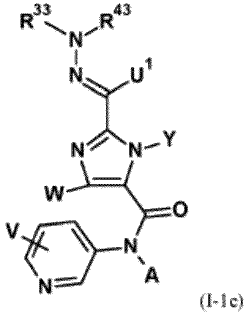

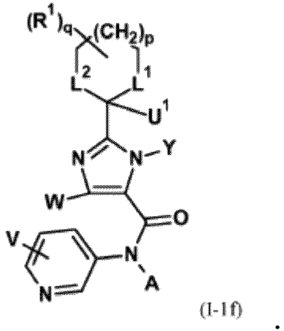

8. Agent, doté d'une teneur d'au moins un composé selon l'une quelconque des revendications 1 à 7 et de diluants et/ou de substances tensioactives habituel(le)s en particulier pour la lutte contre des parasites animaux.

9. Procédé pour la lutte contre des parasites animaux, dans lequel on laisse agir au moins un composé selon l'une quelconque des revendications 1 à 7 ou un agent selon la revendication 8 sur les parasites animaux et/ou sur leur lieu de vie, le traitement chirurgical, thérapeutique et diagnostique du corps humain ou animal étant exclu.

10. Utilisation d'au moins un composé selon l'une quelconque des revendications 1 à 7 ou d'un agent selon la revendication 8 pour la lutte contre des parasites animaux, le traitement chirurgical, thérapeutique et diagnostique du corps humain ou animal étant exclu.

11. Utilisation d'au moins un composé selon l'une quelconque des revendications 1 à 7 pour la protection du matériel de propagation de végétaux.

12. Formulation agrochimique contenant au moins un composé selon l'une quelconque des revendications 1 à 7 en des teneurs biologiquement efficaces comprises entre 0,00000001 et 98 % en poids, par rapport au poids de la formulation agrochimique, ainsi que des diluants et/ou des substances tensioactives.

13. Formulation agrochimique selon la revendication 12, contenant de plus un principe actif agrochimique supplémentaire.
